# EUROPEAN PATENT SPECIFICATION

(11) **EP 1 762 567 B1**
(45) Date of publication and mention of the grant of the patent: **02.05.2012**
(21) Application number: 06126433.9
(22) Date of filing: 04.04.2002
(51) Int. Cl.: C07D 231/18, C07D 231/20, A61K 31/415, A61K 31/4162, A61K 31/4155, A61P 31/18, C07D 403/06, C07D 413/06, C07D 401/12, C07D 403/12, C07D 405/12, C07D 403/04, C07D 498/04, C07D 401/06, C07D 471/04

(54) **Pyrazole derivatives for treating HIV**
Pyrazolderivate zur Behandlung von HIV
Dérivés de pyrazole pour le traitment de HIV

(30) Priority: 10.04.2001 GB 0108999; 15.11.2001 GB 0127426
(43) Date of publication of application: 14.03.2007
(62) Divisional of application: 02708600.8
(73) Proprietor: Pfizer Limited, Sandwich, Kent CT13 9NJ (GB); Pfizer Inc., New York, NY 10017 (US)
(72) Inventor: Jones, Lyn Howard, Ramsgate Road Sandwich, Kent CT13 9NJ (GB); Mowbray, Charles Eric, Ramsgate Road Sandwich, Kent CT13 9NJ (GB); Price, Davis Anthony, Ramsgate Road Sandwich, Kent CT13 9NJ (GB); Selby, Matthew Duncan, Ramsgate Road Sandwich, Kent CT13 9NJ (GB); Stupple, Paul Anthony, Ramsgate Road Sandwich, Kent CT13 9NJ (GB)
(74) Representative: Gladwin, Amanda Rachel

(56) References cited:
- US-A- 3 963 742
- GENIN M J ET AL: "NOVEL 1,5-DIPHENYLPYRAZOLE NONNUCLEOSIDE HIV-1 REVERSE TRANSCRIPTASE INHIBITORS WITH ENHANCED ACTIVITY VERSUS THE DELAVIRDINE-RESISTANT P236L MUTANT: LEAD IDENTIFICATION AND SAR OF 3- AND 4-SUBSTITUTED DERIVATIVES" JOURNAL OF MEDICINAL CHEMISTRY, AMERICAN CHEMICAL SOCIETY. WASHINGTON, US, vol. 43, 2000, pages 1034-1040, XP002178918 ISSN: 0022-2623
- SMODIS J ET AL: "The Synthesis and Transformations of Substituted 2-Hydroxy-3-dimethylaminopropenoates. The Preparation of Condensed 3-Hydroxypyran-2-ones" TETRAHEDRON, ELSEVIER SCIENCE PUBLISHERS, AMSTERDAM, NL, vol. 54, no. 33, 13 August 1998 (1998-08-13), pages 9799-9810, XP004127445 ISSN: 0040-4020
- LIPINSKI ET AL: "Bronchodilator and antiulcer Phenoxypyrimidinones" JOURNAL OF MEDICINAL CHEMISTRY., vol. 23, 1980, pages 1026-1031, XP002201971 AMERICAN CHEMICAL SOCIETY. WASHINGTON., US ISSN: 0022-2623
- REGITZ; SCHÄFER: "Stabile enole von aplpha-[Aryl(alkyl)oxy]dibenzoylmethanen" LIEBIGS ANNALEN DER CHEMIE., vol. 7, 1981, pages 1172-1185, XP002201972 VERLAG CHEMIE GMBH. WEINHEIM., DE ISSN: 0170-2041
- MUNCH; HAUSER: "Acylations of certain alpha-alkoxy and alpha-aryloxy ketones and esters" JOURNAL OF THE AMERICAN CHEMICAL SOCIETY., vol. 71, 1949, pages 770-773, XP002201973 AMERICAN CHEMICAL SOCIETY, WASHINGTON, DC., US ISSN: 0002-7863
- ORAZIO ET AL: "Reaction of some 1,2-diaza-1,3-butadienes with activated methine compounds" JOURNAL OF ORGANIC CHEMISTRY., vol. 63, 1998, pages 9880-9887, XP002201974 AMERICAN CHEMICAL SOCIETY. EASTON., US ISSN: 0022-3263
- OLSEN ET AL: "Synthesis of functionalized aryloxy" JOURNAL OF ORGANIC CHEMISTRY., vol. 60, no. 19, 1995, pages 6025-6031, XP002201975 AMERICAN CHEMICAL SOCIETY. EASTON., US ISSN: 0022-3263

## Description

This invention relates to pyrazole derivatives in combination with one or more additional therapeutic agents and the uses of such combinations.

The pyrazole compounds of the present combination bind to the enzyme reverse transcriptase and are modulators, especially inhibitors thereof. Reverse transcriptase is implicated in the infectious lifecycle of HIV, and compounds which interfere with the function of this enzyme have shown utility in the treatment of conditions including AIDS. There is a constant need to provide new and better modulators, especially inhibitors, of HIV reverse transcriptase since the virus is able to mutate, becoming resistant to the effects of known modulators.

The combinations of the present invention are useful in the treatment of a variety of disorders including those in which the inhibition of reverse transcriptase is implicated. Disorders of interest include those caused by Human Immunodificiency Virus (HIV) and genetically related retroviruses, such as

Acquired Immune Deficiency Syndrome (AIDS).

European patent application EP 0 786 455 A1 discloses a class of imidazole compounds which inhibit the growth of HIV. A class of N-phenylpyrazoles which act as reverse transcriptase inhibitors are disclosed in J. Med. Chem., 2000, 43, 1034. Antiviral activity is ascribed to a class of N-(hydroxyethyl)pyrazole derivatives in US patent number 3,303,200.

According to the present invention there is provided a compound of the formula 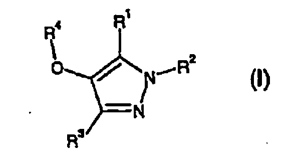 or a pharmaceutically acceptable salt, or solvate thereof, as defined in Claim 1 below, in combination with one or more additional therapeutic agents.

In the definitions, halo means fluoro, chloro, bromo or iodo. Unless otherwise stated, alkyl, alkenyl, alkynyl, alkylene and alkoxy groups containing the requisite number of carbon atoms can be unbranched or branched chain. Examples of alkyl include methyl, ethyl, n-propyl, i-propyl, n-butyl, i-butyl, sec-butyl and t-butyl. Examples of alkenyl include ethenyl, propen-1-yl, propen-2-yl, propen-3-yl, 1-buten-1-yl, 1-buten-2-yl, 1-buten-3-yl, 1-buten-4-yl, 2-buten-1-yl, 2-buten-2-yl, 2-methylpropen-1-yl or 2-methylpropen-3-yl. Examples of alkynyl include ethynyl, propyn-1-yl, propyn-3-yl, 1-butyn-1-yl, 1-butyn-3-yl, 1-butyn-4-yl, 2-butyn-1-yl. Examples of alkylene include methylene, 1,1-ethylene, 1,2-ethylene, 1,1-propylene, 1,2-propylene, 2,2-propylene and 1,3-propylene. Examples of alkoxy include methoxy, ethoxy, n-propoxy, i-propoxy, n-butoxy, i-butoxy, sec-butoxy and t-butoxy. Examples of cycloalkyl include cyclopropyl, cyclobutyl, cyclopentyl, cyclohexyl and cycloheptyl. Where R¹ and R² are taken together, they form, along with the nitrogen atom and the carbon atom of the pyrazole ring to which they are attached, a 5- or 6-membered ring. Where a heterocyclic group R⁸ or R⁹ is attached to an oxygen, sulphur or nitrogen heteroatom the heterocyclic group R⁸ or R⁹ must be linked through a ring carbon atom. Further, where a heterocyclic group R⁹ is attached to an oxygen, sulphur or nitrogen heteroatom the heterocyclic group R⁹ must be linked through a ring carbon atom that is not adjacent to a ring heteratom.

The pharmaceutically acceptable salts of the compounds of the formula (I) include the acid addition and the base salts thereof.

Suitable acid addition salts are formed from acids which form non-toxic salts and examples are the hydrochloride, hydrobromide, hydroiodide, sulphate, bisulphate, nitrate, phosphate, hydrogen phosphate, acetate, maleate, fumarate, lactate, tartrate, citrate, gluconate, succinate, saccharate, benzoate, methanesulphonate, ethanesulphonate, benzenesulphonate, paratoluenesulphonate and pamoate salts.

Suitable base salts are formed from bases which form non-toxic salts and examples are the sodium, potassium, aluminium, calcium, magnesium, zinc and diethanolamine salts.

For a review on suitable salts see Berge et al, *J. Pharm. Sci.*, **66**, 1-19, 1977.

The pharmaceutically acceptable solvates of the compounds of the formula (I) include the hydrates thereof.

Also included within the present scope of the compounds of the formula (I) are polymorphs thereof.

The compounds of formula (I) may be modified to provide pharmaceutically acceptable derivatives thereof at any of the functional groups in the compounds. Examples of such derivatives are described in: Drugs of Today, Volume 19, Number 9, 1983, pp 499 - 538; Topics in Chemistry, Chapter 31, pp 306 - 316; and in "Design of Prodrugs" by H. Bundgaard, Elsevier, 1985, Chapter 1 (the disclosures in which documents are incorporated herein by reference) and include: esters, carbonate esters, hemi-esters, phosphate esters, nitro esters, sulfate esters, sulfoxides, amides, sulphonamides, carbamates, azo-compounds, phosphamides, glycosides, ethers, acetals and ketals.

A compound of the formula (I) may contain one or more asymmetric carbon atoms and therefore exist in two or more stereoisomeric forms. The present invention includes the individual stereoisomers of the compounds of the formula (I) together with, where appropriate, the individual tautomers thereof, and mixtures thereof.

Separation of diastereoisomers may be achieved by conventional techniques, e.g. by fractional crystallisation, chromatography or high performance liquid chromatography (HPLC) of a stereoisomeric mixture of a compound of the formula (I) or a suitable salt or derivative thereof. An individual enantiomer of a compound of the formula (I) may also be prepared from a corresponding optically pure intermediate or by resolution, such as by HPLC of the corresponding racemate using a suitable chiral support or by fractional crystallisation of the diastereoisomeric salts formed by reaction of the corresponding racemate with a suitable optically active acid or base, as appropriate.

Preferably, R¹, when taken separately, is H, C₁-C₆ alkyl, C₃-C₇ cycloalkyl or -OR⁷, said C₁-C₆ alkyl being optionally substituted by halo, -OR¹⁰, -NR¹⁰R¹¹, -NR⁵COR¹⁰ or R¹⁰.
Preferably, R¹, when taken separately, is H, C₁-C₄ alkyl, cyclopropyl, or -OCH₃, said C₁-C₄ alkyl being optionally substituted by bromo, -OH, -O(C₁-C₂ alkyl), -NR¹⁰R¹¹, -NHCOR¹³ or R¹⁰.
Preferably, R¹, when taken separately, is H, -CH₃, -CH₂CH₃, -CH(CH₃)₂, -C(CH₃)₃, cyclopropyl, -OCH₃, -CH₂OH, -CH₂OCH₃, -CH₂OCH₂CH₃, -CH₂Br, -CH₂NH₂, -CH₂NHCH₃, -CH₂N(CH₃)₂, -CH₂NHCH₂(cyclopropyl), -CH₂NHCH₂CH₂OCH₃, -CH₂NHCH₂CH₂NHCOCH₃, -CH₂NHCO(4-cyanophenyl), -CH₂NHCO(3-cyanophenyl), -CH₂NHCH₂(4-cyanophenyl), -CH₂NHCH₂(4-fluorophenyl), -CH₂NHCH₂(4-methoxyphenyl), -CH2NHCH2(4-aminosulphonylphenyl), -CH₂NHCH₂(4-aminocarbonylphenyl), -CH₂NHCH₂(pyrid-3-yl), -CH₂N(CH₃)(4-cyanophenylmethyl), -CH₂N(CH₂CH₂OH)(4-cyanophenylmethyl), 4-methoxypiperidin-1-ylmethyl, 4-aminocarbonylpiperidin-1-ylmethyl, 4-methylcarbonylaminopipefidin-1-ylmethyl, piperazin-1-ylmethyl, 4-methylpiperazin-1-ylmethyl, 4-methylcarbonylpiperazin-1-ylmethyl, 4-methoxymethylcarbonylpiperazin-1-ylmethyl, 4-methoxycarbonylpiperazin-1-ylmethyl, 4-methylsulphonylpiperazin-1-ylmethyl, morpholin-4-ylmethyl, 2-methylimidazol-1-ylmethyl, pyrazol-1-ylmethyl or 1,2,4-triazol-1-ylmethyl. Preferably, R¹, when taken separately, is, -CH₃, -CH₂CH₃, cyclopropyl, -CH₂NHCH₂(4-cyanophenyl), -CH₂NHCH₂(4-fluorophenyl), -CH₂NHCH₂(4-methoxyphenyl), -CH₂NHCH₂(4-aminosulphonylphenyl) or -CH₂NHCH₂(4-aminocarbonylphenyl).

Preferably, R², when taken separately, is H, C₁-C₆ alkyl, C₃-C₆ alkenyl or R⁹, said C₁-C₆ alkyl being optionally substituted by -OR⁵, -OR¹², -CN, -CO₂R⁷, -CONR⁵R⁵, -C(=NR⁵)NR⁵OR⁵, -CONR⁵NR⁵R⁵, -NR⁶R⁶, -NR⁵R¹², -NR⁵COR⁸, -NR⁵COR¹², -NR⁵CO₂R⁵, R⁸ or R⁹.

Preferably, R², when taken separately, is H, C₁-C₃ alkyl, propenyl or R⁹, said C₁-C₃ alkyl being optionally substituted by -OH, -OCH₃, -OCH₂CH₂NH₂, -CN, -CO₂CH₃, -CO₂CH₂CH₃, -CONH₂, -C(=NH)NHOH, -CONHNH₂, -NH₂, -NHCH₃, -N(CH₃)₂, -NHCH₂CH₂NHCOCH₃, -NHCH₂CH₂OCH₃, -NHCH₂R⁹, -NHCOR⁸, -NHCOCH₂OCH₃, -NHCO₂C(CH₃)₃, R⁸ or R⁹.

Preferably, R², when taken separately, is H, methyl, -CH₂CH=CH₂, -CH₂CN, -CH₂OCH₃, -CH₂CONH₂, -CH₂CONHNH₂, -CH₂CO₂CH₃, -CH₂CO₂CH₂CH₃, -CH₂C(=NH)NHOH, -CH₂CH₂OH, -CH₂CH₂OCH₃, -CH₂CH₂NH₂, -CH₂CH₂NHCOCH₂OCH₃, -CH₂CH₂NHCO₂C(CH₃)₃, 2-(pyrid-2-ylcarbonylamino)eth-1-yl, 2-(pyrazin-2-ylcarbonylamino)eth-1-yl, -CH₂CH₂OCH₂CH₂NH₂, -CH₂CH₂NHCH₃, -CH₂CH₂N(CH₃)₂, -CH₂CH₂NHCH₂CH₂NHCOCH₃, -CH₂CH₂NHCH₂CH₂OCH₃, -CH₂CH(OH)CH₃, (3-hydroxypyrazol-5-yl)methyl, 2-hydroxy-1,3,4-oxadiazol-5-ylmethyl, 2-amino-1,3,4-oxadiazol-5-yl, 5-hydroxy-1,2,4-oxadiazol-3-ylmethyl, 6-hydroxy-2-methylpyrimidin-4-ylmethyl, 6-hydroxy-2-aminopyrimidin-4-ylmethyl, 2-(morpholin-4-yl)eth-1-yl, 2-(4-methylcarbonylpiperazin-1-yl)eth-1-yl, morpholin-3-ylmethyl, (2-(tetrahydrofuran-2-ylmethylamino)eth-1-yl, 1-methylazetidin-3-yl or azetidin-3-yl.
Preferably, R², when taken separately, is H, -CH₂CH₂OH or -CH₂CH₂NH₂.

Preferably, R¹ and R², when taken together, represent unbranched propylene wherein one methylene group is replaced by an oxygen atom or unbranched butylene wherein one methylene group is replaced by a nitrogen atom, said propylene and butylene being optionally substituted by oxo and said nitrogen atom being optionally substituted by R¹⁰.
Preferably, R¹ and R², when taken together, represent ^{x}-OCH₂CH₂-^{y}, ^{x}-CONHCH₂CH₂-^{y}, ^{x}-CH₂NHCH₂CH₂-^{y}, ^{x}-CH₂N(CH₃)CH₂CH₂-^{y}, ^{x}-CH₂N(4-cyanophenylmethyl)CH₂CH₂-^{y} or ^{x}-CH₂N(4-methoxyphenylmethyl)CH₂CH₂-^{y} wherein 'x' represents the point of attachment to the carbon atom of the pyrazole ring and 'y' represents the point of attachment to the nitrogen atom of the pyrazole ring.

Preferably, R³ is H or C₁-C₆ alkyl.
Preferably, R³ is H or C₁-C₄ alkyl.
Preferably, R³ is H, -CH₃, -CH₂CH₃, -CH(CH₃)₂ or -C(CH₃)₃.
Preferably, R³ is -CH₃, -CH₂CH₃, -CH(CH₃)₂ or cyclopropyl.

Preferably, R⁴ is phenyl optionally substituted by R⁸, halo, -CN, C₁-C₆ alkyl, C₁-C₆ haloalkyl, C₃-C₇ cycloalkyl or C₁-C₆ alkoxy.
Preferably, R⁴ is phenyl substituted by R⁸, halo, -CN, C₁-C₆ alkyl, C₁-C₆ haloalkyl, C₃-C₇ cycloalkyl or C₁-C₆ alkoxy.
Preferably, R⁴ is phenyl substituted by halo, -CN or C₁-C₆ alkyl.
Preferably, R⁴ is phenyl substituted by fluoro, chloro, -CN or methyl.
Preferably, R⁴ is 3-cyanophenyl, 4-chlorophenyl, 3-chlorophenyl, 2-chlorophenyl, 3-fluorophenyl, 2-fluorophenyl, 3,5-dichlorophenyl, 2,6-dichlorophenyl, 2,3-dichlorophenyl, 2,4-dichlorophenyl, 3,4-dichlorophenyl, 2,5-dichlorophenyl, 2,6-difluorophenyl, 2,3-difluorophenyl, 3,5-difluorophenyl, 2,5-difluorophenyl, 3,5-dicyanophenyl, 3,5-dimethylphenyl, 4-fluoro-3-methylphenyl, 3-cyano-4-fluorophenyl, 3-cyano-5-fluorophenyl, 2-chloro-4-cyanophenyl, 3-chloro-5-cyanophenyl, 3-cyano-5-methylphenyl or 4-cyano-2,6-dimethylphenyl. Preferably, R⁴ is 3,5-dicyanophenyl, 3-cyano-5-fluorophenyl, 3-chloro-5-cyanophenyl or 3-cyano-5-methylphenyl.

### In an alternative preference:

Preferably, R⁴ is phenyl substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹³, SoₓR⁶, O-(C₁-C₆ alkylene)-CONR⁵R⁵, O-(C₁-C₆ alkylene)-NR⁵R⁵, or O-(C₁-C₆ alkylene)-OR⁶.

Preferably, R⁸ is pyrrolyl, imidazolyl, pyrazolyl, 1,2,3-triazolyl, 1,2,4-triazolyl, tetrazolyl, oxazolyl, isoxazolyl, thiazolyl, isothiazolyl, 1,2,4-oxadiazolyl, 1,3,4-oxadiazolyl, furanyl, thienyl, pyridinyl, pyridazinyl, pyrimidinyl or pyrazinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OR⁵, -NR⁵R⁵ or C₁-C₆ alkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OH, -NH₂ or methyl.

Preferably, R⁸ is pyrazol-1-yl, 2-methylimidazol-1-yl, 1,2,4-triazol-1-yl, 3-hydroxypyrazol-5-yl, 2-hydroxy-1,3,4-oxadiazol-5-yl, 2-amino-1,3,4-oxadiazol-5-yl, 5-hydroxy-1,2,4-oxadiazol-3-yl, 2-methyl-4-hydroxypyrimidin-6-yl, 2-amino-4-hydroxypyrimidin-6-yl, pyridin-3-yl, pyridin-2-yl or pyrazin-2-yl.

Preferably, R⁹ is azetidinyl, tetrahydropyrrolyl, piperidinyl, azepinyl, oxetanyl, tetrahydrofuranyl, tetrahydropyranyl, oxepinyl, morpholinyl, piperazinyl or diazepinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN. Preferably, R⁹ is azetidinyl, piperidinyl, tetrahydrofuranyl, piperazinyl or morpholinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.

Preferably, R⁹ is azetidinyl, piperidinyl, tetrahydrofuranyl, piperazinyl or morpholinyl, each being optionally substituted by C₁-C₆ alkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by -OR⁵ or -NR⁵COR⁵.
Preferably, R⁹ is azetidinyl, piperidinyl, tetrahydrofuranyl, piperazinyl or morphoninyl, each being optionally substituted by -CH₃, -SO₂CH₃, -CONH₂, -COOCH₃, -COCH₂OCH₃ or -COCH₃ and optionally substituted on a carbon atom which is not adjacent to a heteroatom by -OCH₃ or -NHCOCH₃.
Preferably, R⁹ is 4-methoxypiperidin-1-yl, 4-aminocarbonylpiperidin-1-yl, 4-methylcarbonylaminopiperidin-1-yl, piperazin-1-yl, 4-methylpiperazin-1-yl, 4-methylcarbonylpiperazin-1-yl, 4-methoxymethylcarbonylpiperazin-1-yl, 4-methoxycarbonylpiperazin-1-yl, 4-methylsulphonylpiperazin-1-yl, morpholin-4-yl, tetrahydrofuran-2-yl, morpholin-3-yl, azetidin-3-yl or 1-methylazetidin-3-yl.

Preferably, R¹⁰ is H, R⁸, R⁹, R¹³, C₁-C₆ alkyl or -(C₁-C₆ alkyl)-(C₃-C₇ cycloalkyl), said C₁-C₆ alkyl being optionally substituted by -OR⁵, -OR¹³, R⁸, R⁹, R¹³ or -COR¹³.
Preferably, R¹⁰ is H, R⁸, R⁹, R¹³, C₁-C₆ alkyl or -(C₁-C₆ alkyl)-(C₃-C₇ cycloalkyl), said C₁-C₆ alkyl being optionally substituted by -OR⁵ or R¹³.
Preferably, R¹⁰ is H, R⁸, R⁹, R¹³, -CH₃, -CH₂CH₃ or -CH₂(cyclopropyl), said -CH₃ and -CH₂CH₃ being optionally substituted by -OCH₃ or R¹³.
Preferably, R¹⁰ is H, R⁸, R⁹, R¹³, -CH₃, -CH₂CH₃, -CH₂CH₂OCH₃, -CH₂(cyclopropyl), 4-cyanophenylmethyl, 4-fluorophenylmethyl, 4-methoxyphenylmethyl, 4-aminosulphonylphenylmethyl or 4-aminocarbonylphenylmethyl.

Preferably, R¹¹ is H or C₁-C₆ alkyl, said C₁-C₆ alkyl being optionally substituted by -OR⁵, -NR⁵R⁵, -NR⁵COR⁵, -CONR⁵R⁵, R⁸ or R⁹.

Preferably, R¹¹ is H or C₁-C₆ alkyl, said C₁-C₆ alkyl being optionally substituted by -OR⁵ or -NR⁵COR⁵.

Preferably, R¹¹ is H, -CH₃ or -CH₂CH₃, said -CH₃ and -CH₂CH₃ being optionally substituted by -OH or -NHCOCH₃.
Preferably, R¹¹ is H, -CH₃, -CH₂CH₂NHCOCH₃ or -CH₂CH₂OH.

Preferably, R¹² is C₁-C₄ alkyl substituted by R⁸, R⁹, -OR⁶, -CONR⁵R⁵, -NR⁵COR⁵ or -NR⁶R⁶.
Preferably, R¹² is C₁-C₄ alkyl substituted by R⁹, -OR⁶, -NR⁶COR⁶ or -NR⁵R⁶.
Preferably, R¹² is C₁-C₂ alkyl substituted by tetrahydrofuranyl, -OCH₃, -NHCOCH₃ or-NH₂.
Preferably, R¹² is -CH₂CH₂NH₂, -CH₂CH₂OCH₃, tetrahydrofuran-2-ylmethyl, -CH₂CH₂NHCOCH₃ or -CH₂OCF₃.

Preferably, R¹³ is phenyl substituted by halo, -CN, -COR⁵, -CONR⁵R⁵, -SO₂NR⁵R⁵, -NR⁵SO₂R⁶, -OR⁵, -NR⁵R⁵, -(C₁-C₆ alkylene)-NR⁶R⁵, C₁-C₆ alkyl, halo(C₁-C₆)alkyl or C₃-C₇ cycloalkyl.
Preferably, R¹³ is phenyl substituted by halo, -CN, -CONR⁶R⁵, -SO₂NR⁵R⁵ or -OR⁵.
Preferably, R¹³ is phenyl substituted by fluoro, -CN, -CONH₂, -SO₂NH₂ or -OCH₃.
Preferably, R¹³ is 4-cyanophenyl, 3-cyanophenyl, 4-fluorophenyl, 4-methoxyphenyl, 4-aminocarbonylphenyl or 4-aminosulphonylphenyl.

Preferred groups of compounds according to the invention include all combinations of the preferred definitions for individual substituents given above.

Also preferred according to the invention are the compounds of formula (I) 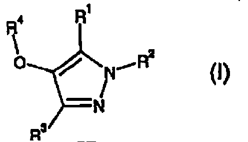 or a pharmaceutically acceptable salt, or solvate thereof, wherein:
R¹ is H, C₁-C₆ alkyl, -OC₁-C₆ alkyl, -OC₃-C₇ cycloalkyl, said C₁-C₈ alkyl being optionally substituted by R¹⁵;
R² is H, C₁-C₃ alkyl, propenyl or C-linked R¹⁵, said C₁-C₃ alkyl being optionally substituted by -OH, -OCH₃, -OCH₂CH₂NH₂, -CN, -CO₂CH₃, -CONH₂, - C(=NH)NH₂, -CONHNH₂, -NH₂, -NHCH₃, -N(CH₃)₂, -NHCH₂CH₂NHCOCH₃, - NHCH₂CH₂OCH₃, -NHCH₂R¹⁵, -NHCOR¹⁵, -NHCOCH₂OCH₃, or R¹⁵
R³ is C₁-C₆ alkyl;
R⁴ is phenyl optionally substituted by halo, -CN, C₁-C₆ alkyl, C₁-C₆ haloalkyl, C₃-C₇ cycloalkyl or C₁-C₆ alkoxy; and
R¹⁵ is azetidinyl, tetrahydrofuranyl, morpholinyl, piperazinyl, pyrazolyl, oxadiazolyl, pyridinyl or pyrimidinyl each being optionally substituted by -OH, - NH₂, oxo or C₁-C₆ alkyl or -CO(C₁-C₆ alkyl).

Preferred individual compounds according to the invention include the Examples below, particularly Examples 117, 118, 119, 120, 122, 123, 124, 125, 126, 127 and 128, and the pharmaceutically acceptable salts and solvates thereof.

All of the compounds of the formula (I) can be prepared by conventional routes such as the procedures described in the general methods presented below or by the specific methods described in the Examples section, or by similar methods thereto. The present invention also encompasses any one or more of these processes for preparing the compounds of formula (I), in addition to any novel intermediates used therein.

In the following general methods, R¹, R², R³ and R⁴ are as previously defined for a compound of the formula (I) unless otherwise stated.

Except where either R¹ or R³ is halo, -OR⁸ or -CN, compounds of the formula (I) may be prepared using the route shown in Scheme 1 that follows.

In Scheme 1, compounds of the formula (I) may be prepared by the condensation of a compound of the formula (II) with a compound of the formula

H₂NNHR² (V),

or a salt or hydrate thereof, optionally in the presence of an acid or a base, the base preferably being a tertiary amine base such as triethylamine and the acid preferably being acetic acid. In a typical procedure, a solution of the compound of the formula (II) in a suitable solvent, such as ethanol, is treated with the compound of the formula (V), or the salt or hydrate thereof, and, if used, the appropriate acid or base, at a temperature of from room temperature to the reflux temperature of the solvent. In a preferred procedure, the reaction mixture is heated under reflux.

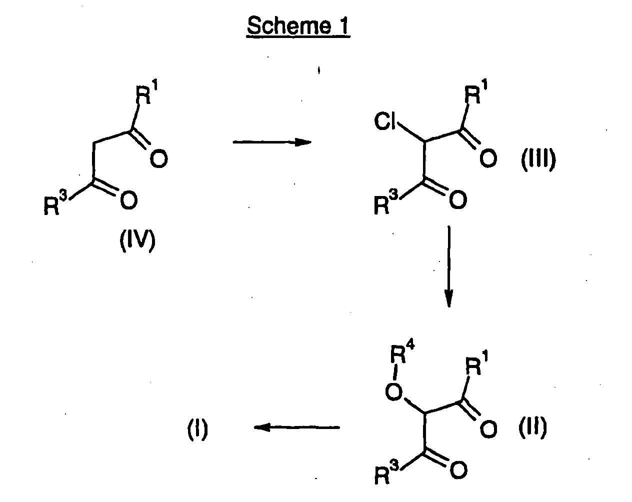

Functional equivalents of compounds of the formula (II) may also be used in this reaction. These include compounds of the formula (VI) or (VII), in which L¹ and L², respectively, are each suitable leaving groups, preferably -N(C₁-C₆ alkyl)₂, most preferably -N(CH₃)₂.

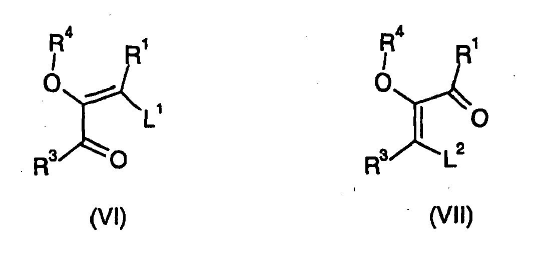

Thus, a compound of the formula (I) may be prepared by the condensation of a compound of the formula (VI) or (VII) with a compound of the formula (V), or a salt or hydrate thereof, optionally in the presence of an acid or a base, the base preferably being a tertiary amine base such as triethylamine and the acid preferably being acetic acid. In a typical procedure, a solution of the compound of the formula (VI) or (VII) in a suitable solvent, such as ethanol, is treated with the compound of the formula (V), or the salt or hydrate thereof, and, if used, the appropriate acid or base, at a temperature of from room temperature to the reflux temperature of the solvent. In a preferred procedure, the reaction mixture is heated under reflux. Compounds of the formula (VI) or (VII) are particularly suitable for the synthesis of compounds of the formula (I) in which R¹ or R³, respectively, is H.

Compounds of the formula (VI) in which R¹ is H and L¹ is dimethylamino may be prepared by the reaction of a compound of the formula (VIII) with dimethylformamide dimethylacetal at an elevated temperature, preferably at about 100°C. Compounds of the formula (VII) in which R¹ is H and L¹ is dimethylamino may be prepared by the reaction of a compound of the formula (IX) under the same conditions. Other compounds of the formula (VI) or (VII) in which L¹ or L² is dimethylamino may be prepared analogously.

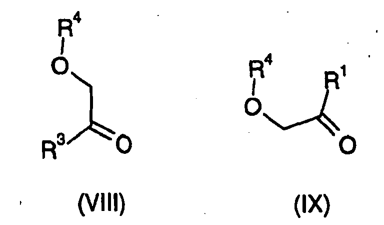

Compounds of the formula (VIII) are either commercially available or may be prepared by the reaction of a compound of the formula

R³COCH₂Br (X)

with a compound of the formula

R⁴OH (XI).

In a typical procedure, a solution of the compound of the formula (XI) in a suitable solvent, such as acetone, is treated with a suitable base, such as caesium carbonate, and the compound of the formula (X). In a preferred procedure, the reaction mixture is heated, for example under reflux. Optionally, a nucleophilic catalyst such as sodium iodide or tetrabutylammonium iodide may be added

Compounds of the formula (IX) are either commercially available or may be prepared from a compound of the formula

R¹COCH₂Br (XII)

and a compound of the formula (XI) in the same way that a compound of the formula (VIII) may be prepared from a compound of the formula (X).

Compounds of the formula (II) may be prepared by the reaction of a compound of the formula (III) with a compound of the formula (XI).

In a typical procedure, a solution of the compound of the formula (III) in a suitable solvent such as acetone is treated with a compound of the formula (XI) and a suitable base, such as potassium or caesium carbonate, and heated, preferably under reflux. Optionally, a nucleophilic catalyst such as sodium iodide or tetrabutylammonium iodide may be added.

Compounds of the formula (III) are either commercially available or may be prepared by the reaction of a compound of the formula (IV) with a chlorinating reagent. In a typical procedure, a cooled solution of the compound of the formula (IV) in a suitable solvent such as acetonitrile is treated first with tetrabutylammonium bromide and chlorotrimethylsilane and then dry dimethylsulphoxide. In another typical procedure, the compound of the formula (IV) is treated with sulphuryl chloride, optionally in the presence of a suitable solvent such as dichloromethane.

Compounds of the formula (I) in which R¹ or R³ is -OR⁸ may be prepared using the route shown in Scheme 2 that follows, in which R^{a} is C₁-C₆ alkyl and L³ is a suitable leaving group, preferably trifluoromethanesulphonate.

In Scheme 2, compounds of the formula (I) in which R¹ is -OR⁸ may be prepared by the reaction of a compound of the formula (XIII) with an alcohol of the formula

R⁸OH (XXI)

in the presence of a suitable palladium catalyst and carbon monoxide. In a typical procedure a mixture of the compound of the formula (XIII), a suitable palladium catalyst such as 1,1'-bis(diphenylphosphino)ferrocenepalladium(II)chloride, the alcohol of the formula (XXI) and, optionally, a suitable solvent such as N,N-dimethylformamide is heated, preferably to about 50°C, under an atmosphere of carbon monoxide, preferably at a pressure of 345 kPa.

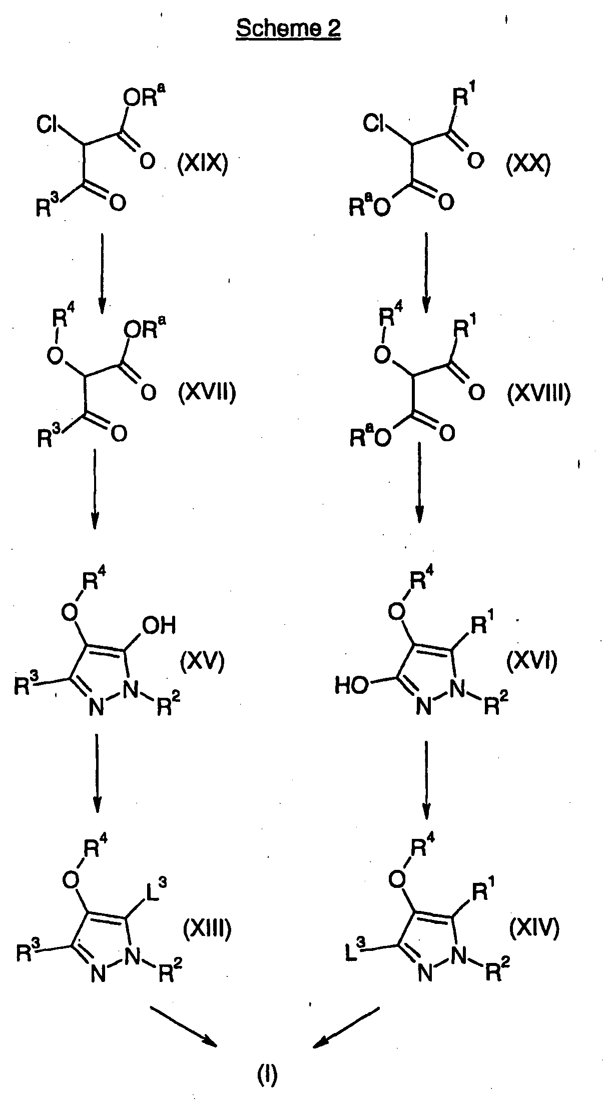

Compounds of the formula (XIII) may be prepared by the derivatisation of a compound of the formula (XV). In the case where L³ is trifluoromethanesulphonate a suitable derivatising agent is phenyltriflamide. In a typical procedure, where L³ is trifluoromethanesulphonate, a solution of the compound of the formula (XV) and a suitable base, preferably a trialkylamine base such as triethylamine, in a suitable solvent such as dichloromethane is treated with phenyltriflamide.

Compound of the formula (XV) may be prepared by the reaction of a compound of the formula (XVII) with a compound of the formula (V), or a salt or hydrate thereof, optionally in the presence of an acid or a base, the base preferably being a tertiary amine base such as triethylamine and the acid preferably being acetic acid. In a typical procedure, a solution of the compound of the formula (XVII) in a suitable solvent, such as ethanol, is treated with the compound of the formula (V), or the salt or hydrate thereof, and, if used, the appropriate acid or base, at a temperature of from room temperature to the reflux temperature of the solvent. In a preferred procedure, the reaction mixture is heated under reflux.

Compounds of the formula (XVII) may be prepared by the reaction of a compound of the formula (XIX) with a compound of the formula (XI). In a typical procedure, a solution of the compound of the formula (XVII) in a suitable solvent such as acetone is treated with a compound of the formula (XI) and a suitable base, such as potassium or caesium carbonate, and heated, preferably under reflux. Optionally, a nucleophilic catalyst such as sodium iodide or tetrabutylammonium iodide may be added.

In Scheme 2, compounds of the formula (I) in which R³ is -OR⁸ may be prepared from a compound of the formula (XX) in the same way that a compound of the formula (I) in which R¹ is -OR⁸ is prepared from a compound of the formula (XIX), as set out above, *mutatis mutandis*.

Chloroketoesters of the formula (XIX) and (XX) are either commercially available or may be prepared by the chlorination of the corresponding ketoesters, for instance using sulphonyl chloride.

Alternatively, compounds of the formula (I) in which R¹ or R³ is -OR⁸ may be prepared from compounds of the formula (XV) or (XVI), respectively, by reaction with a compound of the formula (XXI) under dehydrating conditions, e.g. using the Mitsunobu reaction. In a typical procedure, a solution of the compound of the formula (XV) or (XVI) in a suitable solvent, such as tetrahydrofuran is treated with diethylazodicarboxylate, triphenylphosphine and a compound of the formula (XXI).

Compounds of the formula (I) in which R¹ or R³ is halo can be prepared by the reaction, respectively, of a compound of the formula (XV) or a compound of the formula (XVI) with a suitable halogenating agent. In a typical procedure, the compound of the formula (XV) or (XVI) is treated with POCl₃, optionally in the presence of a suitable solvent such as dimethylformamide, to give a compound of the formula (I) in which R¹ or R³, respectively, is chloro.

It will be appreciated by those skilled in the art that, in many cases, compounds of the formula (I) may be converted into other compounds of the formula (I) by functional group transformations. For instance:
(a) compounds of the formula (I) in which R² is H may be converted into compounds of the formula (I) in which R² is optionally substituted C₁-C₆ alkyl by reaction with an appropriate alkylating agent. In a typical procedure, a solution of a compound of the formula (I) in which R² is H in a suitable solvent such as ethanol or N,N-dimethylformamide is treated with an alkyl bromide and a base such as sodium ethoxide or sodium hydride and heated at a temperature of from room temperature to the reflux temperature of the solvent. A preferred combination is N,N-dimethylformamide as the solvent, sodium hydride as the base and room temperature as the temperature. Examples of specific alkylating agents include bromoacetonitrile, ethyl 4-chloroacetoacetate, methyl bromoacetate and chloroethylamine hydrochloride. The use of further specific alkylating agents is illustrated by the Examples below;
(b) compounds of the formula (I) in which R¹, R² or R³ contains an ester functionality may be reduced with a suitable reducing agent, such as lithium aluminium hydride, to give corresponding compounds of the formula (I) in which R¹, R² or R³ contains a hydroxy group. In a typical procedure, a solution of the compound of the formula (I), in which R¹, R² or R³ contains an ester group, in a suitable solvent, such as diethyl ether, is treated with lithium aluminium hydride, preferably with cooling to a temperature of from -78°C to 0°C;
(c) compounds of the formula (I) in which R¹, R² or R³ are substituted by a heterocycle of the formula R⁶ may be prepared by standard heterocycle-forming reactions well known to the skilled man (see, for example, Advanced Organic Chemistry, 3rd Edition, by Gerry March or Comprehensive Heterocyclic Chemistry, A.R. Katritzky, C.W. Rees, E.F.V. Scriven, Volumes 1-11). For instance, compounds of the formula (I) in which R² is (2-amino-6-hydroxypyrimidin-4-yl)methyl may be prepared by the sequential reaction of a compound of the formula (I) in which R² is H with chloroacetoacetate and then guanidine hydrochloride. This and other similar heterocyle-forming reactions are illustrated by the Examples below; and
(d) compounds of the formula (I) in which R¹ or R³ is -CO₂R⁵, wherein R⁵ is other than H, may be converted into compounds of the formula (I) in which R¹ or R³, respectively, is -CO₂H by hydrolysis. Typically the reaction will be carried out in a suitable solvent, such as aqueous ethanol, or aqueous 1,4-dioxan and in the presence of a base such as sodium hydroxide. Such an acid may be converted to a primary amide by reaction with ammonia and a suitable coupling agent, such as a carbodiimide, e.g. dicyclohexylcarbodiimide. Such a primary amide may then be converted into a nitrile by dehydration with a suitable dehydrating agent, such as phosphoryl chloride.
(e) compounds of the formula (I) in which R¹ or R³ is C₁-C₆ alkyl may be converted into the compounds of the formula (I) in which R¹ or R³, respectively, is
   C₁-C₆ alkyl substituted by halo (such as bromo), by halogenation, using a suitable halogenating agent. Conveniently the reaction is effected in the presence of a solvent, such as a haloalkane (e.g. dichloromethane) and at ambient temperature. Suitable halogenating agents include halogens (e.g. bromine) or N-halosuccinimides (e.g. N-bromsuccinimide).
   Compounds of the formula (I) containing an -OH, -NH- or -NH₂ group may be prepared by the deprotection of the corresponding compound bearing an -OP¹, - NP¹- or -NHP¹ group, respectively, wherein the group P¹ is a suitable protecting group. Examples of suitable protecting groups will be apparent to the skilled person [see, for instance, 'Protecting groups in Organic Synthesis (Second Edition)' by Theodora W. Green and Peter G. M. Wuts, 1991, John Wiley and Sons]. Such compounds bearing an -OP¹, -NP¹- or -NHP¹ group may be prepared using the routes described above, *mutatis mutandis*.

Compounds of the formula (IV), (V) and (XXI) are either commercially available or easily prepared by methods well known to those skilled in the art.

The compounds of the formula (I) can be administered alone but will generally be administered in admixture with a suitable pharmaceutical excipient, diluent or carrier selected with regard to the intended route of administration and standard pharmaceutical practice.

For example, the compounds of the formula (I) can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the formula (I) may also be administered as fast-dispersing or fast-dissolving dosage forms or in the form of a high energy dispersion or as coated particles. Suitable formulations of the compounds of the formula (I) may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain from 0.01 mg to 500mg of active compound whilst tablet fill weights may range from 50mg to 1000mg. An example of a formulation for a 10mg tablet is illustrated below:

| Ingredient | %w/w |
|---|---|
| Compound of the formula (I) or salt | 10.000* |
| Lactose | 64.125 |
| Starch | 21.375 |
| | |
| Croscarmellose sodium | 3.000 |
| | |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the formula (I) may be combined with various sweetening or flavouring agents, colouring matter or dyes, with emulsifying and/or suspending agents and with diluents such as water, ethanol, propylene glycol and glycerin, and combinations thereof.

The compounds of the formula (I) can also be administered parenterally, for example, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the formula (I) will usually be from 0.01 to 30 mg/kg, preferably from 0.01 to 5 mg/kg (in single or divided doses).

Thus tablets or capsules of the compound of the formula (I) may contain from 1 to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that, in the treatment of certain conditions the compounds of the formula (I) may be taken as a single dose as needed or desired.

The compounds of formula (I) can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the formula (I) and a suitable powder base such as lactose or starch.

Alternatively, the compounds of the formula (I) can be administered in the form of a suppository or pessary, or they may be applied topically in the form of a gel, hydrogel, lotion, solution, cream, ointment or dusting powder. The compounds of the formula (I) may also be dermally or transdermally administered, for example, by the use of a skin patch. They may also be administered by the pulmonary or rectal routes.

They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the formula (I) can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the formula (I) may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

It is to be appreciated that all references herein to treatment include curative, palliative and prophylactic treatment.

Oral administration is preferred.

The compounds of formula (I) are used for the co-administration with one or more additional therapeutic agents, and compositions containing a compound of the present invention along with one or more additional therapeutic agents. Such a combination therapy is especially useful for the prevention and/or treatment of infection by HIV and related retroviruses which may evolve rapidly into strains resistant to any monotherapy. Alternatively, additional therapeutic agents may be desirable to treat diseases and conditions which result from or accompany the disease being treated with the compound of the present invention. For example, in the treatment of an HIV or related retroviral infection, it may be desirable to additionally treat opportunistic infections, neoplasms and other conditions which occur as a result of the immuno-compromised state of the patient being treated.

Preferred combinations of the present invention include simultaneous or sequential treatment with a compound of the formula (I), as defined above, or a pharmaceutically acceptable salt thereof, and:
(a) one or more reverse transcriptase inhibitors such as zidovudine, didanosine, zalcitabine, stavudine, lamivudine, abacavir and adefovir;
(b) one or more non-nucleoside reverse transcriptase inhibitors such as nevirapine, delavirdine and efavirenz;
(c) one or more HIV protease inhibitors such as indanivir, ritonavir, saquinavir and nelfinavir;
(d) one or more CCR5 antagonists such as TAK-779;
(e) one or more CXCR4 antagonists such as AMD-3100;
(f) one or more integrase inhibitors;
(g) one or more inhibitors of viral fusion such as T-20;
(h) one or more investigational drugs such as trizivir, KNI-272, amprenavir, GW-33908, FTC, PMPA, S-1153, MKC-442, MSC-204, MSH-372, DMP450, PNU-140690, ABT-378, KNI-764, DPC-083, TMC-120 or TMC-125; or
(i) one or more antifungal or antibacterial agents such as fluconazole.

The activity of the compounds of the invention as reverse transcriptase inhibitors and as agents for treating HIV infections may be measured using the following assays.

### A. Inhibition of HIV-1 reverse transcriptase enzyme

The reverse transcriptase activity of the compounds of the invention may be assayed as following. Using the purified recombinant HIV-1 reverse transcriptase (RT, EC, 2.7.7.49) obtained by expression in Escherichia Coli, a 96-well plate assay system was established for assaying a large number of samples using either the Poly(rA)-oligo(dT) Reverse Transcriptase [3H]-SPA enzyme assay system (Amersham NK9020) or the [3H]-flashplate enzyme assay system (NEN - SMP 103) and following the manufacturer's recommendations. The compounds were dissolved in 100% DMSO and diluted with the appropriate buffer to a 5% final DMSO concentration. The inhibitory activity was expressed in percent inhibition relative to the DMSO control. The concentration at which the compound inhibited the reverse transcriptase by 50% was expressed as the IC₅₀ of the compound. The compounds of examples 7, 20 and 51, when tested according to the above procedure, had IC₅₀ values of, respectively, 39000, 3200 and 248 nanomolar.

### B. Anti-Human Immunodeficiency Virus (HIV-1) cell culture assay

The anti-HIV activity of selected Examples of the invention was assayed by the following procedures.
1) SupT1 cells were cultured in an RPMI-1640 medium supplemented with 10% foetal calf serum and were split so that they were in growth phase on the day of use.
2) The compounds were dissolved in 100% DMSO and diluted with the above culture medium to predetermined concentrations and distributed in 20µl aliquots into a 96-well microtiter plate (0.1 % DMSO final concentration).
3) To prepare infected cells, 100µl of RF viruses (TCID50 of 10⁷/ml) were added to 10⁶ cells and incubated for 1 hour at 37°C. The cells were then washed twice in PBS and resuspended in the culture medium at a density of 2.2 x10⁵cells/ml. 180µl of these infected cells was transferred to wells of the 96 well plate containing the compounds.
4) The plate was incubated in a CO₂ incubator at 37°C for 4 days. The cell survival rates were measured following the manufacturer's recommendations (CellTiter 96^{®} AQᵤₑₒᵤₛ Non-Radioactive Assay - Promega (cat no: G5430)). The concentration at which the compound inhibited the cytotoxic effect of the virus by 50% was expressed as the EC₅₀.

Thus the invention provides:
(i) a compound of the formula (I) or a pharmaceutically acceptable salt, solvate thereof in combination with one or more additional therapeutic agents.
(ii) said combination for use as a medicament;
(iii) said combination for use in the treatment of an HIV, or genetically-related retroviral, infection or a resulting acquired immune deficiency syndrome (AIDS); and
(iii) the use of said combination for the manufacture of a medicament for the treatment of an HIV, or genetically-related retroviral, infection or a resulting acquired immune deficiency syndrome (AIDS).

The following Examples illustrate the preparation of the compounds of the formula (I). The synthesis of certain intermediates used therein are described in the Preparations section that follows the Examples.

¹H Nuclear magnetic resonance (NMR) spectra were in all cases consistent with the proposed structures. Characteristic chemical shifts (δ) are given in parts-per-million downfield from tetramethylsilane using conventional abbreviations for designation of major peaks: e.g. s, singlet; d, doublet; t, triplet; q, quartet; m, multiplet; br, broad. The following abbreviations have been used: HRMS, high resolution mass spectrometry; hplc, high performance liquid chromatography; nOe, nuclear Overhauser effect; m.p., melting point; CDCl₃, deuterochloroform; D₆-DMSO, deuterodimethylsulphoxide; CD₃OD, deuteromethanol. Where thin layer chromatography (TLC) has been used it refers to silica gel TLC using silica gel 60 F₂₅₄ plates, R_{f} is the distance travelled by a compound divided by the distance travelled by the solvent front on a TLC plate.

### EXAMPLE 1

### 2-[4-(3,5-Dichlorophenoxy)-3,5-dimethyl-1H-pyrazol-1-yl]ethanol

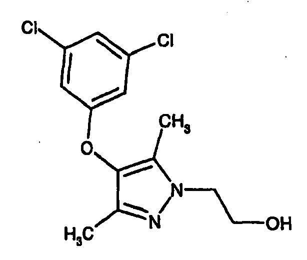

2-Hydroxyethyl hydrazine (21.5µL, 0.316mmol) was added to a stirred solution of the β-diketone of Preparation 1 (75mg, 0.287mmol) in ethanol (2.9ml) at room temperature under nitrogen and the resulting orange solution was heated under reflux for 18 hours. After cooling, the mixture was concentrated under reduced pressure. The residue was dissolved in dichloromethane (20ml) and washed with 2M hydrochloric acid (10ml) and brine (10ml) and then dried over magnesium sulphate, filtered and concentrated under reduced pressure to leave a viscous orange oil. The crude product was purified by flash chromatography on silica gel eluting with pentane:ethyl acetate (10:1, by volume) then dichloromethane to provide the title compound (32mg) as a white powder, m.p. 114-115°C.
¹H-NMR (400MHz, CDCl₃): δ = 2.08 (s, 3H), 2.10 (s, 3H), 3.30 (t, 1H), 4.06 (m, 4H), 6.79 (s, 2H), 7.01 (s, 1 H).
LRMS (thermospray): m/z [MH⁺] 301.
Microanalysis: Found: C, 51.76; H, 4.64; N, 9.20. C₁₃H₁₄Cl₂N₂O₂ requires C, 51.85; H, 4.69; N, 9.30%.

### EXAMPLE 2

### 2-[4-(3,5-Dichlorophenoxy)-3,5-diethyl-1H-pyrazol-1-yl]ethanol

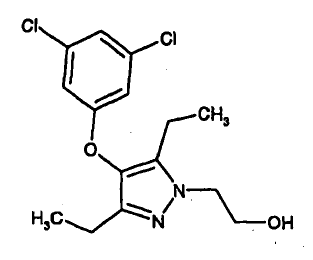

3,5-Dichlorophenol (501mg, 3.07mmol), potassium carbonate (467mg, 3.38mmol) and finally sodium iodide (461 mg, 3.07mmol) were added sequentially to a stirred solution of the chloroketone of Preparation 2 (500mg, 3.07mmol) in acetone (15ml), at room temperature and under nitrogen, producing an orange/red suspension. The mixture was heated under reflux for 22½ hours producing a yellow suspension. After cooling the mixture was diluted with water (10ml) and the acetone was removed under reduced pressure in a fumehood (caution: possible residual lachrymator). The residue was diluted with 2M hydrochloric acid and extracted with dichloromethane (1x20ml, 2x10ml). The combined organic layers were washed with brine (20ml), dried over magnesium sulphate, filtered and concentrated under reduced pressure to leave crude 4-(3,5-dichlorophenoxy)-3,5-heptanedione as an orange oil (777mg). A portion of the crude 4-(3,5-dichlorophenoxy)-3,5-heptanedione (250mg, *ca*. 0.865mmol) was dissolved in ethanol (8.6ml) and treated with 2-hydroxethyl hydrazine (65µL, 0.951mmol). The resulting solution was heated under reflux for 16 hours producing a red solution. After cooling, the mixture was concentrated under reduced pressure and the residue was dissolved in dichloromethane (20ml). The resulting solution was washed with 2M hydrochloric acid (10ml), 1 N sodium hydroxide solution (10ml) and brine (10ml), dried over magnesium sulphate, filtered and concentrated under reduced pressure to leave an orange oil (102mg). The crude product was purified by flash chromatography on silica gel eluting with methanol:dichloromethane (5:95, by volume) to provide the title compound (23mg) as an orange oil which solidified to a waxy solid on standing.
¹H-NMR (400MHz, CDCl₃): δ = 1.08 (t, 3H), 1.12 (t, 3H), 2.38 (q, 2H), 2.48 (q, 2H), 3.69 (br.s, 1 H), 4.02 (m, 4H), 6.76 (s, 2H), 6.97 (s, 1H).
LRMS (thermospray): m/z [MH⁺] 329.

### EXAMPLE 3

### 4-(3,5-Dichlorophenoxy)-3,5-diethyl-1H-pyrazole

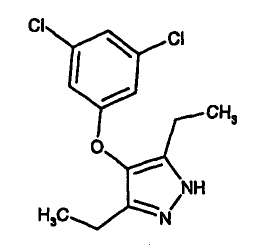

A mixture of the chloroketone of Preparation 2 (5g, 30.8mmol), 3,5-dichlorophenol (5g, 30.8mmol), caesium carbonate (10g, 30.8mmol) and acetone (40ml) was heated under reflux for 18 hours. After cooling, a solid was removed by filtration and washed with dichloromethane (100ml). The combined filtrates were concentrated under reduced pressure. The crude product was dissolved in ethanol (20ml), hydrazine hydrate (1.5ml, 30.8mmol) was added and the mixture was heated at 60°C for 30 minutes under nitrogen. After cooling, the mixture was concentrated under reduced pressure and the residue was purified by flash chromatography on silica gel eluting with ether:pentane (1:1, by volume) to provide the title compound (5.5g) as a yellow oil which solidified on standing to leave a yellow solid, m.p. 114-115°C.
¹H-NMR (300MHz, CDCl₃): δ = 1.15 (6H, t), 2.48 (4H, q), 6.78 (2H, s), 6.95 (1H, s).
LRMS (thermospray): m/z [MH⁺] 285.
Microanalysis: Found: C, 54.93; H, 5.05; N, 9.94. C₁₃H₁₄Cl₂N₂O requires C, 54.75; H, 4.95; N, 9.82%.

### EXAMPLE 4

### [4-(3,5-Dichlorophenoxy)-3,5-diethyl-1H-pyrazol-1-yl]acetonitrile

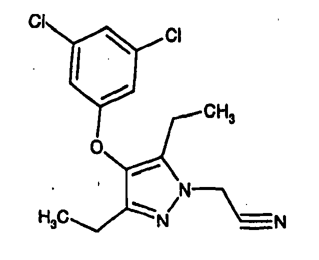

Sodium hydride (60% dispersion in oil, 470mg, 11.8mmol) was added to a stirred solution of 4-(3,5-dichlorophenoxy)-3,5-diethyl-1*H*-pyrazole (3g, 10.5mmol, Example 3) in dry N,N-dimethylformamide (20ml) at 0°C under nitrogen. The mixture was stirred for 5 minutes during which time hydrogen was evolved and then bromoacetonitrile (0.81 ml, 11.6mmol) was added. The yellow solution turned dark brown and a precipitate formed. Further dry N,N-dimethylformamide (5ml) was added to aid dissolution and after 45 minutes the reaction mixture was quenched by the addition of water (1ml). The mixture was partitioned between water (150ml) and diethyl ether (2x150ml). The combined organic layers were washed with water (50ml) and brine (100ml), dried over magnesium sulphate and concentrated under reduced pressure. The crude product was purified by flash chromatography on silica gel eluting with dichloromethane to provide the title compound (3.2g) as a yellow powder, m.p. 70-72°C.
¹H-NMR (400MHz, CDCl₃): δ = 1.14 (6H, m), 2.38 (2H, q), 2.56 (2H, q), 4.92 (2H, s), 6.75 (2H, s), 7.00 (1 H, s).
Microanalysis: Found: C, 55.43; H, 4.69; N, 12.71. C₁₅H₁₅Cl₂N₃O requires C, 55.57; H, 4.60; N, 12.96%.

### EXAMPLE 5

### 5-{[4-(3,5-Dichlorophenoxy)-3,5-diethyl-1H-pyrazol-1-yl]methyl}-1H-pyrazol-3-ol

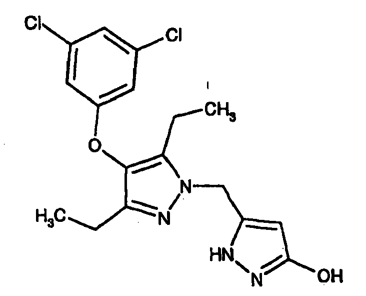

A mixture of the ester (120mg, 0.29mmol) of Preparation 3, hydrazine hydrate (16mg, 0.29mmol) and ethanol (5ml) was stirred and heated at 60°C for 2 hours under nitrogen. After cooling, the mixture was concentrated under reduced pressure and the resulting white solid was stirred in ethyl acetate and then collected by filtration to give the title compound (60mg) as a white solid, m.p. 142-144°C.
¹H-NMR (400MHz, DMSO-d₆): δ = 0.89 (3H, t). 0.99 (3H, t), 2.26 (2H, q), 2.45 (2H, q), 5.01 (2H, s), 5.19 (1 H, s), 6.88 (2H, s), 7.21 (1 H, s).
LRMS (electrospray): m/z [M-H⁺] 379.
Microanalysis: Found: C, 55.39; H, 4.72; N, 14.69. C₁₇H₁₈Cl₂N₄O₂ requires C, 53.56; H, 4.76; N, 14.69%.

### EXAMPLE 6

### 6-{[4-(3,5-Dichlorophenoxy)-3,5-diethyl-1H-pyrazol-1-yl]methyl}-2-methyl-4(3H)-pyrimidinone

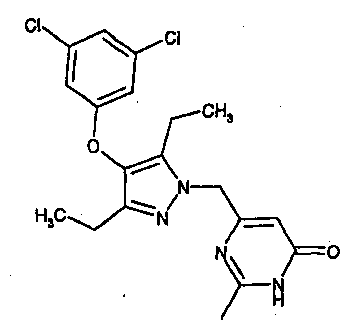

A mixture of the ester (140mg, 0.34mmol) of Preparation 3, acetamidine hydrochloride (95mg, 1.0mmol), sodium ethoxide (68mg, 1.0mmol) and ethanol (5ml) was stirred and heated at 70°C for 1 hour under nitrogen. After cooling, the mixture was concentrated under reduced pressure. The resulting oil was dissolved in dichloromethane (50ml), washed with water (20ml), dried over magnesium sulphate and concentrated under reduced pressure to leave the title compound as a white foam (100mg).
¹H-NMR (300MHz, CDCl₃): δ = 1.10 (3H, t), 1.19 (3H, t), 2.48 (7H, m), 5.08 (2H, s), 5.72 (1 H, s), 6.82 (2H, s), 7.03 (1 H, s).
LRMS (thermospray): m/z [MH⁺] 407.

### EXAMPLE 7

### 2-Amino-6-{[4-(3,5-dichlorophenoxyl-3,5-diethyl-1H-pyrazol-1-yl]methyl}-4(3H)-pyrimidinone

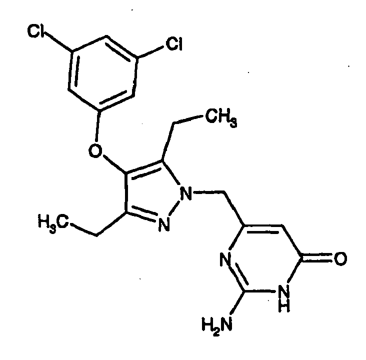

A mixture of the ester (150mg, 0.365mmol) from Preparation 3 and guanidine hydrochloride (104mg, 1.08mmol) and sodium ethoxide (73mg, 1.08mmol) in ethanol (5ml) was stirred and heated at 70°C for 3 hours under nitrogen. After cooling the mixture was concentrated under reduced pressure and the resulting oil was dissolved in dichloromethane (50ml), washed with water (20ml), dried over magnesium sulphate and concentrated under reduced pressure. The crude product was purified by chromatography on silica gel eluting with dichloromethane:methanol:ammonia (90:10:1, by volume) to give the title compound as a white solid (30mg), m.p. 238-240°C.
¹H-NMR (400MHz, DMSO-d₆): δ = 0.91 (3H, t), 0.99 (3H, t), 2.29 (2H, q), 2.44 (2H, q), 4.75 (1 H, s), 4.81 (2H, s), 6.58 (2H, br.s), 6.87 (2H, s), 7.22 (1 H, s).
LRMS (thermospray): m/z [MH⁺] 408.

### EXAMPLE 8

### 2-[4-(3,5-Dichlorophenoxy)-3,5-diethyl-1H-pyrazol-1-yl]-N-hydroxyethanimidamide

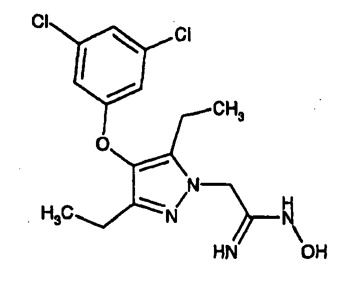

Hydroxylamine hydrochloride (1.1 g, 15.8mmol) and potassium carbonate (2.1 g, 15.2mmol) were added to a suspension of the nitrile (1 g, 3.1mmol) of Example 4 in a mixture of methanol (25ml) and water (10ml) which was then heated under reflux for 3 days. After cooling, the mixture was extracted with dichloromethane (2x250ml) and the combined organic layers were washed with brine (100ml), dried over magnesium sulphate, filtered and concentrated under reduced pressure to afford the product as a white solid (1.1g), m.p. 128-130°C.
¹H-NMR (300MHz, CD₃OD): δ = 1.10 (6H, m), 2.40 (2H, q), 2.60 (2H, q), 4.65 (2H, s), 6.90 (2H, s), 7.10 (1H, s).
LRMS (electrospray): m/z [MH⁺] 357.

### EXAMPLE 9

### Methyl [4-(3,5-dichlorophenoxy)-3,5-diethyl-1H-pyrazol-1-yl]acetate

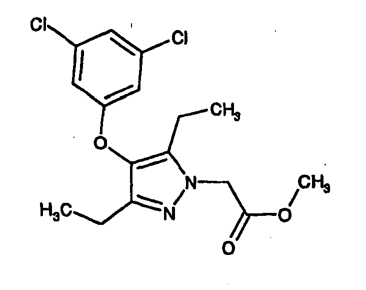

Methyl bromoacetate (984µL, 10mmol) and then sodium hydride (60% dispersion in oil, 801 mg, 20.1 mmol) were added to a stirred solution of the pyrazole (2.6g, 9.12mmol) of Example 3 in dry N,N'-dimethylformamide (25ml) at 0°C under nitrogen. After stirring for 1 hour at 0°C ice-water (100ml) was added and the mixture was extracted with ether (3x50ml). The combined ether layers were dried over magnesium sulphate, filtered and concentrated under reduced pressure. The residue was purified by flash chromatography on silica gel eluting with ethyl acetate:pentane (20:80, by volume) to provide the title compound (780mg) as a yellow oil which partly crystallised on standing.
¹H-NMR (400MHz, CDCl₃): δ = 1.10 (6H, m), 2.44 (4H, m), 3.78 (3H, s), 4.80 (2H, s), 6.69 (2H, s), 6.99 (1 H, s).
LRMS (thermospray): m/z [MH⁺] 357.

### EXAMPLE 10

### 2-[4-(3,5-Dichlorophenoxy)-3,5-diethyl-1H-pyrazol-1-yl]acetamide

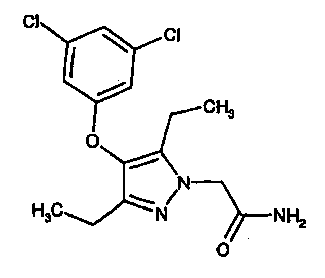

1,1'-Carbonyl diimidazole (71 mg, 0.44mmol) was added to stirred solution of the acid (125mg, 0.36mmol) of Preparation 4 in dry N,N-dimethylformamide at room temperature and the reaction mixture was stirred for 30 minutes. Concentrated aqueous ammonia (d=0.88og/cm³, *ca*. 0.1ml, *ca*. 1.8mmol) was added and stirring was continued for 10 minutes. The solvent was removed under reduced pressure and the residue was partitioned between water (10ml) and ethyl acetate (10ml). The organic layer was concentrated under reduced pressure and the residue was purified by chromatography on silica gel, eluting with ethyl acetate, to give the title compound as a white solid (60mg), m.p. 164-166°C.
¹H-NMR (300MHz, CDCL₃): δ = 1.15 (6H, m), 2.50 (4H, m), 4.70 (2H, s), 5.50 (1 H, br. s), 6.21 (1 H, br. s), 6.78 (2H, s), 7.04 (1 H, s).
LRMS (thermospray): m/z [MH⁺] 342.

### EXAMPLE 11

### 2-[4-(3,5-Dichlorophenoxy)-3,5-diethyl-1H-pyrazol-1-yl]acetohydrazide

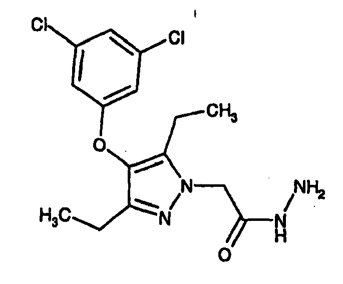

Hydrazine hydrate (520µL, 10.9mmol) was added to a solution of the ester (780mg, 2.18mmol) of Example 9 in ethanol (25ml) and the resulting mixture was heated under reflux for 18 hours. After cooling, the precipitate was collected by filtration and washed with ether (50ml) to afford the title compound (550g) as a white solid, m.p. >250°C.
¹H-NMR (300MHz, CD₃OD): δ = 1.10 (6H, m), 2.39 (2H, q), 2.55 (2H, q), 4.72 (2H, s), 6.93 (2H, s), 7.09 (1 H, s).
LRMS (electrospray): m/z [MH⁺] 357.

### EXAMPLE 12

### 5-{[4-(3,5-Dichlorophenoxy)-3,5-diethyl-1H-pyrazol-1-yl]methyl}-1,3,4-oxadiazol-2(3H)-one

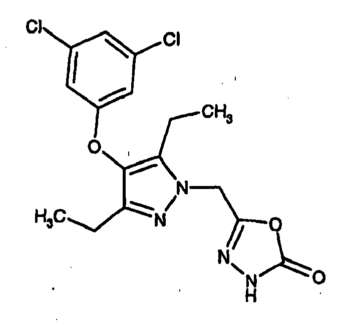

A stirred solution of the hydrazide (275mg, 0.77mmol) of Example 11 and 1,1'-carbonyl diimidazole 187mg, 1.16mmol) in dioxane (50ml) was heated under reflux for 18 hours. After cooling, the mixture was concentrated under reduced pressure and the residue was dissolved in dichloromethane (50ml) and washed with water (25ml). The organic layer was dried over magnesium sulphate, filtered and concentrated under reduced pressure. The residue was purified by flash chromatography on silica gel eluting with dichloromethane:methanol (95:5, by volume) to afford the title compound (112mg) as a white solid m.p. 138-142°C.
¹H-NMR (400MHz, CDCl₃): δ = 1.10 (6H, m), 2.40 (2H, q), 2.55 (2H, q), 5.07 (2H, s), 6.76 (2H, s), 6.98 (1 H, s), 10.45 (1 H, br. s).
LRMS (electrospray): m/z [MH⁺] 383.

### EXAMPLE 13

### 2-[4-(3,5-Dichlorophenoxy)-3,5-diethyl-1H-pyrazol-1-yl]ethylamine

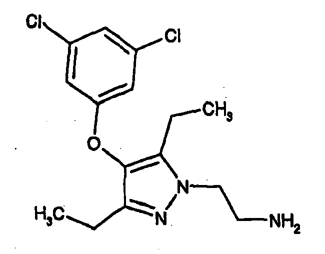

A mixture of the pyrazole (390mg, 1.37mmol) of Example 3 and chloroethylamine hydrochloride (238mg, 2.05mmol) was stirred and heated at 150°C for 24 hours. After cooling, the mixture was partitioned between saturated aqueous sodium bicarbonate solution (100ml) and dichloromethane (2x50ml). The combined organic layers were washed with brine (30ml), dried over magnesium sulphate, filtered and concentrated under reduced pressure. The resulting brown oil was purified by flash chromatography on silica gel eluting with dichloromethane:methanol (90:10, by volume) to afford the title compound (244mg) as a brown oil.
¹H-NMR (400MHz, CDCl₃): δ = 1.09 (6H, m), 2.41 (2H, q), 2.52 (2H, q), 3.18 (2H, t), 4.02 (2H, t), 6.78 (2H, s), 6.99 (1 H, s).
LRMS (electrospray): m/z [MH⁺] 330.
Microanalysis: Found: C, 52.28; H, 5.70; N, 11.75. C₁₅H₁₉Cl₂N₃O.H₂O requires C, 52.03; H, 6.11; N, 12.14%.

### EXAMPLE 14

### 3-{[4-(3,5-Dichlorophenoxy)-3,5-diethyl-1H-pyrazol-1-yl]methyl}-1,2,4-oxadiazol-5-ol

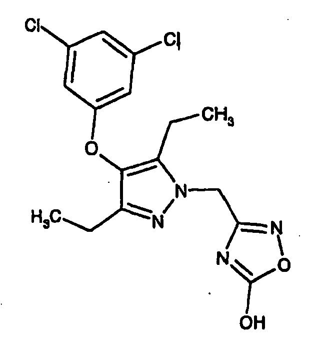

Ethylchloroformate (0.30ml, 3.08mmol) was added to a stirred solution of the amidoxime of Example 8 (500mg, 1.39mmol) in pyridine (8ml) at 0°C under nitrogen and the resulting solution was stirred for 10 minutes. The mixture was concentrated under reduced pressure and the residue was dissolved in a mixture of water (4ml), tetrahydrofuran (4ml) and 1 M aqueous sodium hydroxide solution (2ml). The mixture was heated under reflux for 1 hour, cooled to room temperature and stirred for a further 2 days. The resulting solution was diluted with 2M aqueous hydrochloric acid (20ml) and extracted with ethyl acetate (2x5Oml). The combined organic layers were washed with brine (50ml), dried over magnesium sulphate, filtered and evaporated under reduced pressure to leave a yellow oil. The oil was purified by flash column chromatography on silica gel eluting with pentane:ethyl acetate (50:50, by volume) to yield a white solid. The solid was dissolved in a mixture of tetrahydrofuran (1ml) and 1 M aqueous sodium hydroxide solution (10ml) and then heated under reflux for 24 hours. The resulting solution was diluted with 2M hydrochloric acid (20ml) and extracted with dichloromethane (2x50ml). The combined organic layers were washed with brine (50ml), dried over magnesium sulphate, filtered and evaporated under reduced pressure to give the title compound (113mg) as a white solid m.p. 94-96°C.
¹H-NMR (400MHz, CDCl₃): δ = 1.14 (m, 6H), 2.56 (m, 4H), 5.06 (s, 2H), 6.75 (s, 2H), 7.03 (s, 1 H).
LRMS (electrospray): m/z [M-(H⁺)] 381.

### EXAMPLE 15

### 5-{[4-(3,5-Dichlorophenoxy)-3,5-diethyl-1H-pyrazol-1-yl]methyl}-1,3,4-oxadiazol-2-amine

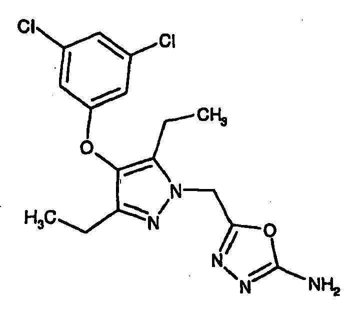

Cyanogen bromide (49mg, 0.462mmol) was added to a stirred solution of the hydrazide of Example 11 (150mg, 0.420mmol) in ethanol (30ml), at room temperature, under nitrogen and the resulting solution was heated to reflux for 2.5 hours. After cooling, the mixture was concentrated under reduced pressure to leave a brown oil. The crude product was purified by flash column chromatography on silica gel eluting with dichloromethane:methanol:ammonia (98:1.75:0.25, by volume) to provide the title compound (71 mg) as a white powder, m.p. 226-228°C.
¹H-NMR (400MHz, CDCl₃): δ = 1.00 (m, 6H), 2.29 (m, 2H), 2.55 (m, 2H), 5.34 (s, 2H), 6.90 (s, 2H), 7.07 (s, 2H), 7.24 (s, 1H).
LRMS (electrospray): m/z [MH⁺] 382.
Microanalysis: Found: C, 49.82; H, 4.52; N, 17.81. C₁₆H₁₇Cl₂N₅O₂.0.25H₂O requires C, 49.69; H, 4.56; N, 18.11 %.

### EXAMPLE 16

### N-{2-[4-(3,5-Dichlorophenoxy)-3,5-diethyl-1H-pyrazol-1-yl]ethyl}-2-methoxyacetamide

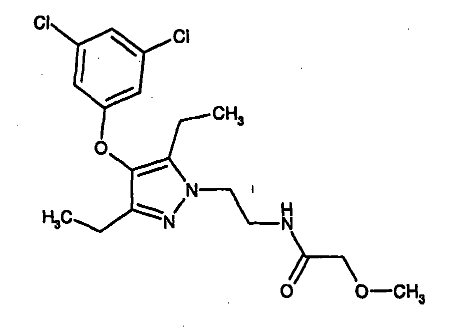

A solution of the pyrazole of Example 13 (53mg, 0.161mmol), 1-(3-(dimethylamino)propyl)-3-ethylcarbodiimide hydrochloride (34mg, 0.178mmol) and 4-(dimethylamino)pyridine (22mg, 0.178mmol) in dichloromethane (1ml) was added to a stirred solution of methoxyacetic acid (14.2µL, 0.178mmol) in dichloromethane (1ml) at room temperature. The reaction was stirred for 12 hours and then concentrated under a stream of nitrogen to leave a yellow solid. The crude product was purified by flash column chromatography on silica gel eluting with dichloromethane:methanol (98:2, by volume) to provide the title compound (54mg) as a brown solid, m.p. 75-76°C.
¹H-NMR (400MHz, CDCl₃): δ = 1.08 (t, 3H), 1.18 (t, 3H), 2.42 (q, 2H), 2.52 (q, 2H), 3.39 (s, 3H), 3.75 (m, 2H), 3.90 (s, 2H), 4.13 (t, 2H), 6.79 (s, 2H), 6.99 (s, 1H), 7.21 (br s, 1H).
LRMS (electrospray): m/z [MH⁺] 400; [M-(H⁺)] 398.
Microanalysis: Found: C, 54.09; H, 5.79; N, 10.39. C₁₈H₂₃Cl₂N₃O₃ requires C, 54.01; H, 5.79; N, 10.50%.

### EXAMPLES 17 AND 18

The compounds of the following tabulated Examples of the general formula: 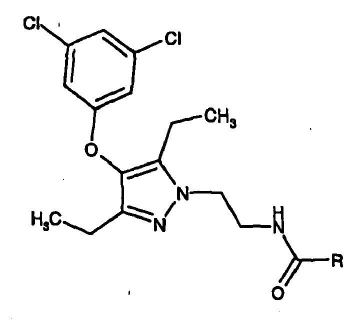 were prepared by a similar method to that of Example 16 using the appropriate acid starting material and the pyrazole of Example 13.

| Example No. | R | LRMS (thermospray) | Analytical Data |
|---|---|---|---|
| 17 | 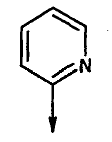 | m/z [MH⁺] 433 | ¹H-NMR (400MHz, CDCl₃): δ = 1.06 (t, 3H), 1.18 (t, 3H), 2.44 (q, 2H), 2.52 (q, 2H), 3.92 (m, 2H), 4.24 (t, 2H), 6.79 (s, 2H), 6.99 (s, 1H), 7.40 (m, 1H), 7.82 (m, 1H), 8.19 (m, 1H), 8.52 (br s, 2H), 8.55 (m, 1H). |
| | | | Microanalysis: Found: C, 57.01; H, 5.08; N, 11.94. C₂₁H₂₂Cl₂N₄O2 requires C, 58.21; H, 5.12; N, 12.03%. |
| 18 | 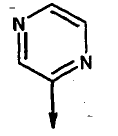 | m/z [MH⁺] 434 | ¹H-NMR (400MHz, CDCl₃): δ = 1.08 (t, 3H), 1.18 (t, 3H), 2.42 (q, 2H), 2.52 (q, 2H), 3.96 (m, 2H), 4.24 (t, 2H), 6.79 (s, 2H), 7.01 (s, 1H), 8.22 (br s, 1H), 8.54 (d, 1H), 8.78 (d, 1H), 9.40 (s, 1H). |

### EXAMPLE 19

### 3-{[3,5-Diethyl-1-(2-hydroxyethyl)-1H-pyrazol-4-yl]oxy}benzonitrile

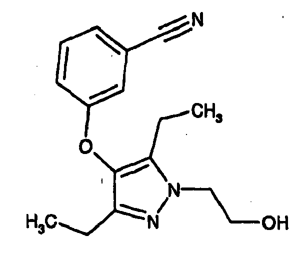

A mixture of the chloroketone of Preparation 2 (243mg, 1.50mmol), 3-cyanophenol (155mg, 1.50mmol), cesium carbonate (488mg, 1.50mmol) and acetone (10ml) was heated under reflux for 2 hours. After cooling, the solid was removed by filtration and the filtrate was concentrated under reduced pressure to leave a brown oil. The oil was dissolved in ethanol (10ml), hydroxyethylhydrazine (114mg, 1.50mmol) was added and the mixture was heated at 60°C for 18 hours. After cooling, the mixture was concentrated under reduced pressure. A solution of the residue in dichloromethane (10ml) was washed with 2M aqueous hydrochloric acid (5ml) and water (5ml), dried over magnesium sulphate, filtered and concentrated under reduced pressure to leave a yellow oil. The crude product was purified by flash column chromatography on silica gel eluting with ethyl acetate to provide the title compound (80mg) as a colourless oil.
¹H-NMR (400MHz, CDCl₃): δ = 1.10 (m, 6H), 2.40 (q, 2H), 2.50 (q, 2H), 3.68 (br s, 1 H), 4.07 (m, 4H), 7.12 (s, 1 H), 7.14 (d, 1H), 7.28 (d, 2H).
LRMS (electrospray): m/z [MH⁺] 286; [MNa⁺] 308.

### EXAMPLES 20 TO 38

The compounds of the following tabulated Examples of the general formula: 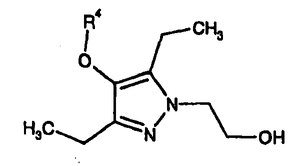 were prepared by a similar method to that of Example 19 using the appropriate phenols and the chloroketone of Preparation 2.

| Example No. | R⁴ | LRMS (electrospray) | Analytical Data |
|---|---|---|---|
| 20 | 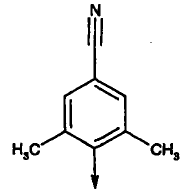 | m/z [MH⁺] 314. | ¹H-NMR (400MHz, CDCl₃): δ = 0.99 (t, 3H), 1.09 (t,3H), 2.18 (s, 6H), 2.25 (q, 2H), 2.40 (q, 2H), 3.78 (br s, 1H), 4.00 (m, 4H), 7.34 (s, 2H). |
| 21 | 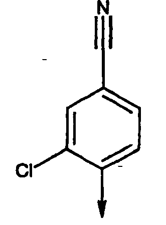 | m/z [MH⁺] 320. | ¹H-NMR (400MHz, CDCl₃): δ =1.10 (m, 6H), 2.40 (q, 2H), 2.53 (q, 2H), 3.56 (br s, 1H), 4.04 (m, 2H), 4.08 (m, 2H), 6.80 (d, 1H), 7.44 (d, 1H), 7.72 (s, 1H). |
| | | | Accurate Mass: Found: 320.1165 [MH⁺]; C₁₆H₁₈ClN₃O₂ requires 320.1161 [MH⁺]. |
| 22 | 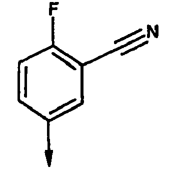 | m/z [MH⁺] 304. | ¹H-NMR (400MHz, CDCl₃): δ = 1.10 (m, 6H), 2.39 (q, 2H), 2.50 (q, 2H), 4.60 (m, 4H), 7.05 (m, 1H), 7.14 (m, 2H). |
| 23 | 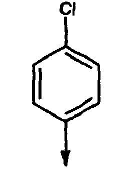 | m/z [MH⁺] 295. | ¹H-NMR (400MHz, CDCl₃): δ = 1.09 (m, 6H), 2.41 (m, 2H), 2.51 (m, 2H), 3.78 (br s, 1H), 4.06 (m, 4H), 6.81 (m, 2H), 7.21 (m, 2H). Microanalysis: Found: C, 60.88; H, 6.49; N, 9.40. C₁₅H₁₉ClN₂O₂ requires C, 61.12; H, 6.50; N, 9.50%. |
| 24 | 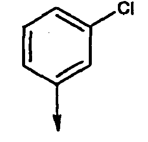 | m/z [MH⁺] 295. | ¹H-NMR (400MHz, CDCl₃): δ =1.09 (t, 3H), 1.14 (t, 3H), 2.41, (q, 2H), 2.52 (q, 2H), 3.79 (br s, 1H), 4.05 (m, 4H), 6.78 (d, 1H), 6.88 (s, 1H), 6.98 (d, 1H), 7.19 (t, 1H). |
| 25 | 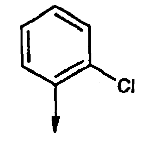 | M/z [MH⁺] 295. | ¹H-NMR (400MHz, CDCl₃): δ = 1.08 (t, 3H), 1.15 (t, 3H), 2.44 (q, 2H), 2.54 (q, 2H), 4.02 (m, 2H), 4.09 (m, 2H), 6.69 (d, 1H), 6.94 (t, 1H), 7.10(t, 1H), 7.40(d, 1H). |
| 26 | 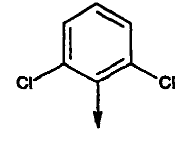 | M/z [MH⁺] 329. | ¹H-NMR (400MHz, CDCl₃): δ =1.01 (t, 3H), 1.10 (t, 3H), 2.38 (q, 2H), 2.49 (q, 2H), 3.84 (br s, 1H), 3.99 (m, 4H), 7.01 (t, 1H), 7.30 (d, 2H). |
| | | | Accurate Mass: Found: 329.0822 [MH⁺]; C₁₆H₁₈Cl₂N₂O₂ requires 329.0818 [MH⁺]. |
| 27 | 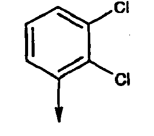 | m/z [M-(H⁺)] 328. | ¹H-NMR (400MHz, CDCl₃): δ = 1.09 (t, 3H), 1.14 (t, 3H), 2.41 (q, 2H), 2.51 (q, 2H), 4.03 (m, 2H), 4.07 (m, 2H), 6.60 (d, 1H), 7.04 (t, 1H), 7.10 (t, 1H). |
| 28 | 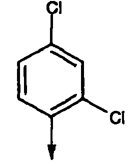 | m/z [MH⁺] 329. | ¹H-NMR (400MHz, CDCl₃): 8 =1.08 (t, 3H), 1.14 (t, 3H), 2.41 (q, 2H), 2.51 (q, 2H), 4.03 (m, 2H), 4.08 (m, 2H), 6.62 (d, 1H), 7.09 (d, 1H). |
| | | | Microanalysis: Found: C, 54.66; H, 5.54; N, 8.12. C₁₅H₁₈Cl₂N₂O₂ requires C, 54.72; H, 5.51; N, 8.51%. - |
| 29 | 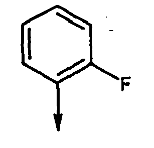 | m/z [MH⁺] 279. | ¹H-NMR (400MHz, CDCl₃): δ=1.10 (t, 3H), 1.14 (t, 3H), 2.44 (q, 2H), 2.55 (q, 2H), 3.79 (br s, 1H), 4.06 (m, 4H), 6.71 (m, 1H), 6.98 (m, 2H), 7.12 (m, 1H). |
| 30 | 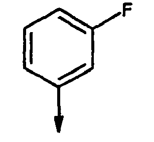 | m/z [MH⁺] 279. | ¹H-NMR (400MHz, CDCl₃): δ =1.09 (t, 3H), 1.12 (t, 3H), 2.43 (q, 2H), 2.52 (q, 2H), 3.78 (br s, 1H), 4.04 (m, 4H), 6.59 (m, 1H), 6.75 (m, 2H), 7.20 (m, 2H). |
| 31 | 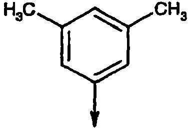 | m/z [MH⁺] 289. | 6H), 2.42 (q, 2H), 2.52 (q, 2H), 3.90 (br s, 1H), 4.05 (m, 4H), 6.49 (s, 2H), 6.62 (s, 1H). |
| 32 | 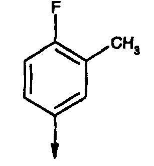 | m/z [MH⁺] 293. | ¹H-NMR (400MHz, CDCl₃): δ = 1.09 (t, 3H), 1.14 (t, 3H), 2.22 (s, 3H), 2.42 (q, 2H), 2.31 (q, 2H), 3.83 (br s, 1H), 4.03 (m, 4H), 6.60 (m, 1H), 6.70 (m, 1H), 6.88 (m, 1H). |
| 33 | 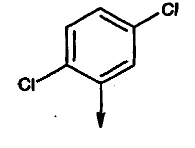 | m/z [MH⁺] 329. | ¹H-NMR (400MHz, CDCl₃): δ = 1.11 (t, 3H), 1.17 (t, 3H), 2.42 (q, 2H), 2.54 (q, 2H), 4.06 (m, 4H), 6.69 (s, 1H), 6.94 (d, 1H), 7.34 (d, 1H). |
| | | | Microanalysis: Found: C, 54.84; H, 5.67; N, 8.48. C₁₅H₁₈Cl₂N₂O₂ requires C, 54.72; H, 5.51; N, 8.51 %. |
| 34 | 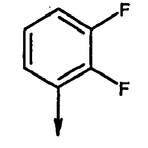 | m/z [MH⁺] 297. | ¹H-NMR (400MHz, CDCl₃): δ =1.09 (3, 3H), 1.12 (t, 3H), 2.42 (q, 2H), 2.50 (q, 2H), 3.68 (br s, 1H), 4.01 (m, 4H), 6.47 (m, 1H), 6.77 (m, 1H), 6.86 (m, 1H). |
| | | | Microanalysis: Found: C, 60.57; H, 6.23; N, 9.52. C₁₅H₁₈F₂N₂O₂ requires C, 60.80; H, 6.12; N, 9.45%. , |
| 35 | 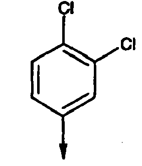 | m/z [MH⁺] 329. | ¹H-NMR (400MHz, CDCl₃): δ =1.08 (t, 3H), 1.12 (t, 3H), 2.41 (q, 2H), 2.51 (q, 2H), 3.73 (br s, 1H), 4.08 (m, 4H), 6.75 (d, 1H), 6.98 (s, 1H), 7.31 (d, 1H). |
| | | | Microanalysis: Found: C, 54.70; H, 5.54; N, 8.50. C₁₅H₁₈Cl₂N₂O₂ requires C, 54.72; H, 5.51; N, 8.51%. |
| 36 | 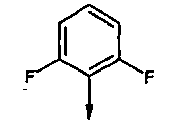 | m/z [MH⁺] 297. (q, | ¹H-NMR (400MHz, CDCl₃): δ =1.08 (t, 3H), 1.12 (t, 3H), 2.49 2H), 2.60 (q, 2H), 3.81 (br s, 1H), 3.99 (m, 4H), 6.91 (m, 2H), 6.99 (m, 1H). |
| 37 | 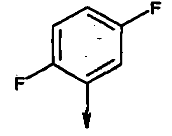 | m/z [MH⁺] 297. | ¹H-NMR (400MHz, CDCl₃): δ = 1.09 (t, 3H), 1.15 (t, 3H), 2.45 (q, 2H), 2.55 (q, 2H), 3.70 (br s, 1H), 4.06 (m, 4H), 6.46 (m, 1H), 6.62 (m, 1H), 7.08 (m, 1H). |
| 38 | 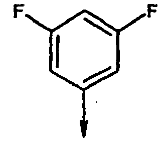 | m/z [MH⁺] 297. | ¹H-NMR (400MHz, CDCl₃): δ=1.08 (t, 3H), 1.14 (t, 3H), 2.41 (q, 2H), 2.53 (q, 2H), 3.72 (br s, 1H), 4.05 (m, 4H), 6.43 (m, 3H). |

### EXAMPLE 39

### 4-(3,5-Dichlorophenoxy)-3,5-diethyl-1-(2-methoxyethyl)-1H-pyrazole

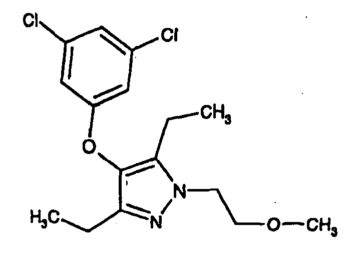

Sodium hydride (60% dispersion in oil, 34mg, 0.850mmol) was added to a stirred solution of 4-(3,5-dichlorophenoxy)-3,5-diethyl-1*H*-pyrazole of Example 3 (200mg, 0.701 mmol) and methoxyethyl bromide (117mg, 0.850mmol) in dry *N,N-*dimethylformamide (2ml) at 0°C under nitrogen. The mixture was stirred at 0°C for 45 minutes during which time hydrogen was evolved and the yellow solution turned dark brown. The reaction mixture was quenched by the addition of water (5ml) and the mixture concentrated under reduced pressure. The residue was dissolved in ethyl acetate (20ml) and washed with water (10ml) and brine (10ml) and then dried over magnesium sulphate, filtered and concentrated under reduced pressure to leave a brown oil. The crude product was purified by flash column chromatography on silica gel eluting with pentane:diethyl ether (80:20, by volume) to provide the title compound (140mg) as a colourless oil.
¹H-NMR (300MHz, CDCl₃): δ = 1.09 - 1.15 (m, 6H), 2.41 - 2.49 (q, 2H), 2.51 - 2.57 (q, 2H), 3.34 (s, 3H), 3.74 - 3.78 (t, 2H), 4.15 - 4.17 (t, 2H), 6.81 (s, 2H), 7.01 (s, 1 H).
LRMS (thermospray): m/z [MH⁺] 343.
Microanalysis: Found: C, 56.25; H, 5.94; N, 7.95. C₁₆H₂₀Cl₂N₂O₂ requires C, 55.99; H, 5.87; N, 8.16%.

### EXAMPLES 40 AND 41

The compounds of the following tabulated Examples of the general formula: 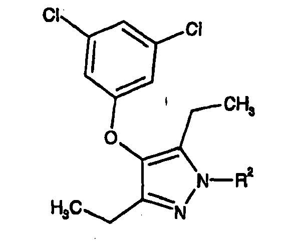 were prepared by a similar method to that of Example 39 using the appropriate halides and the pyrazole of Example 3.

| Example No. | R² | LRMS (thermospray) | Analytical Data |
|---|---|---|---|
| 40 | 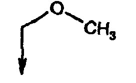 | m/z [MH⁺] 329. | ¹H-NMR (300MHz; CDCl₃): δ = 1.13 - 1.18 (m, 6H), 2.45 (q, 2H), 2.60 (q, 2H), 3.37 (s, 3H), 5.34 (s, 2H), 6.80 (s, 2H), 7.02 (s, 1H). |
| | | | Microanalysis: Found: C, 54.72; H, 5.46; N, 8.40. C₁₅H₁₈Cl₂N₂O₂ requires C, 54.72; H, 5.51; N, 8.51%. |
| 41 | 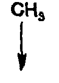 | m/z [MH⁺] 299. | ¹H-NMR (300MHz, CDCl₃): δ=1.15 (m, 6H), 2.48 (m, 4H), 3.79 (s, 3H), 6.82 (s, 2H), 7.01 (s, 1H). |
| | | | Microanalysis: Found: C, 56.08; H, 5.37; N, 9.29. C₁₄H₁₆Cl₂N₂O requires C, 56.20; H, 5.39; N, 9.36%. |

### EXAMPLE 42

### 4-(3,5-Dichlorophenoxy)-3-ethyl-1H-pyrazole

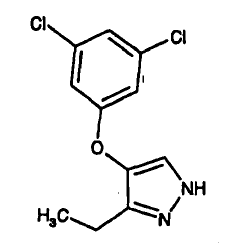

A solution of the enamine of Preparation 6 (2.88g, 10.0mmol) and hydrazine hydrate (0.49ml, 10.0mmol) in ethanol (10ml) was heated under reflux for 12 hours. After cooling further hydrazine hydrate (0.49ml, 10.0mmol) was added and the reaction was heated under reflux for 3 hours. After cooling the mixture was concentrated under reduced pressure and the residue was purified by flash column chromatography on silica gel eluting with cyclohexane:ethyl acetate (80:20, by volume) and then cyclohexane:ethyl acetate (60:40, by volume) to provide the title compound (620mg) as a yellow oil.
¹H-NMR (400MHz, CDCl₃): δ = 1.23 (t, 3H), 2.66 (q, 2H), 6.87 (s, 2H), 7.02 (s, 1 H), 7.40 (s, 1 H).
LRMS (electrospray): m/z [MH⁺] 257; [M-(H⁺)] 255.

### EXAMPLE 43

### 4-{2-[4-(3,5-Dichlorophenoxy)-3,5-diethyl-1H-pyrazol-1-yl]ethyl}morpholine

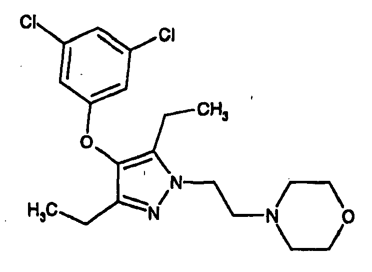

Osmium tetroxide (1.00ml of a 2.5% w/v solution in *tert*-butanol) was added dropwise to a stirred solution of the pyrazole of Example 64 (3.00g, 9.23mmol) and sodium periodate (4.93g, 23.1 mmol) in acetone (90ml) and water (30ml) at room temperature. A white precipitate formed after 5 minutes and the suspension was stirred for a further 3 hours. The solid was removed by filtration and the filtrate was concentrated under reduced pressure. The residue was partitioned between ethyl acetate (300ml) and water (100ml) and the organic phase was separated, dried over magnesium sulphate, filtered and concentrated under reduced pressure to yield an intermediate aldehyde. An aliquot of the aldehyde (100mg, 0.305mmol) was dissolved in dichloromethane (5ml) and morpholine (30mg, 0.344mmol) and glacial acetic acid (17.1µL, 0.305mmol) were added. After stirring at room temperature for 5 minutes sodium triacetoxyborohydride (95mg, 0.451mmol) was added in one portion and the reaction was stirred for 1 hour. After this time the resultant mixture was diluted with dichloromethane (20ml) and partitioned between water (30ml) and dichloromethane (20ml). The organic phase was washed with 2M aqueous sodium hydroxide solution (10ml), dried over magnesium sulphate, filtered and concentrated under reduced pressure. The crude product was purified by flash chromatography on silica gel eluting with dichloromethane:methanol:ammonia (95:4:1, by volume) to provide the title compound (125mg) as a colourless oil.
¹H-NMR (400MHz, CDCl₃): δ = 1.06 (m, 6H), 2.12 (m, 8H), 2.75 (t, 2H), 3.64 (m, 4H), 4.04 (t, 2H), 6.73 (s, 2H), 6.95 (s, 1 H).
LRMS (thermospray): m/z [MH⁺] 398.
Microanalysis: Found: C, 57.18; H, 6.31; N, 10.36. C₁₉H₂₅Cl₂N₃O₂ requires C, 57.29; H, 6.33; N, 10.55%.

### EXAMPLES 44 TO 49

The compounds of the following tabulated Examples of the general formula: 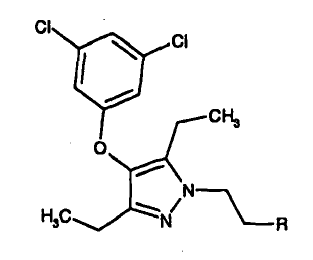 were prepared by a similar method to that of Example 43 using the appropriate amine starting material and the pyrazole of Example 64.

| Example No. | R | LRMS (thermospray) | Analytical Data |
|---|---|---|---|
| 44 | 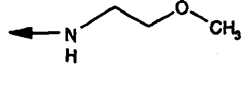 | m/z [MH⁺] 386. | ¹H-NMR (300MHz, CDCl₃): δ=1.09 -1.17 (m, 6H), 2.40 - 2.47 (q, 2H), 2.50 - 2.56 (q, 2H), 2.80 - 2.82 (t, 2H), 3.07 - 3.11 (t,2H), 3.36 (s, 3H), 3.47 - 3.51 (t, 2H), 4.09 - 4.11 (t, 2H), 6.81 (s, 2H), 7.01 (s, 1H). |
| 45 | 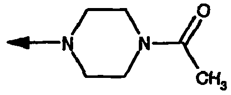 | m/z [MH⁺] 439. | ¹H-NMR (400MHz, CDCl₃): δ = 1.04 (m, 6H), 2.00 (s, 3H), 2.38 (m, 6H), 2.44 (q, 2H), 2.77 (q, 2H), 3.38 (m, 2H), 3.55 (m, 2H), 4.05 (m, 2H), 6.71 (s, 2H), 6.92 (s, 1H). |
| 46 | 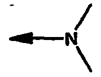 | m/z [MH⁺] 356. | ¹H-NMR (400MHz, CDCl₃): δ = 1.05 (m, 6H), 2.23 (s, 6H), 2.38 (q, 2H), 2.45 (q, 2H), 2,69 (m, 2H), 4.03 (m, 2H), 6.75 (s, 2H), 6.95 (s, 1H). |
| 47 | 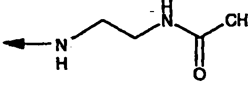 | m/z [MH⁺] 413. | ¹H-NMR (400MHz, CDCl₃): δ =1.08 (m, 6H), 1.59 (br s, 1H), 1.91 (s, 3H), 2.38 (q, 2H), 2.48 (q, 2H), 2.71 (m, 2H), 2.99 (m, 2H), 3.28 (m, 2H), 4.02 (m, 2H), 6.09 (br s, 1H), 6.71 (s, 2H), 6.95 (s, 1H). |
| | | | Microanalysis: Found: C, 54.86; H, 6.32; N, 13.33. C₁₉H₂₆Cl₂N₄O₂ requires C, 55.21; H, 6.34; N, 13.55%. m.p. 69-70°C. |
| 48 | 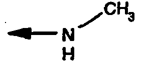 | m/z [MH⁺] 342. | ¹H-NMR (400MHz, CDCl₃): δ=1.08 (m, 7H), 2.39 (m, 2H), 2.42 (s, 3H), 2.49 (q, 2H), 3.00 (m, 2H), 4.05 (m, 2H), 6.78 (s, 2H), 6.96 (s, 1H). |
| 49 | 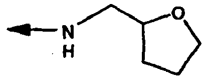 | m/z [MH⁺] 412. | ¹H-NMR (400MHz, CDCl₃): δ=1.05 (m, 6H), 1.49 (m, 1H), 1.81 (m, 4H), 2.42 (q, 2H), 2.52 (q, 2H), 2.64 (m, 2H), 3.08 (t, 2H), 3.76 (m, 1H), 3.79 (m, 1H), 4.00 (m, 1H), 6.78 (s, 2H), 6.98 (s, 1H). |
| | | | Microanalysis: Found: C, 57.78; H, 6.68; N, 9.90. C₂₀H₂₇Cl₂N₃O₂ requires C, 58.13; H, 6.61; N, 10.17%. |

### EXAMPLE 50

### 3-{[4-(3,5-Dichlorophenoxy)-3,5-diethyl-1H-pyrazol-1-yl]methyl}morpholine

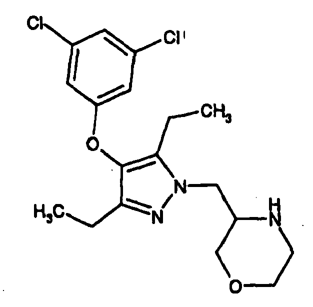

Sodium hydride (60% dispersion in oil, 37mg, 0.925mmol) was added to a stirred solution of the mesylate of Preparation 11 (273mg, 0.925mmol) and the pyrazole of Example 3 (220mg, 0.772mmol) in dry *N,N*-dimethylformamide (4ml) at 0°C under nitrogen. The mixture was heated at 50°C for 3 hours during which time the yellow solution turned dark brown. The reaction mixture was quenched by the addition of water (5ml) and the mixture was concentrated under reduced pressure. A solution of the residue in ethyl acetate (20ml) was washed with water (10ml) and brine (10ml), dried over magnesium sulphate, filtered and concentrated under reduced pressure to leave a brown oil. The oil was dissolved in dichloromethane (3ml), trifluoroacetic acid (1ml) was added and the reaction was stirred at room temperature for 12 hours. The mixture was concentrated under reduced pressure and the residue was dissolved in ethyl acetate (10ml) and washed with 1M aqueous hydrochloric acid (2x5ml). The combined aqueous phases were neutralised with solid sodium carbonate and extracted with ethyl acetate (3x20ml). The combined ethyl acetate layers were dried over magnesium sulphate, filtered and concentrated under reduced pressure. The crude product was purified by flash column chromatography on silica gel eluting with dichloromethane:methanol:ammonia (95:4:1, by volume) to provide the title compound (3mg) as a colourless oil.
¹H-NMR (400MHz, CDCl₃): δ=1.15 (m, 6H), 2.41 (q, 2H), 2.51 (q, 2H), 2.89 (m, 2H), 3.30 (m, 2H), 3.58 (m, 1 H), 3.78 (m, 2H), 3.87 (d, 2H), 6.88 (s, 2H), 7.00 (1 H, s).
LRMS (thermospray): m/z [MH⁺] 384.

### EXAMPLE 51

### 1-(3-Azetidinyl)-4-(3,5-dichlorophenoxy)-3,5-diethyl-1H-pyrazole

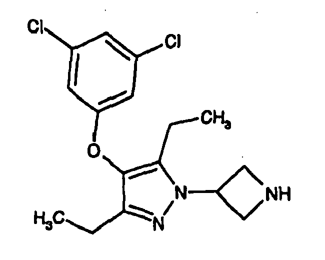

Sodium hydride (60% dispersion in oil, 30mg, 0.750mmol) was added to a stirred solution of the pyrazole of Example 3 (200mg, 0.702mmol) and 1-benzhydryl-3-azetidinyl methanesulfonate (222mg, 0.702mmol) (see J. Org. Chem., 1972, 37, 3953) in *N,N*-dimethylformamide (5ml) at 0°C under nitrogen. The mixture was heated at 50°C for 4 hours and then cooled to room temperature. The reaction mixture was quenched by the addition of water (30ml) and the aqueous mixture was extracted with ether (2x50ml). The combined organic phases were washed with water (10ml) and brine (10ml), dried over magnesium sulphate, filtered and concentrated under reduced pressure to leave a brown oil. The oil was purified by flash column chromatography on silica gel eluting with dichloromethane to provide the intermediate (60mg) as a yellow oil. The oil was dissolved in dichloromethane (5ml) and 1-chloroethylchloroformate (20µL, 0.182mmol) was added at room temperature under nitrogen. The mixture was heated under reflux for 4 hours, cooled to room temperature and concentrated under reduced pressure to leave a yellow oil. The oil was dissolved in methanol (5ml) and the resulting solution was heated under reflux for 1 hour, cooled to room temperature and concentrated under reduced pressure. The crude product was purified by flash column chromatography on silica gel eluting with dichloromethane:methanol:ammonia (95:4:1, by volume) to provide the title compound (17mg) as a colourless oil.
¹H-NMR (400MHz, CDCl₃): δ = 1.08 (t, 3H), 1.16 (t, 3H), 2.48 (m, 4H), 3.87 (t, 2H), 4.40 (t, 2H), 5.07 (q, 1 H), 6.79 (s, 2H), 7.01 (m, 1H).
LRMS (thermospray): m/z [MH⁺] 340.

### EXAMPLE 52

### 7-(3,5-Dichlorophenoxy)-6-ethyl-2,3-dihydropyrazolo[5,1-b][1,3]oxazole

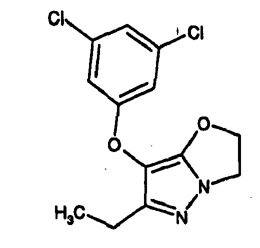

The triflate of Preparation 15 (282mg, 0.500mmol), tributylvinyltin (175µL, 0.600mmol), palladium dibenzylidene acetone (23mg, 0.025mmol), triphenyl arsine (12mg, 0.040mmol) and lithium chloride (64mg, 1.50mmol) were heated in *N,N-*dimethylformamide (3ml) at 80°C under nitrogen for 12 hours. The reaction was cooled to room temperature and partitioned between water (20ml) and ethyl acetate (20ml). The organic layer was washed with brine (10ml), dried over magnesium sulphate, filtered and concentrated under reduced pressure. The crude product was purified by flash column chromatography on silica gel eluting with pentane:ethyl acetate (90:10, by volume) to provide the title compound (34mg) as a colourless oil.
¹H-NMR (400MHz, CDCl₃): δ = 1.16 (t, 3H), 2.45 (q, 2H), 4.29 (t, 2H), 5.03 (t, 2H), 6.89 (s, 2H), 7.02 (s, 1 H).
LRMS (thermospray): m/z [MH⁺] 299.

### EXAMPLE 53

### 4-(3,5-Dichlorophenoxy)-3,5-dimethyl-1H-pyrazole

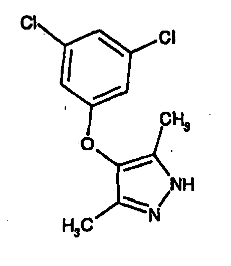

A mixture of 3-chloro-2,4-pentanedione (5.00g, 37.0mmol), 3,5-dichlorophenol (6.03g, 37.0mmol), cesium carbonate (12.0g, 37.0mmol) and acetone (40ml) was heated under reflux for 18 hours. After cooling the solid was removed by filtration and the filtrate concentrated under reduced pressure. The intermediate was dissolved in ethanol (30ml) and hydrazine hydrate (1.85g, 37.0mmol) was added and the mixture heated at 60°C for 30 minutes. After cooling the mixture was concentrated under reduced pressure and the residue purified by flash column chromatography on silica gel eluting with ethyl acetate:pentane (30:70, by volume) to provide the title compound (3.00g) as a yellow oil which solidified on standing to leave a yellow solid, m.p. 85-87°C.
¹H-NMR (300MHz, CDCl₃): δ = 2.14 (s, 6H), 5.24 (br s, 1H), 6.81 (s, 2H), 7.02 (s, 1 H).
LRMS (thermospray): m/z [MH⁺] 257.
Microanalysis: Found: C, 49.58; H, 4.06; N, 11.05. C₁₁H₁₀Cl₂N₂O.0.4H₂O requires C, 49.98; H, 4.12; N, 10.60%.

### EXAMPLE 54

### 1-[4-(3,5-Dichlorophenoxy)-3,5-diethyl-1H-pyrazol-1-yl]-2-propanol

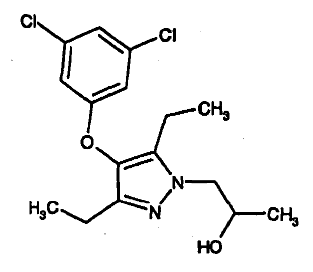

Osmium tetroxide (1.00ml of a 2.5% w/v solution in *tert*-butanol) was added dropwise to a stirred solution of the pyrazole of Example 64 (3.00g, 9.23mmol) and sodium periodate (4.93g, 23.1 mmol) in acetone (90ml) and water (30ml) at room temperature. A white precipitate formed after 5 minutes and the suspension was stirred for a further 3 hours. The solid was removed by filtration and the filtrate was concentrated under reduced pressure. The residue was partitioned between ethyl acetate (300ml) and water (100ml) and the organic phase was separated, dried over magnesium sulphate, filtered and concentrated under reduced pressure to yield the intermediate aldehyde. An aliquot of the aldehyde (250mg, 0.765mmol) was dissolved in tetrahydrofuran (5ml) and stored under nitrogen. In a separate flask, anhydrous cerium trichloride (377mg, 1.53mmol) was added to a stirred solution of methyl magnesium bromide (0.51ml of a 3M solution in ether, 1.53mmol) in tetrahydrofuran (5ml) at room temperature under nitrogen. The mixture was stirred at room temperature for 1.5 hours and the aldehyde in tetrahydrofuran was added dropwise. The mixture was stirred for 12 hours and the reaction was then quenched with 1M aqueous acetic acid at room temperature. The mixture was diluted with dichloromethane (20ml), washed with water (5ml) and brine (5ml), dried over magnesium sulphate, filtered and concentrated under reduced pressure. The crude product was purified by flash column chromatography on silica gel eluting with pentane:ethyl acetate (70:30, by volume) to provide the title compound (30mg) as a colourless oil.
¹H-NMR (400MHz, CDCl₃): δ = 1.05 (t, 3H), 1.10 (t, 3H), 1.21 (d, 2H), 2.40 (q, 2H), 2.47 (q, 2H), 3.79 (dd, 1 H), 3.97 (dd, 1 H), 4.24 (s, 1H), 6.76 (s, 2H), 6.98 (s, 1H).
LRMS (thermospray): m/z [MH⁺] 343.

### EXAMPLE 55

### 2-{2-[4-(3,5-Dichlorophenxoy)-3,5-diethyl-1H-pyrazol-1-yl)ethoxy}ethanamine

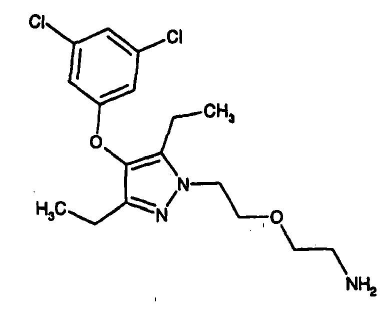

Sodium hydride (60% dispersion in oil, 24mg, 0.600mmol) was added to a stirred solution of the pyrazole of Example 2 (100mg, 0.303mmol) in dry *N,N-*dimethylformamide (4ml) at 0°C under nitrogen. The mixture was stirred at 0°C for 30 minutes and 2-chloroethylamine hydrochloride (53mg, 0.455mmol) was added.

The reaction mixture was stirred at 0°C for 30 minutes and then stirred at room temperature for 30 minutes. The reaction was cooled to 0°C, further sodium hydride (60% dispersion in oil, 24mg, 0.600mmol) and 2-chloroethylamine hydrochloride (53mg, 0.455mmol) were added and the reaction was stirred for 1 hour. The reaction was quenched by the addition of water (5ml) and extracted with ether (10ml). The organic layer was washed with 2M aqueous hydrochloric acid (30ml). The acid was neutralised with solid sodium carbonate and extracted with ether (3x20ml). The combined ether layers were dried over magnesium sulphate, filtered and concentrated under reduced pressure. The crude product was purified by flash column chromatography on silica gel eluting with dichloromethane:methanol:ammonia (95:4:1, by volume) to provide the title compound (21 mg) as a colourless oil.
¹H-NMR (400MHz, CDCl₃): δ = 1.19 (m, 6H), 2.42 (q, 2H), 2.58 (q, 2H), 2.80 (t, 2H), 3.38 (t, 2H), 3.81 (t, 2H), 4.18 (t, 2H), 6.78 (s, 2H), 7.02 (s, 1H).
LRMS (thermospray): m/z [MH⁺] 372.

### EXAMPLE 56

### 4-{[4-(3,5-Dichlorochenoxy)-3-methyl-1H-pyrazol-5-yl]methyl}morcholine

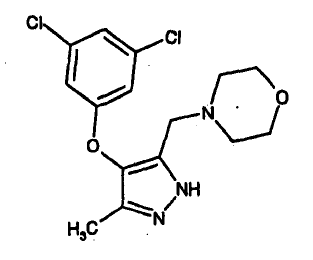

Morpholine (140µL, 1.59mmol) was added in one portion to a stirred solution of the bromide of Preparation 8 (200mg, 0.531mmol) in isopropanol (4ml) at room temperature. The mixture was heated at 50°C for 1 hour, cooled to room temperature and concentrated under reduced pressure to leave a yellow oil. The crude product was purified by flash column chromatography on silica gel eluting with ethyl acetate to provide the title compound (60mg) as a colourless oil.
¹H-NMR (400MHz, CDCl₃): δ = 2.13 (s, 3H), 2.42 (m, 4H), 3.38 (s, 2H), 3.64 (m, 4H), 6.79 (s, 2H), 7.02 (s, 1H).
LRMS (thermospray): m/z [MH⁺] 342.

### EXAMPLE 57

### 4-(3,5-Dichlorophenoxy)-3-methyl-5-[(2-methyl-1H-imidazol-1-yl)methyl]-1H-pyrazole

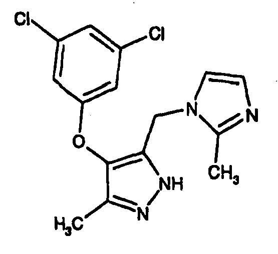

Sodium hydride (60% dispersion in oil, 32mg, 0.800mmol) was added to a stirred solution of 2-methylimidazole (65mg, 0.800mmol) in *N,N*-dimethylformamide (5ml) at 0°C under nitrogen. The mixture was stirred for 10 minutes and then the bromide of Preparation 8 (100mg, 0.261 mmol) was added and the reaction was stirred at room temperature for 1 hour. The reaction mixture was quenched by the addition of 1 M aqueous sodium hydroxide solution (5ml) and the mixture was concentrated under reduced pressure. A solution of the residue in ethyl acetate (20ml) was washed with water (10ml) and brine (10ml), dried over magnesium sulphate, filtered and concentrated under reduced pressure to leave a brown oil. The crude product was purified by flash column chromatography on silica gel eluting with dichloromethane:methanol:ammonia (95:4.5:0.5, by volume) to provide the title compound (10mg) as a colourless oil.
¹H-NMR (300MHz, CDCl₃): δ = 2.14 (s, 3H), 2.35 (s, 3H), 4.89 (s, 2H), 6.68 (s, 2H), 6.78 (s, 1H), 6.82 (s, 1 H), 7.03 (s, 1H).
LRMS (thermospray): m/z [MH⁺] 337.

### EXAMPLE 58

### 2-[4-(3,5-Dichlorophenoxy)-3-ethyl-5-methoxy-1H-pyrazol-1-yl]ethanol

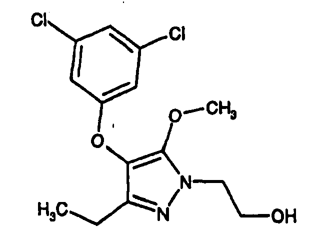

The triflate of Preparation 15 (282mg, 0.500mmol) was dissolved in methanol (3ml) and 1,1'-bis(diphenylphosphino)ferrocenepalladium(II)chloride (18mg, 0.025mmol) was added in one portion at room temperature. The mixture was heated at 50°C under an atmosphere of carbon monoxide (345 kPa, 50 psi) for 10 hours. The reaction was cooled to room temperature and concentrated under reduced pressure to leave a brown oil. The oil was dissolved in a mixture of tetrahydrofuran (0.5ml), glacial acetic acid (1.0ml) and water (0.5ml) and stirred at room temperature for 12 hours. The solvent was removed under a stream of nitrogen to leave a yellow solid and the crude product was purified by flash column chromatography on silica gel eluting with dichloromethane:acetonitrile (95:5, by volume) and then dichloromethane:acetonitrile (90:10, by volume) to provide the title compound (6mg) as a colourless oil.
¹H-NMR (300MHz, CDCl₃): δ = 1.13 (t, 3H), 2.41 (q, 2H), 3.44 (br s, 1 H), 3.94 (s, 3H), 4.23 (m, 4H), 6.87 (s, 2H), 7.09 (s, 1H).
LRMS (thermospray): m/z [MH⁺] 331.

### EXAMPLE 59

### 1-{[4-(3,5-Dichlorophenoxy)-3-methyl-1H-pyrazol-5-yl]methyl]-1H-1,2,4-triazole

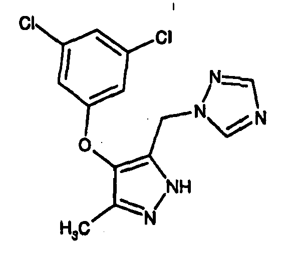

A suspension of the bromide of Preparation 8 (100mg, 0.264mmol), 1,2,4-triazole (92mg, 1.32mmol) and sodium carbonate (140mg, 1.32mmol) in toluene (5ml) was heated at 100°C for 12 hours. The suspension was cooled to room temperature and 1 M aqueous sodium hydroxide solution (5ml) was added. The mixture was extracted with ethyl acetate (3x20ml) and the combined organic phases were dried over magnesium sulphate, filtered and concentrated under reduced pressure to leave a clear oil. The crude product was purified by flash column chromatography on silica gel eluting with dichloromethane:methanol:ammonia (95:4.5:0.5, by volume) to provide the title compound (62mg) as a colourless oil.
¹H-NMR (300MHz, CDCl₃): δ = 2.16 (s, 3H), 5.25 (s, 2H), 6.70 (s, 2H), 7.04 (s, 1H), 7.89 (s, 1H), 8.04 (s, 1 H).
LRMS (thermospray): m/z [MH⁺] 324.

### EXAMPLE 60

### 3-[(3,5-Diethyl-1H-pyrazol-4-yl)oxy]benzonitrile

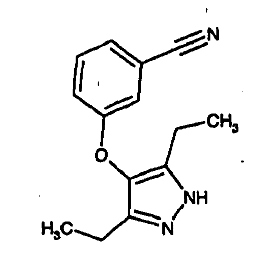

Hydrazine hydrate (153µL, 3.14mmol) was added to a stirred solution of the β-diketone of Preparation 9 (771 mg, 3.14mmol) in ethanol (16ml) and the resulting solution was heated under reflux for 12 hours. After cooling the mixture was concentrated under reduced pressure and the residue was purified by flash column chromatography on silica gel eluting with pentane:ethyl acetate (75:25, by volume) to provide the title compound (712mg) as a yellow solid, m.p. 81-84°C.
¹H-NMR (400MHz, CDCl₃): δ = 1.15 (t, 6H), 2.47 (q, 4H), 7.11 (m, 2H), 7.24 (d, 1 H), 7.35 (t, 1 H).
LRMS (thermospray): m/z [MH⁺] 242.
Microanalysis: Found: C, 69.03; H, 6.43; N, 17.20. C₁₄H₁₅N₃O₃.0.13H₂O requires C, 69.02; H, 6.31; N, 17.25%.

### EXAMPLE 61

### 3-{[1-2-Aminoethyl)-3,5-diethyl-1H-pyrazol-4-yl]oxy}benzonitrile

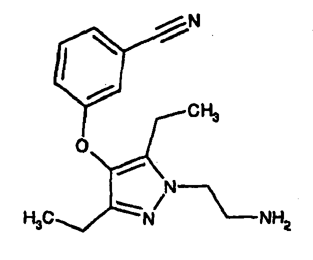

The pyrazole of Example 60 (200mg, 0.829mmol) and 2-chloroethylamine hydrochloride (144mg, 1.24mmol) were heated as a melt at 150°C for 17 hours. After cooling the solid was dissolved in saturated aqueous sodium hydrogencarbonate (15ml) and extracted with dichloromethane (2x10ml). The combined organic phases were washed with 2M aqueous hydrochloric acid (20ml) and the aqueous layer was neutralised with solid sodium carbonate and extracted with dichloromethane (3x10ml). The combined organic phases were dried over magnesium sulphate, filtered and concentrated under reduced pressure to leave an orange gum. The crude product was purified by flash column chromatography on silica gel eluting with dichloromethane:methanol (90:10) then dichloromethane:methanol:ammonia (90:9:1, by volume) to provide the title compound (124mg) as a pale yellow oil.
¹H-NMR (400MHz, CDCl₃): δ = 1.11 (m, 6H), 2.41 (q, 2H), 2.52 (q, 2H), 3.18 (t, 2H), 4.04 (t, 2H), 7.15 (m, 2H), 7.29 (d, 1 H), 7.38 (t, 1H).
LRMS (thermospray): m/z [MH⁺] 285.

### EXAMPLE 62

### 2-[4-(3-Cyanophenoxy)-3,5-diethyl-1H-pyrazol-1-yl]acetamide

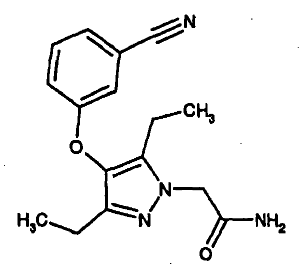

A saturated solution of ammonia in methanol (2.3ml) was added to the ester of Example 63 (75mg, 0.229mmol) in a vial at room temperature then the vial was sealed and heated at 75°C for 17 hours. After cooling to room temperature the mixture was concentrated under reduced pressure to leave a cream solid. The crude product was purified by flash column chromatography on silica gel eluting with dichloromethane then dichloromethane:methanol (99:1, by volume) to provide the title compound (49mg) as a white solid, m.p. 159-160°C.
¹H-NMR (400MHz, CDCl₃): δ = 1.10 (t, 3H), 1.17 (t, 3H), 2.44 (q, 2H), 2.53 (q, 2H), 4.69 (s, 2H), 5.44 (br s, 1 H), 6.22 (br s, 1 H), 7.14 (m, 2H), 7.31 (d, 1 H), 7.40 (t, 1H).
LRMS (thermospray): m/z [MH⁺] 299.
Microanalysis: Found: C, 64.20; H, 6.12; N, 18.79. C₁₆H₁₈N₄O₂ requires C, 64.41; H, 6.08; N, 18.78%.

### EXAMPLE 63

### Ethyl [4-(3-cyanophenoxy)-3,5-diethyl-1H-pyrazol-1-yl]acetate

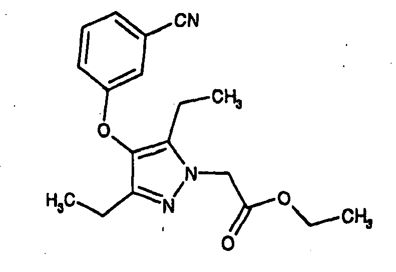

A solution of ethylhydrazinoacetate (88mg, 0.571mmol) in ethanol (2.0ml) was added to a stirred solution of the β-diketone of Preparation 9 (140mg, 0.571 mmol) and triethylamine (88µL, 0.628ml) in ethanol (1.0ml) and the resulting solution was heated under reflux for 18 hours. After cooling, the mixture was concentrated under reduced pressure and the residue was partitioned between dichloromethane (20ml) and water (10ml). The organic layer was separated, washed with brine (10ml), dried over magnesium sulphate, filtered and concentrated under reduced pressure. The crude product was purified by flash column chromatography on silica gel eluting with pentane:ethyl acetate (75:25, by volume) and then ethyl acetate to provide the title compound (131 mg) as a yellow oil.
¹H-NMR (400MHz, CDCl₃): δ = 1.08 (m, 6H), 1.25 (t, 3H), 2.40 (m, 4H), 4.20 (q, 2H), 4.77 (s, 2H), 7.12 (m, 2H), 7.23 (d, 1 H), 7.34 (t, 1H).
LRMS (thermospray): m/z [MH⁺] 328.

### EXAMPLE 64

### 1-Allyl-4-(3,5-dichloroghenoxy)-3,5-diethyl-1H-pyrazole

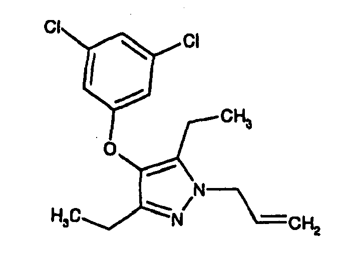

Sodium hydride (60% dispersion in oil, 770mg, 19.2mmol) was added to a stirred solution of allyl bromide (1.70ml, 19.2mmol) and the pyrazole of Example 3 (5.00g, 17.5mmol) in *N,N*-dimethylformamide (20ml) at 0°C under nitrogen. The reaction was warmed to room temperature and stirred for 1 hour. The reaction mixture was quenched by the addition of water (100ml) and the aqueous phase was extracted with ether (2x50ml). The combined organic phases were washed with water (30ml) and brine (30ml), dried over magnesium sulphate, filtered and concentrated under reduced pressure to leave a brown oil. The crude product was purified by flash column chromatography on silica gel eluting with pentane:ethyl acetate (80;20, by volume) to provide the title compound (5.00g) as a yellow oil.
¹H-NMR (400MHz, CDCl₃): δ = 1.11 (m, 6H), 2.46 (m, 4H), 4.65 (d, 2H), 5.04 (d, 1 H), 5.22 (d, 1 H), 5.99 (m, 1 H), 6.79 (s, 2H), 6.99 (s, 1H).
LRMS (thermospray): m/z [MH⁺] 325.

### EXAMPLE 65

### N-{[4-(3,5-Dichlorophenoxy)-3-methyl-1H-pyrazol-5-yl]methyl}-N-(4-methoxybenzyl)amine

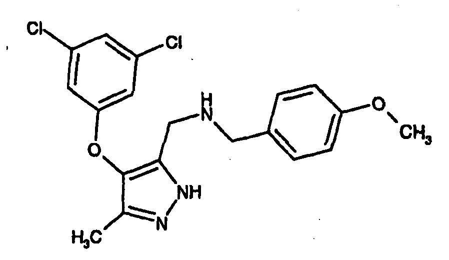

4-Methoxybenzylamine (0.104ml, 0.800mmol) was added in one portion to a stirred solution of the bromide of Preparation 8 (100mg, 0.265mmol) in isopropanol (2ml) at room temperature. The mixture was heated at 50°C for 1 hour, cooled to room temperature and concentrated under reduced pressure to leave a yellow oil. The oil was diluted with diethyl ether (20ml), washed with saturated aqueous sodium hydrogen carbonate (5ml) and water (5ml), dried over magnesium sulphate, filtered and concentrated under reduced pressure. The crude product was purified by flash column chromatography on silica gel eluting with dichloromethane:methanol:ammonia (95:4:1, by volume) to provide the title compound (50mg) as a colourless oil.
¹H-NMR (300MHz, CDCl₃): δ = 2.13 (s, 3H), 3.68 (s, 2H), 3.71 (s, 2H), 3.80 (s, 3H), 6.83 (m, 4H), 7.03 (s, 1 H), 7.17 (m, 2H).
LRMS (thermospray): m/z [MH⁺] 392.

### EXAMPLES 66 TO 75

The compounds of the following tabulated Examples of the general formula: 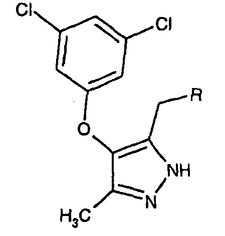 were prepared by a similar method to that of Example 65 using the appropriate amine starting material and the bromide of Preparation 8.

| Example No. | R | LRMS (thermospray) | Analytical Data |
|---|---|---|---|
| 66 | 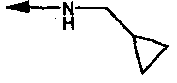 | m/z [MH⁺] 326. | ¹H-NMR (300MHz, CDCl₃): 8 = 0.09 (m, 2H), 0.49 (q, 2H), 0.90 (m, 1H), 2.34 (s, 3H), 2.47 (d, 2H), 3.73 (s, 2H), 6.82 (s, 2H), 7.03 (s, 1H). |
| 67 | 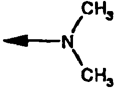 | m/z [MH⁺] 300. | ¹H-NMR (400MHz, CDCl₃): δ = 2.08 (s, 3H), 2.20 (s, 6H), 3.31 (s, 2H), 6.76 (s, 2H), 6.97 (s, 1H). |
| 68 | 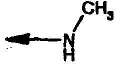 | m/z [MH⁺] 286. | ¹H-NMR (400MHz, CDCl₃): 8 = 2.12 (s, 3H), 2.42 (s, 3H), 3.65 (s, 2H), 6.80 (s, 2H), 7.02 (s, 1H). |
| 69 | 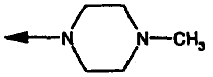 | m/z [MH⁺] 355. | ¹H-NMR (400MHz, CDCl₃): δ = 2.08 (s, 3H), 2.22 (s, 3H), 2.31 (m, 8H), 3.36 (s, 2H), 6.76 (s, 2H), 6.97 (s, 1H). |
| 70 | 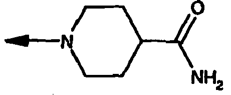 | m/z [MH⁺] 385. | ¹H-NMR (400MHz, CDCl₃): 8 = 1.50 (m, 2H), 1.60 (m, 2H), 1.90 (m, 4H), 2.41 (m, 2H), 3.25 (s, 2H), 3.75 (m, 2H), 5.52 (s, 1H), 5.80 (s, 1H), 6.67 (s, 2H), 6.86 (s, 1H). |
| 71 | 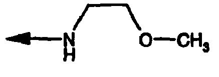 | m/z [MH⁺] 330. | ¹H-NMR (400MHz, CDCl₃): δ = 2.08 (s, 3H), 2.74 (m, 2H), 3.30 (s, 3H), 3.44 (m, 2H), 3.68 (s, 2H), 6.76 (s, 2H), 6.98 (s, 1H). |
| 72 | 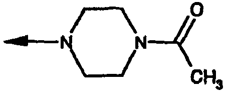 | m/z [MH⁺] 383. | ¹H-NMR (400MHz, CDCl₃): δ = 2.02 (s, 3H), 2.10 (s, 3H), 2.38 (m, 4H), 3.34 (m, 2H), 3.38 (s, 2H), 3.51 (m, 2H), 6.76 (s, 2H), 6.98 (s, 1H). |
| 73 | 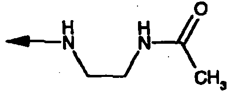 | m/z [MH⁺] 357. | ¹H-NMR (400MHz, CDCl₃): δ = 1.92 (s, 3H), 2.09 (s, 3H), 2.70 (m, 2H), 3.29 (m, 2H), 3.65 (s, 2H), 6.01 (s, 1H), 6.76 (s, 2H), 6.99 (s, 1H). |
| 74 | 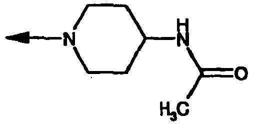 | m/z [MH⁺] 397. | ¹H-NMR (400MHz, CDCl₃): δ = 1.30 (m, 2H), 1.80 (m, 2H), 1.92 (s, 3H), 2.09 (m, 5H), 2.70 (m, 2H), 3.34 (s, 2H), 3.71 (m, 1H), 6.76 (s, 2H), 6.98 (s, 1H). |
| 75 | 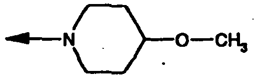 | m/z [MH⁺] 370. | ¹H-NMR (300MHz, CDCl₃): δ = 1.60 (m, 2H), 1.80 (m, 2H), 2.13 (s, 3H), 2.20 (m, 2H), 2.71 (m, 2H), 3.22 (m, 1H), 3.33 (s, 3H), 3.39 (s, 2H), 6.81 (s, 2H), 7.03 (s, 1H). |

### EXAMPLE 76

### 3-Chloro-5-[(3,5-dimethyl-1H-pyrazol-4-yl)oxy]benzonitrile

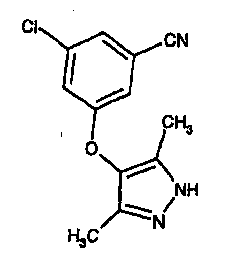

Hydrazine hydrate (1.10ml, 21.9mol) was added to a stirred solution of the β-diketone of Preparation 16 (5.50g, 21.9mmol) in glacial acetic acid (22ml) and the resulting solution was stirred at room temperature for 14 hours. The mixture was concentrated under reduced pressure and the residue was purified by flash column chromatography on silica gel eluting with dichloromethane and then dichloromethane:ethyl acetate (85:15, by volume) to provide the title compound (4.80g) as a yellow solid, m.p. 136-140°C.
¹H-NMR (400MHz, CDCl₃): δ = 2.09 (s, 6H), 7.02 (m, 1H), 7.10 (m, 1H), 7.25 (m, 1 H).
LRMS (electrospray): m/z [MH⁺] 248.
Microanalysis: Found: C, 57.91; H, 4.03; N, 16.79. C₁₂H₁₀N₃OCl requires C, 58.19; H, 4.07; N, 16.97%.

### EXAMPLE 77

### 3-{[5-(Aminomethyl)-3-methyl-1H-pyrazol-4-yl]oxy}-5-chlorobenzonitrile

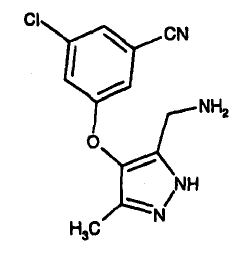

The bromide of Preparation 18 (300mg, 0.800mmol) was added to a saturated solution of ammonia in isopropanol (50ml) at 0°C. The reaction was stirred for 2 hours and allowed to slowly warm to room temperature. The mixture was concentrated under reduced pressure and the resulting yellow oil was dissolved in dichloromethane (50ml). The dichloromethane solution was washed with 1 M aqueous sodium carbonate solution (20ml), dried over magnesium sulphate, filtered and concentrated under reduced pressure to provide the title compound (220mg) as a white foam.
¹H-NMR (300MHz, CDCl₃): δ = 2.14 (s, 3H), 3.79 (s, 2H), 7.08 (1H, s), 7.16 (1 H, s), 7.31 (1 H, s).
LRMS (thermospray): m/z [MH⁺] 263.

### EXAMPLE 78

### 3-Chloro-5-{[3-methyl-5-(1-piperazinylmethyl)-1H-pyrazol-4-yl]oxy}benzonitrile

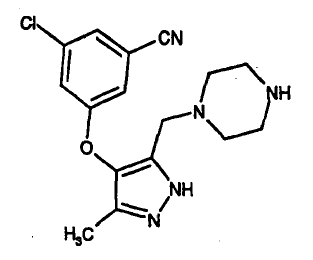

t-Butyl-1-piperazinecarboxylate (1.17g, 6.30mmol) was added in one portion to a stirred solution of the bromide of Preparation 18 (500mg, 1.40mmol) in isopropanol (20ml) at room temperature. The mixture was heated at 60°C for 1 hour, cooled to room temperature and concentrated under reduced pressure to leave a yellow oil. The oil was dissolved in dichloromethane (100ml) and the resulting solution was washed with 1 M aqueous sodium carbonate (20ml) and brine (20ml), dried over magnesium sulphate, filtered and concentrated under reduced pressure. The crude product was purified by flash column chromatography on silica gel eluting with dichloromethane:methanol:ammonia (95:4:1, by volume) to provide a yellow foam. The foam was dissolved in dichloromethane (10ml), the resulting solution was cooled to 0°C and trifluoroacetic acid (2ml) was added. The reaction was allowed to warm to room temperature and stirred for 24 hours. The mixture was diluted with dichloromethane (50ml), washed with 1 M aqueous sodium carbonate (20ml) and brine (20ml), dried over magnesium sulphate, filtered and concentrated under reduced pressure. The crude product was purified by flash column chromatography on silica gel eluting with dichloromethane:methanol:ammonia (90:9:1, by volume) to provide the title compound (400mg) as a white foam.
¹H-NMR (300MHz, CDCl₃): δ = 2.14 (s, 3H), 2.40 (m, 4H), 2.83 (m, 4H), 3.38 (s, 2H), 7.09 (s, 1 H), 7.16 (s, 1 H), 7.30 (s, 1H).
LRMS (thermospray): m/z [MH⁺] 332.

### EXAMPLE 79

### 3-Chloro-5-[(5-{[(4-cyanobenzyl)amino]methyl}-3-methyl-1H-pyrazol-4-yl)oxy]benzonitrile

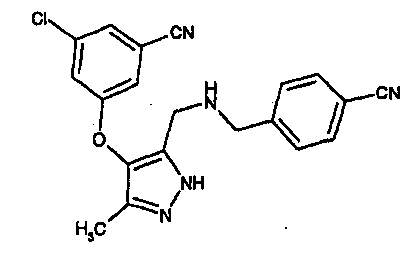

A mixture of 4-cyanobenzaldehyde (60mg, 0.460mmol), the amine of Example 77 (120mg, 0.460mmol), magnesium sulphate (500mg) and dichloromethane (5ml) was stirred under nitrogen at room temperature for 3 days. The mixture was concentrated under reduced pressure and the crude product was purified by flash column chromatography on silica gel eluting with methanol:ethyl acetate (5:95, by volume) to provide a foam. The foam was dissolved in methanol (5ml), sodium borohydride (50mg, 1.31mmol) was added in one portion at room temperature and the reaction was stirred for 30 minutes. The mixture was concentrated under reduced pressure and the residue was dissolved in dichloromethane (20ml). The resulting solution was washed with 1 M aqueous sodium carbonate solution (10ml) and brine (10ml), dried over magnesium sulphate, filtered and concentrated under reduced pressure. The crude product was purified by flash column chromatography on silica gel eluting with dichloromethane:methanol:ammonia (95:4:1, by volume) to provide the title compound (35mg) as a white foam.
¹H-NMR (300MHz, CDCl₃): δ = 2.15 (s, 3H), 3.69 (s, 2H), 3.84 (s, 2H), 7.06 (s, 1H), 7.15 (s, 1 H), 7.31 (s, 1 H), 7.38 (d, 2H), 7.60 (d, 2H).
LRMS (thermospray): m/z [MH⁺] 378.

### EXAMPLE 80

### 3-Chloro-5-[(3-methyl-5-{[4-(methylsulfonyl)-1-piperazinyl]methyl}-1H-pyrazol-4-yl)oxy]benzonitrile

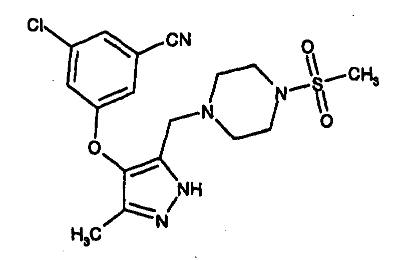

Methanesulphonyl chloride (19µl, 0.240mmol) was added dropwise to a stirred solution of the amine of Example 78 (80mg, 0.240mmol) and triethylamine (45µL, 0.288mmol) in dichloromethane (3ml) at room temperature under nitrogen. The reaction was stirred for 30 minutes and then concentrated under reduced pressure to leave a yellow oil. The crude product was purified by flash column chromatography on silica gel eluting with dichloromethane and then dichloromethane:methanol:ammonia (95:4:1, by volume) to provide the title compound (65mg) as a white foam.
¹H-NMR (400MHz, CDCl₃): δ = 2.14 (s, 3H), 2.51 (m, 4H), 2.72 (s, 3H), 3.12 (m, 4H), 3.39 (s, 2H), 7.08 (m, 1 H), 7.13 (m, 1 H), 7.26 (s, 1H).
LRMS (thermospray): m/z [MH⁺] 410.

### EXAMPLE 81

### 3-Chloro-5-[(5-{[4-(methoxyacetyl)-1-piperazinyl]methyl}-3-methyl-1H-pyrazol-4-yl)oxy]benzonitrile

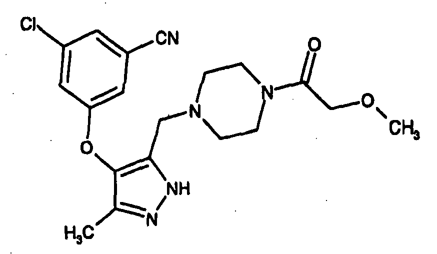

*N*-Benzyl-*N*'-cyclohexylcarbodiimide polymer bound (624mg of 1.3mmol/g, 0.480mmol) was added in one portion to a stirred solution of methoxyacetic acid (37µL, 0.480mmol) and the amine of Example 78 (80mg, 0.240mmol) in dichloromethane (5ml) at room temperature under nitrogen. The reaction was stirred for 1 hour and the polymer bound reagent was removed by filtration. The filtrate was concentrated under reduced pressure and the crude product was purified by flash column chromatography on silica gel eluting with dichloromethane:methanol:ammonia (95:4:1, by volume) to provide the title compound (45mg) as a white foam.
¹H-NMR (400MHz, CDCl₃): δ = 2.11 (s, 3H), 2.38 (m, 4H), 3.37 (m, 7H), 3.51 (m, 2H), 4.04 (s, 2H), 7.04 (m, 1 H), 7.10 (m, 1 H), 7.26 (m, 1H).
LRMS (thermospray): m/z [MH⁺] 404.

### EXAMPLE 82

### Methyl 4-{[4-(3-chloro-5-cyanophenoxy)-3-methyl-1H-pyrazol-5-yl]methyl}-1-piperazinecarboxylate

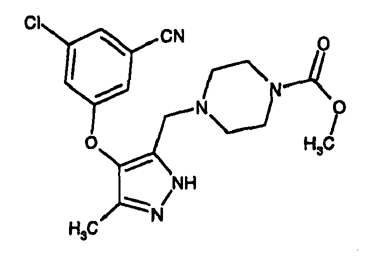

Methyl chloroformate (19µl, 0.240mmol) was added dropwise to a stirred solution of the amine of Example 78 (80mg, 0.240mmol) and triethylamine (45µl, 0.288mmol) in dichloromethane (5ml) at room temperature under nitrogen. The reaction was stirred for 90 minutes and then concentrated under reduced pressure to leave a yellow oil. The crude product was purified by flash column chromatography on silica gel eluting with dichloromethane and then dichloromethane:methanol:ammonia (95:4:1, by volume) to provide the title compound (55mg) as a white foam.
¹H-NMR (400MHz, CDCl₃): δ = 2.09 (s, 3H), 2.34 (m, 4H), 3.36 (m, 6H), 3.64 (s, 3H), 7.02 (m, 1 H), 7.10 (m, 1 H), 7.25 (m, 1H).
LRMS (thermospray): m/z [MH⁺] 390.

### EXAMPLE 83

### 4-[({[4-(3-Chloro-5-cyanophenoxy)-3-methyl-1H-pyrazol-5-yl]methyl}amino)methyl]benzenesulfonamide

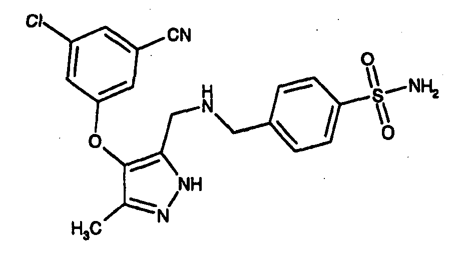

Triethylamine (125µl, 0.860mmol) was added in one portion to a stirred suspension of 4-aminomethylbenzenesulphonamide hydrochloride (144mg, 0.590mmol) and the bromide of Preparation 18 (100mg, 0.270mmol) in isopropanol (5ml) at room temperature under nitrogen. The reaction was heated at 70°C for 1 hour and then cooled to room temperature. The mixture was concentrated under reduced pressure and the crude product was purified by flash column chromatography on silica gel eluting with dichloromethane and then dichloromethane:methanol:ammonia (90:9:1, by volume) to provide a foam. The foam was further purified using a Phenomenex Luna C18 column eluting with diethylamine:methanol (0.1:99.1, by volume) to provide the title compound (8mg) as a white foam.
¹H-NMR (400MHz, CD₃OD): δ = 2.06 (s, 3H), 3.27 (s, 2H), 3.62 (s, 2H), 3.79 (s, 2H), 7.17 (s, 1 H), 7.21 (s, 1 H), 7.40 (m, 3H), 7.77 (d, 2H).
LRMS (thermospray): m/z [MH⁺] 432.

### EXAMPLE 84

### 4-(3,5-Dichlorophenoxy)-5-(methoxymethyl)-3-methyl-1H-gyrazole

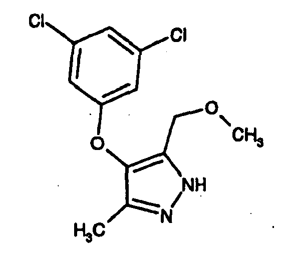

Tetrakis(triphenylphosphine)palladium (60mg) was added in one portion to a stirred solution of the bromide of Preparation 8 (590mg, 1.56mmol) in methanol (20ml) and tetrahydrofuran (20ml) at room temperature. The mixture was heated at 80°C under an atmosphere of carbon monoxide (690kPa, 100psi) for 18 hours. The reaction was cooled to room temperature and concentrated under reduced pressure to leave a brown oil. The oil was dissolved in dichloromethane (100ml) and the resulting solution was washed with water (50ml), dried over magnesium sulphate, filtered and evaporated under reduced pressure. The crude product was purified by flash column chromatography on silica gel eluting with ether to provide the title compound (110mg) as a colourless oil.
¹H-NMR (400MHz, CDCl₃): δ = 2.15 (s, 3H), 3.34 (s, 3H), 4.35 (s, 2H), 6.83 (s, 2H), 7.03 (s, 1 H).
LRMS (thermospray): m/z [MH⁺] 287.

### EXAMPLE 85

### 3-tert-Butyl-4-(3,5-dichlorophenoxy)-5-methyl-1H-pyrazole

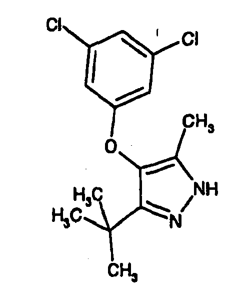

A mixture of the dione of Preparation 19 (1.00g, 5.68mmol), 3,5-dichlorophenol (930mg, 5.68mmol), cesium carbonate (1.85g, 5.68mmol) and acetone (20ml) was heated at reflux for 18 hours. After cooling the solid was removed by filtration and the filtrate was concentrated under reduced pressure. The intermediate was dissolved in ethanol (20ml), hydrazine hydrate (284mg, 5.68mmol) was added and the mixture was heated at 60°C for 1 hour. After cooling the mixture was concentrated under reduced pressure and the residue was purified by flash column chromatography on silica gel eluting with ethyl acetate:pentane (25:75, by volume) to provide the title compound (200mg) as a yellow oil.
¹H-NMR (400MHz, CDCl₃): δ = 1.30 (s, 9H), 2.06 (s, 3H), 6.81 (s, 2H), 7.02 (s, 1H).
LRMS (thermospray): m/z [MH⁺] 299.

### EXAMPLE 86

### 4-(3,5-Dichlorophenoxy)-3-ethyl-5-methyl-1H-pyrazole

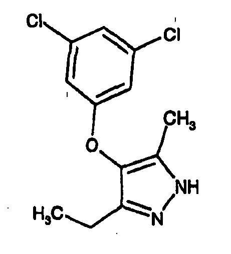

A mixture of the dione of Preparation 50 (4.50g, 30.8mmol), 3,5-dichlorophenol (5.00g, 30.8mmol), caesium carbonate (10.0g, 30.8mmol) and acetone (40ml) was heated at reflux for 18 hours. After cooling the solid was removed by filtration and the filtrate was concentrated under reduced pressure. The intermediate was dissolved in ethanol (40ml), hydrazine hydrate (1.00ml, 30.8mmol) was added and the mixture was heated at 60°C for 1 hour. After cooling the mixture was concentrated under reduced pressure and the residue was purified by flash column chromatography on silica gel eluting with ethyl acetate:pentane (20:80, by volume) to provide the title compound (1.50g) as an orange oil.
¹H-NMR (400MHz, CDCl₃): δ = 1.18 (t, 3H), 2.11 (s, 3H), 2.53 (q, 2H), 6.79 (s, 2H), 7.01 (s, 1H).
LRMS (thermospray): m/z [MH⁺] 271.

### EXAMPLE 87

### 4-Cyano-N-{[4-(3,5-dichlorophenoxy)-3-methyl-1H-pyrazol-5-yl]methyl}benzamide

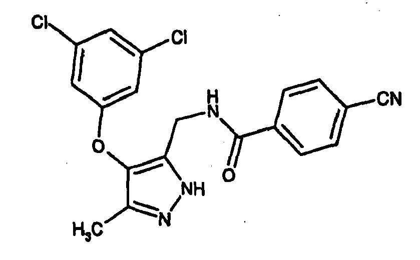

1-(3-(Dimethylamino)propyl)-3-ethylcarbodiimide (93mg, 0.490mmol) was added in one portion to a stirred solution of the amine of Example 109 (120mg, 0.440mmol) and 4-cyanobenzoic acid (71mg, 0.490mmol) in dichloromethane (5ml) at room temperature under nitrogen. The reaction was stirred for 20 minutes and then washed with 1M aqueous sodium hydroxide solution (10ml), 1M aqueous hydrochloric acid (10ml) and water (10ml). The organic layer was dried over magnesium sulphate, filtered and evaporated under reduced pressure to leave a yellow foam. The crude product was purified by flash column chromatography on silica gel eluting with dichloromethane:methanol:ammonia (95:4:1, by volume) to provide the title compound (110mg) as a white foam.
¹H-NMR (400MHz, CDCl₃): δ = 2.09 (s, 3H), 4.91 (d, 2H), 6.74 (s, 2H), 6.95 (s, 1 H), 6.98 (d, 1 H), 7.65 (d, 2H), 7.77 (d, 2H).
LRMS (thermospray): m/z [MNH₄⁺] 418.

### EXAMPLE 88

### 3-Cyano-N-{[4-(3,5-dichlorophenoxy)-3-methyl-1H-pyrazol-5-yl]methyl}benzamide

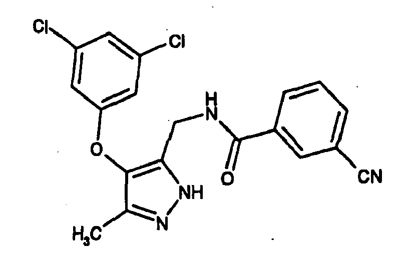

1-(3-(Dimethylamino)propyl)-3-ethylcarbodiimide (93mg, 0.490mmol) was added in one portion to a stirred solution of the amine of Example 109 (120mg, 0.440mmol) and 3-cyanobenzoic acid (71 mg, 0.490mmol) in dichloromethane (5ml) at room temperature under nitrogen. The reaction was stirred for 10 minutes and then washed with 1 M aqueous sodium hydroxide solution (10ml), 1 M aqueous hydrochloric acid (10ml) and brine (10ml). The organic layer was dried over magnesium sulphate, filtered and evaporated under reduced pressure to leave a cream foam. The crude product was purified by flash column chromatography on silica gel eluting with dichloromethane:methanol:ammonia (95:4:1, by volume) to provide the title compound (100mg) as a white foam.
¹H-NMR (400MHz, CDCl₃): δ = 2.14 (s, 3H), 4.53 (d, 2H), 6.78 (s, 2H), 6.98 (m, 2H), 7.54 (dd, 1 H), 7.76 (d, 1 H), 7.95 (d, 1 H), 7.99 (s, 1H).
LRMS (thermospray): m/z [MH⁺] 401.

### EXAMPLE 89

### N-{[4-(3,5-Dichlorophenoxy)-3-methyl-1H-pyrazol-5-yl]methyl}-N-(3-pyridinylmethyl)amine

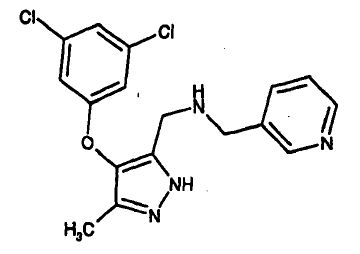

A mixture of 3-pyridinecarboxaldehyde (55mg, 0.514mmol), the amine of Example 109 (140mg, 0.514mmol), magnesium sulphate (500mg) and dichloromethane (5ml) was stirred under nitrogen at room temperature for 18 hours. Sodium triacetoxyborohydride (163mg, 0.771mmol) was added in one portion and then acetic acid (3 drops) was added. After 5 minutes the mixture was filtered. Tthe filtrate was washed with 1 M aqueous sodium carbonate solution (10ml), water (10ml) and brine (10ml), dried over magnesium sulphate, filtered and concentrated under reduced pressure to leave a clear oil. The crude product was purified by flash column chromatography on silica gel eluting with dichloromethane:methanol:ammonia (95:4:1, by volume) to provide the title compound (60mg) as a colourless oil.
¹H-NMR (400MHz, CDCl₃): δ = 2.09 (s, 3H), 3.66 (s, 2H), 3.74 (s, 2H), 6.75 (s, 2H), 6.97 (s, 1 H), 7.17 (m, 1 H), 7.55 (d, 1 H), 8.49 (m, 2H).
LRMS (electrospray): m/z [MH⁺] 363.

### EXAMPLE 90

### 3-({5-[(4-Acetyl-1-piperazinyl)methyl]-3-methyl-1H-pyrazol-4-yl}oxy)-5-chlorobenzonitrile

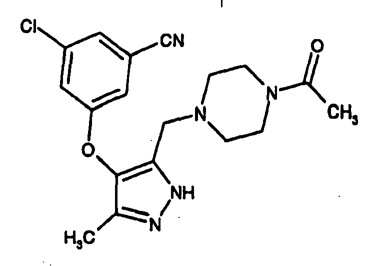

*N*-Acetylpiperazine (104mg, 0.810mmol) was added in one portion to a stirred solution of the bromide of Preparation 18 (100mg, 0.271 mmol) in isopropanol (5ml) at room temperature. The mixture was heated at 50°C for 1 hour, cooled to room temperature and concentrated under reduced pressure to leave a yellow oil. The crude product was purified by flash column chromatography on silica gel eluting with dichloromethane:methanol:ammonia (95:4:1, by volume) to provide the title compound (90mg) as a colourless oil.
¹H-NMR (300MHz, CDCl₃): δ = 2.08 (s, 3H), 2.16 (s, 3H), 2.43 (m, 4H), 3.42 (m, 4H), 3.55 (m, 2H), 7.08 (s, 1 H), 7.16 (s, 1 H), 7.31 (s, 1H).
LRMS (thermospray): m/z [MH⁺] 374.

### EXAMPLE 91

### 3-Chloro-5-[(5-{[(4-cyanobenzyl)(methyl)amino]methyl}-3-methyl-1H-pyrazol-4-yl)oxy]benzonitrile

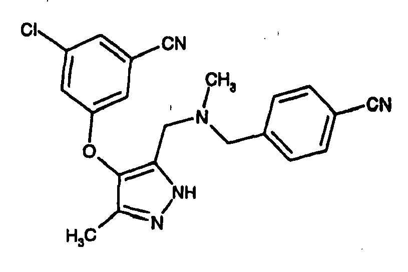

The amine of Preparation 20 (127mg, 0.870mmol) was added in one portion to a stirred solution of the bromide of Preparation 18 (100mg, 0.271 mmol) in isopropanol (5ml) at room temperature. The mixture was heated at 50°C for 12 hours, cooled to room temperature and concentrated under reduced pressure to leave a yellow oil. The oil was dissolved in 1 M hydrochloric acid and the aqueous solution was washed with ethyl acetate (10ml). Solid sodium carbonate was added until effervescence ceased and the mixture was extracted with ethyl acetate (3x20ml). The combined organic phases were dried over magnesium sulphate, filtered and concentrated under reduced pressure. The residue was purified by flash column chromatography on silica gel eluting with dichloromethane:methanol:ammonia (95:4:1, by volume) to provide the title compound (45mg) as a colourless oil.
¹H-NMR (300MHz, CDCl₃): δ = 2.14 (s, 3H), 2.17 (s, 3H), 3.45 (s, 2H), 3.55 (s, 2H), 7.05 (s, 1 H), 7.14 (s, 1 H), 7.31 (m, 3H), 7.59 (d, 2H).
LRMS (thermospray): m/z [MH⁺] 392.

### EXAMPLE 92

### 3-Chloro-5-[(5-{[(4-cyanobenzyl)(2-hydroxyethyl)amino]methyl}-3-methyl-1H-pyrazol-4-yl)oxy]benzonitrile

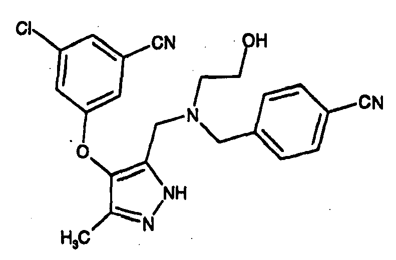

The amine of Preparation 21 (153mg, 0.870mmol) was added in one portion to a stirred solution of the bromide of Preparation 18 (100mg, 0.271 mmol) in isopropanol (5ml) at room temperature. The mixture was heated at 50°C for 12 hours, cooled to room temperature and concentrated under reduced pressure to leave a yellow oil. The oil was dissolved in 1 M aqueous sodium hydroxide solution and the resulting solution was stirred at room temperature for 1 hour. The aqueous was extracted with ethyl acetate (3x20ml) and the combined organic phases were dried over magnesium sulphate, filtered and concentrated under reduced pressure. The residue was purified by flash column chromatography on silica gel eluting with dichloromethane:methanol:ammonia (95:4:1, by volume) to provide the title compound (20mg) as a colourless oil.
¹H-NMR (300MHz, CDCl₃): δ = 2.14 (s, 3H), 2.71 (m, 2H), 3.50 (s, 1 H), 3.58 (s, 2H), 3.67 (m, 2H), 3.72 (s, 2H), 6.99 (s, 1 H), 7.09 (s, 1 H), 7.31 (s, 1 H), 7.41 (d, 2H), 7.58 (d, 2H).
LRMS (thermospray): m/z [MH⁺] 422.

### EXAMPLE 93

### 3-Chloro-5-({3-methyl-5-[(2-methyl-1H-imidazol-1-yl)methyl]-1H-pyrazol-4-yl}oxy)benzonitrile

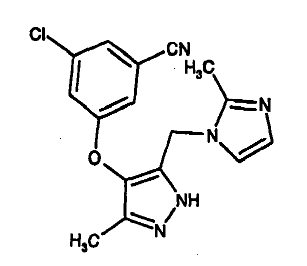

A suspension of the bromide of Preparation 18 (100mg, 0.264mmol), 2-methylimidazole (111mg, 1.35mmol) and sodium carbonate (143mg, 1.35mmol) in toluene (5ml) was heated at 100°C for 12 hours. The suspension was cooled to room temperature, 1 M aqueous sodium hydroxide solution (5ml) was added and the mixture was stirred for 1 hour. The mixture was extracted with ethyl acetate (3x20ml) and the combined organic phases were dried over magnesium sulphate, filtered and concentrated under reduced pressure to leave a white solid. The crude product was purified by flash column chromatography on silica gel eluting with dichloromethane:methanol:ammonia (95:4.5:0.5, by volume) to provide the title compound (77mg) as a white solid, m.p. 212-214°C.
¹H-NMR (300MHz, CDCl₃): δ = 2.14 (s, 3H), 2.33 (s, 3H), 4.92 (s, 2H), 6.76 (s, 1H), 6.79 (s, 1 H), 6.86 (s, 1 H), 7.27 (s, 2H).
LRMS (thermospray): m/z [MH⁺] 328.

### EXAMPLE 94

### 2-(4-(3,5-Dichlorophenoxy)-3-methyl-5-{[(3-pyridinylmethyl)amino]methyl}-1H-pyrazol-1-yl)ethanol

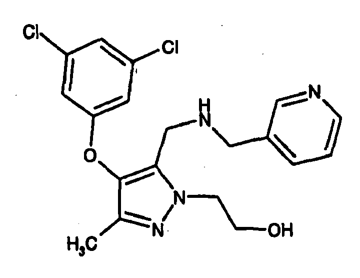

Tetrabutylammonium fluoride (0.58ml of a 1.0M solution in tetrahydrofuran, 0.580mmol) was added in one portion to a stirred solution of the amine of Preparation 22 (150mg, 0.290mmol) in dichloromethane (5ml) at room temperature. The reaction was stirred for 12 hours and concentrated under reduced pressure to leave a colourless oil. The crude product was purified by flash column chromatography on silica gel eluting with dichloromethane:methanol:ammonia (95:4:1, by volume) to provide the title compound (100mg) as a colourless oil.
¹H-NMR (400MHz, CDCl₃): δ = 2.07 (s, 3H), 3.65 (s, 2H), 3.76 (s, 2H), 3.96 (m, 2H), 4.24 (m, 2H), 6.76 (s, 2H), 7.02 (s, 1 H), 7.26 (m, 1 H), 7.59 (d, 1 H), 8.50 (m, 2H).
LRMS (thermospray): m/z [MH⁺] 407.

### EXAMPLE 95

### 5-[(3-Isopropyl-5-methyl-1H-pyrazol-4-yl)oxy]isophthalonitrile

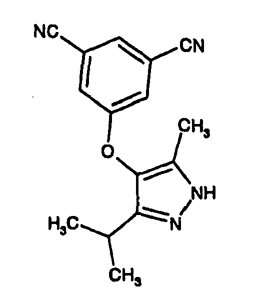

Hydrazine hydrate (110µl, 2.24mmol) was added to a stirred solution of the β-diketone of Preparation 24 (550mg, 2.04mmol) in glacial acetic acid (5ml) and the resulting solution was stirred at room temperature for 1 hour. The mixture was concentrated under reduced pressure and the residue was purified by flash column chromatography on silica gel eluting with pentane:ethyl acetate (60:40, by volume) to provide the title compound (350mg) as a yellow solid, m.p. 142-144°C.
¹H-NMR (300MHz, CDCl₃): δ = 1.21 (d, 6H), 2.09 (s, 3H), 2.90 (sept, 1 H), 7.40 (s, 2H), 7.60 (s, 1H).
LRMS (thermospray): m/z [MH⁺] 267.

### EXAMPLE 96

### 5-{[1-(2-Hydroxyethyl)-3-isopropyl-5-methyl-1H-pyrazol-4-yl]oxy}isophthalonitrile

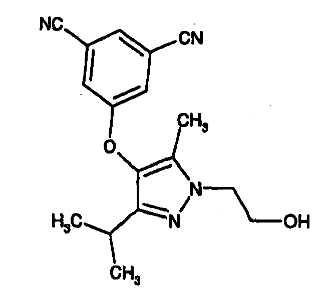

Tetrabutylammonium fluoride (0.28ml of a 1.0M solution in tetrahydrofuran, 0.280mmol) was added in one portion to a stirred solution of the pyrazole of Preparation 25 (60mg, 0.140mmol) in dichloromethane (5ml) at room temperature. The reaction was stirred for 12 hours and concentrated under reduced pressure to leave a colourless oil. The crude product was purified by flash column chromatography on silica gel eluting with pentane:ethyl acetate (20:80, by volume) to provide the title compound (30mg) as a white solid.
¹H-NMR (400MHz, CDCl₃): δ = 1.17 (d, 6H), 2.08 (s, 3H), 2.76 (sept, 1 H), 3.52 (m, 2H), 4.10 (m, 2H), 7.40 (s, 2H), 7.59 (s, 1 H).
LRMS (electrospray): m/z [MH⁺] 311.
Microanalysis: Found: C, 65.44; H, 5.87; N, 17.91. C₁₇H₁₈N₄O₂ requires C, 65.79; H, 5.85; N, 18.05%.

### EXAMPLE 97

### 3-(3,5-Dichlorophenoxy)-2-ethyl-6,7-dihydropyrazolo[1,5-a]pyrazin-4(5H)-one

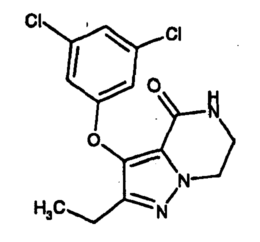

Lithium diisopropylamide (18.0ml of a 1.5M solution in cyclohexane, 27.0mmol) was added dropwise to a stirred solution of the pyrazole of Preparation 26 (12.3g, 24.6mmol) in tetrahydrofuran (120ml) at -78°C under nitrogen. The reaction was stirred for 14 hours, slowly warming to room temperature, and cautiously quenched with saturated aqueous ammonium chloride solution (20ml). The mixture was concentrated under reduced pressure and the residue was dissolved in dichloromethane (200ml). The resulting solution was washed with saturated aqueous ammonium chloride solution (100ml), dried over magnesium sulphate, filtered and concentrated under reduced pressure to leave a white solid. The solid was triturated with a mixture of dichloromethane and pentane (100ml and 100ml) to give the title compound (2.63g) as a white solid, m.p. 220-223°C.
¹H-NMR (400MHz, D₆ DMSO): δ = 1.08 (t, 3H), 2.44 (q, 2H), 3.60 (m, 2H), 4.24 (t, 2H), 7.00 (s, 2H), 7.26 (s, 1 H), 8.15 (s, 1H).
LRMS (thermospray): m/z [MNH₄⁺] 343.
Microanalysis: Found: C, 51.52; H, 3.98; N, 12.74. C₁₄H₃₁Cl₂N₃O₂ requires C, 51.55; H, 4.02; N, 12.88%.

### EXAMPLE 98

### 3-(3,5-Dichlorophenoxy)-2-ethyl-4,5,6,7-tetrahydropyrazolo[1,5-a]pyrazine

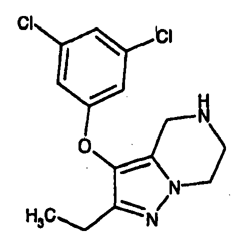

Borane (2.00ml of a 1.0M solution in tetrahydrofuran, 2.00mmol) was added to a stirred solution of the pyrazole of Example 97 (326mg, 1.00mmol) in tetrahydrofuran (10ml) at room temperature under nitrogen. The reaction was heated under reflux for 5 hours and further borane (3.00ml of a 1.0M solution in tetrahydrofuran, 3.00mmol) was added. The reaction was heated under reflux for 14 hours and further borane (2.00ml of a 1.0M solution in tetrahydrofuran, 2.00mmol) was added. The reaction was heated under reflux for 3 hours and further borane (2.00ml of a 1.0M solution in tetrahydrofuran, 2.00mmol) was added. The mixture was cooled to room temperature, 2M hydrochloric acid (10ml) was added and the mixture was heated under reflux for 1 hour. The mixture was cooled to room temperature and concentrated under reduced pressure. The residue was dissolved in dichloromethane (40ml), washed with 1 M aqueous potassium carbonate solution (30ml), dried over magnesium sulphate, filtered and concentrated under reduced pressure. The crude product was purified by flash column chromatography on silica gel eluting with dichloromethane:methanol (98:2, by volume), then dichloromethane:methanol (95:5, by volume) and then dichloromethane:methanol:ammonia (90:9:1, by volume) to provide the title compound (219mg) as a white solid, m.p. 76-77°C.
¹H-NMR (400MHz, CDCl₃): δ = 1.10 (t, 3H), 2.42 (q, 2H), 3.24 (t, 2H), 3.80 (s, 2H), 4.05 (t, 2H), 6.76 (s, 2H), 6.95 (s, 1H).
LRMS (thermospray): m/z [MH⁺] 312.
Microanalysis: Found: C, 53.79; H, 4.88; N, 13.14. C₁₄H₁₅Cl₂N₃O requires C, 53.86; H, 4.84; N, 13.46%.

### EXAMPLE 99

### 3-(3,5-Dichlorophenoxy)-2-ethyl-5-methyl-4,5,6,7-tetrahydropyrazolo[1,5-a]pyrazine

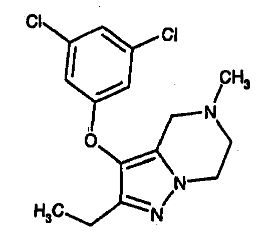

Methyl iodide (11µl, 0.176mmol) was added to a stirred solution of potassium carbonate (24mg, 0.176mmol) and the amine of Example 98 (50mg, 0.160mmol) in *N,N*-dimethylformamide (2ml) at room temperature under nitrogen. The reaction was stirred for 3 hours and then concentrated under reduced pressure. The residue was dissolved in ethyl acetate (20ml), washed with 1 M aqueous potassium carbonate solution (20ml), dried over magnesium sulphate, filtered and concentrated under reduced pressure. The crude product was purified by flash column chromatography on silica gel eluting with dichloromethane:methanol (98:2, by volume) to provide the title compound (18mg) as a colourless oil.
¹H-NMR (400MHz, CDCl₃): δ = 1.11 (t, 3H), 2.42 (m, 5H), 2.84 (t, 2H), 3.37 (s, 2H), 4.11 (t, 2H), 6.77 (s, 2H), 6.98 (s, 1 H).
LRMS (thermospray): m/z [MH⁺] 326.

### EXAMPLE 100

### 4-[(3-(3,5-Dichlorophenoxy)-2-ethyl-6,7-dihydropyrazolo[1,5-a]pyrazin-5(4H)-yl)methyl]benzonitrile

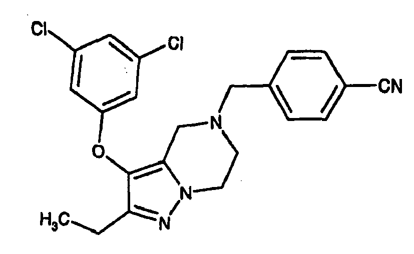

4-Cyanobenzylbromide (35mg, 0.176mmol) was added to a stirred solution of potassium carbonate (24mg, 0.176mmol) and the amine of Example 98 (50mg, 0.160mmol) in *N,N*-dimethylformamide (2ml) at room temperature under nitrogen. The reaction was stirred for 14 hours and then concentrated under reduced pressure. The residue was dissolved in ethyl acetate (20ml) and the resulting solution was washed with 1 M aqueous potassium carbonate solution (15ml), dried over magnesium sulphate, filtered and concentrated under reduced pressure. The crude product was purified by flash column chromatography on silica gel eluting with dichloromethane:methanol (98:2, by volume) to provide the title compound (66mg) as a white solid, m.p. 149-150°C.
¹H-NMR (400MHz, CDCl₃): δ = 1.13 (t, 3H), 2.44 (q, 2H), 2.92 (t, 2H), 3.42 (s, 2H), 3.71 (s, 2H), 4.13 (t, 2H), 6.74 (s, 2H), 6.97 (s, 1 H), 7.42 (d, 2H), 7.60 (d, 2H).
LRMS (thermospray): m/z [MH⁺] 427.

### EXAMPLE 101

### 3-(3,5-Dichlorophenoxy)-2-ethyl-5-(4-methoxybenzyl)-4,5,6,7-tetrahydropyrazolo[1,5-a]pyrazine

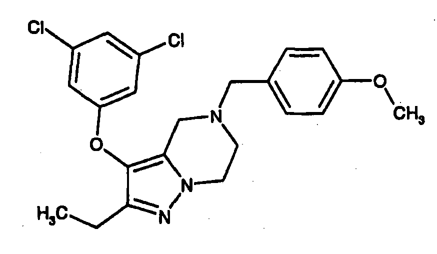

4-Methoxybenzylchloride (24µl, 0.176mmol) was added to a stirred solution of potassium carbonate (24mg, 0.176mmol) and the amine of Example 98 (50mg, 0.160mmol) in *N,N*-dimethylformamide (6ml) at room temperature under nitrogen. The reaction was stirred for 14 hours and then potassium carbonate (12mg, 0.088mmol) and 4-methoxybenzylchloride (12µl, 0.088mmol) added. The reaction was stirred for 3 hours and then concentrated under reduced pressure. The residue was dissolved in ethyl acetate (20ml) and the resulting solution was washed with 1 M aqueous potassium carbonate solution (20ml), dried over magnesium sulphate, filtered and concentrated under reduced pressure. The crude product was purified by flash column chromatography on silica gel eluting with dichloromethane:methanol (99:1, by volume) to provide the title compound (50mg) as a colourless oil.
¹H-NMR (400MHz, CDCl₃): δ = 1.13 (t, 3H), 2.45 (q, 2H), 2.92 (t, 2H), 3.44 (s, 2H), 3.60 (s, 2H), 3.80 (s, 3H), 4.10 (t, 2H), 6.77 (s, 2H), 6.85 (d, 2H), 7.00 (s, 1H), 7.23 (d, 2H).
LRMS (thermospray): m/z [MH⁺] 432.

### EXAMPLE 102

### [1-(2-Aminoethyl)-4-(3,5-dichlorophenoxy)-3-ethyl-1H-pyrazol-5-yl]methanol

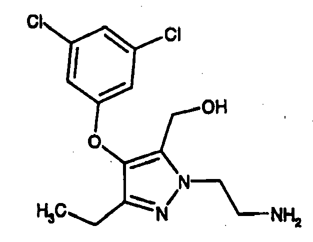

Hydrogen chloride (0.50ml of a 4.0M solution in dioxane, 2.00mmol) was added to a stirred solution of the pyrazole of Example 135 (86mg, 0.200mmol) in dioxane (0.5ml) at room temperature under nitrogen. The reaction was stirred for 24 hours and concentrated under reduced pressure. The residue was dissolved in dichloromethane (20ml) and the resulting solution was washed with 1 M aqueous potassium carbonate solution (10ml), dried over magnesium sulphate, filtered and concentrated under reduced pressure. The crude product was purified by flash chromatography on silica gel eluting with dichloromethane:methanol (99:1, by volume) to provide the title compound (40mg) as a white solid, m.p. 105-107°C.
¹H-NMR (400MHz, CDCl₃): δ = 1.10 (t, 3H), 2.42 (q, 2H), 2.55 (s, 2H), 3.13 (t, 2H), 4.13 (t, 2H), 4.37 (s, 2H), 6.79 (s, 2H), 6.98 (s, 1H).
LRMS (thermospray): m/z [MH⁺] 330.
Microanalysis: Found: C, 50.61; H, 5.23; N, 12.31. C₁₄H₁₇Cl₂N₃O₂ requires C, 50.92; H, 5.19; N, 12.73%.

### EXAMPLE 103

### 2-[4-(3,5-Dichlorophenoxy)-5-(ethoxymethyl)-3-ethyl-1H-pyrazol-1-yl]ethylamine

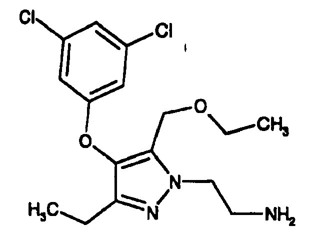

Hydrogen chloride (0.50ml of a 4.0M solution in dioxane, 2.00mmol) was added to a stirred solution of the pyrazole of Example 136 (60mg, 0.130mmol) in dioxane (0.5ml) at room temperature under nitrogen. The reaction was stirred for 2 days and concentrated under reduced pressure. The residue was dissolved in dichloromethane (20ml) and the resulting solution was washed with 1 M aqueous potassium carbonate solution (10ml), dried over magnesium sulphate, filtered and concentrated under reduced pressure. The crude product was purified by flash chromatography on silica gel eluting with dichloromethane:methanol:ammonia (99:9:1, by volume) to provide the title compound (32mg) as a colourless oil.
¹H-NMR (400MHz, CDCl₃): δ = 1.10 (m, 6H), 2.42 (q, 2H), 3.15 (t, 2H), 3.40 (q, 2H), 4.11 (t, 2H), 4.29 (s, 2H), 6.79 (s, 2H), 6.98 (s, 1H).
LRMS (thermospray): m/z [MH⁺] 358.

### EXAMPLES 104 TO 106

The compounds of the following tabulated Examples of the general formula: 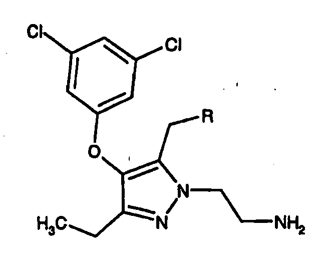 were prepared by a similar method to that of Example 103 using the appropriate starting material.

| Example No. (Starting material) | R | LRMS (thermospray) | Analytical Data |
|---|---|---|---|
| 104 (Example 140) | 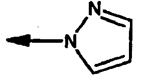 | m/z [MH⁺] 380. | ¹H-NMR (400MHz, CDCl₃): δ = 1.10 (t, 3H), 2.40 (q, 2H), 2.97 (t, 2H), 4.15 (t, 2H), 5.20 (s, 2H), 6.16 (s, 1H), 6.71 (d, 2H), 6.97 (s, 1H), 7.15 (s, 1H), 7.42 (s, 1H). |
| | | | Microanalysis: Found: C, 52.78; H, 5.09; N, 17.86. C₁₇H₁₉Cl₂N₅O.0.12CH₂Cl₂ requires C, 52.66; H, 4.97; N, 17.94%. |
| 105 (Example 142) | 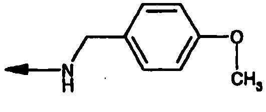 | m/z [MH⁺] 449. | ¹H-NMR (400MHz, CDCl₃): 8 = 1.10 (t, 3H), 2.42 (q, 2H), 3.11 (t, 2H), 3.55 (s, 2H), 3.60 (s, 2H), 3.75 (s, 3H), 4.07 (t, 2H), 6.73 (s, 2H), 6.79 (d, 2H), 6.97 (s, 1H), 7.10 (d, 2H). |
| | | | Microanalysis: Found: C, 56.88; H, 5.67; N, 11.88. C₂₂H₂₆Cl₂N₄O₂.0.23CH₂Cl₂ requires C, 56.94; H, 5.69; N, 11.95%. |
| 106 (Example 143) | 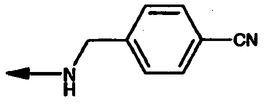 | m/z [MH⁺] 444. | ¹H-NMR (400MHz, CDCl₃): δ = 1.10 (t, 3H), 2.41 (q, 2H), 3.15 (t, 2H), 3.60 (s, 2H), 3.74 (s, 2H), 4.10 (d, 2H), 6.73 (s, 2H), 6.97 (s, 1H), 7.29 (d, 2H), 7.53 (d, 2H). |
| | | | Microanalysis: Found: C, 57.53; H, 5.09; N, 15.05. C₂₂H₂₃Cl₂N₅O.0.22CH₂Cl₂ requires C, 57.64; H, 5.10; N, 15.12%. - |

### EXAMPLE 107

### 2-[5-[(4-Acetyl-1-piperazinyl)methyl]-4-(3,5-dichlorophenoxy)-3-ethyl-1H-pyrazol-1-yl]ethylamine

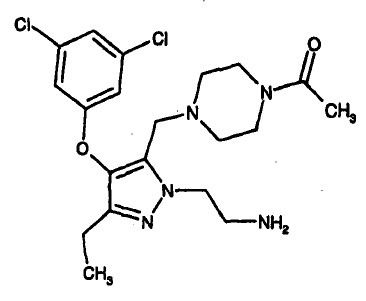

Trifluoroacetic acid (1ml) was added to a stirred solution of the pyrazole of Example 139 (150mg, 0.28mmol) in dichloromethane (2ml) at room temperature under nitrogen. The reaction was stirred for 3 hours and the mixture was concentrated under reduced pressure. The residue was dissolved in dichloromethane (20ml) and the resulting solution was washed with 1 M aqueous potassium carbonate solution (30ml), dried over magnesium sulphate, filtered and concentrated under reduced pressure. The crude product was purified by flash chromatography on silica gel eluting with dichloromethane:methanol:ammonia (90:9:1, by volume) to provide the title compound (103mg) as a colourless oil.
¹H-NMR (400MHz, CDCl₃): δ = 1.11 (t, 3H), 2.05 (s, 3H), 2.32 (m, 4H), 2.42 (q, 2H), 3.13 (m, 2H), 3.33 (s, 2H), 3.34 (m, 2H), 3.52 (m, 2H), 4.15 (t, 2H), 6.73 (s, 2H), 6.97 (s, 1 H).
LRMS (thermospray): m/z [MH⁺] 440.

### EXAMPLE 108

### N-[2-({[1-(2-Aminoethyl)-4-(3,5-dichlorophenoxy)-3-ethyl-1H-pyrazol-5-yl]methyl}amino)ethyl]acetamide

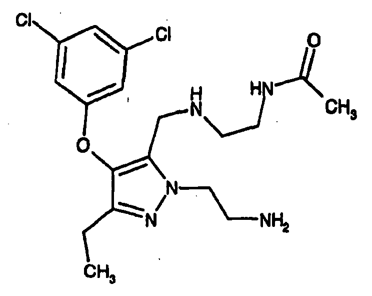

Trifluoroacetic acid (1ml) was added to a stirred solution of the pyrazole of Example 141 (122mg, 0.24mmol) in dichloromethane (2ml) at room temperature under nitrogen. The reaction was stirred for 3 hours and the mixture was concentrated under reduced pressure. The residue was dissolved in dichloromethane (50ml) and the resulting solution was washed with 1 M aqueous potassium carbonate solution (30ml), dried over magnesium sulphate, filtered and concentrated under reduced pressure. The crude product was purified by flash chromatography on silica gel eluting with dichloromethane:methanol:ammonia (90:9:1, by volume) to provide the title compound (64mg) as a colourless oil.
¹H-NMR (400MHz, CDCl₃): δ = 1.15 (t, 3H), 1.95 (s, 3H), 2.45 (q, 2H), 2.69 (t, 2H), 3.20 (t, 2H), 3.27 (m, 2H), 3.65 (s, 2H), 4.15 (t, 2H), 6.31 (s, 1 H), 6.81 (s, 2H), 7.02 (s, 1 H).
LRMS (thermospray): m/z [MH⁺] 414.

### EXAMPLE 109

### [4-(3,5-Dichlorophenoxy)-3-methyl-1H-pyrazol-5-yl]methanamine hydrobromide

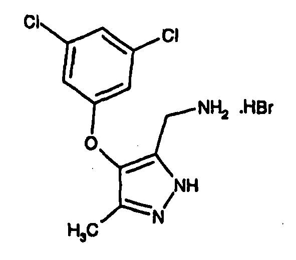

The bromide of Preparation 8 (500mg, 1.30mmol) was added portionwise to a saturated solution of ammonia in isopropanol (50ml) at 0°C. The reaction was stirred for 2 hours and allowed to slowly warm to room temperature. The mixture was concentrated under reduced pressure and the resulting solid was triturated with diethyl ether to provide the title compound (340mg) as a white solid.
¹H-NMR (400MHz, CDCl₃): δ = 2.38 (s, 3H), 4.78 (s, 2H), 6.88 (s, 2H), 7.19 (s, 1H).
LRMS (thermospray): m/z [MH⁺] 272.

### EXAMPLE 110

### N-{[4-(3,5-Dichlorophenoxy)-3-methyl-1H-pyrazol-5-yl]methyl}-N-(4-fluorobenzyl)amine

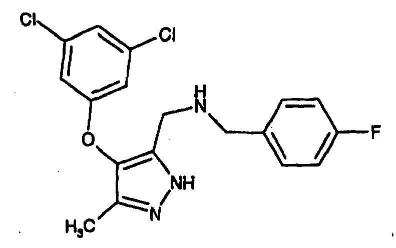

Sodium triacetoxyborohydride (36mg, 0.160mmol) was added in one portion to a stirred solution of the pyrazole of Example 109 (150mg, 0.400mmol), 4-fluorobenzaldehyde (11mg, 0.080mmol) and acetic acid (3 drops) in dichloromethane (15ml) at room temperature under nitrogen. The reaction was stirred for 3 hours and then concentrated under reduced pressure. The crude product was purified by flash chromatography on silica gel eluting with dichloromethane:methanol:ammonia (90:9:1, by volume) to provide the title compound (6mg) as a colourless oil.
¹H-NMR (300MHz, CDCl₃): δ = 2.17 (s, 3H), 3.67 (s, 2H), 3.73 (s, 2H), 6.81 (s, 2H), 6.99 (s, 2H), 7.02 (s, 1 H), 7.22 (s, 2H).
LRMS (electrospray): m/z [M-H⁺] 378.

### EXAMPLE 111

### 4-[({[4-(3,5-Dichlorophenoxy)-3-methyl-1H-pyrazol-5-yl]methyl}amino)methyl]benzonitrile

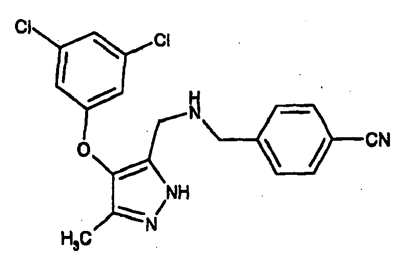

Sodium triacetoxyborohydride (216mg, 1.09mmol) was added in one portion to a stirred solution of the pyrazole of Example 109 (300mg, 0.850mmol), 4-cyanobenzaldehyde (111mg, 0.850mmol) and acetic acid (3 drops) in dichloromethane (25ml) at room temperature under nitrogen. The reaction was stirred for 14 hours and then washed with 1M aqueous sodium carbonate solution (2x10ml) and brine (10ml), dried over magnesium sulphate, filtered and concentrated under reduced pressure. The crude product was purified by flash chromatography on silica gel eluting with dichloromethane:methanol:ammonia (95:4:1, by volume) to provide the title compound (10mg) as a colourless oil.
¹H-NMR (300MHz, CDCl₃): δ = 2.16 (s, 3H), 3.70 (s, 2H), 3.85 (s, 2H), 6.78 (s, 2H), 7.01 (s, 2H), 7.35 (d, 2H), 7.58 (d, 2H).

LRMS (electrospray): m/z [MH⁺] 387.

### EXAMPLE 112

### 3-Chloro-5-[(1,3,5-trimethyl-1H-pyrazol-4-yl)oxy]benzonitrile

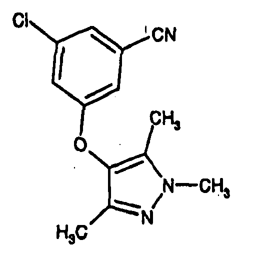

Methyl hydrazine (250mg, 5.17mol) was added to a stirred solution of the β-diketone of Preparation 16 (1.00g, 3.97mmol) in glacial acetic acid (10ml) and the resulting solution was stirred at room temperature for 2 days. The mixture was concentrated under reduced pressure and the resulting orange oil was purified by flash column chromatography on silica gel eluting with pentane:ethyl acetate (50:50, by volume) to provide the title compound (500mg) as a white solid, m.p. 114-116°C.
¹H-NMR (300MHz, CDCl₃): δ = 1.85 (s, 3H), 1.87 (s, 3H), 3.61 (s, 3H), 6.88 (s, 1H), 6.98 (s, 1 H), 7.11 (s, 1H).
LRMS (thermospray): m/z [MH⁺] 262.
Microanalysis: Found: C, 59.48; H, 4.60; N, 15.88. C₁₃H₁₂N₃OCl requires C, 59.66; H, 4.62; N, 16.06%.

### EXAMPLE 113

### 3-Chloro-5-[(5-{[(4-cyanobenzyl)amino]methyl}-1,3-dimethyl-1H-pyrazol-4-yl)oxy]benzonitrile

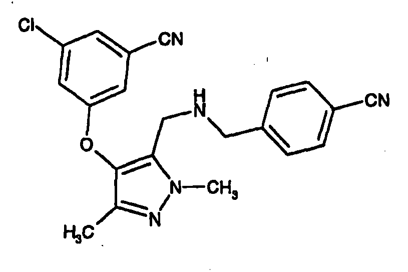

4-Cyanobenzylamine (155mg, 1.17mmol) was added in one portion to a stirred solution of the bromide of Example 144 (100mg, 0.300mmol) in isopropanol (10ml) at room temperature. The mixture was heated at 50°C for 1 hour, cooled to room temperature and concentrated under reduced pressure to leave a yellow oil. The crude product was purified by flash column chromatography on silica gel eluting with dichloromethane:methanol:ammonia (95:4:1, by volume) to provide the title compound (97mg) as a colourless oil.
¹H-NMR (300MHz, CDCl₃): δ = 2.03 (s, 3H), 3.66 (s, 2H), 3.79 (s, 2H), 3.84 (s, 3H), 7.02 (s, 1 H), 7.13 (s, 1 H), 7.31 (s, 1 H), 7.37 (d, 2H), 7.58 (d, 2H).
LRMS (thermospray): m/z [MH⁺] 392.

### EXAMPLE 114

### 3-Chloro-5-{[1-(2-hydroxyethyl)-3,5-dimethyl-1H-pyrazol-4-yl]oxy}benzonitrile

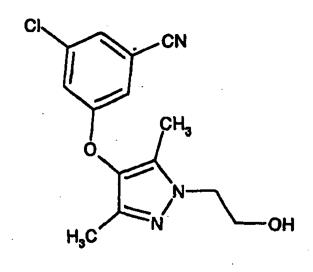

2-Hydroxyethyl hydrazine (1.80g, 24.0mol) was added to a stirred solution of the β-diketone of Preparation 16 (5.80g, 23.0mmol) in glacial acetic acid (30ml) and the resulting solution was stirred at room temperature for 2 days. The mixture was concentrated under reduced pressure and the resulting brown oil was purified by flash column chromatography on silica gel eluting with pentane:ethyl acetate (50:50, by volume) to provide the title compound (4.80g) as a yellow solid, m.p. 114-116°C.
¹H-NMR (300MHz, CDCl₃): δ = 2.04 (s, 3H), 2.12 (s, 3H), 3.24 (s, 1H), 4.08 (m, 4H), 7.03 (s, 1 H), 7.15 (s, 1 H), 7.28 (s, 1 H).
LRMS (thermospray): m/z [MH⁺] 292.
Microanalysis: Found: C, 57.40; H, 4.86; N, 14.14. C₁₄H₁₄N₃O₂Cl requires C, 57.69; H, 4.84; N, 14.40%.

### EXAMPLE 115

### 3-Chloro-5-{[5-{[4-cyanobenzyl)amino]methyl}-1-(2-hydroxyethyl)-3-methyl-1H-pyrazol-4-yl]oxy}benzonitrile

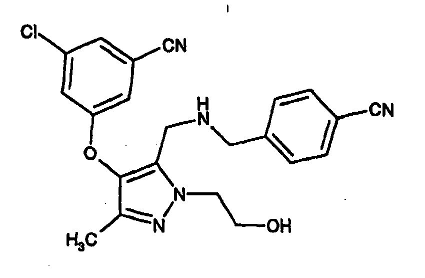

4-Cyanobenzylamine (131mg, 0.910mmol) was added to a stirred solution of the pyrazole of Preparation 30 (120mg, 0.240mmol) in *N*-methylpyrrolidine (10ml) and the resulting solution was heated at 60°C for 3 hours. The mixture was concentrated under reduced pressure and the resulting brown oil was dissolved in acetic acid (10ml) and heated at 40°C for 6 hours. The mixture was concentrated under reduced pressure and the crude product was purified by flash column chromatography on silica gel eluting with dichloromethane:methanol:ammonia (95:4:1, by volume) to provide the title compound (5mg) as a white solid.
¹H-NMR (300MHz, CDCl₃): δ = 2.05 (s, 3H), 3.04 (s, 2H), 3.91 (s, 2H), 3.99 (t, 2H), 4.32 (m, 2H), 7.06 (s, 1 H), 7.11 (s, 1 H), 7.33 (s, 1 H), 7.46 (d, 2H), 7.62 (d, 2H).
LRMS (thermospray): m/z [MNa⁺] 444.

### EXAMPLE 116

### 4-[({[4-(3-Chloro-5-cyanophenoxy)-3-methyl-1H-pyrazol-5-yl]methyl}amino)methyl]benzamide

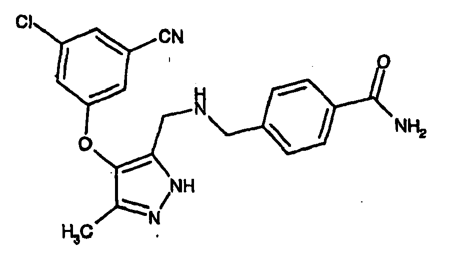

The amine of Preparation 55 (150mg, 0.800mmol) was added to a stirred solution of the pyrazole of Preparation 18 (100mg, 0.270mmol) and triethylamine (81mg, 0.800mmol) in isopropanol (10ml) and *N,N*-dimethylformamide (5ml) and the resulting solution was heated at 60°C for 3 hours. The mixture was concentrated under reduced pressure and the resulting brown oil was dissolved in ethyl acetate (20ml). The solution was washed with 1 M aqueous sodium carbonate solution (2x10ml) and brine (10ml), dried over magnesium sulphate, filtered and concentrated under reduced pressure. The crude product was purified by flash chromatography on silica gel eluting with dichloromethane:methanol:ammonia (90:9:1, by volume) to provide the title compound (5mg) as a colourless oil.
¹H-NMR (300MHz, CDCl₃): δ = 2.16 (s, 3H), 3.68 (s, 2H), 3.82 (s, 2H), 7.05 (s, 1 H), 7.13 (s, 1 H), 7.28 (s, 1 H), 7.32 (d, 2H), 7.76 (d, 2H).
LRMS (electrospray): m/z [MH⁺] 396.

### EXAMPLES 117 TO 120

The compounds of the following tabulated Examples of the general formula: 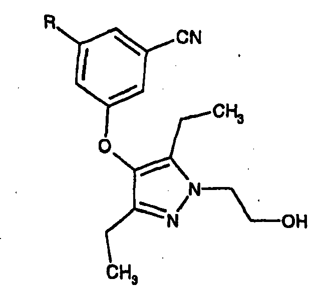 were prepared by a similar method to that of Example 114 using the appropriate diketone starting material and 2-hydroxyethylhydrazine.

| Example No. (Diketone No.) | R | LRMS | Analytical Data |
|---|---|---|---|
| 117 (Preparation 43) | F | m/z [MH⁺] 303. (thermospray) | ¹H-NMR (300MHz, CDCl₃): δ = 1.10 (m, 6H), 2.39 (q, 2H), 2.49 (q, 2H), 4.04 (m, 4H), 6.85 (dd, 1H), 6.99 (s, 1H), 7.00 (dd, 1H). |
| | | | Microanalysis: Found: C, 62.96; H, 5.94; N, 13.75. C₁₆H₁₈N₃O₂F requires C, 63.35; H, 5.98; N, 13.85%. |
| 118 (Preparation 44) | Me | m/z [MH⁺] 300. (electrospray) | ¹H-NMR (400MHz, CDCl₃): δ = 1.09 (t,3H), 1.12 (t, 3H), 2.34 (s, 3H), 2.39 (q, 2H), 2.50 (q, 2H), 3.70 (s, 1H), 4.60 (m, 4H), 6.91 (s, 1H), 6.97 (s, 1H), 7.10 (s, 1H). |
| 119 (Preparation 45) | CN | m/z [MH⁺] 311. (electrospray) | ¹H-NMR (400MHz, CDCl₃): δ = 1.13 (m, 6H), 2.40 (q, 2H), 2.53 (q, 2H), 3.53 (m, 1H), 4.11 (m, 4H), 7.40 (s, 2H), 7.58 (s, 1H). |
| | | | Microanalysis: Found: C, 65.64; H, 5.84; N, 18.05. C₁₇H₁₈N₄O₂ requires C, 65.79; H, 5.85; N, 18.05%. m.p. 120-121 °C. |
| 120 (Preparation 46) | Cl | m/z [MH⁺] 320. (thermospray) | ¹H-NMR (400MHz, CDCl₃): δ = 1.08 (m, 6H), 2.39 (q, 2H), 2.50 (q, 2H), 4.01 (m, 2H), 4.08 (m, 2H), 7.03 (s, 1H), 7.13 (s, 1H), 7.24 (s, 1H). |
| | | | Microanalysis: Found: C, 59.67; H, 5.71; N, 12.99. C₁₆H₁₈N₃O₂Cl requires C, 60.09; H, 5.67; N, 13.14%. |

### EXAMPLES 121 TO 124

The compounds of the following tabulated Examples of the general formula: 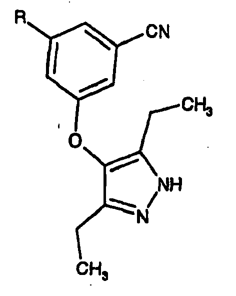 were prepared by a similar method to that of Example 76 using the appropriate diketone starting material and hydrazine.

| Example No. (Diketone No.) | R | LRMS | Analytical Data |
|---|---|---|---|
| 121 (Preparation 43) | F | m/z [MH⁺] 260. (thermospray) | ¹H-NMR (400MHz, CDCl₃): δ = 1.18 (t, 6H), 2.47 (q, 4H), 6.85 (dd, 1H), 6.98 (s, 1H), 7.01 (dd, 1H). |
| 122 (Preparation 45) | CN | m/z [MH⁺] 267. (thermospray) | ¹H-NMR (400MHz, CDCl₃): δ=1.20 (6H, m), 2.47 (q, 4H), 7.39 (s, 2H), 7.59 (s, 1H). |
| 123 (Preparation 44) | Me | m/z [MH⁺] 256. (electrospray) | ¹H-NMR (400MHz, CDCl₃): δ = 1.17 (t, 6H), 2.34 (s, 3H), 2.48 (q, 4H), 6.92 (s, 1H), 6.96 (s, 1H), 7.10 (s, 1H). |
| 124 (Preparation 46) | Cl | m/z [MH⁺] 276. (thermospray) | ¹H-NMR (400MHz, CDCl₃): δ = 1.18 (t, 6H), 2.49 (q, 4H), 7.07 (s, 1H), 7.13 (s, 1H), 7.27 (s, 1H). |

### EXAMPLES 125 TO 128

The compounds of the following tabulated Examples of the general formula: 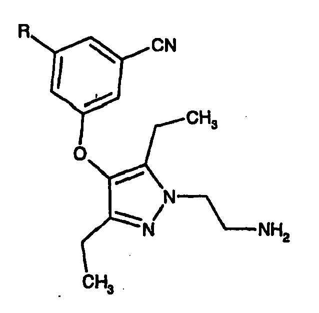 were prepared by a similar method to that of Example 13 using the appropriate pyrazole starting material and chloroethylamine hydrochloride.

| Example No. (Starting pyrazole No.) | R | LRMS | Analytical Data |
|---|---|---|---|
| 125 (Example 123) | Me | m/z [MH⁺] 299. (electrospray) | ¹H-NMR (400MHz, CDCl₃): δ = 1.10 (m, 6H), 2.34 (s, 3H), 2.39 (q, 2H), 2.43 (q, 2H), 3.17 (t, 2H), 4.04 (t, 2H), 6.91 (s, 1H), 6.96 (s, 1H), 7.09 (s, 1H). |
| 126 (Example 124) | Cl | m/z [MH⁺] 319. (thermospray) | ¹H-NMR (400MHz, CDCl₃): δ = 1.09 (m, 6H), 2.40 (q, 2H), 2.51 (q, 2H), 3.15 (m, 2H), 4.02 (m, 2H), 7.04 (s, 1H), 7.12 (s, 1H), 7.28 (s, 1H). |
| 127 (Example 122) | CN | m/z [MH⁺] 310. (thermospray) | ¹H-NMR (400MHz, CDCl₃): δ =1.09 (m, 6H), 2.38 (q, 2H), 2.50 (q, 2H), 3.15 (m, 2H), 4.03 (m, 2H), 7.39 (s, 2H), 7.57 (s, 1H). |
| 128 (Example 121) | F | m/z [MH⁺] 303. (thermospray) | ¹H-NMR (400MHz, CDCl₃): δ = 1.06 (m, 6H), 2.37 (q, 2H), 2.48 (q, 2H), 3.13 (t, 2H), 4.03 (t, 2H), 6.84 (d, 1H), 6.94 (s, 1H), 6.97 (d, 1H). |

### EXAMPLES 129 TO 131

The compounds of the following tabulated Examples of the general formula: 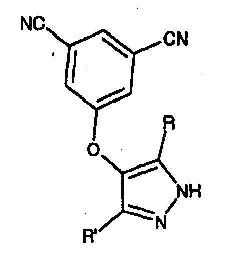 were prepared by a similar method to that of Example 76 using the appropriate diketone starting material and hydrazine.

| Example No. (Diketone No.) | R | R' | LRMS | Analytical Data |
|---|---|---|---|---|
| 129 (Preparation 52) | cycloPr | Et | m/z [MH⁺] 279. (electrospray) | ¹H-NMR (400MHz, CDCl₃): 0.73 (m, 2H), 0.81 (m, 2H), 1.16 (t, 3H), 1.58 (m, 1H), 2.46 (q, 2H), 7.42 (s, 2H), 7.58 (s, 1H). |
| | | | | m.p. 136-141 °C. |
| 130 (Preparation 53) | tBu | Me | m/z [MH⁺] 281. (electrospray) | ¹H-NMR (300MHz, CDCl₃): 1.21 (s, 9H), 1.94 (s, 3H), 7.34 (s, 2H), 7.56 (s, 1H). |
| | | | | Microanalysis: Found: C, 68.18; H, 5.74; N, 19.72. C₁₆H₁₆N₄O requires C, 68.55; H, 5.75; N, 19.99%. |
| | - | | | m.p. 61-63°C. |
| 131 (Preparation 54) | iPr | Et | m/z [MH⁺] 281. (electrospray) | ¹H-NMR (400MHz, CDCl₃): 1.15 (m, 9H), 2.41 (q, 2H), 2.82 (m, 1H), 7.36 (s, 2H), 7.58 (s, 1H). - |
| | - | | | m.p. 136-141 °C. |

### EXAMPLE 132

### 4-(3,5-Dichlorophenoxy)-3,5-diethyl-1-(1-methyl-3-azetidinyl)-1H-pyrazole

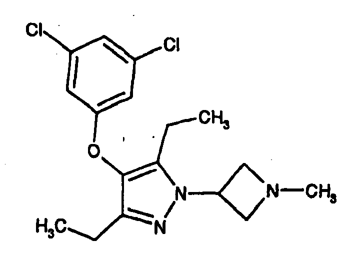

Paraformaldehyde (30mg, 0.330mmol) was added in one portion to a stirred solution of the pyrazole of Example 51 (120mg, 0.330mmol) in formic acid (2ml) at room temperature. The mixture was heated at 100°C for 5 hours, cooled to room temperature and concentrated under reduced pressure to leave a colourless oil. The oil was dissolved in ethyl acetate (50ml) and the resulting solution was washed with saturated aqueous sodium hydrogencarbonate (20ml), water (20ml) and brine (20ml), dried over magnesium sulphate, filtered and concentrated under reduced pressure. The crude product was purified by flash column chromatography on silica gel eluting with dichloromethane:methanol:ammonia (95:4:1, by volume) to provide the title compound (85mg) as a colourless oil.
¹H-NMR (300MHz, CDCl₃): δ = 1.08 (t, 3H), 1.16 (t, 3H), 2.49 (m, 7H), 3.63 (m, 2H), 3.81 (m, 2H), 4.79 (m, 1H), 6.79 (s, 2H), 7.00 (s, 1H).
LRMS (thermospray): m/z [MH⁺] 354.

### EXAMPLES 133-134

### 2-[4-(3,5-Dichlorophenoxy)-3-ethyl-1H-pyrazol-1-yl]ethylamine (Example 133) and 2-[4-(3,5-Dichlorophenoxy)-5-ethyl-1H-pyrazol-1-yl]ethylamine (Example 134)

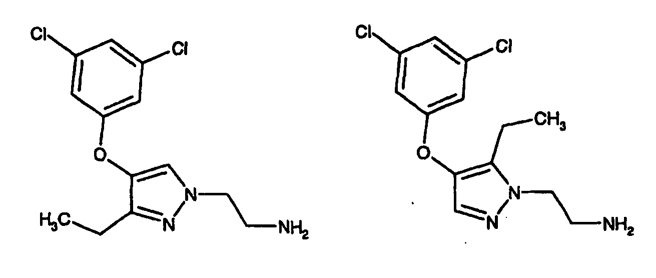

A mixture of the pyrazole (1.03g, 4.00mmol) of Example 42 and chloroethylamine hydrochloride (510mg, 4.40mmol) was stirred and heated at 150°C for 24 hours. After cooling the mixture was partitioned between 1M aqueous potassium carbonate solution (30ml) and dichloromethane (30ml). The organic layer was washed with brine (30ml), dried over magnesium sulphate, filtered and concentrated under reduced pressure. The resulting brown oil was purified by flash chromatography on silica gel eluting with dichloromethane:methanol:ammonia (93:6:1, by volume) to afford the title compounds (768mg) in a 85:15 ratio of regioisomers as a colourless oil.
¹H-NMR (400MHz, CDCl₃): δ = 1.16 (major, t, 3H), 1.16 (minor, t, 3H), 2.48 (major, q, 2H), 2.60 (minor, q, 2H), 3.13 (major, t, 2H), 3.19 (minor, t, 2H), 4.10 (major, t, 2H), 4.10 (minor, t, 2H), 6.85 (major, s, 2H), 6.85 (minor, s, 2H), 7.02 (major, s, 1 H), 7.02 (minor, s, 1H), 7.27 (major, s, 1H), 7.31 (minor, s, 1H).
LRMS (thermospray): m/z [MH⁺] 300.

### EXAMPLE 135

### tert-Butyl 2-[4-(3,5-dichlorophenoxy)-3-ethyl-5-(hydroxymethyl)-1H-pyrazol-1-yl]ethylcarbamate

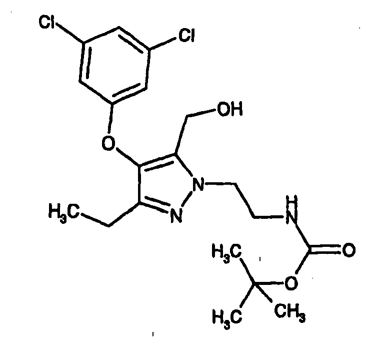

A solution of the pyrazole of Example 97 (1.96g, 6.00mmol) in concentrated hydrochloric acid (50ml) was heated under reflux for 20 hours. The reaction was cooled to room temperature and concentrated under reduced pressure. The residue was dissolved in dioxane (80ml) and water (60ml), di-t-butyldicarbonate (1.44g, 6.60mmol) and sodium hydrogencarbonate (1.26g, 15.0mmol) were added and the reaction was stirred at room temperature for 3 days. The reaction was concentrated under reduced pressure. A solution of the residue in dichloromethane (300ml) was washed with 2M aqueous hydrochloric acid (100ml), dried over magnesium sulphate, filtered and concentrated under reduced pressure. A solution of the crude product in tetrahydrofuran (50ml) was cooled to -40°C under nitrogen and triethylamine (0.79ml, 5.68mmol) and isopropylchloroformate (5.68ml of a 1.0M solution in toluene, 5.68mmol) were added dropwise. The reaction was stirred at - 40°C for 40 minutes and then warmed to 0°C. Sodium borohydride (537mg, 14.2mmol) was added in one portion and then water (3 drops) was added and the reaction was stirred at 0°C for 1 hour and at room temperature for 14 hours. The mixture was concentrated under reduced pressure and a solution of the residue in dichloromethane (100ml) was washed with water (100ml), dried over magnesium sulphate, filtered and concentrated under reduced pressure. The crude product was purified by flash chromatography on silica gel eluting with dichloromethane:methanol (97:3, by volume) to provide the title compound (1.37g) as a white foam.
¹H-NMR (400MHz, CDCl₃): δ = 1.10 (t, 3H), 1.37 (s, 9H), 2.40 (q, 2H), 3.00 (s, 1H), 3.56 (m, 2H), 4.20 (t, 2H), 4.48 (d, 2H), 5.00 (m, 1H), 6.80 (s, 2H), 6.97 (s, 1H).
LRMS (thermospray): m/z [MH⁺] 430.

### EXAMPLE 136

### tert-Butyl 2-[4-(3,5-dichlorophenoxy)-5-(ethoxymethyl)-3-ethyl-1H-pyrazol-1-yl]ethylcarbamate

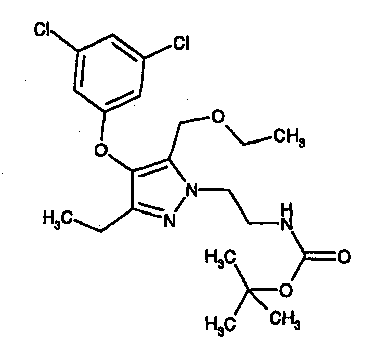

Silver(I)oxide (210mg, 0.900mmol) was added in one portion to a stirred solution of the alcohol of Example 135 (129mg, 0.300mmol) in ethyl iodide (1.75ml) at room temperature under nitrogen. The reaction was heated at 40°C for 1 day and then cooled to room temperature. The mixture was filtered and the residual solid was washed with dichloromethane (10ml). The filtrate was concentrated under reduced pressure. The crude product was purified by flash chromatography on silica gel eluting with dichloromethane:methanol (99:1, by volume) to provide the title compound (60mg) as a colourless oil.
¹H-NMR (400MHz, CDCl₃): δ=1.15 (m, 6H), 1.44 (s, 9H), 2.45 (q, 2H), 3.45 (q, 2H), 3.58 (m, 2H), 4.18 (m, 2H), 4.29 (s, 2H), 5.26 (m, 1 H), 6.92 (s, 2H), 7.00 (s, 1 H).
LRMS (electrospray): m/z [MNa⁺] 480.

### EXAMPLE 137

### tert-Butyl 2-[5-(bromomethyl)-4-(3,5-dichlorophenoxy)-3-ethyl-1H-pyrazol-1-yl]ethylcarbamate

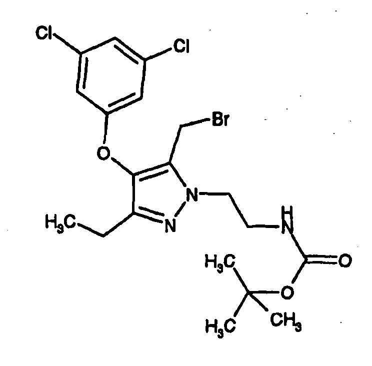

Bromine (160µl, 3.12mmol) was added dropwise to a stirred solution of triphenylphosphine (820mg, 3.12mmol) and imidazole (213mg, 3.12mmol) in dichloromethane (15ml) at room temperature under nitrogen. A solution of the alcohol of Example 135 (1.12g, 2.60mmol) in dichloromethane (5ml) was then added to the reaction. The reaction was stirred at room temperature for 2 hours, diluted with dichloromethane (50ml), washed with brine (20ml), dried over magnesium sulphate, filtered and concentrated under reduced pressure. The crude product was purified by flash chromatography on silica gel eluting with dichloromethane:methanol (98:2, by volume) to provide the title compound (969mg) as a white foam.
¹H-NMR (400MHz, CDCl₃): δ = 1.10 (t, 3H), 1.40 (s, 9H), 2.40 (q, 2H), 3.60 (m, 2H), 4.18 (t, 2H), 4.27 (s, 2H), 4.95 (s, 1 H), 6.82 (s, 2H), 7.00 (s, 1H).
LRMS (electrospray): m/z [MH⁺] 494.
Microanalysis: Found: C, 46.22; H, 4.89; N, 8.44. C₁₉H₂₄BrCl₂N₃O₃ requires C, 46.27; H, 4.90; N, 8.52%.

### EXAMPLE 138

### tert-Butyl 2-[5-(aminomethyl)-4-(3,5-dichlorophenoxy)-3-ethyl-1H-pyrazol-1-yl]ethylcarbamate

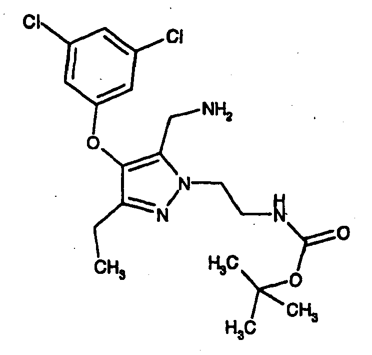

The bromide of Example 137 (444mg, 0.900mmol) was added to a saturated solution of ammonia in isopropanol (25ml) and diisopropylethylamine (173µl, 1.00mmol) at room temperature. The reaction was stirred for 5 hours and then concentrated under reduced pressure. The crude product was purified by flash chromatography on silica gel eluting with dichloromethane:methanol (95:5, by volume) to provide the title compound (359mg) as a white solid, m.p. 112-114°C.
¹H-NMR (400MHz, CDCl₃): δ = 1.11 (t, 3H), 1.40 (s, 9H), 2.40 (q, 2H), 3.55 (m, 2H), 3.73 (s, 2H), 4.18 (t, 2H), 5.60 (s, 1H), 6.77 (s, 2H), 6.98 (s, 1H).
LRMS (thermospray): m/z [MH⁺] 429.

### EXAMPLE 139

### tert-Butyl 2-[5-[(4-acetyl-1-piperazinyl)methyl]-4-(3,5-dichlorophenoxy)-3-ethyl-1H-pyrazol-1-yl]ethylcarbamate

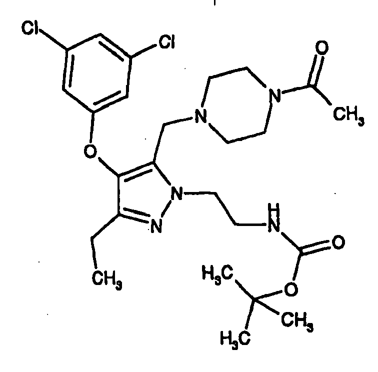

*N*-Acetylpiperazine (42mg, 0.330mmol) in *N,N*-dimethylformamide (1ml) was added to a stirred solution of the bromide of Example 137 (148mg, 0.300mmol) and diisopropylethylamine (57µL, 0.330mmol) in *N,N*-dimethylformamide (2ml) at room temperature. The reaction was stirred for 5 hours and the mixture was concentrated under reduced pressure. A solution of the residue in dichloromethane (30ml) was washed with 1 M aqueous potassium carbonate solution (10ml), dried over magnesium sulphate, filtered and concentrated under reduced pressure. The crude product was purified by flash chromatography on silica gel eluting with dichloromethane:methanol (98:2, by volume) to provide the title compound (150mg) as a colourless oil.
¹H-NMR (400MHz, CDCl₃): δ = 1.15 (t, 3H), 1.42 (s, 9H), 2.06 (s, 3H), 2.44 (m, 6H), 3.32 (s, 2H), 3.47 (m, 2H), 3.60 (m, 2H), 3.65 (m, 2H), 4.23 (m, 2H), 5.89 (s, 1 H), 6.76 (s, 2H), 7.02 (s, 1 H).
LRMS (thermospray): m/z [MH⁺] 540.

### EXAMPLE 140

### tert-Butyl 2-[4-(3,5-dichlorophenoxy)-3-ethyl-5-(1H-pyrazol-1-ylmethyl)-1H-pyrazol-1-yl]ethylcarbamate

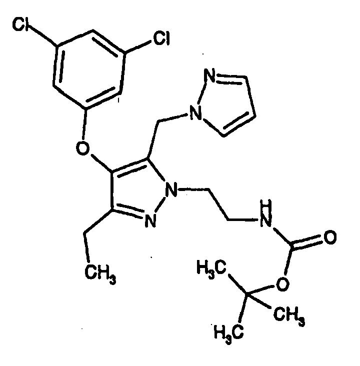

Pyrazole (23mg, 0.330mmol) was added in one portion to a stirred solution of the bromide of Example 137 (148mg, 0.300mmol) and sodium hydride (60% dispersion in oil, 13.2mg, 0.330mmol) in *N,N*-dimethylformamide (2ml) at room temperature under nitrogen. The reaction was stirred for 5 hours, quenched with water (1.00ml) and concentrated under reduced pressure. The residue was dissolved in dichloromethane (30ml) and the resulting solution was washed with 1 M aqueous potassium carbonate solution (10ml), dried over magnesium sulphate, filtered and concentrated under reduced pressure. The crude product was purified by flash chromatography on silica gel eluting with dichloromethane:methanol (98:2, by volume) to provide the title compound (125mg) as a colourless oil.
¹H-NMR (400MHz, CDCl₃): δ = 1.13 (t, 3H), 1.44 (s, 9H), 2.42 (q, 2H), 3.52 (m, 2H), 4.26 (t, 2H), 5.18 (s, 2H), 5.48 (s, 1 H), 6.16 (s, 1 H), 6.73 (s, 2H), 7.00 (s, 1 H), 7.18 (s, 1 H), 7.45 (s, 1 H).
LRMS (thermospray): m/z [MH⁺] 480.

### EXAMPLE 141

### tert-Bulyl 2-[5-({[2-(acetylamino)ethyl]amino}methyl)-4-(3,5-dichlorophenoxy)-3-ethyl-1H-pyrazol-1-yl]ethylcarbamate

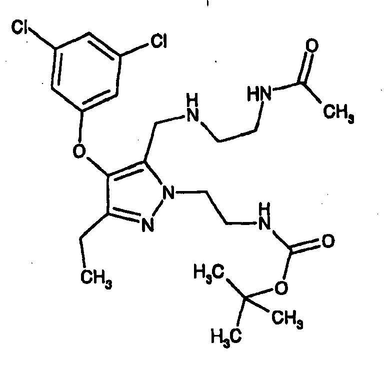

*N*-Acetylethylenediamine (153mg, 1.50mmol) in isopropanol (1ml) was added to a stirred solution of the bromide of Example 137 (148mg, 0.300mmol) and diisopropylethylamine (57µl, 0.330mmol) in isopropanol (2ml) at room temperature. The reaction was stirred for 5 hours and the mixture was concentrated under reduced pressure. A solution of the residue in dichloromethane (50ml) was washed with 1 M aqueous potassium carbonate solution (20ml), dried over magnesium sulphate, filtered and concentrated under reduced pressure. The crude product was purified by flash chromatography on silica gel eluting with dichloromethane:methanol (90:10, by volume) then dichloromethane:methanol:ammonia (90:9:1, by volume) to provide the title compound (122mg) as a colourless oil.
¹H-NMR (400MHz, CDCl₃): δ = 1.13 (t, 3H), 1.42 (s, 9H), 1.94 (d, 3H), 2.44 (q, 2H), 2.74 (m, 2H), 3.35 (m, 2H), 3.58 (m, 4H), 4.19 (m, 2H), 5.68 (s, 1 H), 6.77 (s, 2H), 7.00 (s, 1H), 7.65 (s, 1 H).
LRMS (thermospray): m/z [MH⁺] 514.

### EXAMPLE 142

### tert-Butyl 2-(4-(3,5-dichlorophenoxy)-3-ethyl-5-{[(4-methoxybenzyl)amino]methyl}-1 H-pyrazol-1-yl)ethylcarbamate

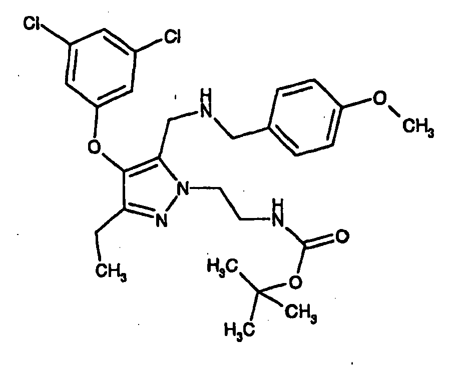

4-Methoxybenzaldehyde (46µl, 0.380mmol), the amine of Example 138 (172mg, 0.400mmol) and magnesium sulphate (200mg) were stirred in dichloromethane (4ml) at room temperature for 4 days. The mixture was filtered and the filtrate was concentrated under reduced pressure to leave a yellow oil. The oil was dissolved in methanol (4ml) and sodium borohydride (18mg, 0.480mmol) was added with vigorous stirring. Once the addition was complete the reaction was stirred for 4 hours and then water (2ml) was added. The mixture was concentrated under reduced pressure and the residue was dissolved in dichloromethane (50ml). The resulting solution was washed with 1 M aqueous potassium carbonate solution (20ml), dried over magnesium sulphate, filtered and concentrated under reduced pressure. The crude product was purified by flash chromatography on silica gel eluting with dichloromethane:methanol (99:1, by volume) and then dichloromethane:methanol (95:5, by volume) to provide the title compound (142mg) as a colourless oil.
¹H-NMR (400MHz, CDCl₃): δ = 1.10 (t, 3H), 1.40 (s, 9H), 2.42 (m, 2H), 3.55 (m, 5H), 3.66 (s, 2H), 3.77 (s, 2H), 4.15 (m, 2H), 6.11 (s, 1H), 6.74 (s, 2H), 6.80 (d, 2H), 7.00 (s, 1 H), 7.11 (d, 2H).
LRMS (thermospray): m/z [MH⁺] 549.

### EXAMPLE 143

### tert-Butyl 2-[5-{[(4-cyanobenzyl)amino]methyl}-4-(3,5-dichlorophenoxy)-3-ethyl-1H-pyrazol-1-yl]ethylcarbamate

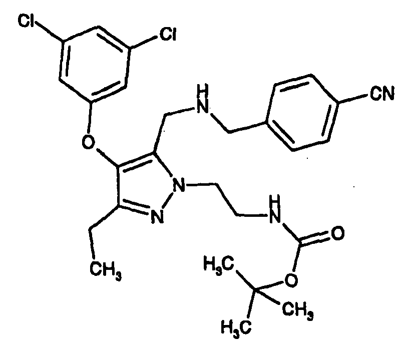

A mixture of 4-cyanobenzaldehyde (50mg, 0.380mmol), the amine of Example 138 (172mg, 0.400mmol), magnesium sulphate (200mg) and dichloromethane (4ml) was stirred at room temperature for 4 days. The mixture was filtered and the filtrate was concentrated under reduced pressure to leave a yellow oil. The oil was dissolved in methanol (4ml) and sodium borohydride (18mg, 0.480mmol) was added with vigorous stirring. Once the addition was complete the reaction was stirred for 4 hours and then water (2ml) was added. The mixture was concentrated under reduced pressure and the residue was dissolved in dichloromethane (50ml). The resulting solution was washed with 1 M aqueous potassium carbonate solution (20ml), dried over magnesium sulphate, filtered and concentrated under reduced pressure. The crude product was purified by flash chromatography on silica gel eluting with dichloromethane:methanol (99:1, by volume) then dichloromethane:methanol (95:5, by volume) to provide the title compound (120mg) as a colourless oil.
¹H-NMR (400MHz, CDCl₃): δ = 1.10 (t, 3H), 1.35 (s, 9H), 2.40 (q, 2H), 3.55 (m, 2H), 3.58 (s, 2H), 3.76 (s, 2H), 4.16 (m, 2H), 5.45 (s, 1H), 6.73 (s, 2H), 6.98 (s, 1 H), 7.32 (d, 2H), 7.55 (d, 2H).
LRMS (thermospray): m/z [MH⁺] 544.

### EXAMPLE 144

### 3-{[5-(Bromomethyl)-1,3-dimethyl-1H-pyrazol-4-yl]oxy}-5-chlorobenzonitrile

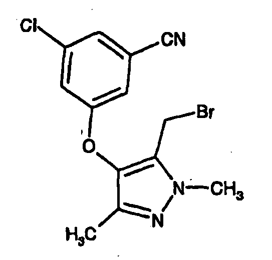

*N*-Bromosuccinimide (340mg, 1.90mmol) was added to a stirred solution of the pyrazole of Example 112 (500mg, 1.90mmol) in carbon tetrachloride (10ml) and azobisisobutyronitrile (20mg) at room temperature under nitrogen. The reaction was heated under reflux for 1 hour, cooled to room temperature and concentrated under reduced pressure. The crude product was purified by flash column chromatography on silica gel eluting with pentane:ethyl acetate (80:20, by volume) to provide the title compound (340mg) as a white solid, m.p. 76-78°C.
¹H-NMR (300MHz, CDCl₃): δ = 2.03 (s, 3H), 3.45 (s, 3H), 4.32 (s, 2H), 7.12 (s, 1 H), 7.19 (s, 1H), 7.34 (s, 1 H).
LRMS (thermospray): m/z [MH⁺] 342.

### EXAMPLE 145

### 3-[(3,5-Diethyl-1-methyl-1H-pyrazol-4-yl)oxy]benzonitrile

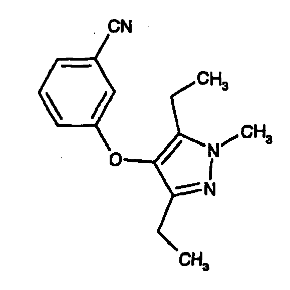

Sodium hydride (60% dispersion in oil, 22mg, 0.53mmol) was added to a solution of the pyrazole from Example 60 (100mg, 0.41mmol) and methyl iodide (34µl, 0.53mmol) in dimethylformamide (1.5ml) at 0°C under nitrogen. The reaction was allowed to warm to room temperature and was stirred for 4 hours. The reaction was quenched with water and the solvent was removed under reduced pressure. The residue was partitioned between ethyl acetate (20ml) and water (10ml) and the organic phase was washed with water (2x10ml), dried over magnesium sulphate and concentrated under reduced pressure. The residual oil was purified by flash chromatography on silica gel eluting with a solvent gradient of 100% pentane changing to 100% ethyl acetate and finally ethyl acetate:methanol (10:1, by volume) to provide the title compound (65mg) as a colourless oil.
¹H NMR (400MHz, CDCl₃): δ = 1.09 (t, 3H), 1.12 (t, 3H), 2.41 (q, 2H), 2.50 (q, 2H), 3.77 (s, 3H), 7.12-7.38 (m, 4H).
LRMS (electrospray) : m/z [MH⁺] 256, [MNa⁺] 278.
Microanalysis: Found C, 70.15; H, 6.78; N, 16.42. C₁₅H₁₅N₃O.0.08H₂O requires C, 70.17; H, 6.74; N, 16.37%.

### EXAMPLE 146

### 3-{[3,5-Diethyl-1-(2-methoxyethyl)-1H-pyrazol-4-yl]oxy}benzonitrile

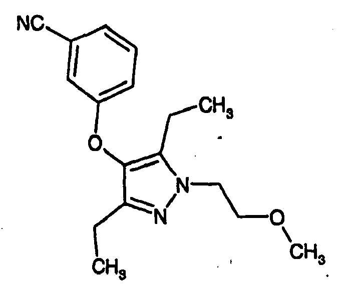

Sodium hydride (60% dispersion in oil, 22mg, 0.54mmol) was added to a solution of the pyrazole from Example 60 (100mg, 0.41mmol) and 1-bromo-2-mathoxy-ethane (51µl, 0.54mmol) in dimethylformamide (1.5ml) at 0°C under nitrogen. The reaction was allowed to warm to room temperature and was stirred for 4 hours. The reaction was quenched with water and the solvent was removed under reduced pressure. The residue was partitioned between ethyl acetate (20ml) and water (10ml) and the organic phase was washed with water (2x10ml), dried over magnesium sulphate and concentrated under reduced pressure. The residual oil was purified by flash chromatography on silica gel eluting with a solvent gradient of 100% pentane changing to 100% ethyl acetate and finally ethyl acetate:methanol (90:10, by volume) to provide the title compound (66mg) as a colourless oil.
¹H NMR (400MHz, CDCl₃): δ = 1.09 (t, 3H), 1.12 (t, 3H), 2.42 (q, 2H), 2.54 (q, 2H), 3.34 (s, 3H), 3.75 (t, 2H), 4.16 (t, 2H), 7.11-7.38 (m, 4H).
LRMS (electrospray): m/z [MH⁺] 300, [MNa⁺] 322.
Microanalysis: Found C, 68.21; H, 7.07; N, 14.04. C₁₇H₂₁N₃O₂ requires C, 67.85; H, 7.12; N, 14.09%.

### EXAMPLE 147

### 3-({5-[2-(Benzyloxy)ethyl]-3-ethyl-1H-pyrazol-4-yl}oxy)-5-fluorobenzonitrile

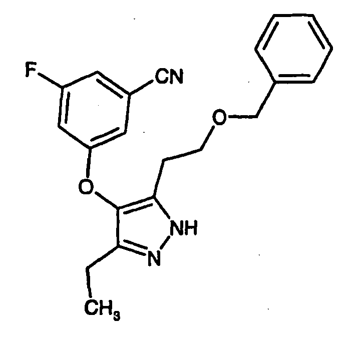

Hydrazine hydrate (390µl, 8.00mmol) was added to a solution of the enol from Preparation 60 (2.47g, 6.69mmol) in acetic acid (5ml) under nitrogen at room temperature. After stirring for 18 hours, the mixture was concentrated under reduced pressure and purified by flash chromatography on silica gel eluting with pentane:ethyl acetate (70:30 changing to 50:50, by volume) to provide the title compound (5.8g) as a yellow oil.
¹H NMR (400MHz, CDCl₃): δ = 1.13 (t, 3H), 2.41 (q, 2H), 2.67 (t, 2H), 3.62 (t, 2H), 4.48 (s, 2H), 6.79 (m, 1H), 6.98 (m, 2H), 7.24 (m, 5H).
LRMS (electrospray) : m/z [M-H⁺] 364.
Microanalysis: Found C, 66.96; H, 5.62; N, 11.25. C₂₁H₂₀N₃O₂F.0.60H₂O requires C, 67.04; H, 5.68; N, 11.17%.

### EXAMPLE 148

### 3-{[3-Ethyl-5-(2-hydroxyethyl)-1H-pyrazol-4-yl]oxy}-5-fluorobenzonitrile

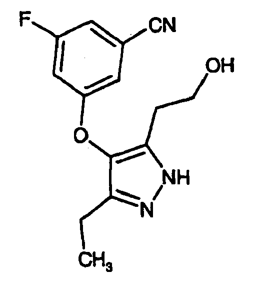

lron(III)chloride (9.30g, 57.5mmol) was added to a solution of the pyrazole from Example 147 (2.10g, 5.75mmol) in dichloromethane (90ml) under nitrogen at room temperature. After stirring for 20 minutes the mixture was diluted with dichloromethane (50ml), washed with water (100ml) then saturated aqueous sodium ethylenediaminetetraacetate solution (70ml), dried over magnesium sulphate and concentrated under reduced pressure. The residue was purified by flash chromatography on silica gel eluting with dichloromethane:methanol (98:2 changing to 95:5, by volume) to provide the title compound (1.2g) as a brown oil which solidified on standing.
¹H NMR (400MHz, CDCl₃): δ = 1.16 (t, 3H), 2.44 (q, 2H), 2.63 (t, 2H), 3.82 (t, 2H), 6.82 (m, 1H), 6.98 (m, 2H).
LRMS (electrospray) : m/z [MH⁺] 276.
Microanalysis: Found C, 60.69; H, 5.12; N, 15.08. C₁₄H₁₄N₃O₂F requires C, 61.08; H, 5.13; N, 15.26%.

### EXAMPLE 149

### 3-({5-[2-(4-Cyanophenoxy)ethyl]-3-ethyl-1H-pyrazol-4-yl}oxy)-5-fluorobenzonitrile

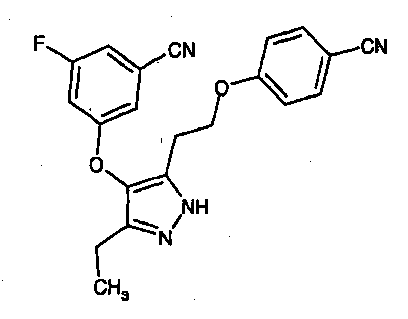

4-Hydroxy-benzonitrile (49mg, 0.41 mmol), triphenylphosphine (106mg, 0.41mmol) and diethylazodicarboxylate (65µl, 0.41 mmol) were added sequentially to a solution of the alcohol from Example 148 (74mg, 0.27mmol) in tetrahydrofuran (2ml) under nitrogen at 0°C. The reaction was allowed to warm to room temperature and was stirred for 18 hours. The mixture was concentrated under reduced pressure and purified by flash chromatography on silica gel eluting with toluene:ethyl acetate (75:25, by volume) to provide the title compound (50mg) as a yellow oil.
¹H NMR (400MHz, CDCl₃): δ = 1.18 (t, 3H), 2.49 (q, 2H), 2.98 (t, 2H), 4.21 (t, 2H), 6.82 (m, 3H), 6.99 (m, 2H), 7.56 (m, 2H).
LRMS (electrospray) : m/z [MH⁺] 377.

### EXAMPLES 150-152

The preparations of the following tabulated Examples of the general formula 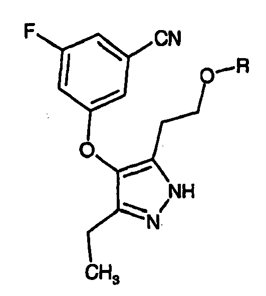 were performed by a similar method to that of Example 149 using the appropriate aryl alcohol as the starting material.

| Example No. | Starting Material Example No. | R | Analytical Data |
|---|---|---|---|
| 150¹ | 148 | 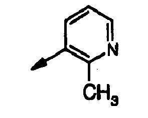 | ¹H NMR (400MHz, CDCl₃): δ = 1.18 (t, 3H), 2.42 (s, 3H), 2.52 (q, 2H), 2.99 (t, 2H), 4.18 (t, 2H), 6.83 (m, 1 H), 6.99 (m, 4H), 8.04 (m, 1 H). LRMS (thermospray) : m/z [MH⁺] 367. |
| 151¹ | 148 | 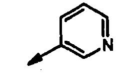 | ¹H NMR (400MHz, CDCl₃): δ = 1.19 (t, 3H), 2.50 (q, 2H), 2.98 (t, 2H), 4.22 (t, 2H), 6.85 (m, 1 H), 6.99 (m, 2H), 7.12 (m, 1 H), 7.18 (m, 1 H), 8.22 (m, 2H). LRMS (thermospray) : m/z [MH⁺] 353. |
| 152¹ | 148 | 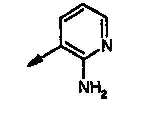 | ¹H NMR (400MHz, CDCl₃): δ = 1.20 (t, 3H), 2.53 (q, 2H), 2.98 (t, 2H), 4.19 (t, 2H), 4.85 (brs, 2H), 6.58 (m, 1 H), 6.83 (m, 2H), 6.99 (m, 2H), 7.63 (d, 1H). LRMS (thermospray) : m/z [MH⁺] 368. |

| | | | |
|---|---|---|---|
| ¹ These compounds were purified on silica gel eluting with a solvent gradient of cyclohexane:ethyl acetate (75:25 then 66:34 then 50:50, by volume) changing to ethyl acetate and finally ethyl acetate:methanol (90:10, by volume). | | | |

### EXAMPLE 153

### 5-({5-[2-(benzyloxy)ethyl]-3-ethyl-1H-pyrazol-4-yl}oxy)isophthalonitrile

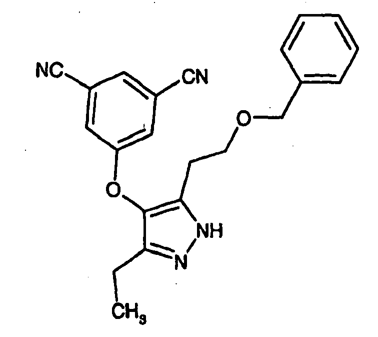

Hydrazine hydrate (177µl, 3.66mmol) was added to a solution of the crude enol from Preparation 61 (917mg, 2.40mmol) in acetic acid (10ml) under nitrogen at room temperature. After stirring for 18 hours, the mixture was concentrated under reduced pressure and purified by flash chromatography on silica gel eluting with pentane:cyclohexane (75:25, by volume) changing to toluene:ethyl acetate (50:50, by volume) to give the product which was further purified by preparative HPLC using a Develosil combi-rp C30 50x4.6mm 3µm column eluting with a solvent gradient of 5:95 0.1% aqueous trifluoroacetic acid in water:acetonitrile to provide the title compound (5mg) as a colourless oil.
¹H NMR (400MHz, CDCl₃): δ = 1.18 (t, 3H), 2.44 (q, 2H), 2.77 (t, 2H), 3.63 (t, 2H), 4.52 (s, 2H), 7.30 (m, 7H), 7.55 (s, 1H).
LRMS (electrospray) : m/z [MH⁺] 231, [MNa⁺] 253.

### EXAMPLE 154

### 5-{[3-Ethyl-5-(2-hydroxyethyl)-1H-pyrazol-4-yl]oxy}isophthalonitrile

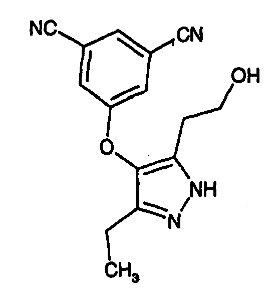

Iron(III)Chloride (217mg, 1.30mmol) was added to a solution of the pyrazole from Example 153 (50mg, 0.13mmol) in dichloromethane (5ml) under nitrogen at room temperature. After stirring for 30 minutes the mixture was diluted with dichloromethane (20ml), washed with water (100ml) then saturated aqueous sodium ethylenediaminetetraacetate solution (20ml), dried over magnesium sulphate and concentrated under reduced pressure. The residue was purified by flash chromatography on silica gel eluting with dichloromethane:methanol (98:2 changing to 95:5, by volume) to provide the title compound (20mg) as a white solid.
¹H NMR (400MHz, CDCl₃): δ = 1.19 (t, 3H), 2.51 (q, 2H), 2.69 (t, 2H), 3.88 (t, 2H), 7.40 (s, 2H), 7.59 (s, 1H).
LRMS (electrospray) : m/z [MH⁺] 283.

### EXAMPLE 155

### 3-{[5-(Aminomethyl)-1-(2-hydroxyethyl)-3-methyl-1H-pyrazol-4-yl]oxy}-5-chlorobenzonitrile

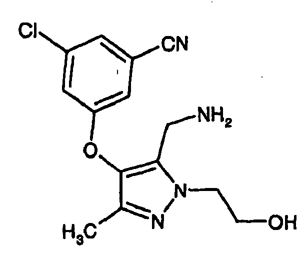

The protected alcohol from Preparation 31 (100mg, 0.23mmol) and tert-butylammonium fluoride (360µl of a 1 M solution in tetrahydrofuran, 0.36mmol) were stirred in dichloromethane (5ml) at room temperature under nitrogen for 3 hours. The reaction mixture was concentrated under reduced pressure and the residue was dissolved in methanol (2ml) and purified on a BondElut® SCX polymer supported sulphonic acid column washing with methanol (2x3ml) to remove impurities and 2N aqueous ammonia to remove the product. This procedure was repeated twice to provide the title compound (40mg) as a colourless oil.
¹H NMR (400MHz, CD₃OD): δ = 1.99 (s, 3H), 3.85 (t, 2H), 4.02 (s, 2H), 4.32 (t, 2H), 7.22 (s, 1 H), 7.28 (s, 1 H), 7.47 (s, 1 H).
LRMS (thermospray) : m/z [MH⁺] 309.
Microanalysis: Found C, 53.32; H, 5.17; N, 16.38. C₁₄H₁₅ClN₄O₂.0.85CH₃OH requires C, 53.40; H, 5.55; N, 16.77%.

### EXAMPLE 156

### 5-[(1-Allyl-3-tert-butyl-5-methyl-1H-pyrazol-4-yl)oxy]isophthalonitrile

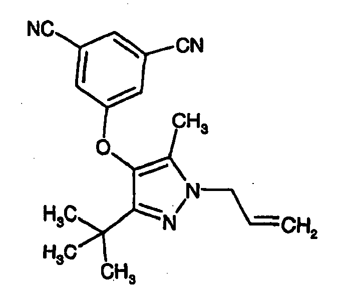

Sodium hydride (60% dispersion in oil, 120mg, 3.15mmol) was added to a solution of the pyrazole from Example 130 (800mg, 2.80mmol) and allyl bromide (345mg, 2.80mmol) in dimethylformamide (30ml) at room temperature under nitrogen and the reaction was stirred for 3 hours. The reaction was diluted with ethyl acetate (50ml), washed with water (2x50ml) then brine (50ml) and the organic phase was concentrated under reduced pressure. The residual oil was purified by flash chromatography on silica gel eluting with a solvent gradient of pentane changing to ethyl acetate:pentane (20:80, by volume) to provide the title compound (600mg) as a colourless oil.
¹H NMR (400MHz, CDCl₃): δ = 1.21 (s, 9H), 1.96 (s, 3H), 4.66 (s, 2H), 5.04 (d, 1 H), 5.24 (d, 1 H), 5.98 (m, 1 H), 7.37 (s, 2H), 7.57 (s, 1 H).
LRMS (thermospray) : m/z [MH⁺] 322.
Microanalysis: Found C, 70.79; H, 6.29; N, 17.11. C₁₉H₂₀N₄O.0.05CH₂Cl₂ requires C, 70.48; H, 6.24; N, 17.26%.

### EXAMPLE 157

### 5-{[3-tert-Bulyl-1-(2-hydroxyethyl)-5-methyl-1H-pyrazol-4-yl]oxy}isophthalonitrile

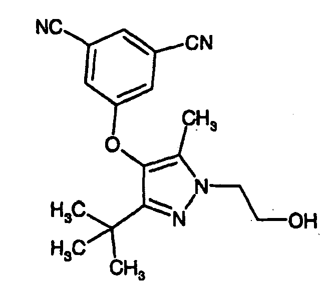

Sodium periodate (1.00g, 4.60mmol), osmium tetroxide (1.5% solution in tert-butanol, 190mg, 0.02mmol) and the pyrazole from Example 156 (600mg, 1.86mmo!) were dissolved in acetone (9ml) and water (3ml) under nitrogen at room temperature, and the reaction was stirred for 5 hours. The acetone was removed under reduced pressure and the residue was extracted with ethyl acetate (30ml). The organic phase was washed with water (2x30ml) then brine (30ml), dried over magnesium sulphate and concentrated under reduced pressure. The crude aldehyde was then dissolved in methanol (15ml) and sodium borohydride (84mg, 2.22mmol) was added portionwise at room temperature under nitrogen. The reaction was stirred for 3 hours and the solvent was removed under reduced pressure. The residue was partitioned between ethyl acetate (10ml) and water (10ml) and the organic phase was washed with water (2x10ml) then brine (10ml), dried over magnesium sulphate and concentrated under reduced pressure. The residual oil was purified by flash chromatography on silica gel eluting with a solvent gradient of pentane changing to ethyl acetate:pentane (50:50, by volume) to provide the title compound (250mg) as a colourless oil.
¹H NMR (400MHz, CDCl₃): δ =1.17 (s, 9H), 1.98 (s, 3H), 3.67 (s, 1 H), 4.04 (m, 4H), 7.35 (s, 2H), 7.54 (s, 1 H).
LRMS (thermospray) : m/z [MH⁺] 325.
Microanalysis: Found C, 64.30; H, 6.10; N, 16.35. C₁₈H₂₀N₄O₂.0.20CH₂Cl₂ requires C, 64.04; H, 6.02; N, 16.41%.

### EXAMPLE 158

### 5-{[1-(2-Aminoethyl)-3-tert-butyl-5-methyl-1H-pyrazol-4-yl]oxy}isophthalonitrile

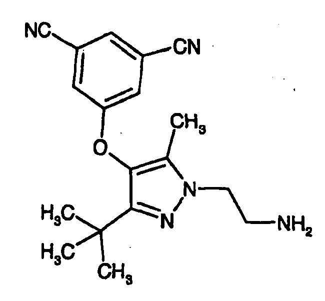

Diphenylphosphorylazide (305mg, 1.10mmol) was dissolved in tetrahydrofuran (5ml) and added to a solution of the pyrazole from Example 157 (180mg, 0.55mmol), triphenylphosphine (291 mg, 1.10mmol) and diethylazodicarboxylate (193mg, 1.10mmol) in tetrahydrofuran (20ml) under nitrogen at room temperature. The reaction was stirred for 18 hours then triphenylphosphine (291 mg, 1.10mmol) was added, and the reaction was stirred for a further 18 hours. Water (180µl, 10.0mmol) was then added and the reaction was stirred for 64 hours. The solvent was removed under reduced pressure and the residual white paste was purified by flash chromatography on silica gel eluting with dichloromethane:methanol:0.88 ammonia (95:4.5:0.5, by volume) to provide the title compound (55mg) as a colourless oil.
¹H NMR (300MHz, CDCl₃): δ = 1.22 (s, 9H), 1.78 (s, 2H), 2.03 (s, 3H), 3.18 (t, 2H), 4.05 (m, 2H), 7.38 (s, 2H), 7.58 (s, 1H).
LRMS (thermospray) : m/z [MH⁺] 324.
Microanalysis: Found C, 64.46; H, 6.48; N, 20.47. C₁₈H₂₁N₅O.0.20CH₂Cl₂ requires C, 64.22; H, 6.34; N, 20.57%.

### EXAMPLE 159

### 3-{[3,5-Diethyl-1-(2-hydroxyethyl)-1H-pyrazol-4-yl]oxy}-5-(1H-1,2,4-triazol-1-yl)benzonitrile

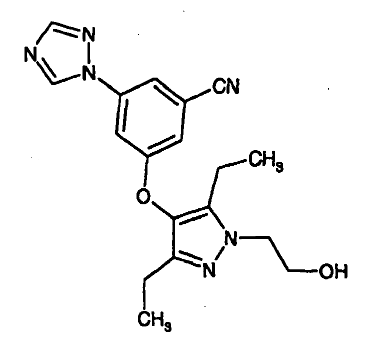

Cesium carbonate (179mg, 0.55mmol) was added to a solution of 1H[1,2,4]triazole (38mg, 0.55mmol) in dimethylsulfoxide (1ml) under nitrogen at room temperature and the reaction was stirred for 10 minutes. The aryl fluoride from Preparation 62 (210mg, 0.5mmol) dissolved in dimethylsulfoxide (1ml) was then added and the reaction was heated to 100°C for 18 hours. After cooling to room temperature the reaction was diluted with water (15ml) and extracted with ethyl acetate (25ml). The organic phase was washed with brine (15ml), dried over magnesium sulphate, concentrated under reduced pressure and the residue was purified by flash chromatography on silica gel eluting with a solvent gradient of dichloromethane:methanol (98:2 changing to 90:10, by volume) to provide the title compound (67.5mg) as a white solid, m.p. 122-124°C.
¹H NMR (400MHz, CDCl₃): δ = 1.10 (m, 6H), 2.39 (q, 2H), 2.51 (q, 2H), 3.61 (brs, 1 H), 4.04 (m, 2H), 4.07 (m, 2H), 7.10 (s, 1 H), 7.52 (s, 1H), 7.60 (s, 1 H), 8.07 (s, 1H), 8.54 (s, 1 H).
LRMS (thermospray) : m/z [MH⁺] 353.
Microanalysis: Found C, 60.69; H, 5.83; N, 22.98. C₁₈H₂₀N₆O₂.0.08CH₂Cl₂ requires C, 60.46; H, 5.66; N, 23.40%.

### EXAMPLES 160-162

The preparation of the following tabulated Examples of the general formula 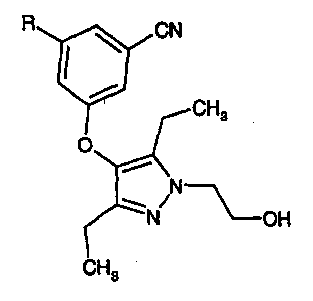 were performed by a similar method to that of Example 159 using the appropriate heterocycle as the starting material.

| Example No. (Starting Material Preparation No) | R | Analytical Data |
|---|---|---|
| 160 (62) | 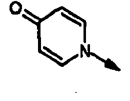 | ¹H NMR (400MHz, CDCl₃): δ = 1.10 (m, 6H), 2.39 (q, 2H), 2.52 (q, 2H), 3.62 (brs, 1 H), 4.02 (t, 2H), 4.08 (t, 2H), 6.44 (d, 2H), 7.14 (s, 1 H), 7.16 (s, 1 H), 7.25 (s, 1H), 7.49 (d, 2H). m.p. 169-170°C. LRMS (thermospray) : m/z [MH⁺] 379. Microanalysis: Found C, 65.68; H, 5.98; N, 14.31. C₂₁H₂₂N₄O₃.0.09CH₂Cl₂ requires C, 65.61; H, 5.79; N, 14.51%. |
| 161¹ (62) | 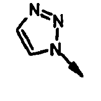 | ¹H NMR (400MHz, CDCl₃): δ = 1.10 (m, 6H), 2.40 (q, 2H), 2.51 (q, 2H), 3.56 (t, 1 H), 4.04 (m, 2H), 4.07 (m, 2H), 7.20 (s, 1 H), 7.65 (s, 2H), 7.85 (s, 1H), 7.98 (s, 1 H). LRMS (thermospray) : m/z [MH⁺] 353. HRMS: [MH⁺] 353.1722. C₁₈H₂₀N₆O₂ requires 353.1720. |
| 162¹ (62) | 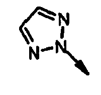 | ¹H NMR (400MHz, CDCl₃): δ = 1.10 (m, 6H), 2.41 (q, 2H), 2.51 (q, 2H), 3.62 (t, 1 H), 4.04 (m, 2H), 4.07 (m, 2H), 7.08 (s, 1 H), 7.80 (s, 2H), 7.87 (s, 1H), 8.02 (s, 1 H). LRMS (thermospray) : m/z [MH⁺] 353. HRMS: [MH⁺] 353.1719. C₁₈H₂₀N₆O₂ requires **353.1720.** |

| | | |
|---|---|---|
| ¹ Both of these compounds were isolated from a single reaction starting from 1,2,3-triazole with Example 161 being the most polar. | | |

### EXAMPLE 163

### 3-{[3,5-Diethyl-1-(2-hydroxyethyl)-1H-pyrazol-4-yl]oxy}-5-fluorobenzamide

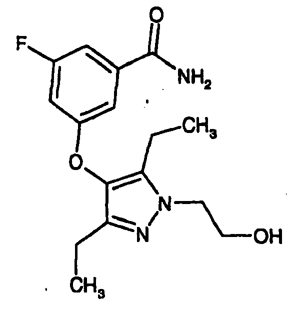

The protected alcohol from Preparation 64 (432mg, 1.07mmol) and p-toluene-sulphonic acid (30.3mg, 0.11mmol) were dissolved in methanol (4ml) and stirred under nitrogen at room temperature for 18 hours. The solvent was removed under reduced pressure and the residue was partitioned between saturated aqueous sodium bicarbonate solution (20ml) and dichloromethane (20ml). The aqueous phase was extracted with dichloromethane (10ml) and the combined organic extracts were dried over magnesium sulphate, concentrated under reduced pressure and the residue was purified by flash chromatography on silica gel eluting with a solvent gradient of dichloromethane:methanol (100:0 changing to 93:7, by volume) to provide the title compound (241 mg) as a white foam.
¹H NMR (400MHz, CDCl₃): δ = 1.10 (m, 6H), 2.39 (q, 2H), 2.49 (q, 2H), 3.68 (brs, 1 H), 4.04 (m, 4H), 5.59 (brs, 1 H), 5.88 (brs, 1 H), 6.71 (d, 1 H), 7.11 (m, 2H).
LRMS (thermospray) : m/z [MH⁺] 322.
Microanalysis: Found C, 57.91; H, 6.32; N, 12.56. C₁₆H₂₀FN₃O₃.0.13CH₂Cl₂.0.12H₂O requires C, 57.91; H, 6.18; N, 12.56%.

### EXAMPLES 164-167

### The preparation of the following tabulated Examples of the general formula

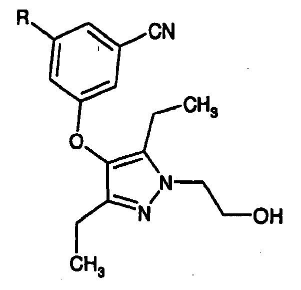

were performed by a similar method to that of Example 163 using the appropriate protected alcohol as the starting material.

| Example No. (Starting Material Preparation No.) | R | Analytical Data |
|---|---|---|
| 164¹ (65) | 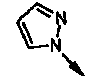 | ¹H NMR (400MHz, CDCl₃): δ = 1.13 (m, 6H), 2.44 (q, 2H), 2.54 (q, 2H), 3.65 (brs, 1 H), 4.07 (t, 2H), 4.11 (t, 2H), 6.51 (s, 1 H), 7.00 (s, 1 H), 7.56 (s, 1 H), 7.63 (s, 1 H), 7.74 (s, 1 H), 7.90 (s, 1H). LRMS (electrospray) : m/z [MH⁺] 352, [MNa⁺] 374. HRMS: [MH⁺] Found 352.1770. C₁₉H₂₂N₅O₂ requires 352.1768. |
| 165¹ (66) | 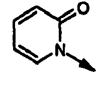 | ¹H NMR (400MHz, CDCl₃): δ = 1.10 (m, 6H), 2.40(q, 2H), 2.50 (q, 2H), , 4.00 (t, 2H), 4.06 (t, 2H), 6.24 (t, 1H), 6.60 (d, 1H), 7.18 (d, 2H), 7.24 (d, 1 H), 7.30 (s, 1H), 7.38 (t, 1H). LRMS (electrospray) : m/z [MH⁺] 379, [MNa⁺] 401. HRMS: [MH⁺] Found 379.1766. C₂₁H₂₃N₄O₃ requires 379.1765 [MNa⁺] Found 401.1585. C₂₁H₂₂N₄O₃Na requires 401.1584. |
| 166¹ (67) | 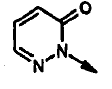 | ¹H NMR (400MHz, CDCl₃): δ = 1.10 (m, 6H), 2.41 (q, 2H), 2.51 (q, 2H), 4.01 (t, 2H), 4.06 (t, 2H), 7.07 (d, 1 H), 7.13 (s, 1 H), 7.22 (m, 1 H), 7.52 (s, 1 H), 7.65 (s, 1H), 7.88 (s, 1 H). LRMS (electrospray) : m/z [MH⁺] 380, [MNa⁺] 402. HRMS: [MH⁺] Found 380.1722. C₂₀H₂₂N₅O₃ requires 380.1717. |
| 167² (68) | 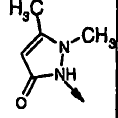 | ¹H NMR (400MHz, CDCl₃): δ = 1.11 (m, 6H), 2.27 (s, 3H), 2.41 (q, 2H), 2.50 (q, 2H), 3.70 (s, 3H), 4.04 (t, 1 H), 4.08 (t, 2H), 5.64 (s, 1 H), 6.81 (s, 1 H), 6.91 (s, 1 H), 6.99 (s, 1 H). LRMS (electrospray) : m/z [MH⁺] 396, [MNa⁺] 418. HRMS: [MH⁺] Found 396.2027. C₂₁H₂₆N₅O₃ requires 396.2030. |

| | | |
|---|---|---|
| ¹ The eluent used for flash column chromatography purification of these compounds was dichloromethane:methanol (99:1 changing to 80:20, by volume). ² The eluent used for flash column chromatography purification of this compound was dichloromethane:methanol (99:1 changing to 98:2, by volume). | | |

### EXAMPLE 168

### 5-{[3-Cyclopropyl-5-ethyl-1-(2-hydroxyethyl)-1H-pyrazol-4-yl]oxy}isophthalonitrile

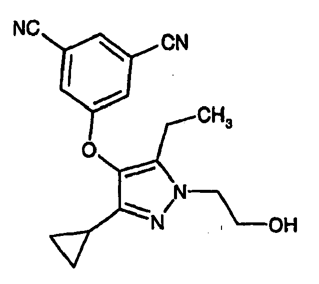

and

### EXAMPLE 169

### 5-{[5-Cyclopropyl-3-ethyl-1-(2-hydroxyethyl)-1H-pyrazol-4-yl]oxy}isophthalonitrile

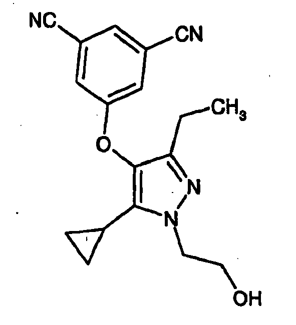

Potassium carbonate (91 mg, 0.66mmol) and 2-(2-bromoethoxy)-tetrahydropyran (91µl, 0.61 mmol) were sequentially added to a solution of the pyrazole from Example 129 (152mg, 0.55mmol) dissolved in dimethylformamide (4ml) and the reaction was heated to 35°C under nitrogen for 5 hours. Starting material still remained, so the temperature was increased to 80°C and the reaction was stirred for a further 18 hours. The reaction was cooled to room temperature, sodium hydride (60% dispersion in oil, 24mg, 0.60mmol) was added and the reaction was stirred at room temperature for 1hour. The mixture was diluted with water (50ml) and extracted with ethyl acetate (2x50ml). The combined organic extracts were washed with brine (30ml), dried over magnesium sulphate, concentrated under reduced pressure and the residue was purified by flash chromatography on silica gel eluting with pentane:cyclohexane (75:25, by volume) to provide a mixture of regioisomers (239mg). The regioisomers (239mg, 0.55mmol) and p-toluenesulphonic acid (10mg, 0.05mmol) were dissolved in methanol (5ml) and stirred under nitrogen at room temperature for 18 hours. The solvent was removed under reduced pressure and the residue was partitioned between saturated aqueous sodium bicarbonate solution (20ml) and dichloromethane (30ml). The organic phase was dried over magnesium sulphate, concentrated under reduced pressure and the residual oil was purified by flash chromatography on silica gel eluting with toluene:ethyl acetate (50:50, by volume) to yield two products as colourless oils.
Least Polar Fraction (Example 168) - 34mg
¹H NMR (400MHz, CDCl₃): δ = 0.76 (m, 4H), 1.05 (t, 3H), 1.45 (m, 1H), 2.48 (q, 2H), 3.39 (brs, 1 H), 4.02 (m, 4H), 7.39 (s, 2H), 7.56 (s, 1 H).
LRMS (electrospray) : m/z [M-H⁺] 321.
Most Polar Fraction (Example 169) - 9mg
¹H NMR (400MHz, CDCl₃): δ = 0.62 (m, 2H), 0.78 (m, 2H), 1.18 (t, 3H), 1.46 (m, 1H), 2.38 (q, 2H), 3.42 (brs, 1 H), 4.02 (m, 2H), 4.21 (t, 2H), 7.38 (s, 2H), 7.57 (s, 1H).
LRMS (electrospray) : m/z [MH⁺] 323, [MH⁻] 321.

### EXAMPLE 170

### 5-{[5-Ethyl-1-(2-hydroxyethyl)-3-isooropyl-1H-pyrazol-4-yl]oxy}isophthalonitrile

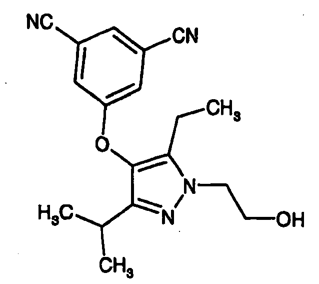

2-(2-Bromoethoxy)-tetrahydropyran (91µl, 0.60mmol) was added to a solution of the pyrazole from Example 131 (153mg, 0.55mmol) dissolved in dimethylformamide (4ml) at room temperature under nitrogen, then sodium hydride (60% dispersion in oil, 24mg, 0.60mmol) was added and the reaction was stirred at room temperature for 3 hours. The mixture was diluted with water (50ml) and extracted with ethyl acetate (2x50ml). The combined organic extracts were washed with brine (30ml), dried over magnesium sulphate, concentrated under reduced pressure and the residue was purified by flash chromatography on silica gel eluting with toluene:ethyl acetate (85:15, by volume) to provide the separated isomers as colourless oils (83mg of Isomer 1, 55mg of Isomer 2).
The least polar isomer (isomer 1) (83mg, 0.20mmol) and p-toluene-sulphonic acid (4mg, 0.02mmol) were dissolved in methanol (5ml) and stirred under nitrogen at room temperature for 18 hours. The solvent was removed under reduced pressure and the residue was partitioned between water (30ml) and dichloromethane (30ml).The aqueous phase was extracted with dichloromethane (20ml) and the combined organic extracts were dried over magnesium sulphate, concentrated under reduced pressure and the residual oil was purified by flash chromatography on silica gel eluting with toluene:ethyl acetate (66:34, by volume) to provide the title compound (39mg) as an oil.
¹H NMR (400MHz, CDCl₃): δ = 1.05 (t, 3H), 1.14 (d, 6H), 2.44 (q, 2H), 2.68 (sept, 1 H), 3.77 (brs, 1 H), 4.06 (m, 4H), 7.38 (s, 2H), 7.58 (s, 1H).
LRMS (electrospray) : m/z [MH⁺] 325.

### EXAMPLE 171

### 5-{[3-Ethyl-1-(2-hydroxyethyl)-5-isopropyl-1H-pyrazol-4yl]oxy}isophthalonitrile

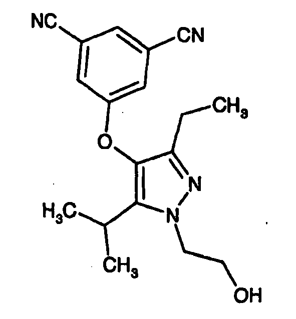

The most polar isomer (isomer 2) from Example 170 (55mg, 0.13mmol) and p-toluene-sulphonic acid (3mg, 0.01mmol) were dissolved in methanol (5ml) and stirred under nitrogen at room temperature for 18 hours. The solvent was removed under reduced pressure and the residue was partitioned between water (30ml) and dichloromethane (30ml). The aqueous phase was extracted with dichloromethane (20ml) and the combined organic extracts were dried over magnesium sulphate, concentrated under reduced pressure and the residual oil was purified by flash chromatography on silica gel eluting with toluene:ethyl acetate 66:33, by volume) to provide the title compound (39mg) as a white solid.
¹H NMR (400MHz, CDCl₃): δ = 1.08 (t, 3H), 1.13 (d, 6H), 2.49 (q, 2H), 2.97 (sept, 1 H), 3.59 (t, 1 H), 4.06 (m, 4H), 7.37 (s, 2H), 7.57 (s, 1H).
LRMS (electrospray) : m/z [MH⁺] 325.

### EXAMPLE 172

### 2-[4-(3,5-Dicyanophenoxy)-3,5-diethyl-1H-pyrazol-1-yl]ethyl carbamate

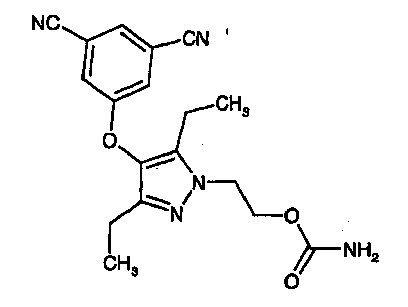

Trichloroacetyl-isocyanate (46µl, 0.38mmol) was added to a solution of the alcohol from Example 119 (100mg, 0.32mmol) dissolved in dichloromethane (3.2ml) under nitrogen at 0°C. After stirring for 2 hours the dichloromethane was removed under reduced pressure and methanol (1.6ml), water (1.6ml) and potassium carbonate (134mg, 0.96mmol) were added and the reaction was stirred for a further 2 hours. The methanol was removed under reduced pressure and the residue was extracted with dichloromethane (3x10ml). The combined organic extracts were dried over magnesium sulphate, concentrated under reduced pressure and the residual solid was purified by flash chromatography on silica gel eluting with dichloromethane:methanol (98:2, by volume) to provide the title compound (60mg) as a white solid.
¹H NMR (400MHz, CDCl₃): δ = 1.10 (m, 6H), 2.39 (q, 2H), 2.48 (q, 2H), 4.26 (m, 2H), 4.44 (m, 2H), 4.62 (brs, 2H), 7.41 (s, 2H), 7.58 (s, 1H).
LRMS (thermospray) : m/z [MH⁺] 354.
Microanalysis: Found C, 60.00; H, 5.55; N, 19.82. C₁₈H₁₉N₅O₃.0.23EtOAc requires C, 60.30; H, 5.67; N, 18.58%.

### EXAMPLE 173

### N-{2-[4-(3,5-Dicyanophenoxy)-3,5-diethyl-1H-pyrazol-1-yl]ethyl}sulfamide

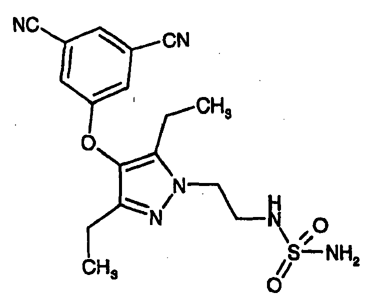

Sulfamide (31 mg, 0.32mmol) was added to a solution of the amine from Example 127 (100mg, 0.32mmol) dissolved in dioxan (0.5ml) under nitrogen at room temperature. The reaction was heated to 100°C for 18 hours, cooled to room temperature and partitioned between ethyl acetate (15ml) and water (15ml). The organic phase was dried over magnesium sulphate, concentrated under reduced pressure and the residual brown oil was purified by flash chromatography on silica gel eluting with dichloromethane:methanol:0.88 ammonia (95:5:0.5, by volume) to provide the title compound (25mg) as a white solid.
¹H NMR (400MHz, CDCl₃): δ = 1.12 (m, 6H), 2.39 (q, 2H), 2.51 (q, 2H), 3.61 (m, 2H), 4.20 (m, 2H), 4.78 (s, 2H), 5.42 (s, 1 H), 7.40 (s, 2H), 7.59 (s, 1H).
Microanalysis: Found C, 50.33; H, 5.07; N, 20.60. C₁₇H₂₀N₆O₃S.0.95H₂O requires C, 50.35; H, 5.44; N, 20.72%.

### EXAMPLE 174

### N-{2-[4-(3,5-Dicyanophenoxy)-3,5-diethyl-1H-pyrazol-1-yl]ethyl}-2-methoxyacetamide

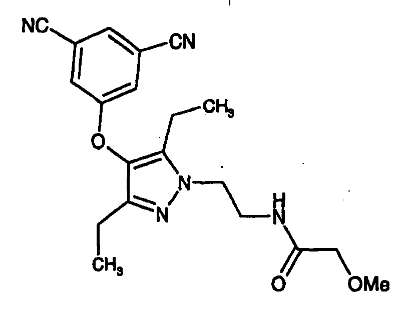

The amine from Example 127 (100mg, 0.32mmol), 1-(3-dimethylaminopropyl)-3-ethylcarbodiimide hydrochloride (68mg, 0.35mmol) and *N,N-*dimethylaminopyridine (43mg, 0.35mmol) were added to a solution of 1-methoxyacetic acid (27µl, 0.35mmol) dissolved in dichloromethane (10ml) under nitrogen at room temperature. The reaction was stirred for 18 hours, concentrated under reduced pressure and the residual yellow oil was purified by flash chromatography on silica gel eluting with dichloromethane:methanol:0.88 ammonia (95:5:0.5, by volume) to provide the title compound (32mg) as a colourless oil.
¹H NMR (400MHz, CDCl₃): δ = 1.11 (t, 3H), 1.16 (t, 3H), 2.38 (q, 2H), 2.47 (q, 2H), 3.41 (s, 3H), 3.77 (dd, 2H), 3.89 (s, 2H), 4.15 (m, 2H), 7.19 (brs, 1 H), 7.40 (s, 2H), 7.59 (s, 1 H).
LRMS (thermospray) : m/z [MH⁺] 382.
Microanalysis: Found C, 61.26; H, 6.18; N, 17.59. C₂₀H₂₃N₆O₃.0.60H₂O requires C, 61.24; H, 6.22; N, 17.85%.

### EXAMPLE 175

### 5-{[1-(3-Azetidinyl)-3,5-diethyl-1H-pyrazol-4-yl]oxy}isophthalonitrile

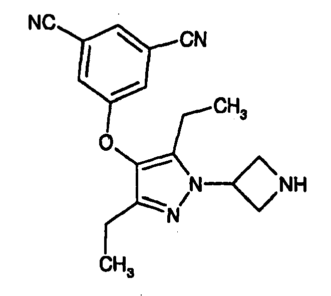

The protected amine from Preparation 69 (178mg, 0.42mmol) was dissolved in 4M hydrochloric acid in dioxan solution (1ml) and dioxan (1ml) and the reaction was stirred at room temperature for 18 hours. The solvent was removed under reduced pressure and the residue was partitioned between dichloromethane (20ml) and saturated aqueous sodium bicarbonate solution (20ml). The organic phase was dried over magnesium sulphate, concentrated under reduced pressure and purified by flash chromatography on silica gel eluting with a solvent gradient of dichloromethane:methanol:0.88 ammonia (100:0:0 then 98:2:0 then 95:5:0 then 95:5:0.5 then 90:10:1 then 80:20:1, by volume) to provide the title compound (33mg) as a white solid.
¹H NMR (400MHz, CDCl₃): δ = 1.05 (t, 3H), 1.11 (t, 3H), 2.44 (m, 4H), 3.85 (m, 2H), 4.38 (m, 2H), 5.05 (m, 1 H), 7.37 (s, 2H), 7.56 (s, 1H).
LRMS (electrospray) : m/z [MH⁺] 322.
Microanalysis: Found C, 65.87; H, 5.94; N, 20.98. C₁₈H₁₉N₅O.0.38H₂O requires C, 65.87; H, 6.07; N, 21.04%.

### EXAMPLE 176

### 5-{[3,5-Diethyl-1-(3-hydroxypropyl)-1H-pyrazol-4-yl]oxy}isophthalonitrile

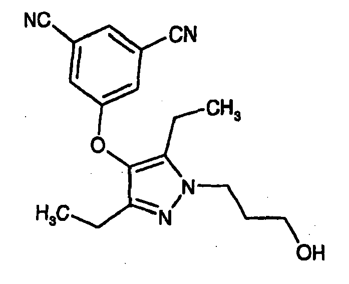

The protected alcohol from Preparation 70 (215mg, 0.53mmol) and p-toluene-sulphonic acid (10mg, 0.05mmol) were dissolved in methanol (2ml) and stirred under nitrogen at room temperature for 18 hours. The solvent was removed under reduced pressure and the residue was partitioned between water (10ml) and dichloromethane (10ml). The organic phase was dried over magnesium sulphate and concentrated under reduced pressure to provide the title compound (148mg) as a pale yellow solid, m.p. 93-95°C.
¹H NMR (400MHz, CDCl₃): δ = 1.11 (m, 6H), 2.04 (tt, 2H), 2.37 (q, 2H), 2.53 (q, 2H), 3.06 (t, 1 H), 3.69 (dt, 2H), 4.18 (t, 2H), 7.38 (s, 2H), 7.58 (s, 1H).
LRMS (electrospray) : m/z [MH⁺] 325, [MNa⁺] 347.
Microanalysis: Found C, 66.27; H, 6.27; N, 17.00. C₁₈H₂₀N₄O₂ requires C, 66.28; H, 6.24; N, 17.18%.

### EXAMPLE 177

### 5-[(3,5-Diethyl-1-methyl-1H-pyrazol-4-yl)oxy]isophthalonitrile

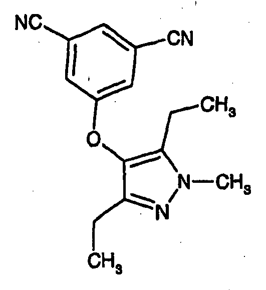

Sodium hydride (60% dispersion in oil, 33mg, 0.82mmol) was added to a solution of the pyrazole from Example 122 (200mg, 0.75mmol) in dimethylformamide (3ml) at 0°C under nitrogen and the reaction was stirred for 10 minutes. Methyl iodide (117mg, 0.82mmol) was added and the reaction was stirred at room temperature for 18 hours. The reaction was quenched with water (0.2ml) and concentrated under reduced pressure. The residue was partitioned between dichloromethane (5ml) and water (5ml) and the organic phase was isolated using a 5µM Whatman PTFE fritted cartridge, then concentrated under reduced pressure. The residue was purified by flash chromatography on silica gel eluting with a solvent gradient of ethyl acetate:pentane (20:80, by volume) changing to ethyl acetate:methanol (90:10, by volume) then dichloromethane:methanol:0.88 ammonia (90:10:1 then 80:20:1, by volume) to provide the title compound (170mg) as a yellow solid.
¹H NMR (400MHz, CDCl₃): δ = 1.10 (m, 6H), 2.39 (q, 2H), 2.49 (q, 2H), 3.80 (s, 3H), 7.40 (s, 2H), 7.56 (s, 1 H).
LRMS (electrospray) : m/z [MH⁺] 281.
Microanalysis: Found C, 68.41; H, 5.71; N, 19.93. C₁₆H₁₆N₄O requires C, 68.55; H, 5.75; N, 19.99%.

### EXAMPLES 178-180

### The preparation of the following tabulated Examples of the general formula

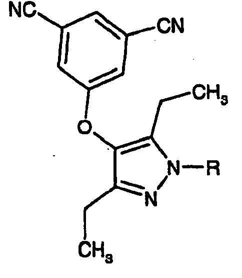

were performed by a similar method to that of Example 177 using the appropriate alkyl halide as the starting material.

| Example No. (Starting Material Example No.) | R | Analytical Data |
|---|---|---|
| 178 (122) | 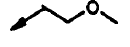 | ¹H NMR (400MHz, CDCl₃): δ = 1.08 (t, 3H), 1.12 (t, 3H), 2.40 (q, 2H), 2.54 (q, 2H), 3.34 (s, 3H), 3.75 (t, 2H), 4.17 (t, 2H), 7.38 (s, 2H), 7.56 (s, 1 H). LRMS (electrospray) : m/z [MH⁺] 325, [MNa⁺] 347. Microanalysis: Found C, 65.73; H, 6.17; N, 17.08. C₁₈H₂₀N₄O₃.0.25H₂O requires C, 65.74; H, 6.28; N, 17.04%. |
| 179^{1,2} (122) | 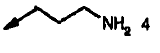 | ¹H NMR (400MHz, CDCl₃): δ = 1.10 (m, 6H), 1.98 (tt, 2H), 2.38 (q, 2H), 2.51 (q, 2H), 2.76 (t, 2H), 4.09 (t, 2H), 7.38 (s, 2H), 7.57 (s, 1 H). LRMS (electrospray) : m/z [MH⁺] 324. Microanalysis: Found C, 64.86; H, 6.51; N, 20.79. C₁₈H₂₁N₅O.0.57H₂O requires C, 64.79; H, 6.69; N, 20.99%. |
| 180³ (122) | 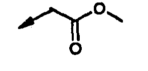 | ¹H NMR (400MHz, CDCl₃): δ = 1.09 (t, 3H), 1.14 (t, 3H), 2.41 (q, 2H), 2.47 (q, 2H), 3.79 (s, 3H), 4.82 (s, 2H), 7.40 (s, 2H), 7.57 (s, 1 H). LRMS (electrospray) : m/z [MH⁺] 339. Microanalysis: Found C, 63.58; H, 5.35; N, 16.35. C₁₈H₁₈N₄O₃.0.10H₂O requires C, 63.56; H, 5.39; N, 16.47%. |

| | | |
|---|---|---|
| ¹ The two reagents were heated together as a melt at 160°C for 24 hours, and the reaction was worked up by partitioning between dichloromethane and saturated sodium bicarbonate solution, extracting the organic phase with 2M aqueous hydrochloric acid and basifying the aqueous phase with sodium carbonate. After extraction with dichloromethane the organic phase was dried and concentrated to give the crude product. ² The eluent used for flash column chromatography purification of this compound was dichloromethane:methanol:0.88 ammonia (95:5:0.5 changing to 80:20:1, by volume). ³ The eluent used for flash column chromatography purification of this compound was pentane:ethyl acetate (75:25 changing to 66:34 then 50:50, by volume). ⁴ The hydrochloride salt of the starting alkyl halide was used. | | |

### EXAMPLE 181

### 2-[4-(3,5-Dicyanophenoxy)-3,5-diethyl-1H-pyrazol-1-yl]acetamide

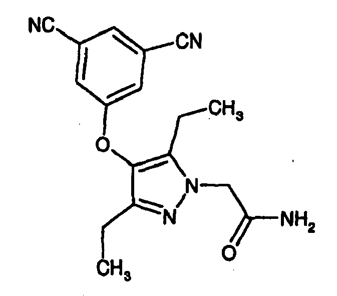

The ester from Example 180 (200mg, 0.59mmol) was dissolved in 2M methanolic ammonia solution (5ml) and the reaction was stirred under nitrogen at 75°C for 18 hours. The mixture was concentrated under reduced pressure and the residue was purified by flash chromatography on silica gel eluting with a solvent gradient of dichloromethane:methanol:0.88 ammonia (95:5:0.5, by volume) to provide the title compound (6mg).
¹H NMR (400MHz, CDCl₃): δ = 1.10 (t, 3H), 1.15 (t, 3H), 2.44 (q, 2H), 2.54 (q, 2H), 4.69 (s, 2H), 5.55 (brs, 1 H), 6.22 (brs, 1 H), 7.38 (s, 2H), 7.59 (s, 1H).
LRMS (electrospray) : m/z [M-H⁺] 322.
Microanalysis: Found C, 68.41; H, 5.71; N, 19.93. C₁₆H₁₆N₄O requires C, 68.55; H, 5.75; N, 19.99%.

### EXAMPLE 182

### 5-{[3,5-Diethyl-1-(hydroxymethyl)-1H-pyrazol-4-yl]oxy}isophthalonitrile

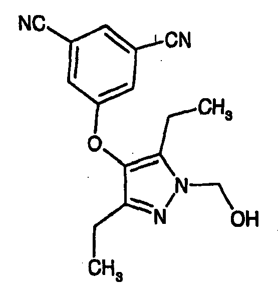

Formaldehyde (37% solution in water, 253µl, 3.14mmol) was added to a solution of the pyrazole from Example 122 (440mg, 1.65mmol) in ethanol (5ml) and the reaction was stirred at 80°C for 18 hours. After cooling to room temperature the solvent was removed under reduced pressure and the residual yellow solid was partitioned between ethyl acetate (15ml) and water (10ml) and the organic phase was removed. The aqueous phase was washed with ethyl acetate (2x15ml) and the combined organic extracts were dried over magnesium sulphate and concentrated under reduced pressure to provide the title compound (490mg) as a white solid.
¹H NMR (400MHz, CDCl₃): δ = 1.13 (t, 3H), 1.14 (t, 3H), 2.39 (q, 2H), 2.61 (q, 2H), 5.49 (s, 2H), 5.68 (brs, 1 H), 7.40 (s, 2H), 7.56 (s, 1H).
LRMS (thermospray) : m/z [MH⁺] 267.
Microanalysis: Found C, 64.28; H, 5.52; N, 18.47. C₁₆H₁₆N₄O₂.0.15H₂O requires C, 64.27; H, 5.49; N, 18.24%.

### EXAMPLE 183

### 3-[({[4-(3-cyano-5-fluorophenoxy)-3-methyl-1H-pyrazol-5-yl]methyl}amino)methyl]benzamide

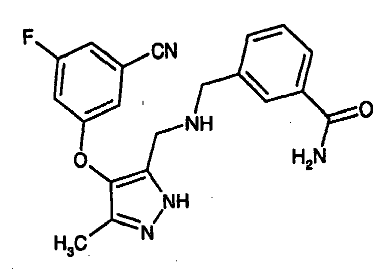

The pyrazole from Preparation 75 (320mg, 0.91 mmol) and the amine from Preparation 80 (680mg, 4.61 mmol) were refluxed in isopropanol (5ml) for 1.5 hours. The solvent was removed under reduced pressure and the residue was purified by flash chromatography on silica gel eluting with dichloromethane:methanol:0.88 ammonia (95:5:0.5, by volume) to give the product which was further purified by preparative HPLC using a Develosil combi-rp C30 50x4.6mm 3µm column eluting with a solvent gradient of 5:95 0.1% aqueous trifluoroacetic acid in acetonitrile:acetonitrile (0-6min 95:5 changing to 50:50; 6-7min 50:50; 7-7.1 min 50:50 changing to 5:95; 7.1-8min 5:95) to provide the title compound (38mg).
¹H NMR (400MHz, CD₃OD): δ = 2.14 (s, 3H), 4.10 (s, 2H), 4.34 (s, 2H), 7.03 (m, 1 H), 7.10 (s, 1 H), 7.25 (m, 1 H), 7.54 (t, 1 H), 7.64 (d, 1 H), 7.92 (d, 1 H), 7.97 (s, 1H).

LRMS (electrospray) : m/z [MH⁺] 380.

Microanalysis: Found C, 51.32; H, 3.91; N, 13.69. C₂₀H₁₈N₅O₂F.1.00CF₃CO₂H1.10H₂O requires C, 51.49; H, 4.16; N, 13.65%.

### EXAMPLES 184-188

The preparation of the following tabulated Examples of the general formula 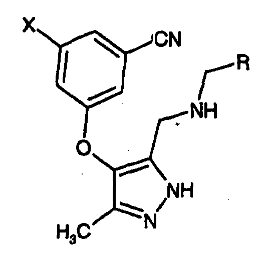 were performed by a similar method to that of Example 183 using as the starting materials the appropriate pyrazole (P) and amine (A).

| Ex. no. | P prep. no. | A prep. no. | X | R | Analytical Data |
|---|---|---|---|---|---|
| 184¹ | 75 | 55 | F | 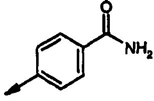 | ¹H NMR (400MHz, CDCl₃): δ = 2.09 (s, 3H), 3.65 (s, 2H), 3.79 (s, 2H), 6.80 (d, 1 H), 6.93 (s, 1H), 6.97 (d, 1 H), 7.31 (d, 2H), 7.72 (d, 2H). LRMS (thermospray) : m/z [MH⁺] 380. |
| 185¹ | 76 | 55 | CN | 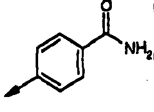 | m.p. 114-116°C ¹H NMR (400MHz, CDCl₃): δ = 2.08 (s, 3H), 3.62 (s, 2H), 3.77 (s, 2H), 7.34 (d, 2H), 7.55 (s, 1 H), 7.77 (d, 2H), 7.79 (s, 1 H). LRMS (thermospray) : m/z [MH⁺] 387. |
| 186¹ | 18 | 80 | Cl | 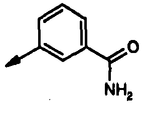 | m.p. 98-101°C ¹H NMR (400MHz, CDCl₃): δ = 2.04 (s, 3H), 3.62 (s, 2H), 3.74 (s, 2H), 6.97 (s, 1 H), 7.07 (s, 1 H), 7.20 (s, 1H), 7.22 (d, 1H), 7.29 (t, 1 H), 7.62 (s, 1H), 7.81 (s, 1 H). LRMS (thermospray) : m/z [MH⁺] 396. Microanalysis: Found C, 56.98; H, 4.58; N, 17.69. C₂₀H₁₈CIN₅O₂.0.40CH₂Cl₂ requires C, 57.01; H, 4.41; N, 16.29%. |
| 187^{1,} 2,3 | 77 | 55 | Me | 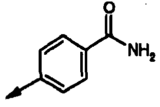 | ¹H NMR (400MHz, CDCl₃): δ = 2.10 (s, 3H), 2.30 (s, 3H), 3.65 (s, 2H), 3.80 (s, 2H), 6.85 (s, 1 H), 6.95 (s, 1 H), 7.10 (s, 1 H), 7.30 (d, 2H), 7.70 (d, 2H). LRMS (electrospray) : m/z [MH⁺] 376, [M-H⁺] 374. Microanalysis: Found C, 65.59; H, 5.65; N, 18.19. C₂₁H₂₁N₅O₂.0.50H₂O requires C, 65.51; H, 5.77; N, 18.22%. |
| 188⁴ | 78 | 55 | H | 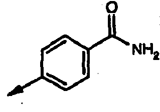 | ¹H NMR (400MHz, CD₃OD): δ = 2.15 (s, 3H), 4.10 (s, 2H), 7.20 (m, 2H), 7.40 (m, 1 H), 7.50 (m, 1 H), 7.55 (d, 2H), 7.90 (d, 2H). Microanalysis: Found C, 53.51; H, 4.13; N, 13.59. C₂₀H₁₉N₅O₂. 1.25 TFA requires C, 53.63; H, 4.05; N, 13.90%. |

| | | | | | |
|---|---|---|---|---|---|
| ¹ No preparative HPLC was required for purification of this compound. ² The eluent used for flash column chromatography purification of this compound was dichloromethane:methanol:0.88 ammonia (95:5:0.5 changing to 90:10:1, by volume). ³ The product was triturated with dichloromethane containing a trace of methanol - a solid crystallised out which was an impurity. This was filtered off and the filtrate was concentrated under reduced pressure and the residue was purified by flash chromatography eluting with dichloromethane:methanol:0.88 ammonia (90:10:1, by volume) to give the title compound. ⁴ The column used for preparative HPLC was a LUNA C18 10µm 150x21.2mm. | | | | | |

### EXAMPLE 189

### 5-[(3,5-Dicyclopropyl-1H-pyrazol-4-yl)oxy]isophthalonitrile

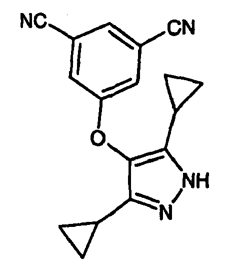

Hydrazine hydrate (133µl, 2.75mmol) was added to a solution of the diketone from Preparation 82 (735mg, 2.50mmol) in acetic acid (25ml) under nitrogen at room temperature. After stirring for 64 hours, the mixture was concentrated under reduced pressure and the residue was partitioned between dichloromethane (25ml) and saturated aqueous sodium bicarbonate solution (25ml). The organic phase was dried over magnesium sulphate, concentrated under reduced pressure and the residue was purified by flash chromatography on silica gel eluting with dichloromethane:methanol (98:2 changing to 96:4, by volume) to provide the title compound (473mg) as a white solid, m.p. 168-170°C.
¹H NMR (400MHz, CDCl₃): δ = 0.77 (m, 4H), 0.85 (m, 4H), 1.59 (m, 2H), 7.44 (s, 2H), 7.59 (s, 1 H).
LRMS (thermospray) : m/z [MH⁺] 291.
Microanalysis: Found C, 69.90; H, 4.85; N, 19.18. C₁₇H₁₄N₄O.0.10H₂O requires C, 69.90; H, 4.90; N, 19.18%.

### EXAMPLE 190

### 5-{[3,5-Dicyclopropyl-1-(2-hydroxyethyl)-1H-pyrazol-4-yl]oxy}isophthalonitrile

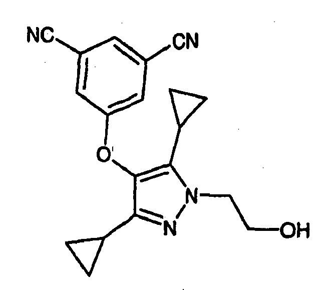

2-Hydroxyethylhydrazine (84mg, 1.10mmol) was added to a solution of the diketone from Preparation 82 (294mg, 1.00mmol) in acetic acid (1 0ml) under nitrogen at room temperature. After stirring for 64 hours, the mixture was concentrated under reduced pressure and the residue was partitioned between dichloromethane (25ml) and saturated aqueous sodium bicarbonate solution (25ml). The organic phase was dried over magnesium sulphate, concentrated under reduced pressure and the residue was purified by flash chromatography on silica gel eluting with dichloromethane:methanol (99:1 changing to 95:5, by volume) to provide the title compound (137mg) as a white solid, m.p. 115-117°C.
¹H NMR (400MHz, CDCl₃): δ = 0.67 (m, 2H), 0.80 (m, 4H), 0.85 (m, 2H), 1.52 (m, 2H), 3.39 (brs, 1 H), 4.05 (m, 2H), 4.22 (t, 2H), 7.42 (s, 2H), 7.58 (s, 1H).
LRMS (thermospray) : m/z [MH⁺] 355.
Microanalysis: Found C, 67.63; H, 5.55; N, 16.35. C₁₉H₁₈N₄O₂.0.17H₂O requires C, 67.63; H, 5.48; N, 16.60%.

### EXAMPLE 191

### 5-{[1-(2-Aminoethyl)-3,5-dicyclopropyl-1H-pyrazol-4-yl]oxy}isophthalonitrile

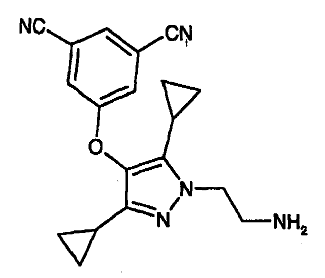

2-Chloroethylamine hydrochloride (192mg, 1.65mmol) and the pyrazole from Example 189 (440mg, 1.50mmol) were heated as a melt at 160°C for 18 hours and the residue was partitioned between dichloromethane (25ml) and 10% aqueous potassium carbonate solution (25ml). The organic phase was dried over magnesium sulphate, concentrated under reduced pressure and the residue was purified by flash chromatography on silica gel eluting with dichloromethane:methanol:0.88 ammonia (95:5:0 changing to 95:5:0.5, by volume) to provide the title compound (9.2mg) as a white solid, m.p. 175-177°C.
¹H NMR (400MHz, CDCl₃): δ = 0.70 (m, 2H), 0.79 (m, 4H), 0.88 (m, 2H), 1.57 (m, 1 H), 1.66 (m, 1 H), 3.46 (t, 2H), 4.41 (t, 2H), 7.62 (s, 2H), 7.58 (s, 1H).

### EXAMPLE 192

### 3-{[3-cyclopropyl-1-(2-hydroxyethyl)-5-methyl-1H-pyrazol-4-yl]oxy}-5-methylbenzonitrile

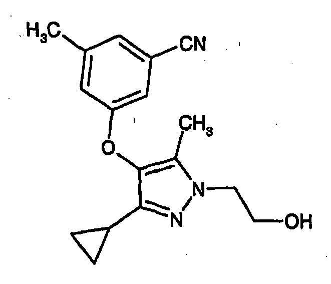

and

### EXAMPLE 193

### 3-{[5-cyclopropyl-1-(2-hydroxyethyl)-3-methyl-1H-pyrazol-4-yl]oxy}-5-methylbenzonitrile

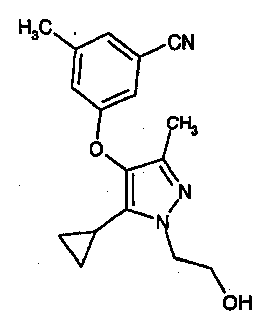

2-Hydroxy-ethyl-hydrazine (326µl, 4.80mmol) was added to a solution of the diketone from Preparation 86 (1.00g, 4.37mmol) in acetic acid (10ml) under nitrogen at room temperature. After stirring for 18 hours, the mixture was concentrated under reduced pressure and the residual orange oil was purified by flash chromatography on silica gel eluting with ethyl acetate:pentane (50:50 changing to 100:0, by volume) to provide two pale yellow oils.
Least Polar Fraction (Example 192) - 419mg
¹H NMR (400MHz, CDCl₃): δ = 0.69 (m, 2H), 0.82 (m, 2H), 1.54 (m, 1 H), 2.00 (s, 3H), 2.35 (s, 3H), 3.46 (brs, 1H), 4.05 (t, 2H), 4.22 (t, 2H), 6.88 (s, 1H), 6.94 (s, 1H), 7.08 (s, 1 H).
LRMS (thermospray) : m/z [MH⁺] 298.
Microanalysis: Found C, 68.29; H, 6.51; N, 13.92. C₁₇H₁₉N₃O₂ requires C, 68.67; H, 6.44; N, 14.13%.
Most Polar Fraction (Example 193) - 201 mg
¹H NMR (400MHz, CDCl₃): δ = 0.75 (m, 4H), 1.58 (m, 1 H), 2.07 (s, 3H), 2.35 (s, 3H), 3.45 (brs, 1H), 4.00 (m, 4H), 6.92 (s, 1 H), 7.00 (s, 1 H), 7.10 (s, 1H).
LRMS (thermospray) : m/z [MH⁺] 298.
Microanalysis: Found C, 68.44; H, 6.49; N, 13.95. C₁₇H₁₉N₃O₂ requires C, 68.67; H, 6.44; N, 14.13%.

### EXAMPLE 194

### 3-[3-Cyclopropyl-1-(2-amino-ethyl)-5-methyl-1H-pyrazol-4-yloxy]-5-methyl-benzonitrile

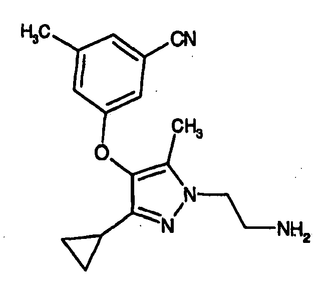

The alcohol from Example 192 (140mg, 0.47mmol), triphenylphosphine (309mg, 1.18mmol) and phthalimide (174mg, 1.18mmol) were dissolved in tetrahydrofuran (9ml) at 0°C under nitrogen and diisopropylazodicarboxylate (232µl, 1.18mmol) dissolved in tetrahydrofuran (2ml) was added over 10 minutes. The reaction was allowed to warm to room temperature and was stirred for 18 hours. The solvent was removed under reduced pressure, the residue was dissoved in ethanol (11 ml) and hydrazine hydrate (114µl, 2.35mmol) was added. The thick white slurry was stirred for 18h at room temperature under nitrogen, methanol (10ml) was added and the mixture was filtered. The filtrate was concentrated under reduced pressure and the residue was dissolved in dichloromethane (20ml). The organic phase was extracted with 2M aqueous hydrochloric acid (20ml) and the aqueous phase was washed with dichloromethane (5x10ml), basified with 1M aqueous sodium hydroxide and extracted with dichloromethane (50ml). The organic phase was dried over magnesium sulphate and concentrated under reduced pressure to provide the title compound (135mg) as a yellow oil.
¹H NMR (400MHz, CDCl₃): δ = 0.70 (m, 4H), 1.56 (m, 1 H), 2.06 (s, 3H), 2.30 (s, 3H), 3.10 (t, 2H), 3.97 (t, 2H), 6.87 (s, 1 H), 6.92 (s, 1 H), 7.05 (s, 1H).
LRMS (electrospray) : m/z [MH⁺] 297.
Microanalysis: Found C, 63.81; H, 6.51; N, 17.30. C₁₇H₂₀N₄O.0.36CH₂Cl₂ requires C, 63.78; H, 6.39; N, 17.14%.

### EXAMPLE 195

### 3-[(3-Cyclopropyl-5-methyl-1H-pyrazol-4-yl)oxy]-5-methylbenzonitrile

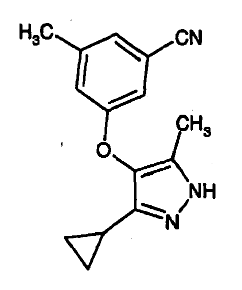

Hydrazine hydrate (31µl), 0.64mmol) was added to a solution of the diketone from Preparation 86 (150mg, 0.58mmol) in acetic acid (1.3ml) under nitrogen at room temperature. After stirring for 24 hours, the mixture was concentrated under reduced pressure and the residue was purified by flash chromatography on silica gel eluting with pentane:ethyl acetate (60:40 changing to 40:60, by volume) to provide the title compound (140mg).
¹H NMR (400MHz, CDCl₃): δ = 0.60 (m, 4H), 1.69 (m, 1 H), 2.09 (s, 3H), 2.34 (s, 3H), 6.95 (s, 1 H), 6.99 (s, 1 H), 7.10 (s, 1H).
LRMS (thermospray) : m/z [MH⁺] 254.
Microanalysis: Found C, 68.35; H, 6.13; N, 15.10. C₁₅H₁₅N₃O.0.29EtOAc requires C, 68.72; H, 6.32; N, 14.88%.

### EXAMPLE 196

### 3-{[1-(3-Aminopropyl)-3,5-diethyl-1H-pyrazol-4-yl]oxy}-5-methylbenzonitrile

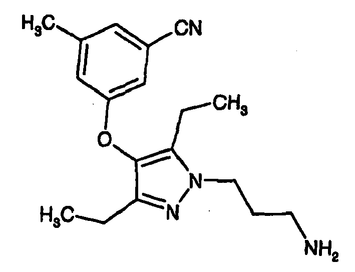

3-Chloropropylamine hydrochloride (62mg, 0.48mmol) and the pyrazole from Example 123 (113mg, 0.44mmol) were heated as a melt at 150°C for 18 hours. After cooling the residue was purified by flash chromatography on silica gel eluting with dichloromethane:methanol:0.88 ammonia (98:2:0 changing to 95:5:0.5, by volume). An impurity remained so the oil was dissolved in acetone (3ml) and (L)-tartaric acid (54mg, 0.44mmol) was added, the mixture was heated to effect dissolution and cooled. The resultant precipitate was isolated by filtration washing with acetone (10ml) to provide the title compound (127mg) as a white solid which was the tartrate salt.
¹H NMR (400MHz, CD₃OD): δ = 1.05 (m, 6H), 2.07 (m, 2H), 2.37 (q, 2H), 2.53 (s, 3H), 2.57 (q, 2H), 2.99 (t, 2H), 4.15 (t, 2H), 4.38 (s, 2H), 6.89 (s, 1 H), 7.01 (s, 1 H), 7.19 (s, 1H).
LRMS (thermospray) : m/z [MH⁺] 313.
Microanalysis: Found C, 56.81; H, 6.57; N, 12.06. C₂₂H₃₀N₄O₇ requires C, 57.13; H, 6.54; N, 12.11%.

### EXAMPLE 197

### 3-{[3,5-Diethyl-1-(2-hydroxyethyl)-1H-pyrazol-4-yl]oxy}-4-methoxybenzonitrile

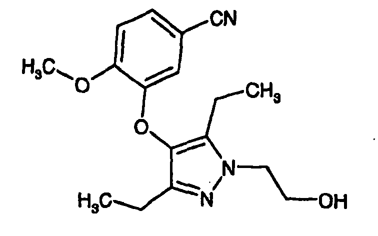

Cesium carbonate (700mg, 2.14mmol) was added to a stirred solution of 2-methoxy-5-cyanophenol (285mg, 2.15mmol) and the dione of Preparation 2 (348mg, 2.15mmol) in acetone (20ml) at room temperature. The reastion was heated at 50°C for 3 hours and then cooled to room temperature. The mixture was concentrated under reduced pressure, dissolved in dichloromethane (5ml) and washed with water (6ml). The organic phase was isolated using a 5µM Whatman PTFE fritted cartridge, then concentrated under reduced pressure. The residue was dissolved in acetic acid (5.4ml) and 2-hydroxy-ethyl-hydrazine (160µl, 2.15mmol) added under nitrogen at room temperature. After stirring for 18 hours, the mixture was concentrated under reduced pressure and the residual orange oil was purified by flash chromatography on silica gel eluting with a solvent gradient of ethyl acetate:pentane (25:75 changing to 50:50, by volume) to provide the title compound (182mg).
¹H NMR (400MHz, CDCl₃): δ = 1.10 (m, 6H), 2.39 (q, 2H), 2.51 (q, 2H), 3.71 (brs, 1 H), 4.00 (s, 3H), 4.08 (m, 2H), 4.09 (m, 2H), 6.89 (s, 1 H), 6.99 (d, 1 H), 7.32 (d, 1H). LRMS (thermospray) : m/z [MH⁺] 316.
Microanalysis: Found C, 64.57; H, 6.73; N, 13.15, C₁₇H₂₁N₃O₃ requires C, 64.74; H, 6.71; N, 13.32%.

### Examples 198-199

The preparation of the following tabulated Examples of the general formula 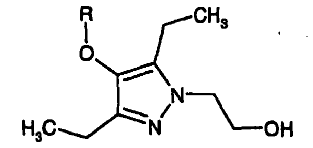 were performed by a similar method to that of Example 197 using the β-diketone of Preparation 2 and the appropriate aryl alcohol as the starting materials.

| Example No. | R | Analytical Data |
|---|---|---|
| 198 | 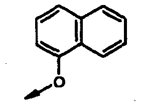 | ¹H NMR (400MHz, CDCl₃): δ = 1.04 (m, 6H), 2.42 (q, 2H), 2.51 (q, 2H), 4.07 (m, 2H), 4.12 (m, 2H), 6.60 (d, 1 H), 7.25 (t, 1 H), 7.49 (d, 1 H), 7.53 (m, 2H), 7.82 (m, 1 H), 8.41 (m, 1 H). LRMS (thermospray) : m/z [MH⁺] 311. |
| 199 | 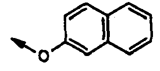 | ¹H NMR (400MHz, CDCl₃): δ = 1.19 (m, 6H), 2.48 (q, 2H), 2.51 (q, 2H), 4.03 (m, 2H), 4.10 (m, 2H), 7.06 (s, 1H), 7.22 (m, 1 H), 7.38 (t, 1 H), 7.42 (m, 1 H), 7.69 (d, 1 H), 7.79 (s, 1H), 7.80 (s, 1 H). LRMS (thermospray) : m/z [MH⁺] 311. Microanalysis: Found C, 72.16; H, 7.20; N, 8.95. C₁₉H₂₂N₂O₂.0.10EtOAc requires C, 72.45; H, 7.19; N, 8.63%. |

### EXAMPLE 200

### 2-{4-[3,5-Di(1H-pyrazol-1-yl)phenoxy]-3,5-diethyl-1H-pyrazol-1-yl}ethanol

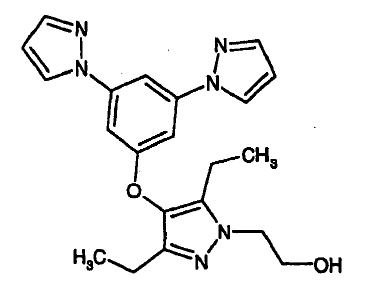

The protected alcohol from Preparation 88 (254mg, 0.53mmol) and p-toluene-sulphonic acid (10mg, 0.05mmol) were dissolved in methanol (4ml) and stirred under nitrogen at room temperature for 18 hours. The solvent was removed under reduced pressure and the residue was partitioned between saturated aqueous sodium bicarbonate solution (20ml) and dichloromethane (20ml). The aqueous phase was extracted with dichloromethane (10ml) and the combined organic extracts were dried over magnesium sulphate, concentrated under reduced pressure and purified by flash chromatography on silica gel eluting with a solvent gradient of dichloromethane:methanol (100:0 changing to 93:7, by volume) to provide the title compound (56mg) as a white solid, m.p. 108-110°C.
¹H NMR (400MHz, CDCl₃): δ = 1.11 (m, 6H), 2.46 (q, 2H), 2.53 (q, 2H), 4.01 (t, 2H), 4.07 (t, 2H), 6.44 (s, 2H), 7.16 (s, 2H), 7.68 (s, 3H), 7.92 (s, 2H).
LRMS (electrospray) : m/z [MH⁺] 393, [MNa⁺] 415.
Microanalysis: Found C, 63.62; H, 6.11; N, 21.11. C₂₁H₂₄N₆O₂.0.06CH₂Cl₂ requires C, 63.63; H, 6.12; N, 21.14%.

### EXAMPLE 201

### 2-{3,5-Diethyl-4-[3-fluoro-5-(1H-pyrazol-1-yl)phenoxy]-1H-pyrazol-1-yl}ethanol

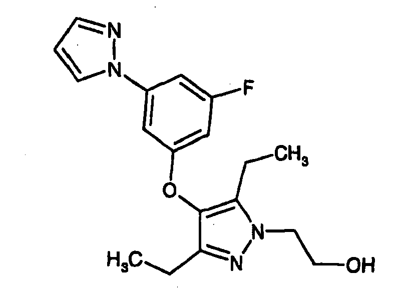

The protected alcohol from Preparation 89 (38.6mg, 0.09mmol) and p-toluene-sulphonic acid (3.5mg, 0.01mmol) were dissolved in methanol (1ml) and stirred under nitrogen at room temperature for 18 hours. The solvent was removed under reduced pressure and the residue was partitioned between 10% aqueous potassium carbonate solution (4ml) and dichloromethane (4ml). The aqueous phase was extracted with dichloromethane (10ml) and the combined organic extracts were dried over magnesium sulphate, concentrated under reduced pressure and purified by flash chromatography on silica gel eluting with a solvent gradient of dichloromethane:methanol (99:1 changing to 98:2, by volume) to provide the title compound (23mg) as a white solid, m.p. 120-122°C.
¹H NMR (400MHz, CDCl₃): δ = 1.14 (m, 6H), 2.46 (q, 2H), 2.55 (q, 2H), 4.06 (m, 2H), 4.09 (m, 2H), 6.47 (s, 1 H), 6.49 (s, 1 H), 7.09 (s, 1H), 7.12 (s, 1 H), 7.71 (s, 1H), 7.86 (s, 1H).
LRMS (electrospray) : m/z [MNa⁺] 367.
HRMS: [MH⁺] Found 345.1717. C₁₈H₂₂FN₄O₂ requires 345.1722.

### EXAMPLE 202

### 3-{[3,5-Diethyl-1-(2-hydroxyethyl)-1H-pyrazol-4-yl]oxy}-5-methoxybenzonitrile

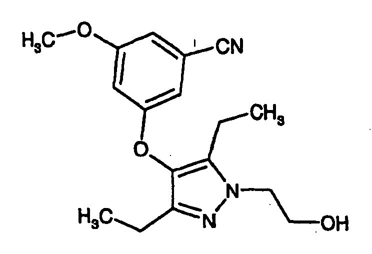

The protected alcohol from Preparation 90 (400mg, 1.00mmol) and p-toluene-sulphonic acid (19mg, 0.10mmol) were dissolved in methanol (10ml) and stirred under nitrogen at room temperature for 18 hours. The solvent was removed under reduced pressure and the residue was partitioned between saturated aqueous sodium bicarbonate solution (20ml) and dichloromethane (20ml). The aqueous phase was extracted with dichloromethane (40ml) and the combined organic extracts were dried over magnesium sulphate, concentrated under reduced pressure and purified by flash chromatography on a silica gel eluting with dichloromethane:methanol (97:3, by volume) to provide the title compound (174mg) as an oil.
¹H NMR (400MHz, CDCl₃): δ = 1.09 (m, 6H), 2.40 (q, 2H), 2.49 (q, 2H), 3.78 (s, 3H), 4.04 (m, 2H), 4.08 (m, 2H), 6.66 (s, 1 H), 6.71 (s, 1 H), 6.79 (s, 1H).
LRMS (electrospray) : m/z [MH⁺] 316.
Microanalysis: Found C, 63.63; H, 6.76; N, 13.06. C₁₇H₂₁N₃O₃.0.08CH₂Cl₂ requires C, 63.68; H, 6.68; N, 13.04%.

### EXAMPLE 203

### 2-[4-(3,5-Difluorophenoxy)-3,5-diethyl-1H-pyrazol-1-yl]ethylamine

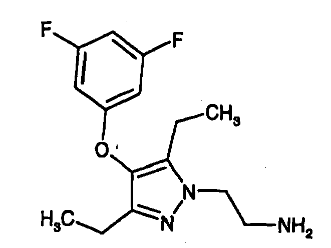

The alcohol from Example 38 (371 mg, 1.25mmol), triphenylphosphine (984mg, 3.75mmol) and phthalimide (552mg, 3.75mmol) were dissolved in tetrahydrofuran (20ml) at 0°C under nitrogen and diisopropylazodicarboxylate (738µl, 3.75mmol) dissolved in tetrahydrofuran (2ml) was added over 10 minutes. The reaction was allowed to warm to room temperature and was stirred for 18 hours. The solvent was removed under reduced pressure, the residue was dissolved in ethanol (25ml) and hydrazine hydrate (303µl, 6.25mmol) was added. The slurry was stirred for 4 hours at 45°C under nitrogen, concentrated under reduced pressure and the residue was dissolved in methanol. The solution was then passed through an SCX column eluting with methanol to remove impurities, then 2M methanolic ammonia solution to elute the product. The product was then purified by flash chromatography on alumina eluting with dichloromethane:methanol:0.88 ammonia (90:10:1, by volume) to provide the title compound (212mg) as an oil.
¹H NMR (400MHz, CDCl₃): δ = 1.12 (m, 6H), 2.43 (q, 2H), 2.54 (q, 2H), 3.21 (t, 2H), 4.07 (t, 2H), 6.43 (m, 3H).
Microanalysis: Found C, 59.78; H, 6.50; N, 14.35. C₁₅H₁₉F₂N₃O.0.26H₂O requires C, 60.05; H, 6.56; N, 14.01%.

### EXAMPLE 204

### 3-{[1-(2-Aminoethyl)-3,5-diethyl-1H-pyrazol-4-yl]oxy}-5-fluorobenzamide

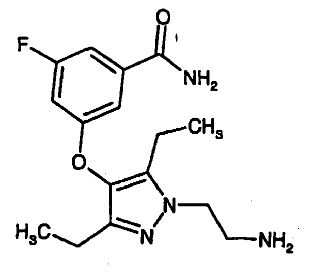

The alcohol from Example 163 (142mg, 0.44mmol), triphenylphosphine (346mg, 1.32mmol) and phthalimide (194mg, 1.32mmol) were dissolved in tetrahydrofuran (8ml) at 0°C under nitrogen and diisopropylazodicarboxylate (260µl, 1.32mmol) dissolved in tetrahydrofuran (1ml) was added over 10 minutes. The reaction was allowed to warm to room temperature and was stirred for 18 hours. The solvent was removed under reduced pressure, the residue was dissolved in ethanol (9ml) and hydrazine hydrate (107µl, 2.2mmol) was added. The slurry was stirred for 4 hours at 45°C under nitrogen, concentrated under reduced pressure and the residue was dissolved in methanol. The solution was then passed through a polymer supported sulphonic acid column eluting with methanol to remove impurities, then 2M methanolic ammonia solution to elute the product. The product was then purified by flash chromatography on alumina eluting with dichloromethane:methanol:0.88 ammonia (90:10:1, by volume) to provide the title compound (60mg) as an oil.
¹H NMR (400MHz, CDCl₃): δ = 1.11 (m, 6H), 2.43 (q, 2H), 2.53 (q, 2H), 3.17 (t, 2H), 4.05 (t, 2H), 6.01 (brs, 1 H), 6.25 (brs, 1 H), 6.75 (d, 1 H), 7.16 (m, 2H).
HRMS: [MH⁺] Found 321.1718. C₁₈H₂₁FN₄O₂ requires 321.1722.

### EXAMPLE 205

### 3-[(3-Isopropyl-5-methyl-1H-pyrazol-4-yl)oxy]-5-methylbenzonitrile

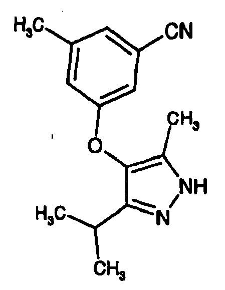

Hydrazine hydrate (100µl, 2.10mmol) was added to a solution of the diketone from Preparation 91 (544mg, 2.10mmol) in acetic acid (10ml) under nitrogen at room temperature. After stirring for 64 hours, the mixture was concentrated under reduced pressure and the residue was purified by flash chromatography on silica gel eluting with pentane:ethyl acetate (66:34, by volume) to provide the title compound (308mg) as a pale yellow oil.
¹H NMR (400MHz, CDCl₃): δ = 1.22 (d, 6H), 2.09 (s, 3H), 2.56 (s, 3H), 2.84 (m, 1 H), 6.91 (s, 1 H), 6.94 (s, 1H), 7.11 (s, 1H).
LRMS (thermospray) : m/z [MH⁺] 256.

### EXAMPLE 206

### 3-{[1-(2-Aminoethyl)-3-isopropyl-5-methyl-1H-pyrazol-4-yl]oxy}-5-methylbenzonitrile

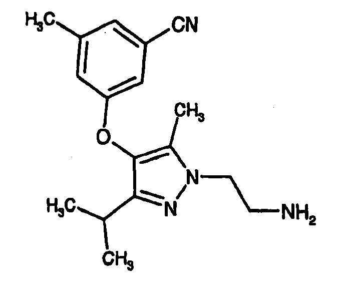

The pyrazole from Example 205 (70mg, 0.27mmol) and 2-chloroethylamine hydrochloride (38mg, 0.33mmol) were heated as a melt at 150°C for 18 hours. The residue was cooled and purified by flash chromatography on silica gel eluting with dichloromethane:methanol:0.88 ammonia (95:5:0.5, by volume) to give the title compound (25mg).
¹H NMR (400MHz, CDCl₃): δ = 1.18 (m, 6H), 2.06 (s, 3H), 2.35 (s, 3H), 2.79 (m, 1 H), 3.19 (m, 2H), 4.04 (m, 2H), 6.89 (s, 1 H), 6.97 (s, 1 H), 7.12 (s, 1H).
LRMS (electrospray) : m/z [MH⁺] 300.

### EXAMPLE 207

### 2-[4-(3,5-Dichlorophenoxy)-3,5-diethyl-1H-pyrazol-1-yl]-N-(2-pyridinylmethyl)acetamide

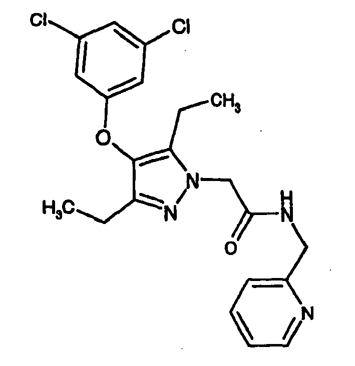

Standard solutions: The acid of Preparation 4 (800mg, 2.33mmol), 1*H*-benzotriazol-1-yl)-*N,N,N',N'*-tetramethyluronium hexafluorophosphate (822mg, 3.50mmol) and diisopropylethylamine (603mg, 4.66mmol) were separately dissolved in *N,N-*dimethylformamide (3x13ml). 2-(Methylamino)pyridine (3mg, 0.029mmol) was treated with the standard solutions of the acid and coupling reagents (3x170µl) in a 96 well plate and the mixture was shaken for 14 hours at room temperature. The solvent was removed under reduced pressure and the mixture dissolved in dimethylsulphoxide (500µl) and purified by HPLC (Magellen C₈(2) 150x10mm column; a gradient mobile phase was used, 5:95 (by volume) to 95:5 (by volume) acetonitrile:(0.1% trifluoroacetic acid in water).
Retention time: 5.69 minutes.
LRMS (electrospray) : m/z [MH⁺] 434.

### EXAMPLE 208

### [4-(3,5-Dichlorophenoxy)-3-methyl-1H-pyrazol-5-yl]acetonitrile

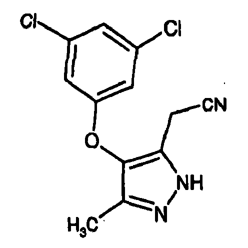

The pyrazole of Preparation 8 (1.00g, 2.60mmol) in tetrahydrofuran (10ml) was added in one portion to a solution of sodium cyanide (284mg, 5.20mmol) in water (10ml) at room temperature. The reaction was heated at 80°C for 14 hours and cooled to room temperature. The solvent was removed under reduced pressure and the resulting brown solid was dissolved in dichloromethane (50ml) and water (50ml). The organic layer was separated, washed with water (50ml), brine (30ml), dried over magnesium sulphate, filtered and the solvent removed under reduced pressure to give a brown solid. The product was purified by flash chromatography on silica gel eluting with pentane:ethyl acetate (50:50, by volume) to give the title compound as a yellow solid (500mg), m.p. 150-152°C.
¹H NMR (400MHz, CDCl₃): δ = 2.17 (s, 3H), 3.56 (s, 2H), 6.77 (s, 2H), 7.02 (s, 1 H).
LRMS (thermospray) : m/z [MH⁺] 282.

### EXAMPLE 209

### 1-{[4-(3,5-Dichlorophenoxy)-3-methyl-1H-pyrazol-5-yl]acetyl}piperidine

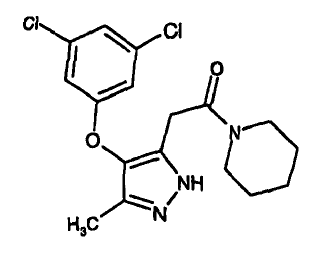

Standard solutions: The acid of Preparation 92 (680mg, 2.16mmol) and *1H-*benzotriazol-1-yl)-*N,N,N',N'*-tetramethyluronium hexafluorophosphate (761 mg, 3.23mmol) were separately dissolved in *N,N*-dimethylacetamide:triethylamine (96:4) (2x17ml).
Piperidine (3mg, 0.031 mmol) was treated with the standard solutions of the acid and coupling reagents (250µl of each) in a 96 well plate and the mixture was shaken for 14 hours at 80°C. The solvent was removed under reduced pressure and the mixture dissolved in dimethylsulphoxide (500µl) and purified by HPLC (Magellen C₁₈(2) 150x10mm column; a gradient mobile phase was used, 5:95 (by volume) to 95:5 (by volume) acetonitrile: (0.1% trifluoroacetic acid in water).
Retention time: 4.7 minutes.
LRMS (electrospray) : m/z [MH⁺] 368.

### EXAMPLES 210-217

The compounds of the following tabulated Examples of the general formula: 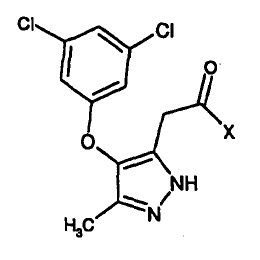 were performed by a similar method to that of Example 209 using the appropriate amine.

| Example No. | X | HPLC retention times / min | LRMS (electrospray) m/z [MH⁺] |
|---|---|---|---|
| 210 | 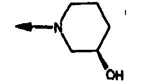 | 3.9 | 384 |
| 211 | 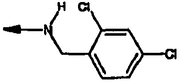 | 5.5 | 459 |
| 212 | 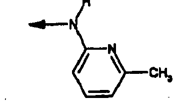 | 5.4 | 476 |
| 213 | 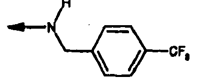 | 5.3 | 458 |
| 214 | 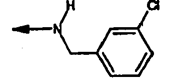 | 5.1 | 424 |
| 215 | 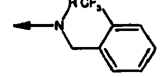 | 5.3 | 458 |
| 216 | 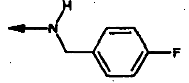 | 4.9 | 408 |
| 217 | 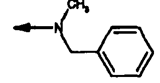 | 5.2 | 404 |

### EXAMPLE 218

### 3-chloro-5-[(5-{[(2-chlorobenzyl)amino]methyl}-3-methyl-1H-pyrazol-4-yl)oxy]benzonitrile

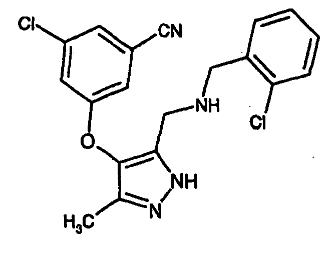

Standard solutions: The bromide of Preparation 18 (850mg, 2.30mmol) was dissolved in *N*-methylpyrolidinone (43ml).
2-Chlorobenzylamine (19mg, 0.13mmol) in a 96 well plate was treated with the solution of the bromide of Preparation 18 (500µl) and the mixture was shaken for 14 hours at 80°C. The solvent was removed under reduced pressure and the mixture dissolved in dimethylsulphoxide (500µl) and purified by HPLC (Magellen C₈(2) 150x10mm column; a gradient mobile phase was used, 5:95 (by volume) to 95:5 (by volume) acetonitrile:(0.1 % trifluoroacetic acid in water).
Retention time: 5.3 minutes.
LRMS (electrospray) : m/z [MH⁺] 386.

### EXAMPLES 219-249

The compounds of the following tabulated Examples of the general formula: 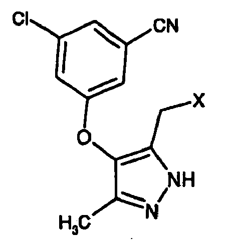 were performed by a similar method to that of Example 218 using the appropriate amine.

| Example No. | X | HPLC retention times / min | LRMS (electrospray) m/z [MH⁺] |
|---|---|---|---|
| 219 | 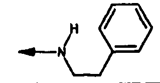 | 4.2 | 367 |
| 220 | 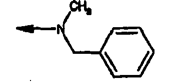 | 4.1 | 366 |
| 221 | 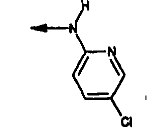 | 3.8 | 374 |
| 222 | 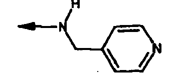 | 3.2 | 353 |
| 223 | 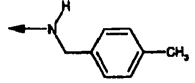 | 4.2 | 366 |
| 224 | 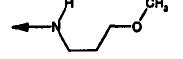 | 3.7 | 334 |
| 225 | 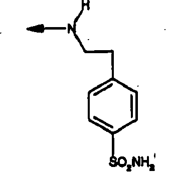 | 3.7 | 445 |
| 226 | 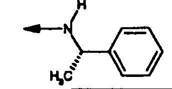 | 4.1 | 366 |
| 227 | 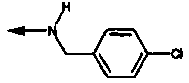 | 4.3 | 387 |
| 228 | 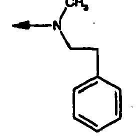 | 4.2 | 380 |
| 229 | 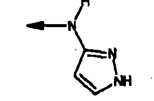 | 3.6 | 328 |
| 230 | 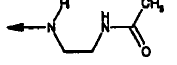 | 3.5 | 347 |
| 231 | 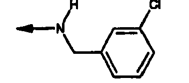 | 4.3 | 387 |
| 232 | 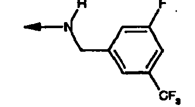 | 4.5 | 438 |
| 233 | 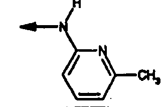 | 3.8 | 353 |
| 234 | 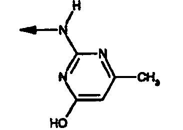 | 3.7 | 370 |
| 235 | 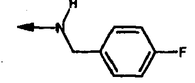 | 4.1 | 370 |
| 236 | 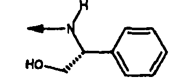 | 4.1 | 396 |
| 237 | 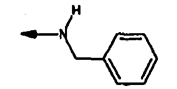 | 4.1 | 352 |
| 238 | 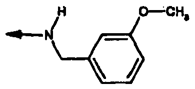 | 4.1 | 382 |
| 239 | 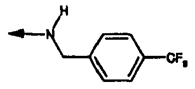 | 4.4 | 420 |
| 240 | 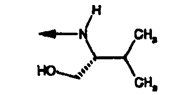 | 4.0 | 362 |
| 241 | 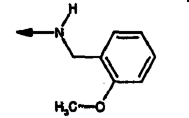 | 4.1 | 382 |
| 242 | 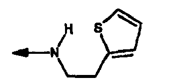 | 4.2 | 372 |
| 243 | 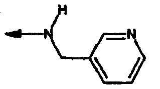 | 3.2 | 353 |
| 244 | 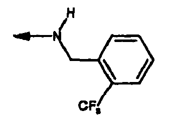 | 4.2 | 420 |
| 245 | 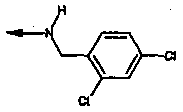 | 4.4 | 421 |
| 246 | 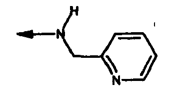 | 3.7 | 353 |
| 247 | 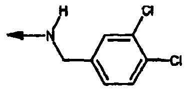 | 4.4 | 421 |
| 248 | 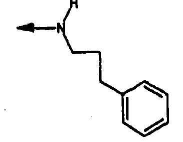 | 4.1 | 382 |
| 249 | 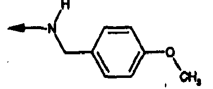 | 4.1 | 382 |

### EXAMPLE 250

### 3-{[3,5-Diethyl-1-(2-hydroxyethyl)-1H-pyrazol-4-yl]oxy}-5-(methylsulfanyl)benzonitrile

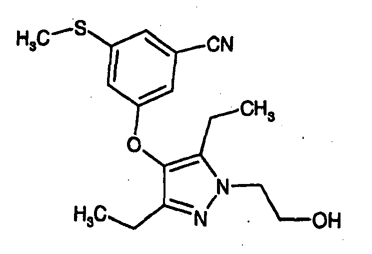

The protected alcohol from Preparation 93 (687mg, 1.65mmol) and p-toluene-sulphonic acid (32mg, 0.17mmol) were dissolved in methanol (16ml) and stirred under nitrogen at room temperature. After 4 hours a second portion of p-toluene-sulphonic acid (32mg, 0.17mmol) was added. After 18 hours the solvent was removed under reduced pressure and the residue was partitioned between saturated aqueous sodium bicarbonate solution (20ml) and dichloromethane (20ml). The aqueous phase was extracted with dichloromethane (40ml) and the combined organic extracts were dried over magnesium sulphate, concentrated under reduced pressure and purified by flash chromatography on silica gel eluting with dichloromethane:methanol (97:3, by volume) to provide the title compound (487mg) as a white solid. m.p. 72 °C.
¹H NMR (400MHz, CDCl₃): δ = 1.14 (m, 6H), 2.44 (q, 2H), 2.49 (s, 3H), 2.53 (q, 3H), 4.08 (m, 2H), 4.14 (m, 2H), 6.84 (s, 1 H), 7.00 (s, 1 H), 7.10 (s, 1H).
LRMS (electrospray) : m/z [MH⁺] 332.
Microanalysis: Found C, 61.36; H, 6.43; N, 12.55. C₁₇H₂₁N₃O₂S requires C, 61.61; H, 6.39; N, 12.68%.

### EXAMPLE 251

### 3-{[3,5-Diethyl-1-(2-hydroxyethyl)-1H-pyrazol-4-yl]oxy}-5-(methylsulfinyl)benzonitrile

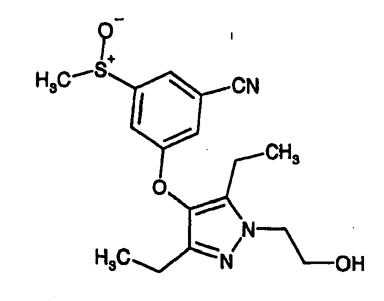

Wet alumina was prepared by adding water (1ml) to Brockman grade I alumina (5g). To a stirred solution of the sulphide from Example 250 (134mg, 0.40mmol) in dichloromethane (2ml) was added of wet alumina (400mg) followed by Oxone® (123mg, 0.4mmol) and the mixture was heated at reflux. After 1 hour a second portion of oxone (123mg, 0.40mmol) was added and the mixture was heated for a further 2 hours. After cooling to room temperature the reaction mixture was filtered and the resulting solids were washed with dichloromethane (20ml). The filtrate was concentrated and was purified by flash chromatography on silica gel eluting with dichloromethane:methanol (a gradient from 99:1 to 90:10, by volume) to provide the title compound (92mg) as an oil.
¹H NMR (400MHz, CDCl₃): δ =1.12 (m, 6H), 2.44 (q, 2H), 2.53 (q, 2H), 2.73 (s, 3H), 4.06 (m, 2H), 4.18 (m, 2H), 7.24 (s, 1 H), 7.45 (s, 1 H), 7.49 (s, 1H).
LRMS (electrospray) : m/z [M+Na⁺] 370.

### EXAMPLE 252

### 3-{[3,5-Diethyl-1-(2-hydroxyethyl)-1H-pyrazol-4-yl]oxy}-5-(methylsulfonyl)benzonitrile

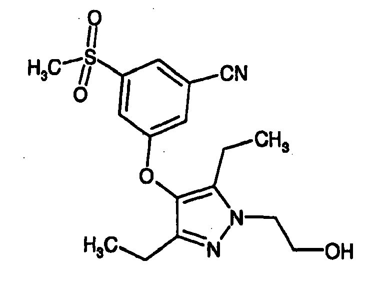

To a stirred solution of the sulphide from Example 250 (133mg, 0.4mmol) in dichloromethane (2ml) at -78°C was added a solution of meta-chloroperoxybenzoic acid (138mg of 50% by weight mixture, 0.4mmol) in dichloromethane (2ml). The cooling bath was removed and the solution was stirred at room temperature for 4 hours. The mixture was quenched by addition of saturated aqueous sodium bicarbonate solution (6ml) and extracted with dichloromethane (3x5ml). The combined organic components were dried over magnesium sulphate and concentrated. Analysis of the ¹H NMR (400MHz, CDCl₃) suggested a mixture of the desired product and the sulphoxide from Example 251. The crude product mixture was dissolved in dichloromethane (2ml), cooled to -78°C and to this was added meta-chloroperoxybenzoic acid (138mg of 50% by weight mixture, 0.4mmol) in dichloromethane (2ml). The cooling bath was removed and the mixture was stirred at room temperature for 1 hour. The mixture was quenched by addition of saturated aqueous sodium bicarbonate solution (6ml) and extracted with dichloromethane (3x5ml). The combined organic components were dried over magnesium sulphate and concentrated. The crude product mixture was purified by flash chromatography on a silica gel eluting with dichloromethane:methanol (98:2, by volume) to provide the title compound contaminated with meta-chloroperxoybenzoic acid. To a solution of this crude product in dichloromethane at -78°C was added dimethylsulphoxide (30µl, 0.4mmol). The cooling bath was removed and the mixture was stirred at room temperature for 15 minutes. The mixture was quenched by addition of 10% aqueous potassium carbonate solution (10ml) and the dichloromethane was evaporated. The remaining aqueous mixture was then extracted with diethyl ether (2x10ml) and ethyl acetate (10ml). The organic components were combined, dried over magnesium sulphate and concentrated to give the crude product mixture which was purified by flash chromatography on a silica gel eluting with dichloromethane:methanol (98:2, by volume) to provide the title compound (26mg) as a white solid. m.p. 133 °C.
¹H NMR (400MHz, CDCl₃): δ = 1.10 (m, 6H), 2.39 (q, 2H), 2.51 (q, 2H), 3.06 (s, 3H), 4.05 (m, 2H), 4.10 (m, 2H), 7.39 (s, 1 H), 7.67 (s, 1 H), 7.84 (s, 1H).
LRMS (electrospray) : m/z [M+Na⁺] 385.
HRMS: [MH⁺] 364.1329. C₁₈H₂₀N₆O₂ requires 364.1326.

### EXAMPLE 253

### 3-{[3,5-Diethyl-1-(2-hydroxyethyl)-1H-pyrazol-4-yl]oxy}-5-[2-(dimethylamino)ethoxy]benzonitrile

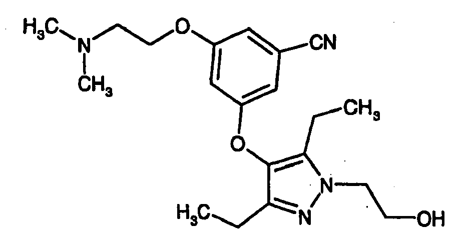

To a stirred solution of the protected alcohol from Preparation 94 (180mg, 0.39mmol) in methanol (4ml) was added para-toluenesulphonic acid (89mg, 0.47mmol). After 18 hours at room temperature the solvent was evaporated under reduced pressure and the residue was partitioned between dichloromethane (5ml) and 10% aqueous potassium carbonate solution (5ml). The aqueous phase was separated and extracted with a dichloromethane (3ml). The organic components were combined, dried over magnesium sulphate and concentrated under reduced pressure. The crude product mixture was purified by flash chromatography on silica gel eluting with dichloromethane:methanol (95:5, by volume) followed by dichloromethane:methanol:ammonia (80:20:1, by volume) to provide the title compound (63mg) as an oil.
¹H NMR (400MHz, CDCl₃): δ =1.13 (m, 6H), 2.43 (m, 8H), 2.52 (q, 2H), 2.85 (m, 2H), 3.81 (broad s, 1 H), 4.08 (m, 6H), 6.70 (s, 1 H), 6.78 (s, 1 H), 6.81 (s, 1H).
LRMS (APCI) : m/z [MH⁺] 373.
HRMS: [MH⁺] 373.2234. C₂₀H₂₉N₄O₃ requires 373.2234.

### EXAMPLES 254-256

The compounds of the following tabulated Examples of the general formula: 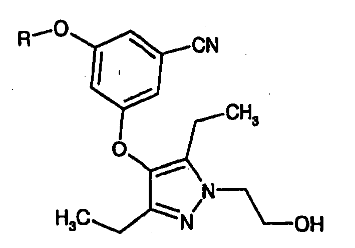 were performed by a similar method to that of Example 253 using as starting material the appropriate protected alcohol (PA) from Preparations 95-97.

| Example No. | PA prep No. | R | Analytical Data |
|---|---|---|---|
| 254 | 95 | CH₂CH₂NHMe | ¹H NMR (400MHz, CDCl₃): δ = 1.13 (m, 6H), 2.42 (q, 2H), 2.53 (q, 2H), 2.59 (s, 3H), 3.12 (t, 2H), 4.05 (m, 2H), 4.09 (m, 2H), 4.16 (t, 2H), 6.75 (s, 1 H), 6.81 (s, 1 H), 6.82 (s, 1 H). LRMS (APCI): m/z [MH⁺] 359 HRMS: [MH⁺] 359.2083. C₁₉H₂₇N₄O₃ requires 359.2078. |
| 255 | 96 | CH₂CONH₂ | ¹H NMR (400MHz, CDCl₃): δ = 1.11 (m, 6H), 2.41 (q, 2H), 2.52 (q, 2H), 4.05 (t, 2H), 4.09 (t, 2H), 4.46 (s, 2H), 5.74 (broad s, 1 H), 6.42 (broad s, 1 H), 6.69 (s, 1 H), 6.85 (s, 2H). LRMS (APCI): m/z 359 (MH⁺) |
| 256 | 97 | CH₂CH₂OCH₃ | ¹H NMR (400MHz, CDCl₃): δ = 1.12 (m, 6H), 2.42 (q, 2H), 2.51 (q, 2H), 3.44 (s, 3H), 3.73 (t, 2H), 4.09 (m, 6H), 6.71 (s, 1 H), 6.77 (s, 1H), 6.83 (s, 1H). LRMS (electrospray): m/z 360 (MH⁺) HRMS: [MH⁺] 360.1920. C₁₉H₂₆N₃O₄ requires 360.1918. |

### EXAMPLE 257

### 3-{[1-(2-Aminoethyl)-3,5-diethyl-1H-pyrazol-4-yl]oxy}-5-methoxybenzonitrile

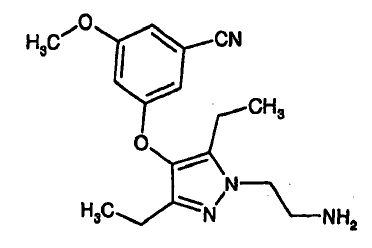

The alcohol from Example 202 (87mg, 0.28mmol), triphenylphosphine (220mg, 0.84mmol) and phthalimide (124mg, 0.84mmol) were dissolved in tetrahydrofuran (5ml) at 0°C under nitrogen and diisopropylazodicarboxylate (165µl, 0.84mmol) dissolved in tetrahydrofuran (1ml) was added dropwise. The reaction was allowed to warm to room temperature and was stirred for 18 hours. The solvent was removed under reduced pressure, the residue was dissolved in ethanol (6ml) and hydrazine hydrate (68µl, 1.40mmol) was added. The slurry was stirred for 48 hours at room temperature under nitrogen, concentrated under reduced pressure and the residue was dissolved in methanol. The solution was then passed through an SCX column eluting with methanol to remove impurities, then 2M ammonia in methanol solution to elute the product. The product was then purified by flash chromatography on silica gel eluting with dichloromethane:methanol (95:5) then dichloromethane:methanol:0.88 ammonia (90:10:1, by volume) to provide the title compound (67mg) as an oil.
¹H NMR (400MHz, CDCl₃): δ = 1.13 (m, 6H), 2.19 (broad s, 2H), 2.43 (q, 2H), 2.54 (q, 2H), 3.19 (t, 2H), 3.60 (s, 3H), 4.06 (t, 2H), 6.68 (s, 1H), 6.73 (s, 1H), 6.80 (s, 1 H).
LRMS (electrospray): m/z 315 (MH⁺)
HRMS: [MH⁺] 315.1819. C₁₇H₂₃N₄O₂ requires 315.1816.

### EXAMPLE 258

### 3-{[1-(2-Aminoethyl)-3,5-diethyl-1H-pyrazol-4-yl]oxy}-5-(1H-pyrazol-1-yl)benzonitrile

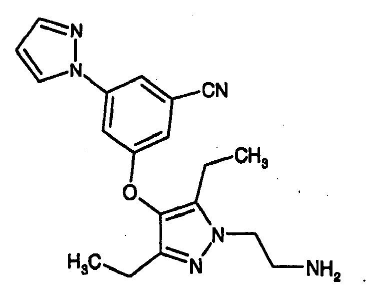

The alcohol from Example 164 (162mg, 0.46mmol), triphenylphosphine (362mg, 1.38mmol) and phthalimide (203mg, 1.38mmol) were dissolved in tetrahydrofuran (8ml) at 0°C under nitrogen and diisopropylazodicarboxylate (272µl, 1.38mmol) dissolved in tetrahydrofuran (1ml) was added dropwise. The reaction was allowed to warm to room temperature and was stirred for 18 hours. The solvent was removed under reduced pressure, the residue was dissolved in ethanol (9ml) and hydrazine hydrate (112µl, 2.3mmol) was added. The slurry was stirred for 48 hours at room temperature under nitrogen, concentrated under reduced pressure and the residue was dissolved in methanol. The solution was then passed through an SCX column eluting with methanol to remove impurities, then 2M ammonia in methanol solution to elute the product. The product was then purified by flash chromatography on silica gel eluting with dichloromethane:methanol (95:5) then dichloromethane:methanol:0.880 ammonia (90:10:1, by volume) to provide the title compound (62mg) as an oil.
¹H NMR (400MHz, CD₃OD): δ = 1.15 (m, 6H), 2.46 (q, 2H), 2.63 (q, 2H), 3.13 (t, 2H), 4.13 (t, 2H), 6.54 (s, 1 H), 7.17 (s, 1 H), 7.69 (s, 1 H), 7.72 (s, 1 H), 7.82 (s, 1 H), 8.32 (s, 1 H).
LRMS (APCI): m/z 351 (MH⁺)
HRMS: [MH⁺] 351.1929. C₁₉H₂₂N₄O₂ requires 351.1928.

### EXAMPLE 259

### 3,5-Dichlorophenyl-3-methyl-5-[(3-methyl-1,2,4-oxadiazol-5-yl)methyl]-1H-pyrazol-4-yl ether

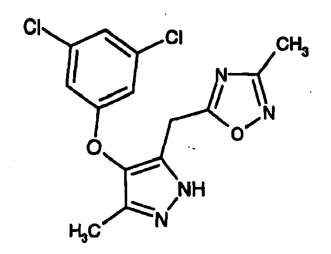

To a stirred solution of the acid (100mg, 0.33mmol) from Preparation 92 in dimethylformamide (2ml) was added carbonyldiimidazole (59mg, 0.36mmol) in one portion. After 30 minutes at room temperature (1*Z*)-*N*'-hydroxyethanimidamide (27mg, 0.36mmol) was added and the reaction mixture was stirred at room temperature for 3 hours. A second portion of carbonyldiimidazole (59mg, 0.36mmol) was added and the mixture was heated at 100°C for 12 hours. After cooling to room temperature water (30ml) was added and the mixture was extracted with ethyl acetate (3 x 20ml). The combined organic components were dried over magnesium sulphate and concentrated under reduced pressure to give a brown oil. The crude product mixture was purified by flash chromatography on silica gel eluting with ethyl acetate:pentane (30:70, by volume) to provide the title compound (40mg) as a pale yellow oil.
¹H NMR (400MHz, CDCl₃): δ = 2.12 (s, 3H), 2.29 (s, 3H), 4.08 (s, 2H), 6.74 (s, 2H), 6.98 (s, 1H).

LRMS (electrospray): m/z 339 (MH⁺)

### EXAMPLE 260

### 3-Fluoro-5-{[1-(2-hydroxyethyl)-5-methyl-3-(trifluoromethyl)-1H-pyrazol-4-yl]oxy}benzonitrile

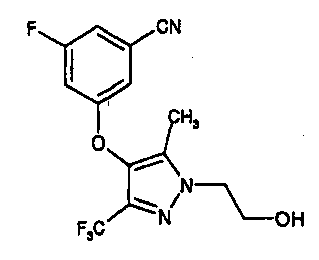

To a stirred solution of the protected alcohol (85mg, 0.21 mmol) from Preparation 99 in methanol (0.5ml) was added para-toluenesulphonic acid (4mg, 0.02mmol). After 5 hours the reaction mixture was concentrated under reduced pressure, dissolved in dichloromethane (20ml), washed with saturated sodium bicarbonate solution (20ml), dried over magnesium sulphate and concentrated under reduced pressure. The crude product mixture was purified by flash chromatography on silica gel eluting with pentane:ethyl acetate (60:40 followed by 40:60, by volume) to provide the title compound (54mg) as a white solid.
¹H NMR (400MHz, CDCl₃): δ = 2.19 (s, 3H), 2.45 (t, 1 H), 4.10 (m, 2H), 4.20 (m, 2H), 6.87 (d, 1 H), 6.96 (s, 1 H), 7.05 (d, 1 H).
LRMS (APCI): m/z 330 (MH⁺)
Microanalysis: Found C, 51.38; H, 3.52; N, 12.37. C₁₄H₁₁F₄N₃O₂ requires C, 51.07; H, 3.37; N, 12.76%.

### EXAMPLE 261

### 5-[(3,5-Diethyl-1-{2-[(2-methoxyethoxy)methoxy]ethyl}-1H-pyrazol-4-yl)oxy]isophthalonitrile

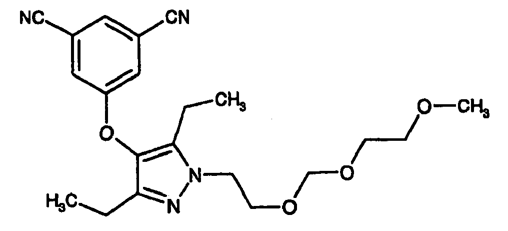

To a stirred solution of the alcohol (5.0g, 16.11mmol) from Example 119 in tetrahydrofuran (65ml) at 0°C was added 2-methoxyethoxymethylchloride (2.39ml, 20.94mmol) followed by sodium hydride (838mg of a 60% by weight dispersion in oil, 20.94mmol). After 10 minutes the reaction mixture was heated at 50°C for 18 hours. After cooling to room temperature, the mixture was diluted with saturated aqueous ammonium chloride solution dropwise (3ml). The mixture was concentrated under reduced pressure and the residue was partitioned between dichloromethane (250ml) and water (200ml). The aqueous phase was separated and extracted with dichloromethane (150ml). The organic components were combined, dried over magnesium sulphate and concentrated under reduced pressure. The crude product mixture was purified by flash chromatography on silica gel eluting with dichloromethane, followed by dichloromethane:methanol (99:1, by volume) to provide the title compound (5.38g) as a colourless oil.
¹H NMR (400MHz, CDCl₃): δ = 1.10 (m, 6H), 2.39 (q, 2H), 2.55 (q, 2H), 3.38 (s, 3H), 3.51 (m, 2H), 3.56 (m, 2H), 3.93 (t, 2H), 4.20 (t, 2H), 4.66 (s, 2H), 7.38 (s, 2H), 7.56 (s, 1 H).
LRMS (APCI): m/z 399 (MH⁺)
Microanalysis: Found C, 62.11; H, 6.67; N, 13.51. C₂₁H₂₆N₄O₄+0.43H₂O requires C, 62.09; H, 6.67; N, 13.79%.

### EXAMPLE 262

### 3-Cyano-5-{[3,5-diethyl-1-(2-hydroxyethyl)-1H-pyrazol-4-yl]oxy}benzamide

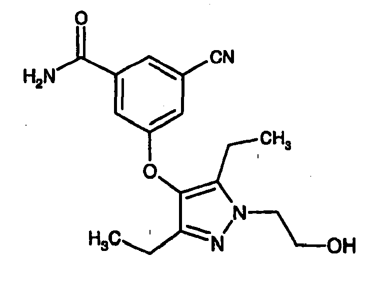

To a stirred solution of the pyrazole (60mg) from Preparation 100 in dichloromethane (4ml) was added aluminium trichloride (134mg, 1mmol). After 18 hours, ice was added, the mixture was neutralised using saturated aqueous sodium bicarbonate solution, diluted with water (30ml) and extracted with dichloromethane (2x40ml). The organic components were combined, dried over magnesium sulphate and concentrated under reduced pressure. The crude product mixture was purified by flash chromatography on silica gel eluting with dichloromethane:methanol (95:5, by volume) to provide the title compound (27mg) as a colourless glass.
¹H NMR (400MHz, CDCl₃): δ = 1.10 (m, 6H), 2.40 (q, 2H), 2.52 (q, 2H), 4.07 (m, 4H), 7.25 (s, 1 H), 7.60 (s, 1 H), 7.65 (s, 1 H).
LRMS (APCI): m/z 329 (MH⁺)

### EXAMPLE 263

### 5-{[5-Ethyl-3-(1-hydroxyethyl)-1H-pyrazol-4-yl]oxy}isophthalonitrile

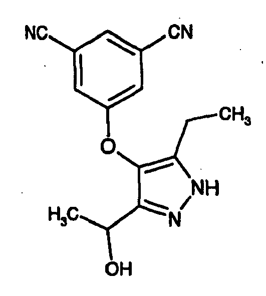

To a stirred solution of the pyrazole from Preparation 102 (219mg, 0.57mmol) in tetrahydrofuran (2.5ml) was added saturated aqueous sodium carbonate solution (0.5ml). The reaction mixture was stirred at room temperature for 4 hours and then heated at reflux for 18 hours. The reaction mixture was concentrated under reduced pressure and the residue was partitioned between dichloromethane (20ml) and water (20ml). The organic phase was dried over magnesium sulphate and concentrated under reduced pressure. The crude product mixture was purified by flash chromatography on silica gel eluting with dichloromethane:methanol (a gradient from 100:0 to 90:10, by volume) to provide the title compound (68mg) as a white solid.
¹H NMR (400MHz, CDCl₃): δ =1.21 (t, 3H), 1.51 (d, 3H), 2.54 (q, 2H), 4.89 (q, 1 H), 7.25 (s, 2H), 7.43 (s, 1 H).

LRMS (APCI): m/z 283 (MH⁺)

### EXAMPLE 264

### 5-{[5-Ethyl-3-(1-hydroxyethyl)-1-(2-hydroxyethyl)-1H-pyrazol-4-yl]oxy}isophthalonitrile

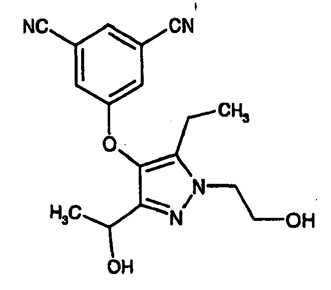

To a stirred solution of the pyrazole from Preparation 103 (80mg, 0.19mmol) in methanol (1ml) was added para-toluenesulphonic acid (4mg, 0.02mmol). After 5 hours at room temperature the reaction mixture was concentrated under reduced pressure and the residue was partitioned between dichloromethane (20ml) and water (20ml). The organic component was dried over magnesium sulphate and concentrated under reduced pressure. The crude product mixture was purified by flash chromatography on silica gel eluting with dichloromethane:methanol (a gradient from 100:0 to 95:5, by volume) to provide the title compound (44mg) white solid.
¹H NMR (400MHz, CDCl₃): δ =1.11 (t, 3H), 1.46 (d, 3H), 2.54 (q, 2H), 4.10 (q, 2H), 4.17 (q, 2H), 4.79 (q, 1 H), 7.44 (s, 2H), 7.57 (s, 1H).

LRMS (APCI): m/z 327 (MH⁺)

### EXAMPLE 265

### 3-{[3,5-Diethyl-1-(2-hydroxyethyl)-1H-pyrazol-4-yl]oxy}-5-(5-trifluoromethyl-1,2,4-oxadiazol-3-yl)benzonitrile

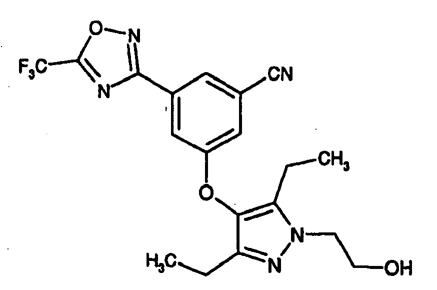

To a stirred solution of the pyrazole from Preparation 105 (235mg, 0.46mmol) in dichloromethane (2ml) was added aluminium trichloride (373mg, 2.8mmol). The reaction mixture was stirred at room temperature for 48 hours, diluted with water (6ml) and extracted with dichloromethane (6ml). The organic component was concentrated under reduced pressure and purified by flash chromatography on silica gel eluting with dichloromethane:methanol (a gradient from 99:1 to 80:20, by volume) followed by dichloromethane:methanol:0.88 ammonia (80:20:1, by volume) to provide an impure sample of the title compound (44mg) as a white solid. The product was further purified by HPLC using a Phenomonex Luna C₁₈ 150x21.2mm column eluting with a solvent gradient of 5:95 0.1 % aqueous trifluoroacetic acid in acetonitrile:acetonitrile (0-1min 80:20; 1-7min 80:20 changing to 0:100; 7-12min 0:100; 12-12.1min 0:100 changing to 80:20; 12.1-15min 80:20) to provide the title compound (38mg) as a white solid.
Retention time 5.7minutes.
LRMS (electrospray): m/z 422 (MH⁺)

### EXAMPLES 266-268

The compounds of the following tabulated Examples of the general formula: 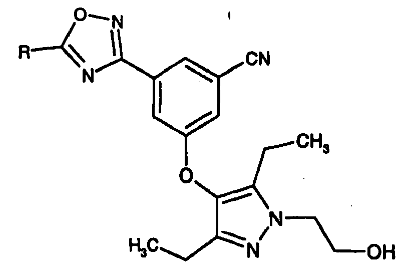 were prepared by a similar method to that of Example 265 using the appropriate protected alcohol (PA) from Preparation 106-108.

| Example No. | PA prep No. | R | Analytical Data |
|---|---|---|---|
| 266 | 106 | Me | Retention time 4.8 minutes LRMS (electrospray): m/z [MH⁺] 368 |
| 267 | 107 | Et | Retention time 5.3 minutes LRMS (electrospray): m/z [MH⁺] 382 |
| 268 | 108 | ⁱPr | Retention time 5.7 minutes LRMS (electrospray): m/z 396 (MH⁺) |

### EXAMPLE 269

### 5-[({[4-(3-Chloro-5-cyanophenoxy)-3-methyl-1H-pyrazol-5-yl]methyl}amino)methyl]nicotinamide

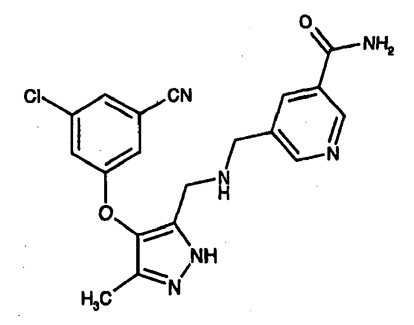

To a stirred solution of the amine from Preparation 111 (650mg, 1.70mmol) in isopropyl alcohol (6ml) was added the pyrazole from Preparation 18 (210mg, 0.57mmol) followed by potassium carbonate (240mg, 1.70mmol). The reaction mixture was heated at reflux for 1.5 hours. After cooling to room temperature the mixture was concentrated under reduced pressure and the crude product mixture was purified by flash chromatography on silica gel eluting with dichloromethane:methanol:0.88 ammonia (95:5:0.5 then 90:10:1 then 80:20:1, by volume) which gave an impure sample of the desired product. Flash chromatography was repeated eluting with dichloromethane:methanol:0.88 ammonia (100:0:0 then 95:5:0.5 then 90:10:1, by volume) to provide the title compound (10mg) as a pale yellow solid.
¹H NMR (400MHz, CD₃OD): δ = 2.05 (s, 3H), 3.62 (s, 2H), 3.79 (s, 2H), 7.16 (m, 1 H), 7.18 (m, 1 H), 7.38 (s, 1 H), 8.15 (s, 1 H), 8.54 (s, 1 H), 8.84 (s, 1H).
LRMS (APCI): m/z 419 (M+Na⁺)
HRMS: [MH⁺] 397.1173. C₁₉H₁₈N₆O₂Cl requires 397.1175.

### EXAMPLE 270

### 2-[({[4-(3-Chloro-5-cyanophenoxy)-3-methyl-1H-pyrazol-5-yl]methyl}amino)methyl]isonicotinamide

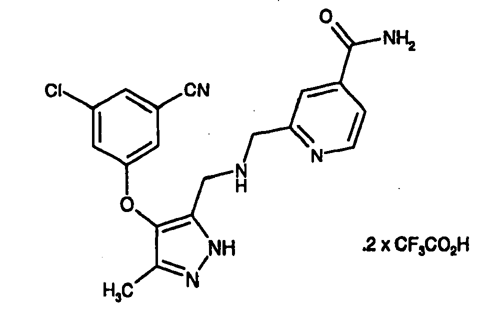

To a stirred suspension of the amine from Preparation 115 (250mg, 1.66mmol) and the pyrazole from Preparation 18 (155mg, 0.42mmol) in isopropanol (6ml) was added tetrahydofuran (2ml). The mixture was heated at reflux for 2 hours after which the reaction mixture was concentrated under reduced pressure. The crude product mixture was purified by flash chromatography on silica gel eluting with dichloromethane:methanol:0.88 ammonia (85:15:1, by volume) to provide an impure sample of the title compound. The product was further purified by HPLC using a Phenomonex Luna C₈(II) 10µM 150x21.2mm column eluting with a solvent gradient of 5:95 0.1% aqueous trifluoroacetic acid in acetonitrile:acetonitrile (0-6min 95:5 changing to 0:100; 6-10min 0:100) to provide the title compound (65mg) as an off-white solid.
Retention time: 3.40 minutes
¹H NMR (400MHz, CD₃OD): δ = 2.14 (s, 3H), 4.21 (s, 2H), 4.50 (s, 2H), 7.19 (s, 1 H), 7.27 (m, 1 H), 7.43 (m, 1 H), 7.48 (m, 1 H), 7.78 (m, 1 H), 8.68 (d, 1 H)
LRMS (electrospray): m/z 397 (MH⁻)
Microanalysis: Found C, 44.56; H, 3.41; N, 14.07. C₁₉H₁₇N₆O₂Cl+1.9.CF₃CO₂H requires C, 44.64; H, 3.11; N, 13.70%.

### EXAMPLE 271

### Di(tert-butyl) 2-[4-(3,5-dicyanophenoxy)-3,5-diethyl-1H-pyrazol-1-yl]ethyl phosphate

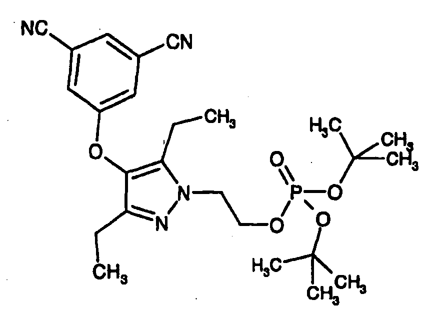

To a stirred solution of the alcohol from Example 119 (500mg, 1.60mmol) in dichloromethane (5ml) was added tetrazole (226mg, 3.20mmol) followed by di-*tert-*butyl-N,N-diisopropylphosphoramidite (1.02ml, 3.20mmol). After stirring for 4 hours at room temperature the reaction mixture was cooled to 0°C and meta-chloroperoxybenzoic acid (1.0g of 50% by weight mixture, 3mmol) was added portionwise (CARE, EXOTHERM). After 10 minutes the mixture was warmed to room temperature and was diluted with dichloromethane (50ml). The solution was washed with saturated aqueous sodium carbonate solution (20ml) and the aqueous component was separated and extracted with dichloromethane (20ml). The combined organic components were washed with brine (20ml), dried over magnesium sulphate and concentrated under reduced pressure. The crude product mixture was purified by flash chromatography on silica gel eluting with dichloromethane:methanol:0.88 ammonia (100:0:0 then 99:1:0.1 then 98:2:0.2, by volume) to provide a sample of the title compound (660mg)
¹H NMR (400MHz, CDCl₃): δ = 1.10 (m, 6H), 1.43 (s, 18H), 2.38 (q, 2H), 2.55 (q, 2H), 4.26 (m, 4H), 7.38 (s, 2H), 7.54 (s, 1H).
LRMS (electrospray): m/z 525 (MH⁺)
Microanalysis: Found C, 57.77; H, 7.38; N, 10.33. C₂₅H₃₅N₄O₅P+H₂O requires C, 57.68; H, 7.16; N, 10.76%.

### EXAMPLE 272

### 2-[4-(3,5-Dicyanophenoxy)-3,5-diethyl-1H-pyrazol-1-yl]ethyl dihydrogen phosphate

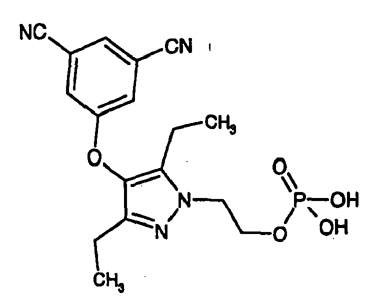

To a stirred solution of the phosphate ester from Example 271 (250mg, 0.48mmol) in dichloromethane (10ml) at 0°C was added trifluoroacetic acid (0.5ml). The reaction mixture was allowed to warm to room temperature and after 4 hours it was concentrated under reduced pressure. The residue was purified by HPLC using a Phenomonex Luna C₈(II) 10µM 150x21.2mm column eluting with a solvent gradient of 5:95 0.1% aqueous trifluoroacetic acid in acetonitrile:acetonitrile (0-1.9min 95:5; 2-10min 90:10 changing to 30:70; 10.0-13.8min 30:70; 13.8-13.9min 30:70 changing to 95:5; 13.9-15min 95:5) to give a sample of the desired product. This sample was further purified by recrystallisation using acetonitrile/water which gave the title compound as a white solid, m.p. 198-199 °C.
Retention time: 2.31 minutes.
¹H NMR (400MHz, CD₃OD): δ = 1.09 (m, 6H), 2.35 (q, 2H), 2.61 (q, 2H), 4.28 (m, 4H), 7.55 (s, 2H), 7.79 (s, 1 H).
LRMS (APCI): m/z 391 (MH⁺)
Microanalysis: Found C, 50.99; H, 4.92; N, 14.06. C₁₇H₁₉N₄O₅P+0.5H₂O requires C, 51.13; H, 5.05; N, 14.03%.

### EXAMPLE 273

### 5-{[3,5-Diethyl-1-(2-hydroxyethyl)-1H-pyrazol-4-yl]oxy}isophthalonitrile sulfate salt

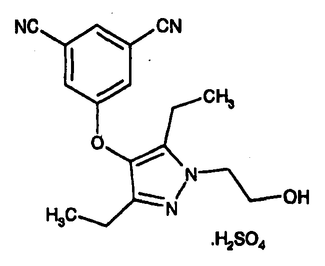

To a stirred solution of the pyrazole from Example 119 (200mg, 0.65mmol) in acetone (5ml) was added sulfuric acid (0.32ml of a 2M aqueous solution, 0.64mmol) and the mixture was stirred at room temperature and the solvent allowed to evaporate. The residue was recrystallised (toluene/acetone) to give the title compound (160mg) as a white powder, m.p. 105-110°C.
¹H NMR (400 MHz, CDCl₃): δ = 1.22 (m, 6H), 2.70 (m, 4H), 4.12 (bs, 1 H), 4.59 (m, 2H), 4.75 (bs, 1 H), 7.66 (s, 1 H), 7.69 (m, 1 H), 7.72 (s, 1H).
Microanalysis: Found C, 50.29; H, 4.90; N, 13.48. C₁₇H₁₈N₄O₂.H₂SO₄ requires C, 49.99; H, 4.93; N, 13.72%.

### EXAMPLE 274

### 5-{[3,5-Diethyl-1-(2-hydroxyethyl)-1H-pyrazol-4-yl]oxy}isophthalonitrile benzenesulfonic acid salt

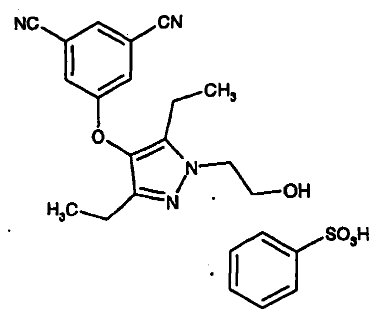

To a stirred solution of the pyrazole from Example 119 (20g, 65mmol) in acetone (200ml) was added benzenesulfonic acid (10.7g, 68mmol) and the mixture was stirred at room temperature for 10 minutes. The reaction mixture was concentrated under reduced pressure and the residue was recrystallised twice (acetone) to give the title compound (16.2g) as a white powder, m.p. 142-144°C.
¹H NMR (400 MHz, CDCl₃): δ = 1.05-1.08 (m, 6H), 2.59 (q, 2H), 2.68 (q, 2H), 4.04 (t, 2H), 4.54 (t, 2H), 7.35-7.42 (m, 3H), 7.55 (s, 1 H), 7.64 (s, 1 H), 7.86 (d, 2H).
Microanalysis: Found C, 58.86; H, 5.13; N, 11.88. C₂₃H₂₄N₄O₅S requires C, 58.96; H, 5.16; N, 11.96%.

### EXAMPLE 275

### 5-{[3,5-Diethyl-1-(2-hydroxyethyl)-1H-pyrazol-4-yl]oxy}isophthalonitrile tosylate salt

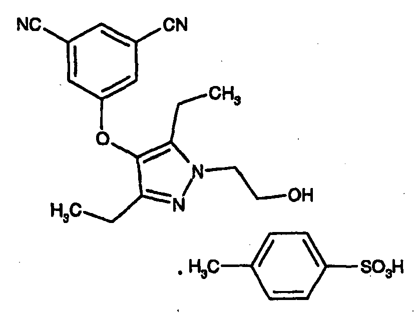

To a stirred suspension of the pyrazole from Example 119 (300mg, 1.00mmol) in ethanol (2ml) was added *p*-toluenesulfonic acid (202mg, 1.10mmol) and the mixture was heated on an oil bath until the solids dissolved. The reaction mixture was cooled to room temperature and concentrated under reduced pressure. The residue was crystallised (diethyl ether), filtered and recrystallised (isopropyl alcohol) to give the title compound (200mg) as a white solid, m.p. 120°C.
¹H NMR (400 MHz, DMSO-*d₆*): δ = 1.00 (m, 6H), 2.24 (m, 5H), 2.49 (m, 2H), 4.00 (m, 2H), 7.11 (d, 2H), 7.45 (d, 2H), 7.73 (s, 2H), 8.09 (s, 1H).
Microanalysis: Found C, 59.64; H, 5.46; N, 11.60. C₂₄H₂₆N₄O₅S requires C, 59.74; H, 5.43; N, 11.61%.

### EXAMPLE 276

### 5-{[3,5-Diethyl-1-(2-hydroxyethyl)-1H-pyrazol-4-yl]oxy}isophthalonitrile mesylate salt

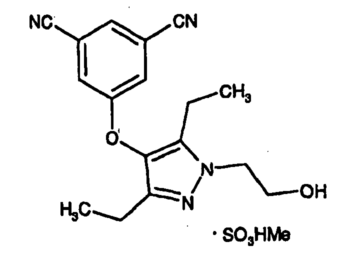

To a stirred suspension of the pyrazole from Example 119 (250mg, 0.83mmol) in isopropyl alcohol (3ml) was added methanesulfonic acid (52µl, 0.91mmol) and the mixture was heated on an oil bath until the solids dissolved. The reaction mixture was cooled to room temperature and concentrated under reduced pressure to a volume of 1 ml. A white solid precipitated out which was washed with cold isopropyl alcohol to give the title compound (239mg) as a white solid, m.p. 144-146°C.
¹H NMR (400 MHz, DMSO-*d*₆): δ =1.02 (m, 6H), 2.32 (q, 2H), 2.43 (s, 3H), 2.52 (m, 2H), 3.73 (m, 2H), 4.02 (m, 2H), 7.75 (s, 2H), 8.11 (s, 1 H).
Microanalysis: Found C, 53.20; H, 5.52; N, 13.68. C₁₈H₂₂N₄O₅S requires C, 53.19; H, 5.46; N, 13.78%.

### EXAMPLE 277

### 3-{[1-(2-Aminoethyl)-3,5-diethyl-1H-pyrazol-4-yl]oxy}-5-methylbenzonitrile bis-mesylate salt

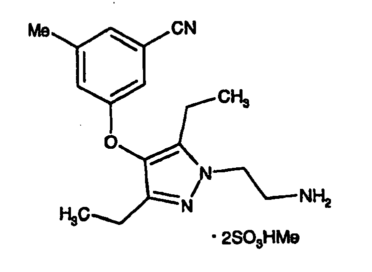

To a stirred solution of the amine from Example 125 (119mg, 0.40mmol) in ethanol (2ml) was added methanesulfonic acid (1.00ml of a 0.84M solution in ethanol, 0.84mmol). The reaction mixture was concentrated under reduced pressure to remove some of the ethanol. A mixture of diethyl ether and acetone were added and a white solid precipitated out which was filtered and washed (diethyl ether/acetone) to give the title compound (153mg) as a white solid, m.p. 146-148°C.
¹H NMR (400 MHz, CD₃OD): δ = 1.09 (m, 6H), 2.33 (s, 3H), 2.39 (q, 2H), 2.55 (q, 2H), 2.68 (s, 6H), 3.42 (t, 2H), 4.29 (t, 2H), 6.93 (s, 1 H), 7.06 (s, 1 H), 7.19 (s, 1H).
LRMS (thermospray): m/z [free base+H⁺] 299
Microanalysis: Found C, 45.83; H, 6.12; N, 11.27. C₁₉H₃₀N₄O₇S₂.0.50H₂O requires C, 45.68; H, 6.25; N, 11.21%.

### EXAMPLE 278

### 3-{[1-(2-Aminoethyl)-3,5-diethyl-1H-pyrazol-4-yl]oxy}-5-methylbenzonitrile phosphate salt

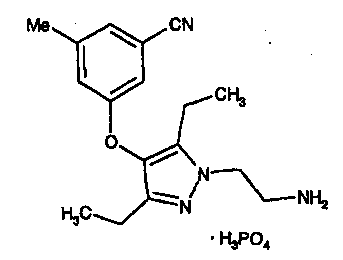

To a stirred solution of the amine from Example 125 (251 mg, 0.84mmol) in ethanol (5ml) was added phosphoric acid (63µl, 0.93mmol). The resulting precipitate was filtered, washed (ethanol then diethyl ether) and dried to give the title compound (265mg) as a white solid, m.p. 210-211°C.
¹H NMR (400 MHz, CD₃OD): δ = 1.08 (m, 6H), 2.32 (s, 3H), 2.39 (q, 2H), 2.56 (q, 2H), 3.39 (m, 2H), 4.29 (m, 2H), 6.93 (s, 1 H), 7.05 (s, 1 H), 7.18 (s, 1H).
LRMS (thermospray): m/z [free base+H⁺] 299
Microanalysis: Found C, 51.26; H, 6.36; N, 14.08. C₁₇H₂₅N₄O₅P requires C, 51.51; H, 6.36; N, 14.14%.

### EXAMPLE 279

### 3-{[1-(2-Aminoethyl)-3,5-diethyl-1H-pyrazol-4-yl]oxy}-5-methylbenzonitrile (L) tartrate salt

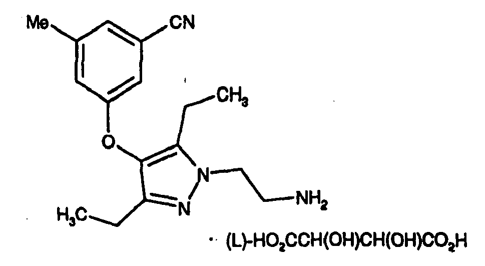

To a stirred solution of the amine from Example 125 (500mg, 1.68mmol) in acetone (15ml) was added (L)-tartaric acid (252mg, 1.68mmol) and the mixture was heated on an oil bath until complete dissolution had occurred. The mixture was cooled to room temperature and a white precipitate formed which was filtered and washed (acetone) to give the title compound (515mg) as a white powder, m.p. 159-161°C.
¹H NMR (400 MHz, CD₃OD): δ = 1.05-1.10 (m, 6H), 2.32 (s, 3H), 2.34-2.41 (m, 2H), 2.53-2.57 (m, 2H), 3.40 (m, 2H), 4.27 (m, 2H), 4.35 (s, 2H), 6.93 (s, 1 H), 7.05 (s, 1 H), 7.17 (s, 1H).
LRMS (electrospray): m/z [free base+H⁺] 299
Microanalysis: Found C, 54.80; H, 6.38; N, 12.11. C₂₁H₂₈N₄O₇.0.65H₂O requires C, 54.81; H, 6.42; N, 12.10%.

### EXAMPLE 280

### 3-{[1-(2-Aminoethyl)-3,5-diethyl-1H-pyrazol-4-yl]oxy}-5-methylbenzonitrile succinate salt

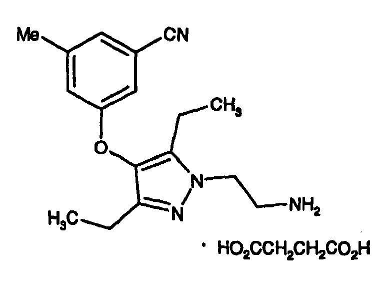

To a stirred solution of the amine from Example 125 (235mg, 0.79mmol) in acetone (7ml) was added succinic acid (93mg, 0.79mmol). After two minutes the mixture was concentrated to - 3ml using a stream of nitrogen gas which resulted in the formation of white crystals. The precipitate was filtered and washed (acetone) to give the title compound (172mg) as white crystals, m.p. 155°C.
¹H NMR (400 MHz, CD₃OD): δ = 1.03-1.07 (m, 6H), 2.34 (s, 3H), 2.40 (q, 2H), 2.50 (s, 4H), 2.59 (q, 2H), 3.34 (t, 2H), 4.23 (t, 2H), 6.95 (s, 1 H), 7.06 (s, 1H), 7.22 (s, 1H).
LRMS (electrospray): m/z [free base+H⁺] 299
Microanalysis: Found C, 60.47; H, 6.77; N, 13.39. C₂₁H₂₈N₄O₅ requires C, 60.56; H, 6.78; N, 13.45%.

### EXAMPLE 281

### 3-{[1-(2-Aminoethyl)-3,5-diethyl-1H-pyrazol-4-yl]oxy}-5-methylbenzonitrile (L) citrate salt

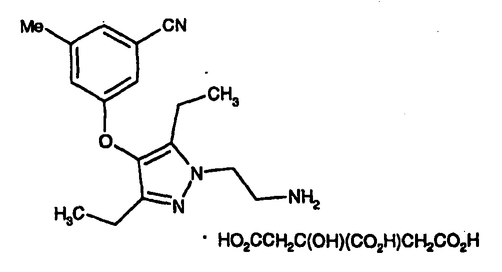

To a stirred solution of the amine from Example 125 (140mg, 0.47mmol) in acetone (3ml) was added citric acid (90mg, 0.47mmol). The mixture was stirred until complete dissolution had occurred. The mixture was concentrated to - 1 ml using a stream of nitrogen gas and cooled in a freezer for 1.5 hours. A precipitate collected which was filtered to give the title compound (149mg) as a white powder, m.p. 180-182°C.
¹H NMR (400 MHz, CD₃OD): δ = 1.04-1.07 (m, 6H), 2.35 (s, 3H), 2.40 (q, 2H), 2.58 (q, 2H), 2.73 (d, 2H), 2.80 (d, 2H), 3.42 (t, 2H), 4.30 (t, 2H), 6.95 (s, 1 H), 7.08 (s, 1H), 7.21 (s, 1 H).
LRMS (electrospray): m/z [free base+H⁺] 299
Microanalysis: Found C, 56.19; H, 6.20; N, 11.31. C₂₃H₃₀N₄O₈ requires C, 56.32; H, 6.16; N, 11.42%.

### EXAMPLE 282

### 5-{[3,5-Diethyl-1-(2-hydroxyethyl)-1H-pyrazol-4-yl]oxy}isophthalonitrile

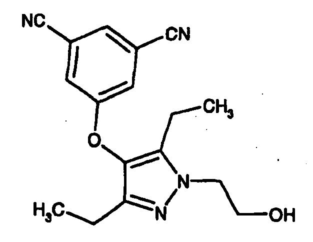

2-Hydroxyethylhydrazine (8.43ml, 124mmol) was added dropwise to a solution of the diketone of Preparation 45 (30.5g, 113mmol) in acetic acid (300ml) at room temperature under nitrogen. The reaction was stirred at room temperature for 90 minutes and the solvent removed under reduced pressure to give an orange solid. This was combined with an orange solid from another reaction carried out in an identical manner to this. The combined crude product was purified by flash column chromatography on silica gel eluting with ethyl acetate:pentane (75:25 by volume) to provide the title compound as a white solid. Analysis of the proton nmr showed minor impurities were present so the product was purified by flash column chromatography on silica gel eluting with ethyl acetate:pentane (50:50 by volume) to provide the title compound (50g) as a white solid, m.p. 125°C.
¹H-NMR (400MHz, CDCl₃): δ = 1.13 (6H, m), 2.40 (2H, q), 2.53 (2H, q), 3.53 (1H, m), 4.11 (4H, m), 7.40 (2H, s), 7.58 (1 H, s).
LRMS (electrospray): m/z [MH⁺] 311.
Microanalysis: Found: C, 65.62; H, 5.85; N, 18.04. C₁₇H₁₈N₄O₂ requires C, 65.64; H, 5.84; N, 18.05%.

### EXAMPLE 283

### 2-[4-(3,5-Dichlorophenoxy)-3-ethyl-1H-pyrazol-1-yl]ethylamine and 2-[4-(3,5-Dichlorophenoxy)-5-ethyl-1H-pyrazol-1-yl]ethylamine

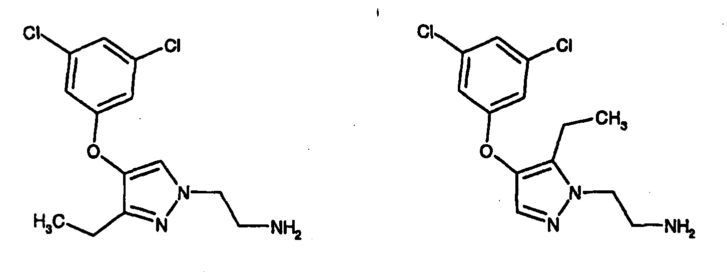

The pyrazole from Example 42 (1.03g, 4.00mmol) and 2-chloroethylamine hydrochloride (510mg, 4.40mmol) were heated as a melt at 150°C for 24 hours. The reaction was cooled and a solution of the residue in dichloromethane (100ml) was washed with an aqueous solution of 1 M potassium carbonate (50ml), brine (50ml), dried over magnesium sulphate, filtered and concentrated under reduced pressure. The crude product was purified by flash chromatography on silica gel eluting with dichloromethane:methanol:ammonia (93:7:1, by volume) to afford the title compounds (768mg) in a 85:15 ratio of regioisomers as a colourless oil.
¹H-NMR (400MHz, CDCl₃): δ = 1.16 (major, t, 3H), 1.16 (minor, t, 3H), 2.47 (major, q, 2H), 2.60 (minor, q, 2H), 3.13 (major, m, 2H), 3.13 (minor, m, 2H), 4.10 (major, m, 2H), 4.10 (minor, m, 2H), 4.24 (major, t, 2H), 4.24 (minor, t, 2H), 6.85 (major, s, 2H), 6.85 (minor, s, 2H), 7.02 (major, s, 1 H), 7.02 (minor, s, 1 H), 7.27 (major, s, 1 H), 7.31 (minor, s, 1H).
LRMS (thermospray): m/z [MH⁺] 300.

The following Preparations describe the preparation of certain intermediates used in the preceding Examples.

### PREPARATION 1

### 3-(3,5-Dichlorophenoxy)-2,4-pentanedione

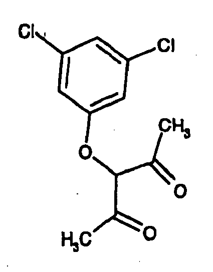

3-Chloro-2,4-pentanedione (183µL, 1.53mmol)) was added to a stirred suspension of 3,5-dichlorophenol (250mg, 1.53mmol) and potassium carbonate (233mg, 1.69mmol) in acetone (7.7ml) at room temperature under nitrogen. The mixture was stirred for 30 minutes and then heated under reflux for 3½ hours. After cooling, sodium iodide (230mg, 1.53mmol) was added and refluxing continued for a further 3½ hours. After cooling again the mixture was diluted with water (5ml) and concentrated under reduced pressure in a fumehood (Caution: possible residual lachrymator) to remove acetone. The resulting red aqueous solution was diluted with 2M hydrochloric acid (5ml) and extracted with dichloromethane (3x10ml). The combined organic layers were washed with saturated aqueous sodium sulphite solution (10ml) and brine (10ml), dried over magnesium sulphate, filtered and evaporated under reduced pressure to leave a red oil (344mg). The crude product was purified by flash chromatography on silica gel eluting with pentane:ethyl acetate (20:1, by volume) to give the title compound (118mg) as a cream solid m.p. 91-92°C.
¹H-NMR (400MHz, CDCl₃): δ = 2.04 (s, 6H), 6.84 (s, 2H), 7.06 (s, 1H), 14.38 (br.s, 1 H)
LRMS (thermospray): m/z [MNH₄⁺] 278.
Microanalysis: Found: C, 50.43; H, 3.84. C₁₁H₁₀Cl₂O₃ requires C, 50.60; H, 3.86%.

### PREPARATION 2

### 4-Chloro-3,5-heptanedione

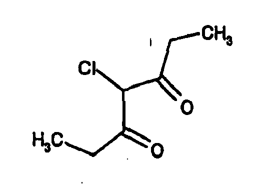

Chlorotrimethylsilane (29.7ml, 0.234mol) was added dropwise to a stirred pale yellow solution of tetrabutylammonium bromide (1.26g, 3.9mmol) in dry acetonitrile (116ml) at room temperature under nitrogen. The resulting solution was cooled in ice and 3,5-heptanedione (10.6ml, 78.0mmol) and then dry dimethylsulphoxide (16.6ml, 0.234mol) were added dropwise over 5 minutes producing a yellow solution which was allowed to warm slowly to room temperature, with stirring, over 4 hours. The mixture was diluted with water (1litre), stirred for 10min and then extracted with ether (1x500ml, 2x250ml). The combined ether layers were dried over magnesium sulphate, filtered and concentrated under reduced pressure to leave a yellow oil. The crude product was purified by distillation under reduced pressure to afford the title compound (5.5g) as a pale yellow oil, b.p. 102-105°C/54mmHg containing ca. 10% 4,4-dichloro-3,5-heptanedione as estimated by microanalysis.
¹H-NMR (400MHz, CDCl₃): δ = 1.12 (t, 6H), 2.59 (q, 4H), 4.77 (s, 0.2H, diketone), 15.50 (s, 0.8H, enol).
LRMS (thermospray): m/z [MNH₄⁺] 180 for title compound and 214 for dichlorinated impurity.

### PREPARATION 3

### Ethyl 4-[4-(3,5-dichlorophenoxy)-3,5-diethyl-1H-pyrazol-1-yl]-3-oxobutanoate

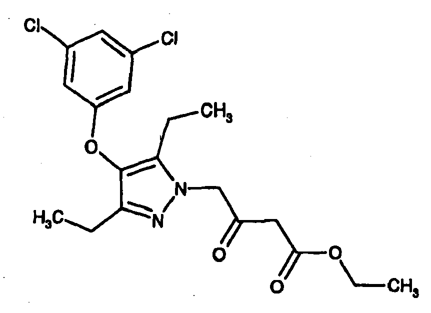

Sodium hydride (60% dispersion in oil, 250mg, 6.17mmol) was added to a stirred solution of 4-(3,5-dichlorophenoxy)-3,5-diethyl-1*H*-pyrazole (800mg, 2.81mmol, Example 3) in dry N,N-dimethylformamide (5ml) at 0°C under nitrogen. The mixture was stirred for 5 minutes during which time hydrogen was evolved and then ethyl 4-chloroacetoacetate (0.42ml, 3.09mmol) was added. After 30 minutes the reaction mixture was quenched by the addition of water (0.5ml) and concentrated under reduced pressure. A solution of the residue in ethyl acetate (50ml) was washed with saturated aqueous ammonium chloride solution (20ml) and water (20ml), dried over magnesium sulphate and concentrated under reduced pressure. The crude product was purified by flash chromatography on silica gel eluting with ethyl acetate:pentane (30:70, by volume) to provide the title compound (1.1g) as a white solid, m.p. 82-84°C.
¹H-NMR (300MHz, CDCl₃): δ = 1.40 (6H, m), 1.26 (3H, t), 2.44 (4H, q), 3.47 (2H, s), 4.22 (2H, q), 4.96 (2H, s), 6.82 (2H, s), 7.02 (1 H, s).
LRMS (thermospray): m/z [MH⁺] 413.
Microanalysis: Found: C, 55.13; H, 5.34; N, 6.98. C₁₅H₁₅Cl₂N₃O requires C, 55.22; H, 5.37; N, 6.78%.

### PREPARATION 4

### [4-(3,5-Dichlorophenoxy)-3,5-diethyl-1H-pyrazol-1-yl]acetic acid

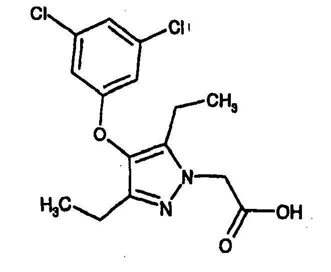

Aqueous sodium hydroxide solution (1 N, 6.2ml, 6.2mmol) was added dropwise to a stirred solution of the ester (2g, 5.6mmol) of Example 9 in tetrahydrofuran (20ml) at 0°C. After 1 hour the solvent was removed under reduced pressure and aqueous hydrochloric acid (20ml) was added with vigorous stirring. The resulting white precipitate was collected by filtration, washed with ether (3x30ml) and dried in a vacuum pistol at 60°C/10mmHg to afford the title compound as a white solid (1.5g), m.p. 157-158°C.
¹H-NMR (300MHz, CDCl₃): δ = 1.13 (6H, m), 2.52 (2H, q), 2.60(2H, q), 5.03 (2H, s), 6.95 (2H, s), 7.14 (1 H, s).
LRMS (electrospray): m/z [M-H⁺] 341.

### PREPARATION 5

### 1-(3,5-Dichlorophenoxy)-2-butanone

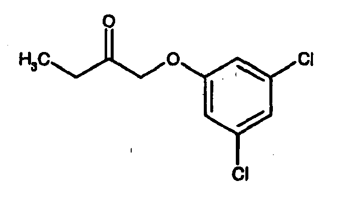

Cesium carbonate (108g, 0.33mol) was added in one portion to a stirred solution of 3,5-dichlorophenol (49g, 0.30mol) in acetone (900ml) at room temperature under nitrogen. To this suspension a solution of 1-bromo-2-butanone (30.6ml, 0.30mol) in acetone (300ml) was added dropwise and the resultant suspension was heated under reflux for 2 hours. The suspension was cooled to room temperature, water (200ml) was added and the acetone was removed under reduced pressure. The mixture was extracted with dichloromethane (2x300ml) and the combined organic phases were dried over magnesium sulphate, filtered and concentrated under reduced pressure to leave a clear oil. The crude product was purified by flash column chromatography on silica gel eluting with dichloromethane:cyclohexane (50:50, by volume) to provide the title compound (65g) as a yellow oil.
¹H-NMR (400MHz, CDCl₃): δ = 1.13 (t, 3H), 2.60 (q, 2H), 4.58 (s, 2H), 6.78 (s, 2H), 7.01 (s, 1 H).
LRMS (thermospray): m/z [MNH₄⁺] 250.

### PREPARATION 6

### 2-(3,5-Dichlorophenoxy)-1-(dimethylamino)-1-penten-3-one

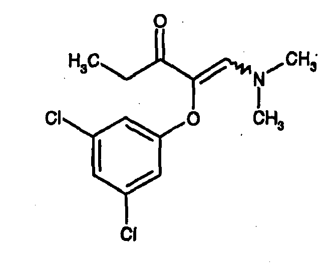

A solution of the ketone of Preparation 5 (65g, 0.28mol) in *N,N*-dimethylformamide dimethylacetal (75ml, 0.56mol) was heated at 100°C using a Dean-Stark apparatus for 10 hours. The reaction was cooled and concentrated under reduced pressure to leave a brown oil. The crude product was purified by flash column chromatography on silica gel eluting with pentane:ethyl acetate (90:10, by volume) and then pentane:ethyl acetate (60:40, by volume) to provide the title compound (55g) as a yellow oil that solidified upon standing. The resultant yellow solid was washed with pentane (100ml) and dried to provide the title compound (28g) as a yellow solid, m.p. 96-97°C.
¹H-NMR (400MHz, CDCl₃): δ = 0.98 (t, 3H), 2.30 (br s, 2H), 2.94 (s, 6H), 6.77 (s, 2H), 6.95 (s, 1H), 7.24 (s, 1 H).
LRMS (thermospray): m/z [MNH₄⁺] 288.

### PREPARATION 7

### 1-Acetyl-4-(3,5-dichlorophenoxy)-3,5-dimethyl-1H-pyrazole

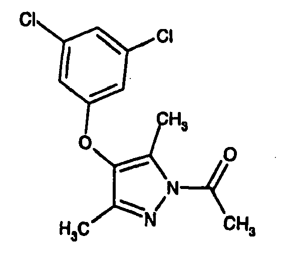

Sodium hydride (60% dispersion in oil, 684mg, 17.1mmol) was added to a stirred solution of acetyl chloride (1.21 ml, 17.1 mmol) and the pyrazole of Example 53 (4.00g, 15.6mmol) in *N,N*-dimethylformamide (20ml) at 0°C under nitrogen. The reaction was stirred at 0°C for 1 hour and then quenched by the addition of water (100ml). The aqueous extracted was with ether (2x5Oml). The combined organic phases were washed with water (30ml) and brine (30ml), dried over magnesium sulphate, filtered and concentrated under reduced pressure to leave a yellow solid. The crude product was purified by flash column chromatography on silica gel eluting with pentane:ether (90:10, by volume) to provide the title compound (3.0g) as a white solid, m.p. <60°C.
¹H-NMR (300MHz, CDCl₃): δ = 2.11 (s, 3H), 2.43 (s, 3H), 2.70 (s, 3H), 6.78 (s, 2H), 7.03 (s, 1 H).

LRMS (thermospray): m/z [MH⁺] 299.

### PREPARATION 8

### 1-Acetyl-3-(bromomethyl)-4-(3,5-dichlorophenoxy)-5-methyl-1H-pyrazole

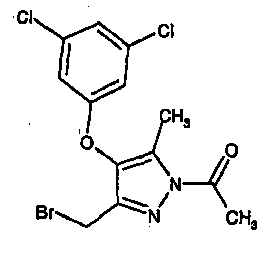

*N*-Bromosuccinimide (2.70g, 15.0mmol) was added to a stirred solution of the pyrazole of Preparation 7 (3.00g, 10.0mmol) in 1,1,1-trichloroethane (40ml) at room temperature under nitrogen. The reaction was heated at 80°C for 1 hour and then azobisisobutyronitrile (2mg) was added and the reaction mixture was heated for a further 3 hours. The reaction was cooled to room temperature and a solid removed by filtration. The filtrate was concentrated under reduced pressure and the resulting yellow oil was dissolved in ethyl acetate (100ml). The ethyl acetate was washed with 1 M aqueous sodium carbonate solution (30ml), water (30ml) and brine (30ml), dried over magnesium sulphate, filtered and concentrated under reduced pressure to leave a yellow solid. The crude product was purified by flash column chromatography on silica gel eluting with pentane:ethyl acetate (90:10, by volume) to provide a yellow solid that was washed with ice cold ether (20ml) to provide the title compound (2.3g) as a white solid, m.p. 111-113°C.
¹H-NMR (300MHz, CDCl₃): δ = 2.10 (s, 3H), 2.73 (s, 3H), 4.73 (s, 2H), 6.86 (s, 2H), 7.11 (s, 1 H).

LRMS (thermospray): m/z [MH⁺] 379.

### PREPARATION 9

### 4-(3-Cyanophenoxy)-3,5-heptanedione

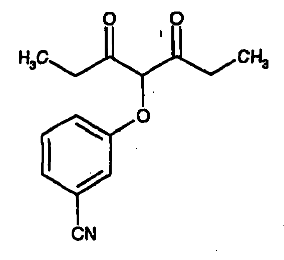

A mixture of the β-diketone of Preparation 2 (1.79g, 11.0mmol), 3-cyanophenol (1.31 g, 11.0mmol), cesium carbonate (3.58g, 11.0mmol) and acetone (44ml) was heated under reflux for 2 hours. After cooling, the mixture was concentrated under reduced pressure and the residue was partitioned between dichloromethane (50ml) and water (25ml). The organic layer was separated, washed with brine (25ml), dried over magnesium sulphate, filtered and concentrated under reduced pressure to leave a yellow oil. The crude product was purified by flash column chromatography on silica gel eluting with ethyl acetate:pentane (10:90, by volume) to provide the title compound (1.10g) as a yellow oil.
¹H-NMR (400MHz, CDCl₃): δ = 1.04 (t, 6H), 2.49 (q, 4H), 7.16 (m, 2H), 7.30 (d, 1H), 7.39 (t, 2H), 14.51 (s, 1 H).
LRMS (thermospray): m/z [MNH₄⁺] 263.

### PREPARATION 10

### tert-Butyl 3-(hydroxymethyl)-4-morpholinecarboxylate

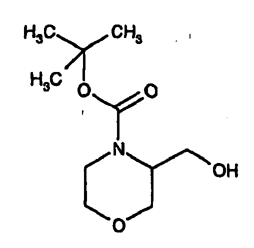

Borane (38.1ml of a 1.0M solution in tetrahydrofuran, 38.1mmol) was added dropwise to a stirred suspension of 3-morpholinecarboxylic acid (1.00g, 7.63mmol) in tetrahydrofuran (50ml) at room temperature under nitrogen. The reaction was heated under reflux and the reaction became homogeneous and heating was continued for 12 hours. The reaction was cooled to room temperature and concentrated under reduced pressure to leave a brown oil. The residue was dissolved in 1 M aqueous sodium hydroxide solution and stirred at room temperature for 5 days. After this time di-*tert*-butyl dicarbonate (1.66g, 7.63mmol) was added and the reaction was stirred for 12 hours. The reaction was diluted with ether (100ml). The organic layer was separated, washed with brine (10ml), dried over magnesium sulphate, filtered and concentrated under reduced pressure. The crude product was purified by flash column chromatography on silica gel eluting with pentane:ethyl acetate (50:50, by volume) and then ethyl acetate to provide the title compound (1.30g) as a colourless oil.
¹H-NMR (400MHz, CDCl₃): δ = 1.48 (s, 9H), 2.05 (s, 1 H), 3.19 (br t, 1 H), 3.47 (td, 1 H), 3.60 (dd, 1 H), 3.87 (m, 6H).
LRMS (thermospray): m/z [MH⁺] 218.

### PREPARATION 11

### tert-Butyl 3-{[(methylsulfonyl)oxy]methyl}-4-morpholinecarboxylate

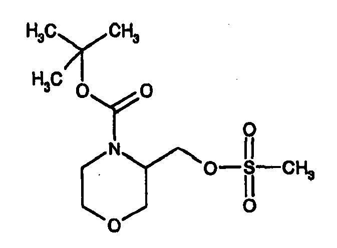

Triethylamine (1.15ml, 8.29mmol) was added dropwise to a stirred solution of the alcohol of Preparation 10 (1.20g, 5.52mmol) and methanesulfonic anhydride (1.44g, 5.52mmol) in dichloromethane (50ml) at room temperature under nitrogen. The reaction was stirred for 1 hour and then poured onto water (50ml). The organic layer was separated, dried over magnesium sulphate, filtered and concentrated under reduced pressure. The crude product was purified by flash column chromatography on silica gel eluting with pentane:ethyl acetate (50:50, by volume) to provide the title compound (1.20g) as a colourless oil.
¹H-NMR (400MHz, CDCl₃): δ = 1.49 (s, 9H), 3.06 (s, 3H), 3.50 (td, 1H), 3.60 (dd, 1 H), 3.80 (m, 4H), 4.26 (br s, 1 H), 4.39 (m, 2H).
LRMS (thermospray): m/z [MNH₄⁺] 313.

### PREPARATION 12

### Methyl-2-(3,5-dichlorophenoxy)-3-oxopentanoate

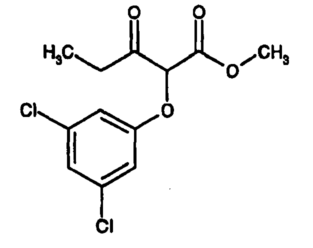

A mixture of methyl-2-chloro-3-pentanoate (25.0g, 152mmol), 3,5-dichlorophenol (24.6g, 152mmol), cesium carbonate (54.4g, 167mmol) and acetone (500ml) was heated under reflux for 2 hours. After cooling the mixture was concentrated under reduced pressure and the residue was partitioned between dichloromethane (100ml) and water (50ml). The organic layer was separated, washed with brine (25ml), dried over magnesium sulphate, filtered and concentrated under reduced pressure to leave an orange oil. The crude product was purified by flash column chromatography on silica gel eluting with pentane:toluene (90:10, by volume) to provide the title compound (40.0g) as a pink oil.
¹H-NMR (300MHz, CDCl₃): δ = 1.16 (t, 3H), 2.60 (m, 2H), 3.77 (s, 3H), 5.13 (s, 1 H), 6.84 (s, 2H), 7.10 (s, 1 H).
LRMS (thermospray): m/z [MNH₄⁺] 308.

### PREPARATION 13

### 4-(3,5-Dichlorophenoxy)-5-ethyl-2-(2-hydroxyethyl)-2,4-dihydro-3H-pyrazol-3-one

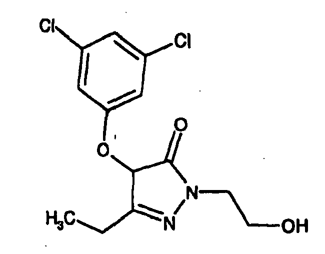

A solution of 2-hydroxyethylhydrazine (4.30g, 56.7mmol) in glacial acetic acid (2.0ml) was added to a stirred solution of the ketoester of Preparation 12 (15.0g, 51.5mol) in glacial acetic acid (100ml) and the resulting solution was stirred at room temperature for 48 hours. The mixture was concentrated under reduced pressure and the crude product was purified by flash column chromatography on silica gel eluting with dichloromethane:methanol (95:5, by volume) to provide the title compound (10.1 g) as a colourless oil.
¹H-NMR (400MHz, CDCl₃): δ = 1.02 (t, 3H), 2.29 (m, 2H), 3.63 (m, 2H), 3.80 (m, 2H), 6.92 (s, 2H), 7.21 (s, 1 H).
LRMS (thermospray): m/z [MH⁺] 317.
Microanalysis: Found: C, 48.86; H, 4.44; N, 9.01. C₁₃H₁₄N₂O₃Cl₂ requires C, 49.23; H, 4.45; N, 8.83%.

### PREPARATION 14

### 2-(2-{[tert-Butyl(dimethyl)silyl]oxy}ethyl)-4-(3,5-dichlorophenoxy)-5-ethyl-2,4-dihydro-3H-pyrazol-3-one

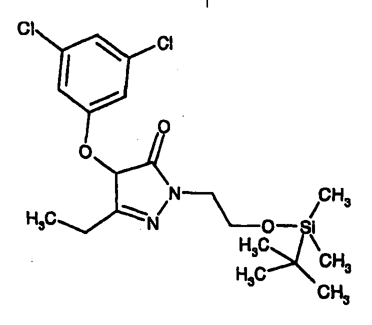

*tert*-Butyldimethylsilyl chloride (8.14g, 54.0mmol) was added in one portion to a stirred solution of the pyrazole of Preparation 13 (14.3g, 45.0mmol) and imidazole (3.98g, 58.5mmol) in *N,N*-dimethylformamide (90ml) and the resulting solution was stirred at room temperature for 48 hours. The mixture was partitioned between ethyl acetate (100ml) and water (300ml). The organic layer was separated, dried over magnesium sulphate, filtered and concentrated under reduced pressure. The crude product was purified by flash column chromatography on silica gel eluting with dichloromethane:methanol (95:5, by volume) to provide the title compound (9.56g) as a colourless oil.
¹H-NMR (400MHz, CDCl₃): δ = 0.15 (s, 6H), 0.94 (s, 9H), 1.16 (t, 3H), 2.45 (m, 2H), 3.94 (m, 4H), 6.85 (s, 2H), 6.97 (s, 1H).
LRMS (thermospray): m/z [MH⁺] 431.
Microanalysis: Found: C, 52.87; H, 6.52; N, 6.46. C₁₉H₂₈N₂O₃Cl₂Si requires C, 52.90; H, 6.54; N, 6.49%.

### PREPARATION 15

### 1-(2-{[tert-Butyl(dimethyl)silyl]oxy}ethyl)-4-(3,5-dichloroghenoxy)-3-ethyl-1H-pyrazol-5-yl trifluoromethanesulfonate

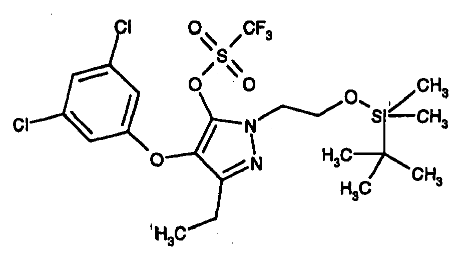

Phenyltriflamide (3.70g, 10.5mmol) was added in one portion to a stirred solution of the pyrazole of Preparation 14 (4.10g, 9.50mmol) and triethylamine (1.60ml, 11.4mmol) in dichloromethane (20ml) at room temperature under nitrogen. The reaction was stirred for 2 hours and then poured onto water (50ml). The organic layer was separated, dried over magnesium sulphate, filtered and concentrated under reduced pressure. The crude product was purified by flash column chromatography on silica gel eluting with dichloromethane to provide the title compound (5.10g) as a purple oil.
¹H-NMR (300MHz, CDCl₃): δ = 0.01 (s, 6H), 0.86 (s, 9H), 1.17 (t, 3H), 2.45 (q, 2H), 4.01 (m, 2H), 4.14 (m, 2H), 6.84 (s, 2H), 7.08 (s, 1H).
LRMS (thermospray): m/z [MH⁺] 563.

### PREPARATION 16

### 3-(1-Acetyl-2-oxopropoxy)-5-chlorobenzonitrile

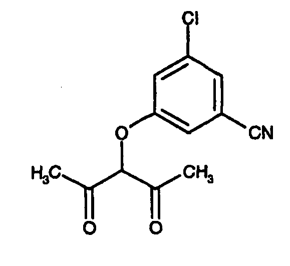

A mixture of 3-chloro-2,4-pentanedione (6.73g, 50.0mmol), the phenol of Preparation 36 (7.67g, 50.0mmol), cesium carbonate (18.0g, 55.4mmol) and acetone (40ml) was heated under reflux for 2 hours. The reaction was cooled to room temperature, *N,N*-dimethylformamide (6ml) and acetone (30ml) were added and the reaction was heated at 70°C for a further 12 hours. After cooling, the solid was removed by filtration and dissolved in 1M aqueous hydrochloric acid (150ml). The resulting solution was extracted with dichloromethane (3x100ml) and the combined organic phases were washed with brine (30ml), dried over magnesium sulphate, filtered and concentrated under reduced pressure to provide the title compound (5.50g) as a brown solid, m.p. 105-108°C.
¹H-NMR (300MHz, CDCl₃): δ = 2.04 (s, 6H), 7.13 (s, 1 H), 7.19 (s, 1 H), 7.35 (s, 1 H), 14.40 (s, 1 H).

### PREPARATION 17

### 3-[(1-Acetyl-3,5-dimethyl-1H-pyrazol-4-yl)oxy]-5-chlorobenzonitrile

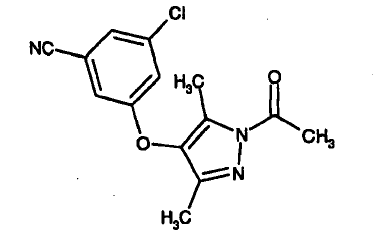

Sodium hydride (60% dispersion in oil, 840mg, 21.0mmol) was added to a stirred solution of acetyl chloride (1.50ml, 21.0mmol) and the pyrazole of Example 76 (4.80g, 19.4mmol) in *N,N*-dimethylformamide (20ml) at 0°C under nitrogen. The reaction was stirred at 0°C for 15 minutes and then quenched by the addition of water (200ml). The reaction mixture was extracted with ethyl acetate (3x120ml). The combined organic phases were washed with water (50ml) and brine (50ml), dried over magnesium sulphate, filtered and concentrated under reduced pressure to leave a yellow solid. The crude product was purified by flash column chromatography on silica gel eluting with dichloromethane to provide the title compound (5.00g) as a white solid, m.p. <60°C.
¹H-NMR (400MHz, CDCl₃): δ = 2.06 (s, 3H), 2.38 (s, 3H), 2.65 (s, 3H), 6.99 (m, 1H), 7.08 (m, 1H), 7.29 (m, 1H).
LRMS (thermospray): m/z [MH⁺] 290.

### PREPARATION 18

### 3-{[1-Acetyl-3-(bromomethyl)-5-methyl-1H-pyrazol-4-yl]oxy}-5-chlorobenzonitrile

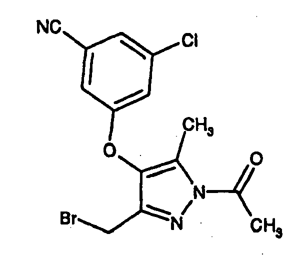

*N*-Bromosuccinimide (4.60g, 25.6mmol) was added to a stirred solution of the pyrazole of Preparation 17 (5.00g, 17.3mmol) in 1,1,1-trichloroethane (70ml) and azobisisobutyronitrile (20mg) at room temperature under nitrogen. The reaction was heated at 80°C for 3 hours and then cooled to room temperature. A second portion of *N*-bromosuccinimide (2.00g, 11.2mmol) was added and the reaction mixture was heated at 80°C for a further 4 hours. The reaction was cooled to room temperature and concentrated under reduced pressure and the resulting yellow oil was purified by flash column chromatography on silica gel eluting with pentane:dichloromethane (25:75, by volume) to provide the title compound (2.30g) as a white solid, m.p. 122-123°C.
¹H-NMR (300MHz, CDCl₃): δ = 2.10 (s, 3H), 2.74 (s, 3H), 4.73 (s, 2H), 7.12 (s, 1 H), 7.22 (s, 1H), 7.39 (s, 1 H).

### PREPARATION 19

### 3-Chloro-5,5-dimethyl-2,4-hexanedione

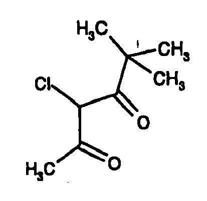

Chlorotrimethylsilane (26.8ml, 0.21 mol) was added dropwise to a stirred pale yellow solution of tetrabutylammonium bromide (1.13g, 3.50mmol) in dry acetonitrile (100ml) at room temperature under nitrogen. The resulting solution was cooled in ice and 5,5-dimethylhexane-2,4-dione (10.0g, 70.4mmol) and then dry dimethylsulphoxide (14.7ml, 0.21 mol) were added dropwise over 5 minutes producing a yellow solution which was allowed to warm slowly to room temperature with stirring over 3 hours. The mixture was diluted with water (1000ml) and stirred for 10min and then extracted with ether (1x500ml, 2x250ml). The combined ether layers were dried over magnesium sulphate, filtered and concentrated under reduced pressure to leave a yellow oil. The crude product was purified by distillation under reduced pressure to provide the title compound (10.0g) as a pale yellow oil, b.p. 220-225°C/60mmHg.
¹H-NMR (400MHz, CDCl₃): δ = 1.25 (s, 9H), 2.39 (s, 3H), 5.10 (s, 1 H).
LRMS (thermospray): m/z [MNH₄⁺] 194.

### PREPARATION 20

### 4-[(Methylamino)methyl]benzonitrile

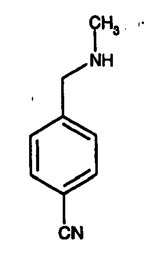

4-Cyanobenzaldehyde (12.0g, 92.0mmol), methylamine (69ml of a 2.0M solution in tetrahydrofuran, 137mmol) and magnesium sulphate (45g) were stirred in dichloromethane (300ml) at room temperature for 5 days. The mixture was filtered and the filtrate was concentrated under reduced pressure to leave a yellow oil. The oil was dissolved in methanol (200ml) and sodium borohydride (4.10g, 109mmol) was added cautiously with vigorous stirring. Once the addition was complete the reaction was stirred for 1 hour and the mixture was concentrated under reduced pressure. The residue was dissolved in 1 M aqueous sodium hydroxide solution (200ml) and the mixture was stirred at room temperature for 1 hour. The resulting solution was extracted with dichloromethane (2x200ml) and the combined organic phases were dried over magnesium sulphate, filtered and concentrated under reduced pressure to provide the title compound (13.4g) as a pale yellow oil.
¹H-NMR (300MHz, CDCl₃): δ = 1.46 (s, 1 H), 2.46 (s, 3H), 3.82 (s, 2H), 7.47 (d, 2H), 7.64 (d, 2H).
LRMS (electrospray): m/z [MH⁺] 147.

### PREPARATION 21

### 4-{[(2-Hydroxyethyl)amino]methyl}benzonitrile

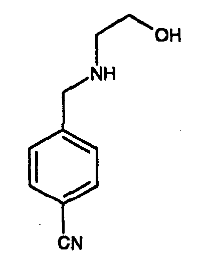

A mixture of 4-Cyanobenzaldehyde (14.1g, 107mmol), ethanolamine (6.56g, 107mmol) and toluene (100ml) was heated under reflux for 14 hours using a Dean-Stark apparatus to remove water. The reaction was cooled to room temperature and concentrated under reduced pressure to leave a yellow oil. The oil was dissolved in dichloromethane (200ml), cooled to 0°C and triethylamine (16.3ml, 117mmol) and chlorotrimethylsilane (14.9ml, 117mmol) were added dropwise. A white precipitate formed and after stirring for 1 hour the mixture was filtered. The filtrate was concentrated under reduced pressure to leave an orange solid (25.0g). The orange solid was dissolved in methanol (200ml) and sodium borohydride (4.50g, 122mmol) was added cautiously with vigorous stirring. Once the addition was complete the reaction was stirred for 1 hour and the mixture was then concentrated under reduced pressure. The residue was dissolved in 1M aqueous sodium hydroxide solution (200ml) and the mixture was stirred at room temperature for 1 hour. The resulting solution was extracted with dichloromethane (3x200ml) and the combined organic phases were dried over magnesium sulphate, filtered and concentrated under reduced pressure. The crude product was purified by flash column chromatography on silica gel eluting with dichloromethane:methanol:ammonia (95:4:1, by volume) to provide the title compound (12.0g) as a pale yellow oil which solidified on standing to leave a yellow solid, m.p. <60°C.
¹H-NMR (300MHz, CDCl₃): δ = 1.84 (s, 2H), 2.84 (t, 2H), 3.68 (t, 2H), 3.89 (s, 2H), 7.45 (d, 2H), 7.65 (d, 2H).
LRMS (thermospray): m/z [MH⁺] 177.

### PREPARATION 22

### N-{[1-(2-{[tert-Butyl(dimethyl)silyl]oxy}ethyl)-4-(3,5-dichlorophenoxy)-3-methyl-1H-pyrazol-5-yl]methyl}-N-(3-pyridinylmethyl)amine

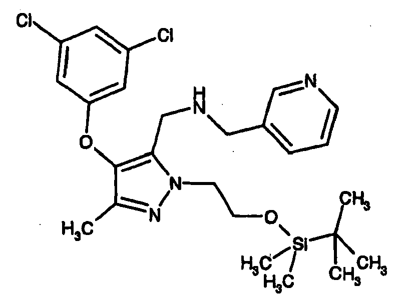

3-(Methylamino)pyridine (327mg, 3.04mmol) was added in one portion to a stirred solution of the bromide of Preparation 28 (300mg, 0.610mmol) in isopropanol (5ml) at room temperature. The mixture was heated at 50°C for 1 hour, cooled to room temperature and concentrated under reduced pressure to leave an orange oil. The crude product was purified by flash column chromatography on silica gel eluting with dichloromethane:methanol:ammonia (95:4:1, by volume) to provide the title compound (50mg) as a colourless oil.
¹H-NMR (400MHz, CDCl₃): δ = 0.15 (s, 6H), 0.77 (s, 9H), 2.02 (s, 3H), 3.64 (s, 2H), 3.70 (s, 2H), 3.95 (t, 2H), 4.17 (t, 2H), 6.75 (s, 2H), 6.97 (s, 1 H), 7.15 (dd, 1 H), 7.53 (d, 1 H), 8.47 (m, 2H).
LRMS (thermospray): m/z [MH⁺] 521.

### PREPARATION 23

### 3-Chloro-5-methyl-2,4-hexanedione

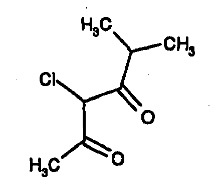

Chlorotrimethylsilane (13.4ml, 105mmol) was added dropwise to a stirred pale yellow solution of tetrabutylammonium iodide (566mg, 1.53mmol) in dry acetonitrile (100ml) at room temperature under nitrogen. The resulting solution was cooled in ice and 5-methylhexane-2,4-dione (4.50g, 35.1mmol) and then dry dimethylsulphoxide (7.47ml, 105mmol) were added dropwise over 5 minutes producing a yellow solution which was allowed to warm slowly to room temperature with stirring over 1 hour. Tetrabutylammonium bromide (566mg, 1.75mmol) was then added in one portion and the reaction was stirred at room temperature for 2 hours. The mixture was diluted with water (200ml), stirred for 10min and then extracted with ether (3x100ml). The combined ether layers were dried over magnesium sulphate, filtered and concentrated under reduced pressure to leave a yellow oil. The crude product was purified by flash column chromatography on silica gel eluting with pentane:ethyl acetate (98:2, by volume) to provide the title compound (2.00g) as a colourless oil.
¹H-NMR (400MHz, CDCl₃): δ = 1.15 (d, 6H), 2.29 (s, 3H), 3.25 (sept, 1H), 15.60 (s, 1 H).
LRMS (thermospray): m/z [MNH₄⁺] 180.

### PREPARATION 24

### 5-(1-Acetyl-3-methyl-2-oxobutoxy)isophthalonitrile

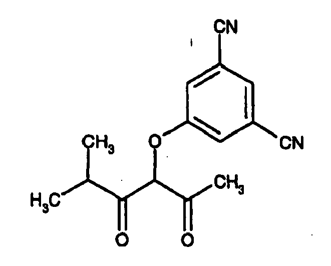

A mixture of the dione of Preparation 23 (1.12g, 6.94mmol), the phenol of Preparation 39 (1.00g, 6.94mmol), cesium carbonate (2.25g, 6.94mmol) and acetone (30ml) was heated under reflux for 4 hours. The reaction was cooled to room temperature and concentrated under reduced pressure to leave a brown solid. The solid was dissolved in 1 M aqueous hydrochloric acid (50ml) and the solution was extracted with dichloromethane (3x30ml). The combined organic phases were washed with brine (30ml), dried over magnesium sulphate, filtered and concentrated under reduced pressure. The crude product was purified by flash column chromatography on silica gel eluting with pentane:ethyl acetate (90:10, by volume) to provide the title compound (580mg) as a yellow solid.
¹H-NMR (300MHz, CDCl₃): δ = 1.08 (d, 6H), 2.02 (s, 3H), 2.24 (sept, 1H), 7.47 (s, 2H), 7.63 (s, 1 H), 14.71 (s, 1 H).
LRMS (electrospray): m/z [M-H⁺] 269.

### PREPARATION 25

### 5-{[1-(2-{[tert-Butyl(dimethyl)silyl]oxy}ethyl)-3-isopropyl-5-methyl-1H-pyrazol-4-yl]oxy}isophthalonitrile

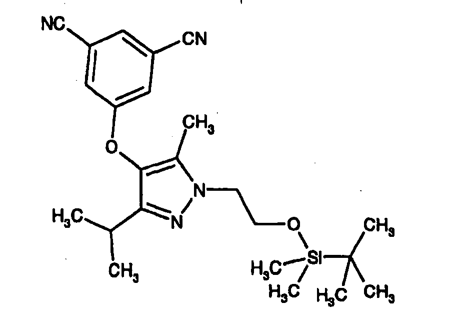

Sodium hydride (60% dispersion in oil, 45mg, 1.12mmol) was added to a stirred solution of 2-bromoethoxy-t-butyldimethylsilane (270mg, 1.12mmol) and the pyrazole of Example 95 (250mg, 0.930mmol) in *N*,*N*-dimethylformamide (5ml) at 0°C under nitrogen. The reaction was warmed to room temperature and stirred for 12 hours. The reaction mixture was quenched by the addition of water (50ml) and the aqueous phase was extracted with ethyl acetate (3x30ml). The combined organic phases were dried over magnesium sulphate, filtered and concentrated under reduced pressure to leave a brown oil. The crude product was purified by flash column chromatography on silica gel eluting pentane:ethyl acetate (80:20, by volume) to provide the title compound (60mg) as a colourless oil.
¹H-NMR (400MHz, CDCl₃): δ = 0.02 (s, 6H), 0.85 (s, 9H), 1.19 (d, 6H), 2.09 (s, 3H), 2.79 (sept, 1 H), 3.99 (m, 2H), 4.10 (m, 2H), 7.39 (s, 2H), 7.57 (s, 1H).
LRMS (electrospray): m/z [MH⁺] 425.

### PREPARATION 26

### di(tert-Butyl) 2-[4-(3,5-dichlorophenoxy)-3-ethyl-1H-pyrazol-1-yl]ethylimidodicarbonate and di(tert-butyl) 2-[4-(3,5-dichlorophenoxy)-5-ethyl-1H-pyrazol-1-yl]ethylimidodicarbonate

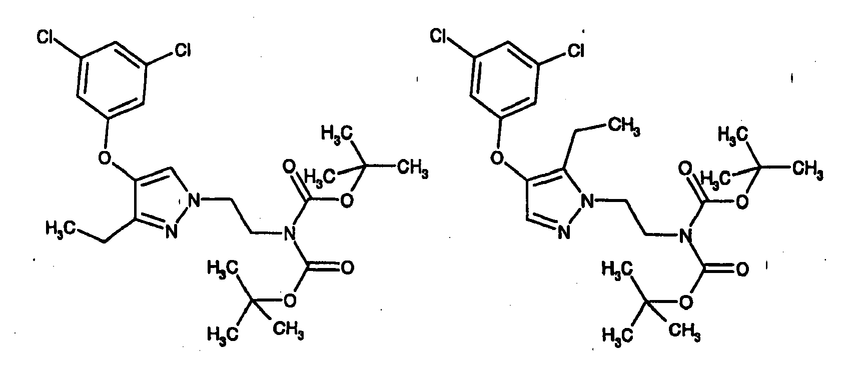

Di-t-butyldicarbonate (14.0g, 64.2mmol) and 4,4-dimethylaminopyridine (630mg, 5.14mmol) were added portionwise to a stirred solution of the amines of Example 283 (7.72g, 25.7mmol) in acetonitrile (128ml) at room temperature under nitrogen. The reaction was stirred for 14 hours and concentrated under reduced pressure. A solution of the residue in dichloromethane (300ml) was washed with water (100ml), dried over magnesium sulphate, filtered and concentrated under reduced pressure. The crude product was purified by flash chromatography on silica gel eluting with dichloromethane:methanol (99:1, by volume) to afford the title compounds (12.3g) in a 85:15 ratio of regioisomers as a colourless oil.
¹H-NMR (400MHz, CDCl₃): δ = 1.15 (major, t, 3H), 1.15 (minor, t, 3H), 1.52 (major, s, 18H), 1.52 (minor, s, 18H), 2.47 (major, q, 2H), 2.56 (minor, q, 2H), 4.00 (major, t, 2H), 4.00 (minor, t, 2H), 4.24 (major, t, 2H), 4.24 (minor, t, 2H), 6.85 (major, s, 2H), 6.85 (minor, s, 2H), 7.00 (major, s, 1 H), 7.00 (minor, s, 1 H), 7.21 (major, s, 1 H), 7.25 (minor, s, 1 H).
LRMS (thermospray): m/z [MH⁺] 500.
Microanalysis: Found: C, 54.94; H, 6.26; N, 8.27. C₂₃H₃₁Cl₂N₃O₅ requires C, 55.20; H, 6.24; N, 8.40%.

### PREPARATION 27

### 1-(2-{[tert-Butyl(dimethyl)silyl]oxy}ethyl)-4-(3,5-dichlorophenoxy)-3,5-dimethyl-1H-pyrazole

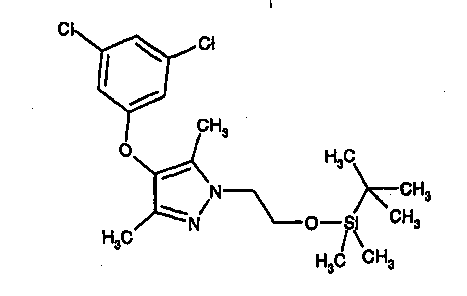

Chloro-t-butyldimethylsilane (1.93g, 12.8mmol) was added in one portion to a stirred solution of the pyrazole of Example 1 (3.50g, 11.6mmol) and imidazole (1.03g, 15.1mmol) in *N,N*-dimethylformamide (23ml) at room temperature under nitrogen. The reaction was stirred for 2 days and water (200ml) was added. The aqueous phase was extracted with diethyl ether (3x200ml) and the combined organic phases were washed with water (2x50ml) and brine (2x50ml), dried over magnesium sulphate, filtered and concentrated under reduced pressure. The crude product was purified by flash chromatography on silica gel eluting with pentane:ethyl acetate (80:20, by volume) to provide the title compound (4.82g) as a colourless oil.
¹H-NMR (400MHz, CDCl₃): δ = 0.09 (s, 6H), 0.78 (s, 9H), 2.01 (s, 3H), 2.05 (s, 3H), 3.88 (q, 2H), 4.02 (q, 2H), 6.76 (s, 2H), 6.88 (s, 1H).
LRMS (thermospray): m/z [MH⁺] 415.

### PREPARATION 28

### 5-(Bromomethyl)-1-(2-{[tert-butyl(dimethyl)silyl]oxy}ethyl)-4-(3,5-dichlorophenoxy)-3-methyl-1H-pyrazole

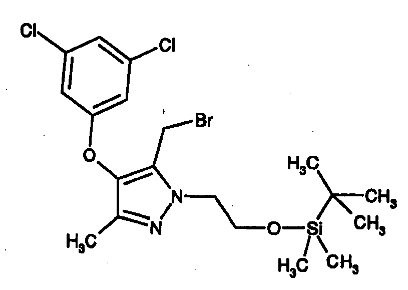

*N*-Bromosuccinimide (640mg, 3.60mmol) was added to a stirred solution of the pyrazole of Preparation 27 (1.00g, 2.40mmol) in carbon tetrachloride (15ml) and azobisisobutyronitrile (20mg) at room temperature under nitrogen. The reaction was heated under reflux for 5 hours then cooled to room temperature and filtered. The filtrate was concentrated under reduced pressure and the crude product was purified by flash column chromatography on silica gel eluting with dichloromethane:methanol:ammonia (97:2.5:0.5, by volume) to provide the title compound (300mg) as a colourless oil.
¹H-NMR (300MHz, CDCl₃): δ = 0.04 (s, 6H), 0.82 (s, 9H), 2.02 (s, 3H), 3.96 (m, 2H), 4.22 (m, 2H), 4.41 (s, 2H), 6.81 (s, 2H), 7.01 (s, 1H).
LRMS (thermospray): m/z [MH⁺] 495.

### PREPARATION 29

### 3-{[1-(2-{[tert-Butyl(dimethyl)silyl]oxy}ethyl)-3,5-dimethyl-1H-pyrazol-4-yl]oxy}-5-chlorobenzonitrile

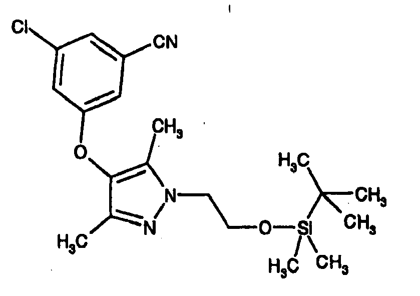

Chloro-t-butyldimethylsilane (2.78g, 18.5mmol) was added in one portion to a stirred solution of the pyrazole of Example 114 (4.89g, 16.8mmol) and imidazole (1.48g, 21.8mmol) in *N,N*-dimethylformamide (30ml) at room temperature under nitrogen. The reaction was stirred for 3 days and water (200ml) was added. The aqueous phase was extracted with diethyl ether (3x200ml) and the combined organic phases were washed with water (2x50ml) and brine (2x50ml), dried over magnesium sulphate, filtered and concentrated under reduced pressure. The crude product was purified by flash chromatography on silica gel eluting with dichloromethane to provide the title compound (5.60g) as a yellow oil.
¹H-NMR (400MHz, CDCl₃): δ = -0.02 (s, 6H), 0.82 (s, 9H), 2.02 (s, 3H), 2.12 (s, 3H), 3.97 (q, 2H), 4.06 (m, 2H), 7.02 (s, 1 H), 7.11 (s, 1 H), 7.24 (s, 1H).
LRMS (thermospray): m/z [MH⁺] 408.
Microanalysis: Found: C, 58.95; H, 6.96; N, 10.22. C₂₀H₂₈N₃O₂ClSi requires C, 59.13; H, 6.95; N, 10.35%.

### PREPARATION 30

### 3-{[5-(Bromomethyl)-1-(2-{[tert-butyl(dimethyl)silyl]oxy}ethyl)-3-methyl-1H-pyrazol-4-yl]oxy}-5-chlorobenzonitrile

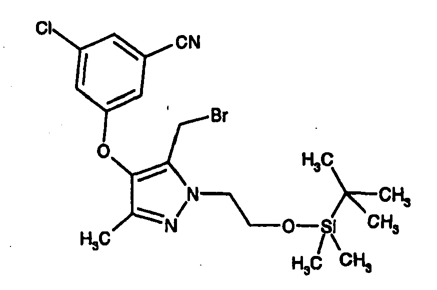

*N*-Bromosuccinimide (2.44g, 13.7mmol) was added to a stirred solution of the pyrazole of Preparation 29 (5,56g, 13.7mmol) in carbon tetrachloride (50ml) and azobisisobutyronitrile (20mg) at room temperature under nitrogen. The reaction was heated under reflux for 1 hour, cooled to room temperature and concentrated under reduced pressure. The crude product was purified by flash column chromatography on silica gel eluting with pentane:dichloromethane acetate (75:25, by volume) to provide the title compound (3.00g) as a colourless oil.
¹H-NMR (300MHz, CDCl₃): δ = -0.02 (s, 6H), 0.83 (s, 9H), 2.04 (s, 3H), 3.97 (q, 2H), 4.25 (m, 2H), 4.43 (s, 2H), 7.09 (s, 1 H), 7.18 (s, 1 H), 7.33 (s, 1H).
LRMS (thermospray): m/z [MH⁺] 486.

### PREPARATION 31

### 3-{[5-(Aminomethyl)-1-(2-{[tert-butyl(dimethy)silyl]oxy}ethyl)-3-methyl-1H-pyrazol-4-yl]oxy}-5-chlorobenzonitrile

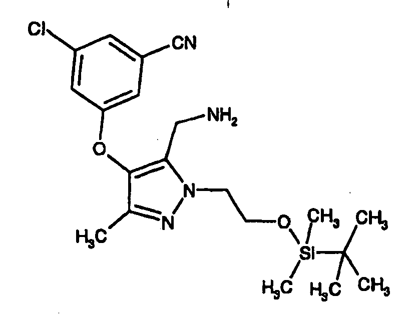

The bromide of Preparation 30 (1.58g, 3.26mmol) was added to a saturated solution of ammonia in isopropanol (50ml) at 0°C. The reaction was stirred for 6 hours and allowed to slowly warm to room temperature. The mixture was concentrated under reduced pressure and the resulting yellow oil was dissolved in dichloromethane (50ml). The solution was washed with 1M aqueous sodium carbonate solution (2x20ml) and brine (20ml), dried over magnesium sulphate, filtered and concentrated under reduced pressure to provide the title compound (1.00g) as a yellow oil.
¹H-NMR (300MHz, CDCl₃): δ = -0.23 (s, 6H), 0.62 (s, 9H), 1.22 (s, 2H), 1.82 (s, 3H), 2.56 (s, 2H), 3.78 (m, 2H), 4.02 (m, 2H), 6.85 (s, 1 H), 6.96 (s, 1 H), 7.06 (s, 1H).

LRMS (thermospray): m/z [MH⁺] 421.

### PREPARATION 32

### 1-Bromo-3-chloro-5-methoxybenzene

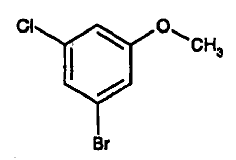

Sodium methoxide (2.20ml of a 4.5M solution in methanol, 10.0mmol) was added dropwise to a stirred solution of 1-fluoro-3-chloro-5-bromobenzene (1.00g, 4.77mmol) in methanol (28ml) at room temperature under nitrogen. The reaction was heated under reflux for 3 days and cooled to room temperature. The mixture was concentrated under reduced pressure and the resulting yellow oil was dissolved in dichloromethane (30ml). The resulting solution was washed with water (2x20ml) dried over magnesium sulphate, filtered and concentrated under reduced pressure. The crude product was purified by flash column chromatography on silica gel eluting with cyclohexane to provide the title compound (302mg) as a colourless oil.
¹H-NMR (400MHz, CDCl₃): δ = 3.77 (s, 3H), 6.82 (s, 1 H), 6.94 (s, 1 H), 7.09 (s, 1H). Microanalysis: Found: C, 37.94; H, 2.75. C₇H₆BrClO requires C, 37.96; H, 2.73%.

### PREPARATION 33

### 3-Fluoro-5-methoxybenzonitrile

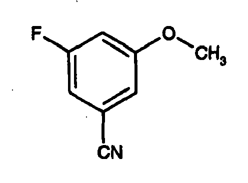

Sodium methoxide (1.50ml of a 4.5M solution in methanol, 7.10mmol) was added dropwise to a stirred solution of 3,5-difluorobenzonitrile (1.00g, 7.10mmol) in *N,N-*dimethylformamide (36ml) at 0°C under nitrogen. The reaction was allowed to warm to room temperature and stirred for 14 hours. The reaction was diluted with ether (40ml), washed with water (3x100ml) and brine (100ml), dried over magnesium sulphate, filtered and concentrated under reduced pressure. The crude product was purified by flash chromatography on silica gel eluting with cyclohexane:ethyl acetate (95:5, by volume) to provide the title compound (418mg) as a yellow oil.
¹H-NMR (400MHz, CDCl₃): δ = 3.84 (s, 3H), 6.82 (dd, 1 H), 6.95 (dd, 1 H), 6.96 (s, 1H).
LRMS (thermospray): m/z [MNH₄⁺] 169.
Microanalysis: Found: C, 63.46; H, 3.95; N, 9.14. C₈H₆NOF requires C, 63.58; H, 4.00; N, 9.27%.

### PREPARATION 34

### 3-Fluoro-5-hydroxybenzonitrile

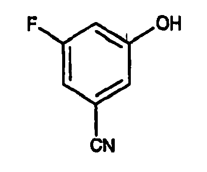

Boron trichloride (1.65ml of a 1.0M solution in dichloromethane, 1.65mmol) was added dropwise to a stirred solution of the nitrile of Preparation 33 (100mg, 0.660mmol) and tetrabutylammonium iodide (268mg, 0.728mmol) in dichloromethane (3ml) at -78°C. The reaction was allowed to warm 0°C, stirred for 2 hours and then allowed to warm to room temperature and stirred for 14 hours. The reaction was cooled to 0°C, cautiously quenched with ice and then concentrated under reduced pressure. The residue was dissolved in ether (40ml) and the resulting solution was washed with water (3x40ml) and brine (40ml), dried over magnesium sulphate, filtered and concentrated under reduced pressure. The crude product was purified by flash chromatography on silica gel eluting with cyclohexane:ethyl acetate (90:10, by volume) to provide the title compound (50mg) as a white solid, m.p. 138-139°C.
¹H-NMR (300MHz, CDCl₃): δ = 5.81 (s, 1 H), 6.80 (dd, 1 H), 6.94 (dd, 1 H), 6.95 (s, 1H).
Microanalysis: Found: C, 60.99; H, 3.01; N, 10.16. C₇H₄NOF requires C, 61.32; H, 2.94; N, 10.22%.

### PREPARATION 35

### 3-Chloro-5-methoxybenzonitrile

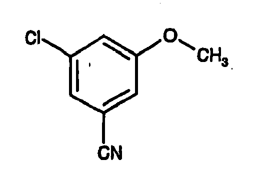

Palladiumtetrakis(triphenylphosphine) (174mg, 0.150mmol) was added in one portion to a stirred solution of the bromide of Preparation 32 (500mg, 2.26mmol) and zinc cyanide (146mg, 1.24mmol) in *N,N*-dimethylformamide (3ml) at room temperature under nitrogen. The reaction was heated at 100°C for 14 hours and cooled to room temperature. The mixture was concentrated under reduced pressure and the crude product was purified by flash chromatography on silica gel eluting with cyclohexane:ethyl acetate (95:5, by volume) to provide the title compound (380mg) as a yellow oil.
¹H-NMR (300MHz, CDCl₃): δ = 3.82 (3H, s), 7.04 (s, 1 H), 7.12 (s, 1 H), 7.23 (s, 1H).

Microanalysis: Found: C, 57.50; H, 3.63; N, 8.16. C₈H₆NOCl requires C, 57.33; H, 3.61; N, 8.36%.

### PREPARATION 36

### 3-Chloro-5-hydroxybenzonitrile

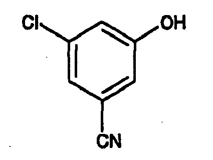

Boron trichloride (26.0ml of a 1.0M solution in dichloromethane, 26.0mmol) was added dropwise to a stirred solution of the nitrile of Preparation 35 (1.80g, 10.0mmol) and tetrabutylammonium iodide (4.36g, 11.0mmol) in dichloromethane (50ml) at -78°C. The reaction was allowed to warm to room temperature and stirred for 14 hours. The reaction was cooled to 0°C, cautiously quenched with ice and diluted with dichloromethane (100ml). The organic phase was washed with water (3x40ml) and brine (40ml), dried over magnesium sulphate, filtered and concentrated under reduced pressure. The crude product was purified by flash chromatography on silica gel eluting with cyclohexane:ethyl acetate (80:20, by volume) to provide the title compound (900mg) as a white solid.
¹H-NMR (400MHz, *d*₆DMSO): δ = 7.12 (m, 2H), 7.38 (s, 1 H), 10.65 (s, 1H).
Microanalysis: Found: C, 54.76; H, 2.81; N, 8.94. C₇H₄NOCl requires C, 54.75; H, 2.63; N, 9.12%.

### PREPARATION 37

### 1,3-Dibromo-5-methoxybenzene

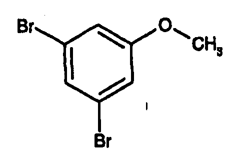

Sodium methoxide (8.80ml of a 4.5M solution in methanol, 41.0mmol) was added dropwise to a stirred solution of 3,5-dibromofluorobenzene (5.00g, 19.0mmol) in *N,N*-dimethylformamide (95ml) at 0°C under nitrogen. The reaction was allowed to warm to room temperature, stirred for 1 hour and then concentrated under reduced pressure. The residue was dissolved in ether and the resulting solution was washed with water (3x300ml) and brine (300ml), dried over magnesium sulphate, filtered and concentrated under reduced pressure to provide the title compound (5.13g) as a white solid.
¹H-NMR (300MHz, CDCl₃): δ = 3.79 (s, 3H), 7.00 (s, 2H), 7.26 (s, 1H).
LRMS (thermospray): m/z [MH⁺] 266.
Microanalysis: Found: C, 31.56; H, 2.29. C₇H₆OBr₂ requires C, 31.62; H, 2.27%.

### PREPARATION 38

### 3,5-Dicyanomethoxybenzene

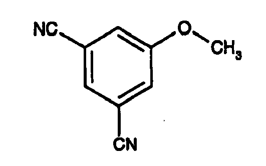

Tris(dibenzylideneacetone)dipalladium (6.53g, 7.15mmol) was added in one portion to a stirred solution of the bromide of Preparation 37 (38.0g, 143mmol) and zinc cyanide (20.0g, 172mmol) in *N,N*-dimethylformamide (300ml) at room temperature under nitrogen. The reaction was heated at 100°C for 14 hours and cooled to room temperature. Water (1500ml) was added and the mixture was extracted with ethyl acetate (3x500ml). The combined organics were filtered and the filtrate was washed with water (500ml), dried over magnesium sulphate, filtered and concentrated under reduced pressure. The resulting solid was triturated with toluene (1000ml) to provide the title compound (18.0g) as a tan solid.
¹H-NMR (300MHz, CDCl₃): δ = 3.83 (3H, s), 7.31 (2H, s), 7.48 (1 H, s).

### PREPARATION 39

### 3,5-Dicyanohydroxybenzene

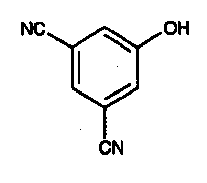

The nitrile of Preparation 38 (9.60g, 60.7mmol) was added portionwise to a stirred suspension of aluminium trichloride (32.4g, 243mmol) in dichloromethane (250ml) at 0°C under nitrogen. The suspension was heated to 45°C and stirred for 6 days. The reaction was cooled to room temperature and cautiously poured onto ice (450ml). Concentrated hydrochloric acid (450ml) was added dropwise and the resulting suspension was stirred for 10 minutes at room temperature. The resulting solid was collected by filtration, washed with water and dried over phosphorus pentoxide to provide the title compound (7.83g) as a tan solid containing approximately 11 % starting material by ¹H-NMR and microanalysis.
¹H-NMR (400MHz, CDCl₃): δ = 7.36 (m, 2H), 7.56 (m, 1H).

### PREPARATION 40

### 3-Methoxy-5-methylphenyl trifluoromethanesulfonate

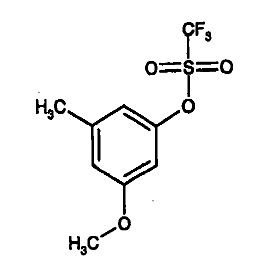

Trifluoromethanesulphonic anhydride (2.02ml, 12.0mmol) was added dropwise to a stirred solution of 3-methoxy-5-methylphenol (1.50g, 10.9mmol) in pyridine (20ml) at -20°C under nitrogen. The reaction was warmed to 0°C, stirred for 90 minutes and re-cooled to -20°C. More trifluoromethanesulphonic anhydride (1.01ml, 6.00mmol) was added dropwise. The reaction was allowed to warm to room temperature, stirred for 14 hours and cautiously poured into water (100ml). The aqueous phase was extracted with ether (150ml) and the organic phases were washed with water (3x75ml), 0.2M hydrochloric acid (3x75ml), 1.0M aqueous sodium carbonate solution (2x75ml), water (75ml) and brine (75ml), dried over magnesium sulphate, filtered and concentrated under reduced pressure to provide the title compound (2.86g) as a pale brown oil.
¹H-NMR (400MHz, CDCl₃): δ = 2.35 (s, 3H), 3.80 (s, 3H), 6.60 (s, 1H), 6.68 (s, 1H), 6.73 (s, 1H).

### PREPARATION 41

### 3-Methoxy-5-methylbenzonitrile

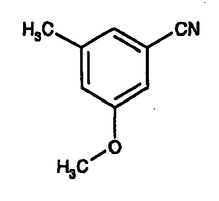

The triflate of Preparation 40 (1.94g, 7.10mmol), dibromobis(triphenylphosphine)nickel (369mg, 0.490mmol), 1,1'bis(diphenylphosphino)ferrocene (331 mg, 0.590mmol) and potassium cyanide (1.38g, 21.3mmol) were added consecutively to a stirred suspension of Rieke® zinc (supplied by the Aldrich chemical company as a suspension; 5g Zinc in 100ml tetrahydrofuran) (74mg, 1.14mmol) in acetonitrile (4ml) at room temperature. The reaction was heated to 75°C for 8 hours and then cooled to room temperature. The mixture was partitioned between ether (200ml) and water (150ml) and the organic phase was separated, washed with water (2x100ml) and brine (75ml), dried over magnesium sulphate, filtered and concentrated under reduced pressure to give a pale brown oil. The crude product was purified by flash chromatography on silica gel eluting with pentane:ethyl acetate (85:15, by volume) to provide the title compound (815mg) as a white solid.
¹H-NMR (400MHz, CDCl₃): δ = 2.34 (s, 3H), 3.80 (s, 3H), 6.93 (s, 1 H), 6.94 (s, 1 H), 7.04 (s, 1 H).

### PREPARATION 42

### 3-Hydroxy-5-methylbenzonitrile

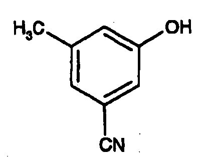

Boron trichloride (17.6ml of a 1.0M solution in dichloromethane, 17.6mmol) was added dropwise to a stirred solution of the nitrile of Preparation 41 (866mg, 5.88mmol) and tetrabutylammonium iodide (2.61 g, 7.05mmol) in dichloromethane (50ml) at -78°C. The reaction was allowed to warm to room temperature and stirred for 20 minutes. The reaction was cooled to 0°C, cautiously quenched with ice and diluted with dichloromethane (100ml). The organic phase was separated, dried over magnesium sulphate, filtered and concentrated under reduced pressure. The crude product was purified by flash chromatography on silica gel eluting with pentane:ethyl acetate (50:50, by volume) to provide the title compound (677mg) as a white solid.
¹H-NMR (400MHz, CDCl₃): δ = 2.32 (s, 3H), 5.05 (s, 1 H), 6.88 (s, 1H), 6.90 (s, 1 H), 7.04 (s, 1 H).

### PREPARATIONS 43 TO 46

The compounds of the following tabulated Preparations of the general formula: 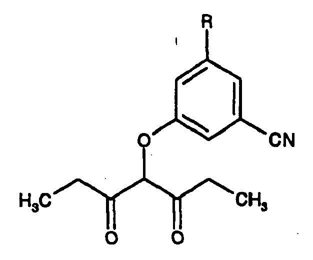 were prepared by a similar method to that of Preparation 9 using the appropriate phenol starting material and the chloride of Preparation 2.

| Preparation No. (Phenol No.) | R | LRMS | Analytical Data |
|---|---|---|---|
| 43 (Phenol Preparation 34) | F | m/z [MNH₄⁺] 281. (thermospray) | ¹H-NMR (300MHz, CDCl₃): δ = 1.05 (t, 6H), 2.27 (q, 4H), 6.89 (m, 1H), 7.03 (s, 1H), 7.04 (m, 1H). |
| 44 (Phenol Preparation 42) | Me | m/z [M-H⁺] 258. (electrospray) | ¹H-NMR (400MHz, CDCl₃): δ = 1.09 (t, 6H), 2.32 (q, 4H), 2.37 (s, 3H), 6.96 (s, 1H), 6.97 (s, 1H), 7.15 (s, 1H), 14.50 (s, 1H). |
| 45 (Phenol Preparation 39) | CN | m/z [M-H⁺] 269. (electrospray) | ¹H-NMR (300MHz, CDCl₃): δ = 1.09 (m, 6H), 2.30 (m, 4H), 7.42 (s, 2H), 7.61 (s, 1H), 14.56 (s, 1H). |
| 46 (Phenol Preparation 36) | Cl | m/z [MH⁺] 280. (thermospray) | ¹H-NMR (400MHz, CDCl₃): δ = 1.08 (m, 6H), 2.31 (q, 4H), 7.12 (s, 1H), 7.19 (s, 1H), 7.31 (s, 1H). |

### PREPARATION 47

### 1-Cyclopropyl-1,3-pentanedione

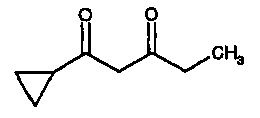

A stirred suspension of magnesium turnings (1.83g, 75.0mmol) in methanol (85ml) was heated under reflux for 90 minutes. The suspension was cooled to room temperature and a solution of 3-ketopentanoic acid (17.4g, 150.0mmol) in methanol (15ml) was added. The white suspension dissolved to give a pale yellow solution. The reaction was stirred at room temperature for 1 hour and then concentrated under reduced pressure to give a pale yellow solid which was dissolved in *N,N-*dimethylformamide (50ml). In a separate flask carbonyldiimidazole (13.4g, 83.0mmol) was added portionwise to a stirred solution of cyclopropanecarboxylic acid (6.46g, 75.0mmol) in *N,N*-dimethylformamide (150ml) at room temperature under nitrogen. The reaction was stirred for 90 minutes and then the magnesium salt previously prepared was added dropwise. The reaction was stirred for 3 days and then poured into 1.0M hydrochloric acid (150ml). The aqueous phase was extracted with ether (3x200ml) and the combined organic phases were dried over magnesium sulphate, filtered and concentrated under reduced pressure. The crude product was purified by flash chromatography on silica gel eluting with pentane:ethyl acetate (90:10, by volume) to provide the title compound (9.33g) as a yellow oil.
¹H-NMR (400MHz, CDCl₃): keto and enol forms present with enol as major component; enol signals δ = 1.00 (m, 7H), 1.60 (m, 1H), 2.25 (m, 2H), 5.59 (s, 1 H), 15.62 (s, 1 H); keto signals δ = 1.00 (m, 7H), 2.01 (m, 1 H), 2.52 (m, 2H), 3.68 (s, 2H).
LRMS (electrospray): m/z [M-H⁺] 139.
Microanalysis: Found: C, 68.35; H, 8.72. C₈H₁₂O₂ requires C, 68.55; H, 8.63%.

### PREPARATION 48

The compound of the following tabulated Preparation of the general formula: 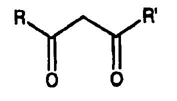 was prepared by a similar method to that of Preparation 47 using the appropriate ketoacid and carboxylic acid starting materials.

| Preparation No. | R | R' | LRMS | Analytical Data |
|---|---|---|---|---|
| 48 | iPr | Et | m/z [M-H⁺] 141. (electrospray) | ¹H-NMR (400MHz, CDCl₃): keto and enol forms present with enol major δ =1.12 (m, 18H, keto and enol), 2.32 (m, 4H, keto and enol), 2.49 (m, 2H, keto and enol), 3.61 (s, 2H, keto), 5.49 (s, 1H, enol), 15.52 (s, 1H, enol). |
| | | | | Microanalysis: Found: C, 67.22; H, 9.95. C₈H₁₄O₂ requires C, 67.57; H, 9.92%. |

### PREPARATIONS 49 TO 51

The compounds of the following tabulated Preparations of the general formula: 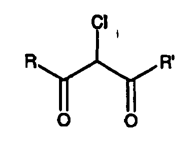 were prepared by a similar method to that of Preparation 2 using the appropriate diketone starting material.

| Preparation No. (Diketone No.) | R | R' | LRMS | Analytical Data |
|---|---|---|---|---|
| 49 (Preparation 47) | cycloPr | Et | m/z [M-H⁺] 173. (electrospray) | ¹H-NMR (400MHz, CDCl₃): 1.10 (m, 7H), 2.41 (m, 1H), 2.61 (m, 2H), 15.90 (s, 1H). |
| 50 (Commercially available diketone used) | Me | Et | m/z [MNH₄⁺] 166. (thermospray) | ¹H-NMR (300MHz, CDCl₃): 1.19 (m, 3H), 2.27 **(s,** 3H), 2.67 (q, 2H), 15.40 (s, 1H). |
| 51 (Preparation 48) | iPr | Et | m/z [M-H⁺] 175. (electrospray) | ¹H-NMR (400MHz, CDCl₃): 1.18 (m, 9H), 2.64 (q, 2H), 3.20 (m, 1H), 15.80 (s, 1H). |

### PREPARATIONS 52 TO 54

The compounds of the following tabulated Preparations of the general formula: 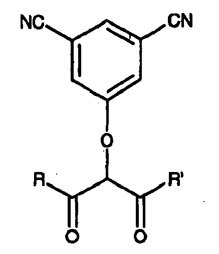 were prepared by a similar method to that of Preparation 9 using the appropriate diketone starting material and the phenol of Preparation 39.

| Preparation No. (Diketone No.) | R | R' | LRMS | Analytical Data |
|---|---|---|---|---|
| 52 (Preparation 49) | cycloPr | Et | m/z [M-H⁺] 282. (electrospray) | ¹H-NMR (400MHz, CDCl₃): 0.93 (m, 2H), 1.12 (t, 3H), 1.21 (m, 2H), 1.78 (m, 1H), 2.29 (q, 2H), 7.49 (s, 2H), 7.61 (s, 1H), 14.87 (s, 1H). |
| 53 (Preparation 19) | tBu | Me | m/z [MNH₄⁺] 301. (thermospray) | ¹H-NMR (400MHz, CDCl₃): 1.08 (s, 9H), 1.84 (s, 3H), 7.30 (s, 1H), 7.57 (s, 2H), 15.42 (s, 1H). |
| 54 (Preparation 51) | iPr | Et | m/z [M-H⁺] 283. (electrospray) | ¹H-NMR (400MHz, CDCl₃): 1.03 (m, 9H), 2.23 (q, 2H), 2.58 (m, 1H), 7.41 (s, 2H), 7.59 (s, 1H), 14.63 (s, 1H). |

### PREPARATION 55

### 4-(Aminomethyl)benzamide

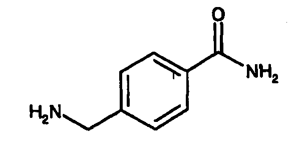

Powdered potassium hydroxide (340mg, 6mmol) was added in one portion to a stirred solution of 4-(aminomethyl)benzonitrile (200mg, 1.5mmol) in 2-methyl-2-propanol (20ml) at reflux under nitrogen. The reaction was heated at reflux for 30 minutes and cooled to room temperature. The mixture was concentrated under reduced pressure and the crude product was purified by flash chromatography on silica gel eluting with dichloromethane:methanol:ammonia (95:5:0.5, by volume) to provide the title compound (150mg) as a white solid.
¹H-NMR (300MHz, CD₃OD): δ = 3.85 (s, 2H), 7.43 (d, 2H), 7.82 (d, 2H).
LRMS (thermospray): m/z [MH⁺] 151.

### PREPARATION 56

### 3-Oxopentanoic acid

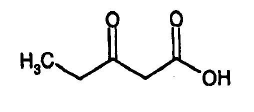

Sodium hydroxide (54g, 1.35mol) was added portionwise to a solution of 3-oxopentanoic acid methyl ester (80g, 0.62mol) in tetrahydrofuran (300ml) and water (300ml) at 0°C. The reaction was allowed to warm to room temperature and was stirred for 18 hours. The reaction mixture was washed with diethylether (500ml) and the aqueous phase was acidified to pH1 at 0°C with concentrated hydrochloric acid (140ml). The aqueous phase was extracted with dichloromethane (2x300ml) and the combined organic extracts dried over magnesium sulphate and concentrated under reduced pressure to provide the title compound (44g) as a white solid.
¹H NMR (400MHz, CDCl₃): δ = 1.12 (t, 3H), 2.59 (q, 2H), 3.49 (s, 2H).

### PREPARATION 57

### 3-(Benzyloxy)propanoic acid

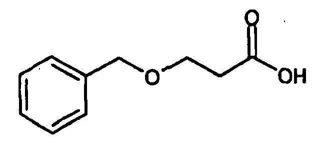

Sodium metal (249mg, 10.8mmol) was added to benzyl alcohol (30g, 278mmol) at room temperature under nitrogen and the reaction was stirred for 30 minutes. Methyl acrylate (25.9ml, 259mmol) was then added dropwise and the reaction was stirred at room temperature for 18h. After quenching with saturated aqueous ammonium chloride solution (200ml) the mixture was extracted with ethyl acetate (2x300ml) and the combined organic extracts were washed with brine (100ml), dried over magnesium sulphate and concentrated under reduced pressure. The residual oil was dissolved in ethanol (300ml) and 1M aqueous sodium hydroxide solution (300ml) was added dropwise. After 3 hours the ethanol was removed under reduced pressure and the aqueous residue was washed with dichloromethane (200ml). The aqueous phase was then acidified with 2N aqueous hydrochloric acid (150ml), extracted with dichloromethane (2x250ml) and the combined organic extracts were dried over magnesium sulphate and concentrated under reduced pressure. The residual oil was dissolved in 10% aqueous potassium carbonate solution (300ml), washed with diethylether (300ml) and the aqueous phase was acidified to pH1 using concentrated hydrochloric acid. The mixture was then extracted with dichloromethane (2x300ml) and the combined organic extracts were dried over magnesium sulphate and concentrated under reduced pressure to provide the title compound (44.4g) as a colourless oil.
¹H NMR (300MHz, CDCl₃): δ = 2.67 (t, 2H), 3.89 (t, 2H), 4.58 (s, 2H), 7.18 (m, 5H).

### PREPARATION 58

### (4Z)-1-(Benzyloxy)-5-hydroxy-4-hepten-3-one

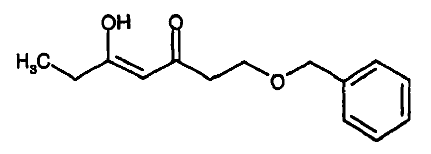

A suspension of magnesium turnings (1.74g, 71.6mmol) in methanol (85ml) was heated to reflux under nitrogen for 1.5 hours, cooled to room temperature and the β-keto acid from Preparation 56 (16.6g, 143mmol) was added. The reaction was stirred for 1.5 hours and the solvent was removed under reduced pressure to give the magnesium salt of the acid as a white solid. Meanwhile, the acid from Preparation 57 (12.9g, 71.6mmol) was dissolved in dimethylformamide (150ml) and carbonyldiimidazole (12.8g, 78.8mmol) was added portionwise under nitrogen at room temperature. This was stirred for 1 hour and the magnesium salt from above was added as a solution in dimethylformamide (50ml). Evolution of gas was noted, and the reaction was allowed to stir at room temperature for 18 hours. The mixture was concentrated under reduced pressure and the residual orange oil was dissolved in dichloromethane (300ml), washed with 0.5M aqueous hydrochloric acid (250ml) containing methanol (10ml) and the aqueous phase was separated and extracted with dichloromethane (2x300ml). The combined organic extracts were washed with brine (300ml) containing methanol (20ml), dried over magnesium sulphate and concentrated under reduced pressure. The residual orange oil was purified by flash chromatography on silica gel eluting with cyclohexane:ethyl acetate (80:20, by volume) to provide the title compound (12.0g) as an orange oil.
¹H NMR (400MHz, CDCl₃): δ =1.17 (t, 3H), 2.33 (q, 2H), 2.58 (t, 2H), 3.76 (t, 2H), 4.53 (s, 2H), 5.57 (s, 1 H), 7.13 (m, 5H).
LRMS (electrospray) : m/z [MNa⁺] 257.
Microanalysis: Found C, 71.77; H, 7.74. C₁₄H₁₈O₃ requires C, 71.76; H, 7.69%.

### PREPARATION 59

### (4E)-1-(Benzyloxy)-4-chloro-5-hydroxy-4-hepten-3-one

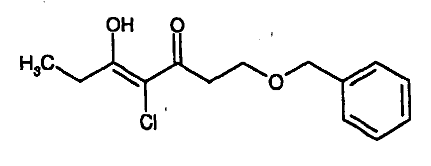

Trimethylsilyl chloride (10ml, 51.3mmol) was added to a solution of the enol from Preparation 58 (4.0g, 17.1mmol) in acetonitrile (25ml) under nitrogen at 0°C. Dimethylsulfoxide (3.6ml, 51.3mmol) followed by tert-butylammonium bromide (275mg, 0.85mmol) were then added and the reaction was stirred at 0°C for 2 hours. The mixture was diluted with water (100ml), extracted with diethylether (100ml) and the organic phase was washed with brine (50ml), dried over magnesium sulphate and concentrated under reduced pressure. The residual pink oil was purified by flash chromatography on silica gel eluting with cyclohexane:ethyl acetate (80:20, by volume) to provide the title compound (3.76g) as a pink oil.
¹H NMR (400MHz, CDCl₃): δ = 1.17 (t, 3H), 2.62 (q, 2H), 2.96 (t, 2H), 3.79 (t, 2H), 4.57 (s, 2H), 7.12 (m, 5H), 15.49 (s, 1H).
LRMS (electrospray) : m/z [MNa⁺] 291.

### PREPARATION 60

### 3-({(1E)-1-[3-(benzyloxy)propanoyl]-2-hydroxy-1-butenyl}oxy)-5-fluorobenzonitrile

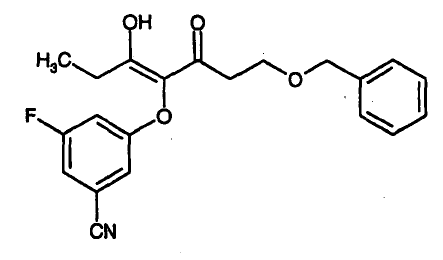

Sodium hydride (60% dispersion in oil, (1.92g, 48.0mmol) was added to a stirred solution of the phenol from Preparation 34 (8.80g, 48.0mmol) in tetrahydrofuran (450ml) under nitrogen at room temperature. After stirring for 1 hour, the enol from Preparation 59 (12.9g, 48.0mmol) was added and the reaction was stirred for 64 hours. The mixture was diluted with water (200ml) and 2N aqueous hydrochloric acid (40ml), extracted with ethyl acetate (2x150ml) and the combined organic extracts were washed with brine (100ml), dried over magnesium sulphate and concentrated under reduced pressure. The residual orange oil was purified by flash chromatography on silica gel eluting with cyclohexane:pentane (10:90, by volume) to provide the title compound (5.80g) as an orange oil.
¹H NMR (400MHz, CDCl₃): δ= 1.08 (t, 3H), 2.31 (q, 2H), 2.59 (t, 2H), 3.75 (t, 2H), 4.45 (s, 2H), 6.92 (m, 1 H), 7.02 (m, 2H), 7.29 (m, 5H), 14.50 (s, 1H).
LRMS (electrospray) : m/z [MNa⁺] 392.

### PREPARATION 61

### 5-({(1E)-1-[3-(Benzyloxy)propanoyl]-2-hydroxy-1-butenyl}oxy)isophthalonitrile

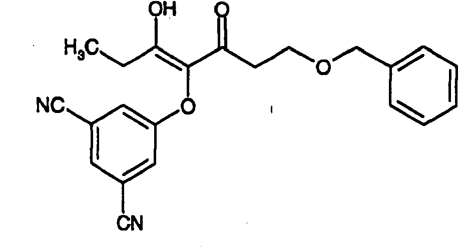

Sodium hydride (60% dispersion in oil, 412mg, 12.3mmol) was added to a stirred solution of the phenol from Preparation 39 (1.48g, 10.3mmol) in tetrahydrofuran (70ml) under nitrogen at room temperature. After stirring for 30 minutes, the enol from Preparation 59 (2.76g, 10.3mmol) was added and the reaction was stirred for 18 hours. Water (100ml) and 2N aqueous hydrochloric acid (10ml) were cautiously added and the mixture extracted with ethyl acetate (2x150ml). The organics were combined, washed with brine (100ml), dried over magnesium sulphate and concentrated under reduced pressure. The residue was purified by flash column chromatography on silica gel eluting with (pentane:ethyl acetate 90:10, by volume) to provide the title compound (1.00g) as a yellow oil.
LRMS (thermospray) : m/z [MH⁺] 375.

### PREPARATION 62

### 3-{[1-(2-{[tert-Butyl(dimethyl)silyl]oxy}ethyl)-3,5-diethyl-1H-pyrazol-4-yl]oxy}-5-fluorobenzonitrile

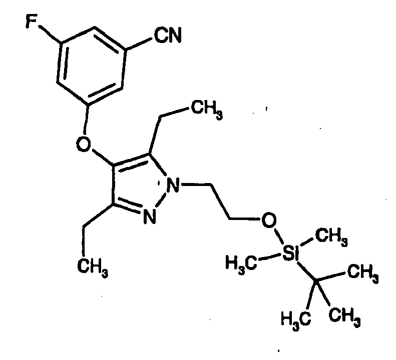

Imidazole (477mg, 7.02mmol) and tert-butyl-dimethyl-silyl chloride (977mg, 6.48mmol) were sequentially added to a solution of the alcohol from Example 117 (1.65g, 5.40mmol) in dimethylformamide (11 ml) at room temperature under nitrogen. The reaction was stirred for 18 hours and the mixture was diluted with water (100ml) and extracted with diethylether (4x50ml). The combined organic extracts were dried over magnesium sulphate, concentrated under reduced pressure and the residue was purified by flash chromatography on silica gel eluting with dichloromethane:methanol (99:1, by volume) to provide the title compound (2.12g) as a colourless oil.
¹H NMR (400MHz, CDCl₃): δ = 0.03 (s, 6H), 0.84 (s, 9H), 1.10 (m, 6H), 2.42 (q, 2H), 2.56 (q, 2H), 4.00 (t, 2H), 4.09 (t, 2H), 6.86 (d, 1 H), 6.99 (m, 2H).
LRMS (thermospray) : m/z [MH⁺] 419.
Microanalysis: Found C, 62.73; H, 7.83; N, 9.75. C₂₂H₃₂FN₃O₂Si.0.06CH₂Cl₂ requires C, 62.68; H, 7.66; N, 9.94%.

### PREPARATION 63

### 3-({3,5-Diethyl-1-[2-(tetrahydro-2H-pyran-2-yloxy)ethyl]-1H-pyrazol-4-yl}oxy)-5-fluorobenzonitrile

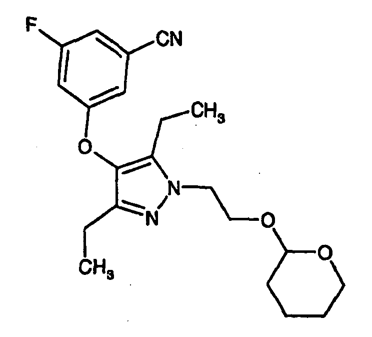

p-Toluene-sulphonic acid (32mg, 0.17mmol) was added to a solution of the alcohol from Example 117 (5.04g, 16.6mmol) and dihydropyran (7.57ml, 83mmol) in dichloromethane (65ml) at room temperature under nitrogen. The reaction was stirred for 2 hours, but starting material still remained so a further aliquot of p-toluene-sulphonic acid (284mg, 1.49mmol) was added and the reaction was stirred for 1 hour. The mixture was diluted with diethylether (90ml) and washed with a mixed aqueous solution (water (50ml), brine (25ml) and saturated aqueous sodium bicarbonate solution (25ml)). The aqueous phase was extracted with diethylether (2x60ml) and the combined organic extracts were dried over magnesium sulphate, concentrated under reduced pressure and the residue was purified by flash chromatography on silica gel eluting with dichloromethane:methanol (98:2, by volume) to provide the title compound (6.31 g) as an oil.
¹H NMR (400MHz, CDCl₃): δ = 1.08 (m, 6H), 1.52 (m, 6H), 2.39 (q, 2H), 2.54 (q, 2H), 3.45 (m, 1 H), 3.64 (m, 1 H), 3.75 (m, 1 H), 4.06 (m, 1 H), 4.17 (t, 2H), 4.51 (s, 1 H), 6.82 (d, 1H), 7.22 (m, 2H).
LRMS (thermospray) : m/z [MH⁺] 388.

### PREPARATION 64

### 3-({3,5-Diethyl-1-[2-(tetrahydro-2H-pyran-2-yloxy)ethyl]-1H-pyrazol-4-yl}oxy)-5-fluorobenzamide

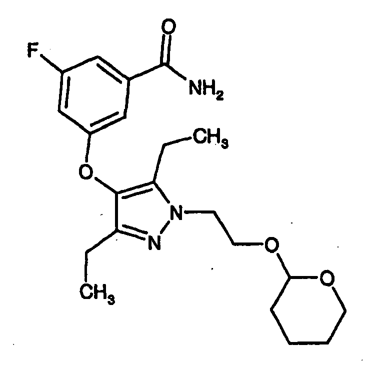

Cesium carbonate (269mg, 0.82mmol) was added to a solution of 3-methyl-3-pyrazolin-5-one (74mg, 0.75mmol) in dimethylsulfoxide (1ml) under nitrogen at room temperature and the reaction was stirred for 15 minutes. The aryl fluoride from Preparation 63 (291 mg, 0.75mmol) dissolved in dimethylsulfoxide (1ml) was then added and the reaction was heated to 100°C for 18 hours. After cooling to room temperature the reaction was diluted with water (7ml) and extracted with diethylether (12ml). The organic phase was washed with brine (3.5ml), dried over magnesium sulphate, concentrated under reduced pressure and the residue was purified by flash chromatography on silica gel eluting with a solvent gradient of dichloromethane:methanol (99:1 changing to 95:5, by volume) to provide the unexpected title compound (108mg) as an oil.
¹H NMR (400MHz, CDCl₃): δ = 1.12 (m, 6H), 1.56 (m, 6H), 2.44 (q, 2H), 2.59 (q, 2H), 3.48 (m, 1H), 3.69 (m, 1H), 3.79 (m, 1 H), 4.08 (m, 1H), 4.20 (t, 2H), 4.54 (s, 1 H), 6.72 (d, 1 H), 7.15 (m, 2H).
LRMS (thermospray) : m/z [MH⁺] 406.
Microanalysis: Found C, 60.57; H, 6.97; N, 9.97. C₂₁H₂₈FN₃O₄.0.08CH₂Cl₂.0.32H₂O requires C, 60.57; H, 6.94; N, 10.05%.

### PREPARATION 65

### 3-({3,5-Diethyl-1-[2-(tetrahydro-2H-pyran-2-yloxy)ethyl]-1H-pyrazol-4-yl}oxy)-5-(1H-pyrazol-1-yl)benzonitrile

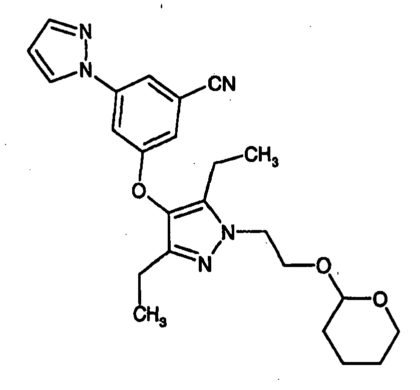

Cesium Carbonate (269mg, 0.82mmol) was added to a solution of pyrazole (51mg, 0.75mmol) in dry dimethylsulfoxide (1ml) under nitrogen at room temperature and the reaction was stirred for 15 minutes. The aryl fluoride from Preparation 63 (291 mg, 0.75mmol) dissolved in dry dimethylsulfoxide (1 ml) was then added and the reaction was heated to 100°C for 18 hours. After cooling to room temperature the reaction was diluted with water (7ml) and extracted with diethylether (10ml). The organic phase was washed with brine (3ml), dried over magnesium sulphate, concentrated under reduced pressure and the residue was purified by flash chromatography on silica gel eluting with a solvent gradient of dichloromethane:methanol (100:0 changing to 90:10, by volume) to provide the title compound (55mg).
¹H NMR (400MHz, CDCl₃): δ = 1.13 (m, 6H), 1.58 (m, 6H), 2.44 (q, 2H), 2.60 (q, 2H), 3.49 (m, 1 H), 3.69 (m, 1 H), 3.80 (m, 1 H), 4.10 (m, 1 H), 4.21 (t, 2H), 4.55 (s, 1 H), 6.50 (s, 1 H), 6.98 (s, 1 H), 7.57 (s, 1 H), 7.63 (s, 1 H), 7.72 (s, 1 H), 7.89 (s, 1 H).
LRMS (thermospray): m/z [MH⁺] 436, [MNa⁺] 458.
HRMS: [MH⁺] Found 436.2352. C₂₄H₃₀N₅O₃ requires 436.2343
[MNa⁺] Found 458.2168. C₂₄H₂₉N₅O₃Na requires 458.2162.

### PREPARATIONS 66-68

The preparation of the following tabulated Preparations of the general formula 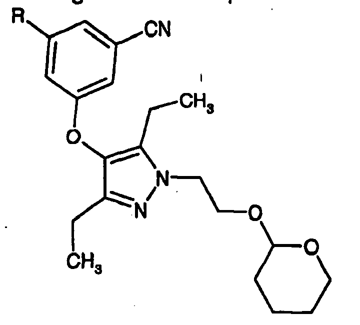 were performed by a similar method to that of Preparation 65 using the appropriate heterocycle as the starting material.

| Preparation No. (Starting material preparation no.) | R | Analytical Data |
|---|---|---|
| 66 (63) | 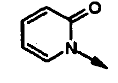 | ¹H NMR (400MHz, CDCl₃): δ = 1.13 (m, 6H), 1.63 (m, 6H), 2.44 (q, 2H), 2.60 (q, 2H), 3.46 (m, 1H), 3.67 (m, 1 H), 3.79 (m, 1 H), 4.08 (m, 1 H), 4.20 (t, 2H), 4.53 (s, 1 H), 6.26 (t, 1 H), 6.64 (d, 1 H), 7.17 (s, 1 H), 7.21 (s, 1 H), 7.34 (s, 1 H), 7.41 (t, 1 H). LRMS (thermospray) : m/z [MH⁺] 463, [MNa⁺] 485. HRMS: [MH⁺] Found 463.2353. C₂₆H₃₁N₄O₄ requires 463.2340 [MNa⁺] Found 485.2166. C₂₆H₃₀N₄O₄Na requires 485.2159. |
| 67 (63) | 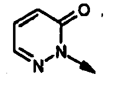 | ¹H NMR (400MHz, CDCl₃): δ = 1.10 (m, 6H), 1.56 (m, 6H), 2.41 (q, 2H), 2.56 (q, 2H), 3.44 (m, 1 H), 3.64 (m, 1 H), 3.75 (m, 1 H), 4.05 (m, 1 H), 4.17 (t, 2H), 4.50 (s, 1 H), 7.00 (d, 1 H), 7.08 (s, 1 H), 7.20 (m, 1 H), 7.51 (s, 1 H), 7.64 (s, 1 H), 7.86 (s, 1 H). LRMS (thermospray) : m/z [MH⁺] 464, [MNa⁺] 486. HRMS: [MH⁺] Found 464.2297. C₂₅H₃₀N₅O₄ requires 464.2293 [MNa⁺] Found 486.2113. C₂₅H₂₉N₅O₄Na requires 486.2112. |
| 68¹ (63) | 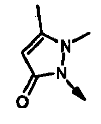 | ¹H NMR (400MHz, CDCl₃): δ = 1.08 (m, 6H), 1.48 (m, 6H), 2.23 (s, 3H), 2.38 (q, 2H), 2.53 (q, 2H), 3.43 (m, 1 H), 3.63 (m, 1 H), 3.66 (s, 3H), 3.73 (m, 1 H), 4.04 (m, 1 H), 4.15 (t, 2H), 4.50 (s, 1 H), 5.59 (s, 1 H), 6.76 (s, 1 H), 6.88 (s, 1 H), 6.95 (s, 1 H). LRMS (thermospray) : m/z [MH⁺] 480, [MNa⁺] 502. |

| | | |
|---|---|---|
| ¹ The eluent used for flash column chromatography purification of this compound was dichloromethane:methanol (99:1 changing to 95:5, by volume). | | |

### PREPARATION 69

### tert-Butyl 3-[4-(3,5-dicyanophenoxy)-3,5-diethyl-1H-pyrazol-1-yl]-1-azetidinecarboxylate

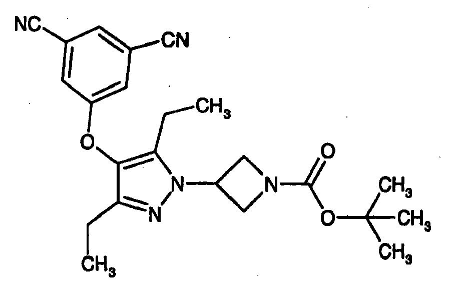

Sodium hydride (60% dispersion in oil, 33mg, 0.82mmol) was added to a solution of the pyrazole from Example 122 (200mg, 0.75mmol) in dimethylformamide (3ml) at 0°C under nitrogen and the reaction was stirred for 10 minutes. 3-lodo-azetidine-1-carboxylic acid tert-butyl ester (234mg, 0.82mmol) was added and the reaction was stirred at room temperature for 18 hours. The reaction was quenched with water (0.2ml) and concentrated under reduced pressure. The residue was partitioned between dichloromethane (5ml) and water (5ml) and the organic phase was isolated using a 5µM Whatman PTFE fritted cartridge, then concentrated under reduced pressure. The residue was purified by flash chromatography on silica gel eluting with a solvent gradient of ethyl acetate:pentane (20:80 then 25:75 then 34:66 then 50:50 then 75:25 then 100:0, by volume) changing to ethyl acetate:methanol (10:1, by volume) then dichloromethane:methanol:0.88 ammonia (90:10:1 then 80:20:1, by volume) to provide the title compound (189mg) as a pale yellow oil.
¹H NMR (400MHz, CDCl₃): δ = 1.03-1.17 (m, 6H), 1.49 (s, 9H), 2.39-2.52 (m, 4H), 4.32 (m, 2H), 4.50 (m, 2H), 4.94 (m, 1 H), 7.38 (s, 2H), 7.56 (s, 1 H).
LRMS (thermospray) : m/z [MH⁺] 422, [MNa⁺] 444.
Microanalysis: Found C, 65.08; H, 6.49; N, 16.48. C₂₃H₂₇N₅O₃.0.18H₂O requires C, 65.04; H, 6.49; N, 16.49%.

### PREPARATION 70

### 5-({3,5-Diethyl-1-[3-(tetrahydro-2H-pyran-2-yloxy)propyl]-1H-pyrazol-4-yl}oxy)isophthalonitrile

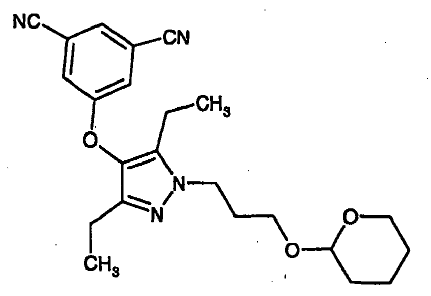

Sodium hydride (60% dispersion in oil, 33mg, 0.82mmol) was added to a solution of the pyrazole from Example 122 (200mg, 0.75mmol) in dimethylformamide (3ml) at 0°C under nitrogen and the reaction was stirred for 10 minutes. 2-(3-bromo-propoxy)-tetrahydro-pyran (184mg, 0.82mmol) was added and the reaction was stirred at room temperature for 18 hours. The reaction was quenched with water (0.2ml) and concentrated under reduced pressure. The residue was partitioned between dichloromethane (5ml) and water (5ml) and the organic phase was isolated using a 5µM Whatman PTFE fritted cartridge, then concentrated under reduced pressure. The residue was purified by flash chromatography on silica gel eluting with a solvent gradient of ethyl acetate:pentane (20:80 then 25:75 then 34:66 then 50:50 then 75:25 then 100:0, by volume) changing to ethyl acetate:methanol (10:1, by volume) then dichloromethane:methanol:0.88 ammonia (90:10:1 then 80:20:1, by volume) to provide the title compound (238mg) as a pale yellow oil.
¹H NMR (400MHz, CDCl₃): δ = 1.09 (m, 6H), 1.47-1.63 (m, 2H), 1.66-1.88 (m, 2H), 2.15 (dd, 2H), 2.38 (q, 2H), 2.53 (q, 2H), 3.37-3.55 (m, 2H), 3.75-3.90 (m, 2H), 4.11 (m, 2H), 4.56 (m, 1 H), 7.37 (s, 2H), 7.55 (s, 1H).
LRMS (electro) : m/z [MH⁺] 409, [MNa⁺] 421.
Microanalysis: Found C, 66.59; H, 6.91; N, 13.40. C₂₃H₂₈N₄O₃.0.36H₂O requires C, 66.57; H, 6.98; N, 13.50%.

### PREPARATION 71

### 3-[(1-Acetyl-3,5-dimethyl-1H-pyrazol-4-yl)oxy]-5-fluorobenzonitrile

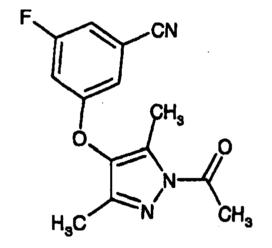

The phenol from Preparation 34 (10.0g, 72.7mmol), 3-chloro-2,4-pentanedione (7.10g, 72.7mmol) and cesium carbonate (23.6g, 72.9mmol) were heated to reflux in acetone (100ml) under nitrogen for 2 hours. The reaction was cooled to room temperature, 1 N aqueous hydrochloric acid (50ml) was added slowly and the mixture was extracted with ethyl acetate (3x100ml). The combined organic extracts dried over magnesium sulphate and concentrated under reduced pressure. The residual yellow oil was dissolved in methanol (100ml), hydrazine (5.3ml, 109mmol) was added and the reaction was stirred at room temperature under nitrogen for 2 hours. The solvent was removed under reduced pressure and the residue was dissolved in dimethylformamide (50ml) at 0°C. Acetyl chloride (5.1ml, 72.0mmol) was added slowly followed by sodium hydride (60% dispersion in oil, 2.8g, 72.0mmol) portionwise. The reaction was stirred for 15 minutes and sat. ammonium chloride solution (50ml) was added, and the reaction was allowed to warm to room temperature. The mixture was extracted with ethyl acetate (3x100ml) and the combined organic extracts were dried over magnesium sulphate and concentrated under reduced pressure giving an oil. After standing for 18 hours, a solid had formed within the oil which was isolated by filtration, washing with diethylether (50ml) to provide the title compound (3.50g) as a white solid, m.p. 109-111 °C.
¹H NMR (400MHz, CDCl₃): δ = 2.06 (s, 3H), 2.37 (s, 3H), 2.65 (s, 3H), 6.81 (d, 1H), 6.91 (s, 1 H), 7.04 (d, 1 H).
LRMS (thermospray) : m/z [MH⁺] 273.
Microanalysis: Found C, 61.62; H, 4.44; N, 15.09. C₁₄H₁₂N₃O₂F requires C, 61.53; H, 4.43; N, 15.38%.

### PREPARATIONS 72-74

The tabulated compounds of the general formula 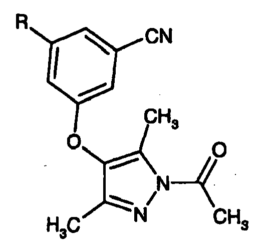 were performed by a similar method to that of Preparation 71 using the appropriate phenol as the starting material.

| Preparation no. (Starting material preparation no.) | R' | Analytical Data |
|---|---|---|
| 72 (39) | CN | m.p. 204-206°C ¹H NMR (400MHz, CDCl₃): δ = 2.06 (s, 3H), 2.38 (s, 3H), 2.66 (s, 3H), 7.33 (s, 2H), 7.58 (s, 1H). LRMS (thermospray) : m/z [MH⁺] 281. Microanalysis: Found C, 63.30; H, 4.25; N, 19.59. C₁₅H₁₂N₄O₂.0.30H₂O requires C, 63.06; H, 4.45; N, 19.61 %. |
| 73¹ (42) | Me | m.p. 152-154°C ¹H NMR (400MHz, CDCl₃): δ = 2.05 (s, 3H), 2.33 (s, 3H), 2.38 (s, 3H), 2.66 (s, 3H), 6.88 (s, 1 H), 6.91 (s, 1H), 7.12 (s, 1H). LRMS (thermospray) : m/z [MH⁺] 270. Microanalysis: Found C, 66.67; H, 5.71; N, 15.25. C₁₅H₁₅N₃O₂ requires C, 66.9; H, 5.61; N, 15.60%. |
| 74² (Commercial) | H | m.p. 131-133°C ¹H NMR (400MHz, CDCl₃): δ = 2.13 (s, 3H), 2.40 (s, 3H), 2.70 (s, 3H), 7.15 (m, 2H), 7.35 (m, 1 H), 7.40 (m, 1 H). LRMS (thermospray) : m/z [MH⁺] 278. Microanalysis: Found C, 65.87; H, 5.11; N, 16.33. C₁₄H₁₃N₃O₂ requires C, 65.87; H, 5.13; N, 14.46%. |

| | | |
|---|---|---|
| ¹ The product was purified by flash column chromatography on silica gel eluting with ethyl acetate:pentane (10:90, by volume). ² The product was purified by flash column chromatography on silica gel eluting with ethyl acetate:pentane (10:90 changing to 20:80, by volume). | | |

### PREPARATION 75

### 3-{[1-Acetyl-3-(bromomethyl)-5-methyl-1H-pyrazol-4-yl]oxy}-5-fluorobenzonitrile

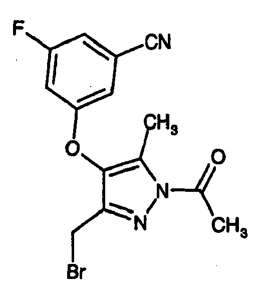

The pyrazole from Preparation 71 (1.00g, 3.66mmol) was dissolved in carbon tetrachloride (20ml) and the solution was degassed by bubbling nitrogen through it for 20 minutes at room temperature. *N-*Bromosuccinimide (973mg, 5.49mmol) followed by 2,2'-azobisisobutyronitrile (30mg) were added and the reaction was heated to 95°C for 1 hour. The reaction was cooled to room temperature, concentrated under reduced pressure and purified by flash chromatography on silica gel eluting with pentane:ethyl acetate (80:20, by volume) to provide the title compound (1.30g) as a pale yellow oil.
¹H NMR (400MHz, CDCl₃): δ = 2.05 (s, 3H), 2.69 (s, 3H), 4.68 (s, 2H), 6.89 (d, 1H), 6.99 (s, 1H), 7.08 (d, 1H).
LRMS (thermospray) : m/z [M-BrH⁺] 272.
Microanalysis: Found C, 45.08; H, 3.14; N, 11.44. C₁₄H₁₁BrN₃O₂F.1.05H₂O requires C, 45.31; H, 3.56; N, 11.32%.

### PREPARATIONS 76-78

The preparation of the following tabulated Preparations of the general formula 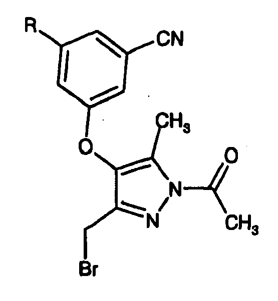 were performed by a similar method to that of Preparation 75 using the appropriate pyrazole as the starting material.

| Preparation no. (Starting material preparation no.) | R | Analytical Data |
|---|---|---|
| 76 (72) | CN | m.p. 132-134 °C ¹H NMR (400MHz, CDCl₃): δ = 2.06 (s, 3H), 2.66 (s, 3H), 4.67 (s, 2H), 7.40 (s, 2H), 7.63 (s, 1H). Microanalysis: Found C, 47.65; H, 3.03; N, 14.79. C₁₅H₁₁BrN₄O₂.0.93H₂O requires C, 47.92; H, 3.45; N, 14.90%. |
| 77^{1,2} (73) | Me | m.p. 107-109°C ¹H NMR (400MHz, CDCl₃): δ = 2.05 (s, 3H), 2.35 (s, 3H), 2.70 (s, 3H), 4.70 (s, 2H), 6.95 (s, 1 H), 6.99 (s, 1 H), 7.18 (s, 1 H). Microanalysis: Found C, 50.34; H, 3.89; N, 11.58. C₁₅H₁₄BrN₃O₂.0.40H₂O requires C, 50.69; H, 4.20; N, 11.82%. |
| 78^{1,3} (74) | H | m.p. 120-124°C ¹H NMR (400MHz, CDCl₃): δ = 2.05 (s, 3H), 2.70 (s, 3H), 4.75 (s, 2H), 7.20 (m, 2H), 7.45 (m, 1 H). Microanalysis: Found C, 49.01; H, 3.47; N, 12.14. C₁₄H₁₂BrN₃O₂.0.50H₂O requires C, 49.00; H, 3.82; N, 12.24%. |

| | | |
|---|---|---|
| ¹ A further aliquot of 2,2'-azobisisobutyronitrile (30mg) was added to this reaction, and refluxing was continued for a further 2 hours. ²The product was purified by flash column chromatography on silica gel eluting with a solvent gradient of ethyl acetate:pentane (0:100 then 2:98 then 5:95 then 10:90 then 15:85 then 30:70, by volume). ³ The product was purified by flash column chromatography on silica gel eluting with ethyl acetate:pentane (10:90 changing to 20:80, by volume). | | |

### PREPARATION 79

### 3-Cyanobenzamide

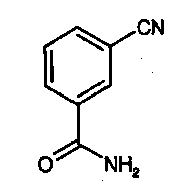

0.88 Ammonia solution (30ml) was slowly added to a solution of 3-cyanobenzoyl chloride (10g, 60.3mmol) in dichloromethane (100ml) at 0°C under nitrogen and the reaction was stirred for 20 minutes. The mixture was filtered and the solid was washed with water (50ml) then diethylether (50ml), azeotroped with toluene and dried *in vacuo* to provide the title compound (9g) as a white solid.
¹H NMR (400MHz, CD₃OD): δ = 7.62 (m, 1 H), 7.86 (m, 1 H), 8.12 (m, 1 H), 8.18 (s, 1 H).

### PREPARATION 80

### 3-(Aminomethyl)benzamide

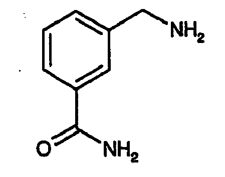

The nitrile from Preparation 79 (6.4g, 43.8mmol) was suspended in acetic acid (60ml) and 10% palladium on carbon (100mg) was added. The reaction was pressurised to 60psi at room temperature with hydrogen, and stirred for 18 hours. Starting material remained, so a further aliquot of 10% palladium on carbon (500mg) was added and the procedure was repeated. The reaction mixture was filtered through arbocel washing with acetic acid and the filtrate was concentrated under reduced pressure. The residue was azeotroped with toluene and purified by flash chromatography on silica gel eluting with dichloromethane:methanol:0.88 ammonia (100:0:0 changing to 90:10:1 then 85:15:1.5, by volume) to provide the title compound (5.3g) as a colourless oil.
¹H NMR (400MHz, CD₃OD): δ = 3.83 (s, 2H), 7.39 (dd, 1 H), 7.49 (d, 1 H), 7.73 (d, 1H), 7.81 (s, 1 H).

### PREPARATION 81

### 2-Chloro-1,3-dicyclopropyl-1,3-propanedione

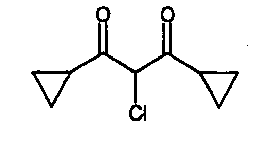

Trimethylsilyl chloride (16.6ml, 130mmol) was added to a solution of tert-butylammonium bromide (0.70g, 2.17mmol) in acetonitrile (50ml) under nitrogen at 0°C. 1,3-Dicyclopropyl-propane-1,3-dione (ref: WO98155438) (6.62g, 43.5mmol) in acetonitrile (15ml) was then added followed by dimethylsulfoxide (9.25ml, 130mmol) dropwise, and the reaction was allowed to warm to room temperature over 4 hours. The mixture was diluted with water (75ml), extracted with diethylether (3x35ml) and the combined organic extracts dried over magnesium sulphate and concentrated under reduced pressure. The residue was purified by flash chromatography on silica gel eluting with pentane:diethylether (95:5, by volume) to provide the title compound (3.76g) as an oil, which was an 80:20 mixture of enol:keto forms.
¹H NMR (400MHz, CDCl₃): δ = 1.02 (m, 4H), 1.17 (m, 4H), 2.24 (m, 0.2H), 2.39 (m, 0.8H), 5.05 (s, 0.2H), 16.34 (s, 0.8H).
Microanalysis: Found C, 57.59; H, 5.89. C₉H₁₁ClO₂.0.02CH₂Cl₂ requires C, 57.92; H, 5.94.

### PREPARATION 82

### 5-[2-Cyclopropyl-1-(cyclopropylcarbonyl)-2-oxoethoxy]isophthalonitrile

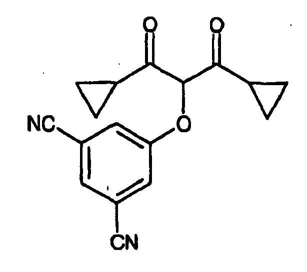

Cesium carbonate (1.97g, 6.06mmol) was added to a stirred solution of the phenol from Preparation 39 (0.865g, 6.00mmol) in acetone (24ml) under nitrogen at reflux. After stirring for 5 minutes, the diketone from Preparation 81 (1.12g, 6.00mmol) in acetone (6ml) was added and the reaction was stirred for 4 hours. After cooling the mixture was diluted with water (25ml) and the acetone was removed under reduced pressure. The aqueous phase was acidified with 2N aqueous hydrochloric acid, extracted with dichloromethane (50ml) and the organic phase was dried over magnesium sulphate and concentrated under reduced pressure. The residue was purified by flash chromatography on silica gel eluting with a solvent gradient of pentane:ethyl acetate (95:5 changing to 90:10 then 80:20, by volume) to provide the title compound (1.03g) as a white solid, which existed as the enol tautomer, m.p. 135-137°C.
¹H NMR (400MHz, CDCl₃): δ = 0.93 (m, 4H), 1.19 (m, 4H), 1.74 (m, 2H), 7.53 (s, 2H), 15.25 (s, 1H).
LRMS (electrospray) : m/z [M-H⁺] 293.
Microanalysis: Found C, 69.18; H, 4.82; N, 9.35. C₁₇H₁₄N₂O₃ requires C, 69.38; H, 4.79; N, 9.52%.

### PREPARATION 83

### 3-Oxobutanoic acid

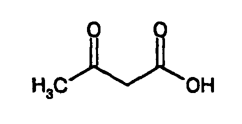

Sodium hydroxide (37.9g, 0.947mol) was dissolved in water (770ml) and added to a solution of 3-oxo-butanoic acid methyl ester (100g, 0.861 mol) at room temperature over 20 minutes. The reaction was stirred for 18 hours, quenched with ammonium sulfate (700g) and acidified slowly with a solution of concentrated Hydrochloric acid (21.5ml) in water (250ml) with ice cooling. The reaction mixture was extracted with diethylether (6x200ml) and the combined organic extracts were dried over magnesium sulphate and concentrated under reduced pressure to provide the title compound (58.2g) as a pale yellow oil which was a mixture of keto:enol tautomers.
¹H NMR (400MHz, CDCl₃): δ = 2.00 (s, 3H-enol), 2.30 (s, 3H-keto), 3.51 (s, 2H-keto), 5.02 (s, 1 H-enol).

### PREPARATION 84

### 1-Cyclopropyl-1,3-butanedione

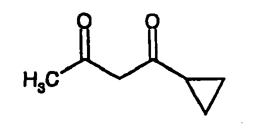

Magnesium turnings (3.04g, 125mmol) suspended in methanol (145ml) were heated to reflux under nitrogen for 1 hour, cooled to room temperature and the β-keto acid from Preparation 83 (25.5g, 250mmol) dissolved in methanol (25ml) was added dropwise with ice-cooling. The reaction was stirred for 1 hour at room temperature and the solvent was removed under reduced pressure to give the magnesium salt of the acid. Meanwhile, cyclopropane-carboxylic acid (9.91 ml, 125mmol) was dissolved in dimethylformamide (200ml) and carbonyldiimidazole (22.4g, 138mmol) was added portionwise under nitrogen at 0°C. This was stirred for 1.5 hour and the magnesium salt from above was added as a solution in dimethylformamide (100ml) at 0°C. The reaction was allowed to stir at room temperature for 92 hours and the mixture was poured into 2M aqueous hydrochloric acid (85ml) then diluted with water (170ml). The mixture was extracted with diethylether (6x200ml) and the combined organic extracts were washed with brine (3x200ml), dried over magnesium sulphate and concentrated under reduced pressure. The residual orange oil was purified by flash chromatography on silica gel eluting with pentane:diethylether (100:0 changing to 90:10 then 80:20, by volume) to provide the title compound (7.39g) as a yellow oil.
¹H NMR (400MHz, CDCl₃): δ = 0.83-0.95 (m, 2H), 1.06-1.10 (m, 2H), 1.54-1.63 (m, 1 H), 2.00 (s, 3H).
LRMS (electrospray) : m/z [MNa⁺] 149.

### PREPARATION 85

### 2-Chloro-1-cyclopropyl-1,3-butanedione

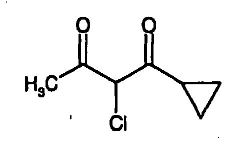

Trimethylsilyl chloride (18.9ml, 174mmol) was added to a solution of tert-butylammonium bromide (932mg, 2.89mmol) in dry acetonitrile (50ml) under nitrogen at room temperature and the mixture was cooled to 0°C. The diketone from Preparation 84 (7.3g, 57.9mmol) in acetonitrile (36ml) was then added followed by dropwise addition of dry dimethylsulfoxide (12.3ml, 174mmol). The reaction was stirred at 0°C for 1.5 hours and the mixture was diluted with water (500ml), extracted with diethylether (2x200ml and 100ml) and the combined organic extracts were dried over magnesium sulphate and concentrated under reduced pressure. The residual oil was purified by flash chromatography on silica gel eluting with pentane:diethylether (100:0 changing to 95:5 then 90:10, by volume) to provide the title compound (5.76g) as a colourless oil.
¹H NMR (400MHz, CDCl₃): δ = 0.99-1.08 (m, 2H), 1.15-1.20 (m, 2H), 2.27 (s, 3H), 2.38-2.46 (m, 1 H).
LRMS (electrospray): m/z [M-H⁺] 159.

### PREPARATION 86

### 3-[1-(Cyclocropylcarbonyl)-2-oxocropoxy]-5-methylbenzonitrile

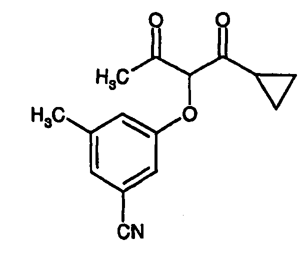

Cesium carbonate (2.45g, 8.30mmol) and the phenol from Preparation 42 (1g, 7.50mmol) were added to a stirred solution of the diketone from Preparation 85 (1.3g, 8.30mmol) in acetone (44ml) under nitrogen at 60°C and the reaction was stirred for 5 hours. After cooling the mixture was quenched with water and the acetone was removed under reduced pressure. The aqueous phase was acidified with 1 N aqueous hydrochloric acid, extracted with ethyl acetate and the organic phase was dried over magnesium sulphate and concentrated under reduced pressure. The residue was purified by flash chromatography on silica gel eluting with pentane:ethyl acetate (85:15, by volume) to provide the title compound (1.03g) as a pale red solid.
¹H NMR (400MHz, CDCl₃): δ = 0.85 (m, 2H), 1.12 (m, 2H), 1.86 (m, 1 H), 1.94 (s, 3H), 2.35 (s, 3H), 6.99 (m, 2H), 7.10 (s, 1H).
LRMS (electrospray) : m/z [M-H⁺] 256.

### PREPARATION 87

### 4-(3,5-Difluorophenoxy)-3,5-diethyl-1-[2-(tetrahydro-2H-pyran-2-yloxy)ethyl]-1H-pyrazole

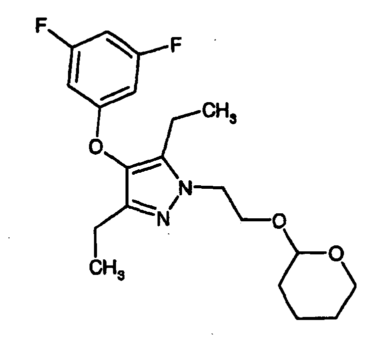

p-Toluene-sulphonic acid (360mg, 1.89mmol) was added to a solution of the alcohol from Example 38 (5.6g, 18.9mmol) and dihydropyran (8.62ml, 94.5mmol) in dichloromethane (75ml) at room temperature under nitrogen. The reaction was stirred for 2 hours, diluted with diethylether (100ml) and washed with a mixed aqueous solution (water (60ml), brine (30ml) and saturated aqueous sodium bicarbonate solution (30ml)). The aqueous phase was extracted with diethylether (2x60ml) and the combined organic extracts were dried over magnesium sulphate, concentrated under reduced pressure and the residue was purified by flash chromatography on silica gel eluting with dichloromethane:methanol (98:2, by volume) to provide the title compound (6.31 g) as an oil.
¹H NMR (400MHz, CDCl₃): δ = 1.09 (m, 6H), 1.57 (m, 6H), 2.40 (q, 2H), 2.55 (q, 2H), 3.44 (m, 1 H), 3.62 (m, 1 H), 3.73 (m, 1 H), 4.05 (m, 1 H), 4.16 (t, 2H), 4.50 (s, 1H), 6.39 (m, 3H).
LRMS (thermospray) : m/z [MH⁺] 381.
Microanalysis: Found C, 62.16; H, 6.92; N, 7.16. C₂₀H₂₆N₂O₃.0.09CH₂Cl₂ requires C, 62.18; H, 6.80; N,7.22%.

### PREPARATION 88

### 4-[3.5-Di(1H-pyrazol-1-yl)phenoxy]-3,5-diethyl-1-[2-(tetrahydro-2H-pyran-2-yloxy)ethyl]-1H-pyrazole

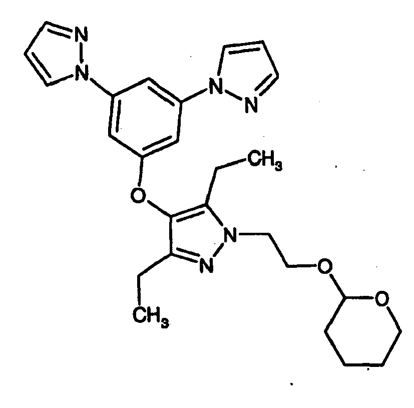

and

### PREPARATION 89

### 3,5-Diethyl-4-[3-fluoro-5-(1H-pyrazol-1-yl)phenoxy]-1-[2-(tetrahydro-2H-pyran-2-yloxy)ethyl]-1H-pyrazole

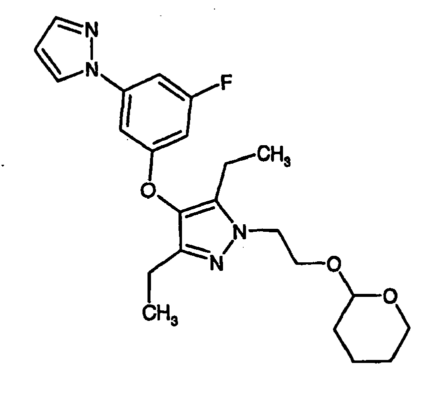

Cesium Carbonate (538mg, 1.65mmol) was added to a solution of pyrazole (102mg, 1.50mmol) in dry dimethylsulfoxide (2ml) under nitrogen at room temperature and the reaction was stirred for 15 minutes. The aryl difluoride from Preparation 87 (570mg, 1.50mmol) dissolved in dry dimethylsulfoxide (2ml) was then added and the reaction was heated to 100°C for 18 hours. After cooling to room temperature the reaction was diluted with water (20ml) and extracted with diethylether (2x2Oml). The organic phase was washed with brine (10ml), dried over magnesium sulphate, concentrated under reduced pressure. Some starting material remained, so the residue was dissolved in dimethylsulfoxide (12ml), pyrazole (510mg, 7.50mmol) followed by cesium carbonate (2.5g, 7.66mmol) were added and the reaction was heated to 100°C for 18 hours. After cooling to room temperature the reaction was diluted with water (6ml), extracted with diethylether (20ml) and the organic phase was washed with brine (10ml), dried over magnesium sulphate, concentrated under reduced pressure and the residue was purified by flash chromatography on silica gel eluting with a solvent gradient of dichloromethane:methanol (100:0 changing to 96:4, by volume). This gave two fractions, the first of which was a single product (least polar) and the other a mixture of two products. The second fraction was re-purified eluting with dichloromethane:acetonitrile (93:7 changing to 90:10, by volume) to provide the most polar product.
Least Polar Product - Preparation 88 (254mg)
¹H NMR (400MHz, CDCl₃): δ = 1.11 (m, 6H), 1.50 (m, 6H), 2.46 (q, 2H), 2.58 (q, 2H), 3.43 (m, 1 H), 3.64 (m, 1 H), 3.75 (m, 1 H), 4.04 (m, 1 H), 4.18 (t, 2H), 4.50 (s, 1 H), 6.42 (s, 2H), 7.15 (s, 2H), 7.67 (s, 3H), 7.90 (s, 2H).
LRMS (electrospray) : m/z [MH⁺] 477, [MNa⁺] 499.
HRMS: [MH⁺] Found 477.2612. C₂₆H₃₃N₆O₃ requires 477.2609.
Most Polar Product - Preparation 89 (37.7mg)
¹H NMR (400MHz, CDCl₃): δ = 1.11 (m, 6H), 1.46 (m, 6H), 2.43 (q, 2H), 2.57 (q, 2H), 3.43 (m, 1 H), 3.64 (m, 1 H), 3.75 (m, 1 H), 4.05 (m, 1 H), 4.17 (t, 2H), 4.51 (s, 1 H), 6.42 (m, 2H), 7.07 (m, 2H), 7.66 (s, 1H), 7.82 (s, 1H).
LRMS (thermospray) : m/z [MH⁺] 429.

### PREPARATION 90

### 3-({3,5-Diethyl-1-[2-(tetrahydro-2H-pyran-2-yloxy)ethyl]-1H-pyrazol-4-yl}oxy)-5-methoxybenzonitrile

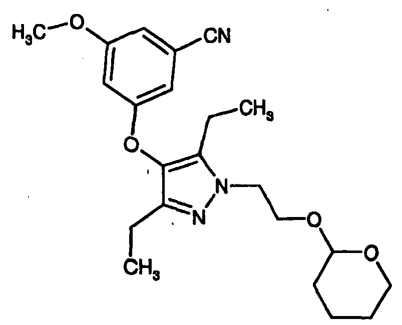

Sodium methoxide (25% w/v in methanol, 230µl, 1.00mmol) was added dropwise to a solution of the aryl fluoride from Preparation 63 (387mg, 1.00mmol) and in dimethylformamide (5ml) at room temperature under nitrogen. The reaction was stirred for 5 hours, diluted with water (10ml) and extracted with diethylether (50ml). The organic phase was dried over magnesium sulphate, concentrated under reduced pressure and the residue was purified by flash chromatography on silica gel eluting with dichloromethane:methanol (97:3, by volume) to provide the title compound (400mg) as an oil.
¹H NMR (400MHz, CDCl₃): δ = 1.09 (m, 6H), 1.49 (m, 6H), 2.41 (q, 2H), 2.55 (q, 2H), 3.46 (m, 1H), 3.66 (m, 1 H), 3.77 (m + s, 4H), 4.07 (m, 1 H), 4.19 (t, 2H), 4.52 (m, 1 H), 6.66 (s, 1 H), 6.69 (s, 1 H), 6.77 (s, 1H).
LRMS (thermospray) : m/z [MH⁺] 400.
Microanalysis: Found C, 65.59; H, 7.32; N, 10.42. C₂₂H₂₉N₃O₄.0.04CH₂Cl₂ requires C, 65.71; H, 7.28; N, 10.43%.

### PREPARATION 91

### 3-(1-Acetyl-3-methyl-2-oxobutoxy)-5-methylbenzonitrile

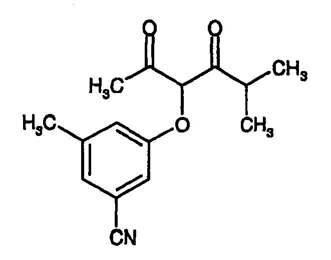

Cesium carbonate (1.50g, 4.61 mmol) and the phenol from Preparation 42 (609mg, 4.61 mmol) were added to a stirred solution of the diketone from Preparation 23 (750mg, 4.61 mmol) in acetone (23ml) under nitrogen at 50°C and the reaction was stirred for 3 hours. After cooling the mixture was quenched with water (10ml) and the acetone was removed under reduced pressure. The aqueous phase was extracted with dichloromethane (4x25ml) and the combined organic extracts were dried over magnesium sulphate and concentrated under reduced pressure. The residue was purified by flash chromatography on silica gel eluting with pentane:ethyl acetate (90:10, by volume) to provide the title compound (544mg).
¹H NMR (400MHz, CDCl₃): δ = 1.10 (m, 6H), 2.09 (s, 3H), 2.42 (s, 3H), 2.69 (m, 1 H), 7.00 (s, 2H), 7.19 (s, 1H).
LRMS (thermospray) : m/z[MNH₄⁺] 277.

### PREPARATION 92

### [4-(3,5-Dichlorophenoxy)-3-methyl-1H-pyrazol-5-yl]acetic acid

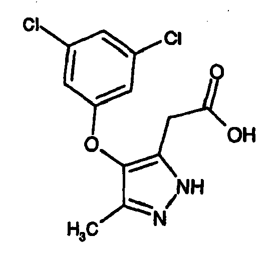

The pyrazole of Example 208 (400mg, 1.41mmol) was stirred at 100°C for 14 hours in concentrated hydrochloric acid (20ml). The mixture was cooled to room temperature and the solvent removed under reduced pressure to give a yellow solid. The solid was dissolved in dichloromethane (50ml) and 1N aqueous hydrochloric acid (50ml) and the organic layer was separated. The organics were washed with 1 N aqueous hydrochloric acid (50ml), dried over magnesium sulphate, filtered and the solvent removed under reduced pressure to provide the title compound (400mg) as pale yellow solid, m.p. 156-158°C.
¹H NMR (400MHz, CD₃OD): δ = 2.02 (s, 3H), 4.89 (s, 2H), 6.82 (s, 2H), 7.02 (s, 1 H).
LRMS (thermospray) : m/z [MH⁺] 303.
Microanalysis: Found C, 47.50; H, 3.50; N, 9.46. C₁₂H₁₀Cl₂N₂O₃ requires C, 47.86; H, 3.35; N, 9.30%.

### PREPARATION 93

### 3-({3,5-Diethyl-1-[2-(tetrahydro-2H-pyran-2-yloxy)ethyl]-1H-pyrazol-4-yl}oxy)-5-(methylsulfanyl)benzonitrile

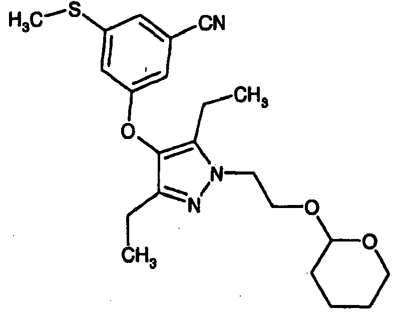

Sodium thiomethoxide (180mg, 2mmol) was added to a stirred solution of the aryl fluoride from Preparation 63 (774mg, 2.00mmol) in dimethylformamide (10ml) at room temperature under nitrogen. The reaction mixture was stirred for 5 hours before being heated at 100°C for 18 hours. A second portion of sodium thiomethoxide (90mg, 1 mmol) was added and the reaction mixture was heated at 100°C for a further 5 hours. After cooling to room temperature the mixture was diluted with water (10ml) and extracted with diethylether (2x50ml). The organic phase was dried over magnesium sulphate, concentrated under reduced pressure and the residue was purified by flash chromatography on silica gel eluting with dichloromethane:methanol (97:3, by volume) to provide the title compound (700mg) as an oil.
¹H NMR (400MHz, CDCl₃): δ = 1.14 (m, 6H), 1.52 (m, 6H), 2.44 (q, 2H), 2.49 (s, 3H), 2.59 (q, 3H), 3.50 (m, 1 H), 3.70 (m, 1 H), 3.80 (m, 1 H), 4.10 (m, 1 H), 4.23 (m, 2H), 4.55 (m, 1 H), 6.82 (s, 1 H), 7.01 (s, 1H), 7.09 (s, 1H).
LRMS (APCI+): m/z [MH⁺] 416.

### PREPARATION 94

### 3-({3,5-Diethyl-1-[2-(tetrahydro-2H-pyran-2-yloxy)ethyl]-1H-pyrazol-4-yl}oxy)-5-[2-(dimethylamino)ethoxy]benzonitrile

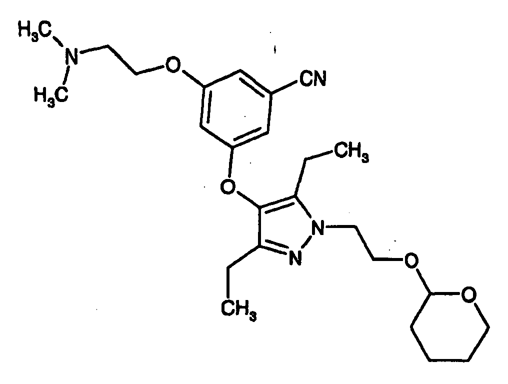

To a stirred solution of N,N-dimethylethanolamine (83µl, 0.83mmol) in dimethylformamide (2ml) was added sodium hydride (36mg of 60% by weight dispersion in oil, 0.90mmol). After 10 minutes a solution of the aryl fluoride from Preparation 63 (291 mg, 0.75mmol) in dimethylformamide (2ml) was added and the reaction mixture was stirred at room temperature for 18 hours. The mixture was diluted with 10% aqueous potassium carbonate solution (12ml) and extracted with diethyl ether (2x7ml). The combined organic components were dried over magnesium sulphate and concentrated under reduced pressure. The crude product mixture was purified by flash chromatography on silica gel eluting with dichloromethane:methanol (a gradient from 99:1 to 90:10, by volume) to provide the title compound (180mg) as an oil.
¹H NMR (400MHz, CDCl₃): δ = 1.09 (m, 6H), 1.50 (m, 6H), 2.39 (q, 2H), 2.47 (s, 6H), 2.55 (q, 2H), 2.87 (m, 2H), 3.47 (m, 1 H), 3.67 (m, 1 H), 3.78 (m, 1 H), 4.05 (m, 1 H), 4.17 (m, 4H), 4.52 (m, 1 H), 6.70 (s, 2H), 6.79 (s, 1H).
LRMS (electrospray): m/z [MH⁺] 457.
HRMS: [MH⁺] 457.2810. C₂₅H₃₇N₄O₄ requires 457.2810.

### PREPARATIONS 95-97

The preparation of the following tabulated Preparations of the general formula 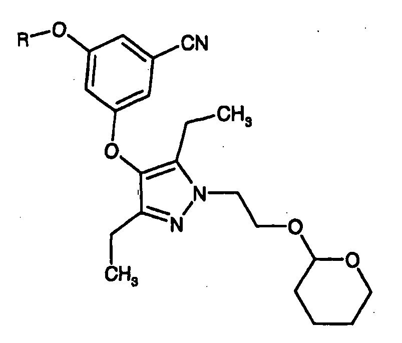 were performed by a similar method to that of Preparation 94 using the appropriate alcohol as the starting material.

| Preparation no. (Starting material preparation no) | R | Analytical Data |
|---|---|---|
| 95 (63) | CH₂CH₂ NHMe | ¹H NMR (400MHz, CDCl₃): δ = 1.09 (m, 6H), 1.50 (m, 6H), 2.39 (q, 2H), 2.54 (m, 5H), 3.04 (t, 2H), 3.46 (m, 1 H), 3.66 (m, 1 H), 3.78 (m, 1 H), 4.05 (m, 1 H), 4.11 (t, 2H), 4.17 (t, 2H), 4.52 (s, 1 H), 6.70 (s, 2H), 6.81 (s, 1 H). LRMS (electrospray): m/z [MH⁺] 443 HRMS: [MH⁺] 443.2654. C₂₄H₃₅N₄O₄ requires 443.2653. |
| 96 (63) | CH₂CONH₂ | ¹H NMR (400MHz, CDCl₃): δ = 1.11 (m, 6H), 1.48 (m, 6H), 2.43 (q, 2H), 2.58 (q, 2H), 3.46 (m, 1H), 3.67 (m, 1H), 3.80 (m, 1 H), 4.08 (m, 1 H), 4.25 (m, 2H), 4.45 (s, 2H), 4.52 (m, 1 H), 5.54 (broad s, 1 H), 6.37 (broad s, 1 H), 6.72 (s, 1 H), 6.85 (s, 2H). LRMS (electrospray): m/z 465 (MH⁺) HRMS: [MH⁺] 443.2282. C₂₃H₃₁N₄O₅ requires 443.2289. |
| 97 (63) | CH₂CH₂OCH₃ | ¹H NMR (400MHz, CDCl₃): δ= 1.10 (m, 6H), 1.50 (m, 6H), 2.41 (q, 2H), 2.55 (q, 2H), 3.41 (s, 3H), 3.47 (m, 1 H), 3.70 (m, 3H), 3.79 (m, 1H), 4.06 (m, 3H), 4.20 (m, 2H), 4.52 (s, 1H), 6.70 (s, 2H), 6.79 (s, 1 H). LRMS (electrospray): m/z 466 (MH⁺) HRMS: [MH⁺] 443.2282. C₂₄H₃₄N₃O₅ requires 443.2289. |

### PREPARATION 98

### 5-Methyl-1-[2-(tetrahydro-2H-pyran-2-yloxy)ethyl]-3-(trifluoromethyl)-1H-pyrazol-4-ol

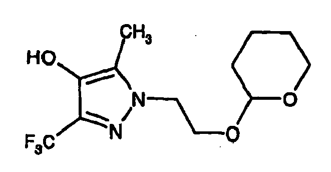

To a stirred solution of 1-(2-hydroxyethyl)-5-methyl-3-(trifluoromethyl)-1*H*-pyrazol-4-ol (600mg, 2.86mmol; Kenkyu Hokoku - Asahi Garasu Kogyo Gijutsu Shoreikai ,1988, 51, 139-49) in dichloromethane (10ml) and ethyl acetate (4ml) was added para-toluenesulphonic acid (27mg, 0.14mmol) followed by 3,4-dihydro-2H-pyran (340µl, 3.7mmol). The reaction mixture was stirred at room temperature for 3 hours before being concentrated under reduced pressure. The crude product mixture was purified by flash chromatography on silica gel eluting with pentane:ethyl acetate (60:40, by volume) to provide the title compound (560mg) as white solid.
¹H NMR (400MHz, CDCl₃): δ = 1.60 (m, 6H), 2.23 (s, 3H), 3.44 (m, 1 H), 3.60 (m, 1 H), 3.72 (m, 1 H), 4.04 (m, 1 H), 4.18 (m, 2H), 4.50 (broad s, 1H).
LRMS (electrospray): m/z [M-H⁺] 293.

### PREPARATION 99

### 3-Fluoro-5-{[5-methyl-1-[2-(tetrahydro-2H-pyran-2-yloxy)ethyl]-3-(trifluoromethyl)-1H-pyrazol-4-yl]oxy}benzonitrile

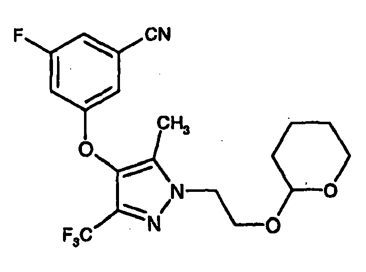

To a stirred solution of the pyrazole (214mg, 0.73mmol) from Preparation 98 in dimethylformamide (0.7ml) was added 3,5-diflurobenzonitrile (304mg, 2.2mmol) and potassium carbonate (304mg, 2.2mmol). The reaction mixture was heated at 90°C for 7 hours. After cooling to room temperature brine (20ml) was added and the mixture was extracted with ethyl acetate (20ml). The organic component was separated, washed with brine (20 ml), dried over magnesium sulphate and concentrated under reduced pressure. The crude product mixture was purified by flash chromatography on silica gel eluting with pentane:ethyl acetate (80:20, by volume) to provide the title compound (267mg) as a colourless oil.
¹H NMR (400MHz, CDCl₃): δ = 1.61 (m, 6H), 2.18 (s, 3H), 3.48 (m, 1H), 3.64 (m, 1 H), 3.75 (m, 1H), 4.30 (t, 2H), 4.50 (broad s, 1 H), 6.85 (d, 1 H), 6.94 (s, 1 H), 7.05 (d, 1 H).
LRMS (electrospray): m/z [M-H⁺] 412.

### PREPARATION 100

### 3-Cyano-5-[(3,5-diethyl-1-{2-[(2-methoxyethoxy)methoxy]ethyl}-1H-pyrazol-4-yl)oxy]benzamide

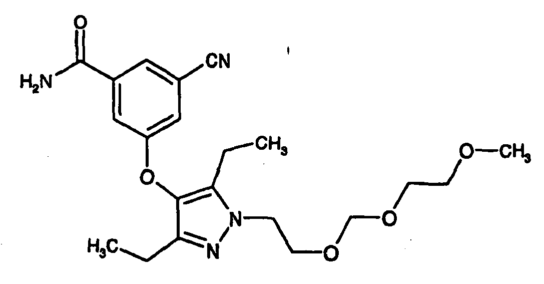

To a stirred solution of the pyrazole from Example 261 (193mg, 0.49mmol) in tetrahydrofuran (2ml) was added 2M aqueous sodium hydroxide solution (8.7µl, 0.49mmol) and the reaction mixture was heated at 65°C for 24 hours. After cooling to room temperature a second portion of 2M sodium hydroxide solution (8.7µl, 0.49mmol) was added and the mixture was heated at 65°C for 24 hours. 6M aqueous sodium hydroxide solution (100µl) was added and the mixture was heated at 65°C for 24 hours. The reaction mixture was concentrated under reduced pressure, diluted with water (75ml), neutralised to pH7 using 2M aqueous hydrochloric acid solution and extracted with dichloromethane (2x25ml). The combined organic components were dried over magnesium sulphate and concentrated under reduced pressure to give a crude product mixture which was purified by flash chromatography on silica gel eluting with dichloromethane:methanol (100:0, 98:2, 96.5:3.5 then 95:5, by volume) to provide the title compound (60mg) as a colourless oil.
¹H NMR (400MHz, CDCl₃): δ = 1.10 (m, 6H), 2.40 (q, 2H), 2.55 (q, 2H), 3.36 (s, 3H), 3.50 (q, 2H), 3.59 (q, 2H), 3.94 (q, 2H), 4.20 (q, 2H), 4.64 (s, 2H), 7.30 (s, 1 H), 7.59 (s, 1H), 7.70 (s, 1H).

### PREPARATION 101

### 5-[(1-Acetyl-3,5-diethyl-1H-pyrazol-4-yl)oxy]isophthalonitrile

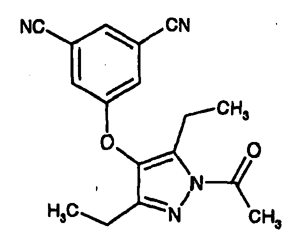

To a stirred solution of the pyrazole from Example 122 (3.0g, 11.3mmol) in dimethylformamide (45ml) at 0°C was added acetyl chloride (1.2ml, 17.0mmol), followed by sodium hydride portionwise (678mg of 60 % by weight dispersion in oil, 17.0mmol). The cooling bath was removed and the reaction mixture was stirred at room temperature for 40 minutes. The reaction was quenched by addition of saturated aqueous ammonium chloride solution (4ml) and concentrated under reduced pressure to give an orange residue. This material was partitioned between ethyl acetate (200ml) and water (200ml). The organic component was washed with water (100ml), brine (75ml) and then dried over magnesium sulphate before being concentrated under reduced pressure. The crude product mixture was purified by flash chromatography on silica gel eluting with dichloromethane:methanol (100:0, 99:1, then 98:2, by volume) to provide the title compound (2.67g) as a white solid.
¹H NMR (400MHz, CDCl₃): δ = 1.15 (t, 3H), 1.19 (t, 3H), 2.43 (q, 2H), 2.72 (s, 3H), 3.85 (q, 2H), 7.38 (s, 2H), 7.61 (s, 1H).
LRMS (electrospray): m/z 331 [M+Na⁺].

### PREPARATION 102

### 5-{[1-Acetyl-3-(1-bromoethyl)-5-ethyl-1H-pyrazol-4-yl]oxy}isophthalonitrile

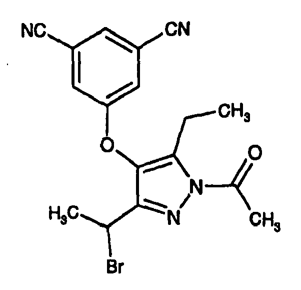

A solution of the pyrazole from Preparation 101 (881mg, 2.86mmol) in carbontetrachloride (12ml) was degassed by passing a stream of nitrogen through the solution for 20 minutes. N-bromosuccinimide (763mg, 4.28mmol) was added followed by AIBN (30mg) and the reaction mixture was heated at 85°C for 4 hours. After cooling to room temperature the mixture was concentrated under reduced pressure and the residue was purified by flash chromatography on silica gel eluting with pentane:ethyl acetate (a gradient from 100:0 to 67:33, by volume) to provide the title compound (348mg) as a colourless oil.
¹H NMR (400MHz, CDCl₃): δ = 1.10 (t, 3h), 2.00 (d, 3H), 2.70 (s, 3H), 2.80 (m, 2H), 4.95 (q, 1 H), 7.42 (s, 2H), 7.60 (s, 1H).
LRMS (electrospray): m/z 283 [MH⁺].

### PREPARATION 103

### 5-({5-Ethyl-3-(1-hydroxyethyl)-1-[2-(tetrahydro-2H-pyran-2-yloxy)ethyl]-1H-pyrazol-4-yl}oxy)isophthalonitrile

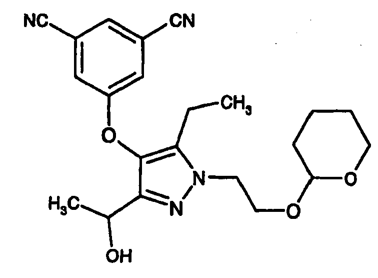

To a stirred solution of the pyrazole from Example 263 (197mg, 0.70mmol) in dimethylformamide (3ml) at 0°C was added 2-(2-bromoethoxy)tetrahydro-2*H-*pyran (105µl, 0.70mmol) followed by sodium hydride (31 mg, 0.77mmol). After 15 minutes the cooling bath was removed and the mixture was stirred at room temperature for 60 hours. The reaction mixture was quenched by addition of saturated aqueous ammonium chloride solution (0.5ml) and then concentrated under reduced pressure. The crude product mixture was purified by flash chromatography on silica gel eluting with dichloromethane:methanol (a gradient from 100:0 to 95:5, by volume) to provide the title compound (84mg) as a white foam which reverts to an oil on standing.
¹H NMR (400MHz, CDCl₃): δ = 1.11 (t, 3H), 1.45 (d, 3H), 1.65 (m, 6H), 2.59 (q, 2H), 3.50 (m, 1 H), 3.70 (m, 1 H), 3.81 (m, 1 H), 4.11 (m, 1 H), 4.25 (t, 2H), 4.56 (m, 1 H), 4.76 (m, 1H), 7.40 (s, 2H), 7.55 (s, 1H).
LRMS (electrospray): m/z 411 [MH⁺].

### PREPARATION 104

### 3-Cyano-5-[(3,5-diethyl-1-{2-[(2-methoxyethoxy)methoxy]ethyl}-1H-pyrazol-4-yl)oxy]-N'-hydroxybenzenecarboximidamide

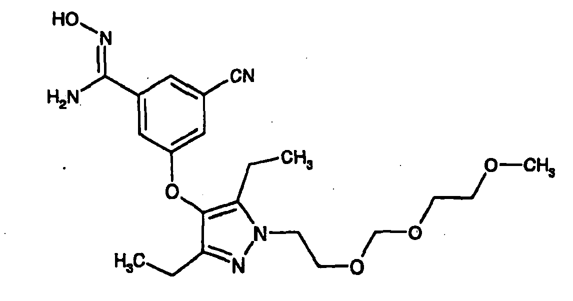

To a stirred solution of the pyrazole from Example 261 (1.5g, 3.76mmol) in ethanol (7.5ml) was added a solution of sodium carbonate (200mg, 1.88mmol) and hydroxylamine hydrochloride (262mg, 3.76mmol) in water (7.5ml). After stirring for 5 hours at room temperature the reaction mixture was concentrated under reduced pressure and the residue was partitioned between dichloromethane (50ml) and water (40ml). The aqueous phase was separated and extracted with dichloromethane (30ml). The organic components were combined, dried over magnesium sulphate and concentrated under reduced pressure. The crude product mixture was purified by flash chromatography on silica gel eluting with dichloromethane:methanol (a gradient from 100:0 to 96:4, by volume) to provide the title compound (1.13mg) as a colourless oil.
¹H NMR (400MHz, CDCl₃): δ = 1.11 (m, 6H), 2.42 (q, 2H), 2.58 (q, 2H), 3.41 (s, 3H), 3.59 (m, 4H), 3.95 (t, 2H), 4.17 (t, 2H), 4.61 (s, 2H), 4.77 (broad s, 2H), 7.38 (m, 1 H), 7.49 (m, 2H).
LRMS (electrospray): m/z 432 [MH⁺].
Microanalysis: Found C, 57.50; H, 6.71; N, 16.01. C₂₁H₂₆N₄O₄+0.4H₂O requires C, 57.50; H, 6.85; N, 15.96%.

### PREPARATION 105

### 3-[(3,5-Diethyl-1-{2-[(2-methoxyethoxy)methoxy]ethyl}-1H-pyrazol-4-yl)oxy]-5-[5-(trifluoromethyl)-1,2,4-oxadiazol-3-yl]benzonitrile

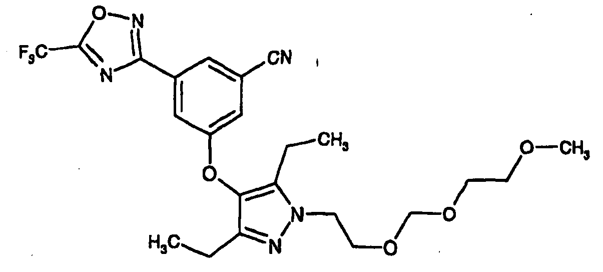

To a stirred solution of the amidoxime from Preparation 104 (300mg, 0.70mmol) in pyridine (3ml) was added trifluoroacetic anhydride (118µl, 0.83mmol). After stirring at room temperature for 2 hours the reaction mixture was heated at 110°C for 18 hours. After cooling to room temperature the mixture was concentrated under reduced pressure and the residue was partitioned between 2M aqueous HCl solution (6ml) and dichloromethane (6ml). The organic phase was separated and concentrated under reduced pressure. The residue was purified by flash chromatography on silica gel eluting with dichloromethane:methanol (a gradient from 100:0 to 90:10, by volume) to provide the title compound (259mg) as a colourless oil.
¹H NMR (400MHz, CDCl₃): δ =1.14 (m, 6H), 2.46 (q, 2H), 2.59 (q, 2H), 3.39 (s, 3H), 3.53 (q, 2H), 3.59 (q, 2H), 3.95 (q, 2H), 4.29 (q, 2H), 4.68 (s, 2H), 7.34 (s, 1 H), 7.87 (s, 1H), 8.04 (s, 1 H).
LRMS (APCI): m/z 532 (MH⁺)

### PREPARATIONS 106-108

The preparation of the following tabulated Preparations of the general formula 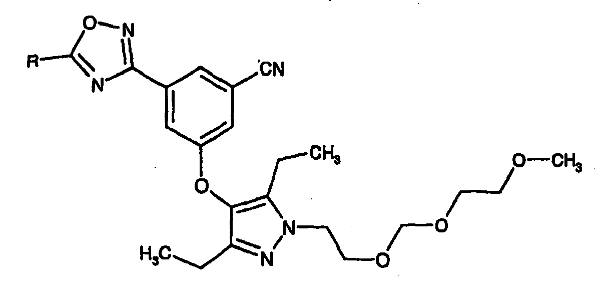 were performed by a similar method to that of Preparation 105 using the appropriate acid chloride as the acylating agent in place of trifluoroacetic anhydride.

| Preparation no. | R | Analytical Data |
|---|---|---|
| 106 | Me | ¹H NMR (400MHz, CDCl₃): δ = 1.14 (m, 6H), 2.46 (q, 2H), 2.59 (q, 2H), 2.67 (s, 3H), 3.39 (s, 3H), 3.55 (q, 2H), 3.59 (q, 2H), 3.95 (q, 2H), 4.22 (q, 2H), 4.68 (s, 2H), 7.27 (s, 1 H), 7.82 (s, 1H), 8.00 (s, 1 H). LRMS (electrospray): m/z 478 [M+Na⁺] Microanalysis: Found C, 59.91; H, 6.27; N, 15.38. C₂₃H₂₉N₅O₅+0.3H₂O requires C, 59.94; H, 6.475; N, 15.19%. |
| 107 | Et | ¹H NMR (400MHz, CDCl₃): δ=1.14 (m, 6H), 1.44 (t, 3H), 2.42 (q, 2H), 2.48 (q, 2H), 2.98 (q, 2H), 3.39 (s, 3H), 3.53 (q, 2H), 3.59 (q, 2H), 3.95 (q, 2H), 4.20 (q, 2H), 4.48 (s, 2H), 7.30 (s, 1 H), 7.84 (s, 1 H), 8.01 (s, 1H). LRMS (electrospray): m/z 492 (M+Na⁺) |
| 108 | ^{l}Pr | ¹H NMR (400MHz, CDCl₃): δ = 1.11 (m, 6H), 1.49 (d, 6H), 2.44 (q, 2H), 2.49 (q, 2H), 3.30 (sept, 1 H), 3.39 (s, 3H), 3.54 (m, 2H), 3.59 (m, 2H), 3.95 (t, 2H), 4.23 (t, 2H), 4.91 (s, 2H), 7.22 (m, 1 H), 7.83 (m, 1 H), 8.02 (m, 1H). LRMS (electrospray): m/z 506 (M+Na⁺) Microanalysis: Found C, 61.87; H, 6.76; N, 14.62. C₂₅H₃₃N₅O₅ requires C, 62.10; H, 6.88; N, 14.48%. |

### PREPARATION 109

### Ethyl 5-{[(tert-butoxycarbonyl)amino]methyl}nicotinate

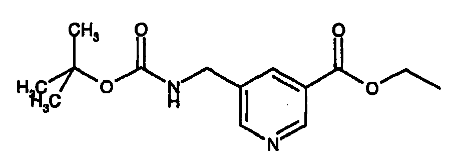

To a stirred solution of ethyl-5-cyanonicotinate (3.0g, 17.0mmol; Annalen Der Chemie, 1959, 621, 106-136) in ethanol (200ml) was added concentrated hydrochloric acid (3.4ml) followed by 5% palladium on carbon (300mg). The reaction mixture was stirred at room temperature under an hydrogen atmosphere (50psi) for 18 hours. The reaction mixture was filtered through Arbocel® and concentrated under reduced pressure. The crude product mixture was purified by flash chromatography on silica gel eluting with dichloromethane:methanol:0.880 ammonia (a gradient from 95:5:0.5 to 85:5:1.5, by volume) to provide the intermediate amine (2.1 g) as a yellow oily solid. This material (2.1 g, 11.7mmol) was suspended in dichloromethane (22ml) to which was added triethylamine (1.8ml, 13.0mmol) followed by di-tert-butyl dicarbonate (2.84g, 13mmol). After 48 hours the reaction mixture was diluted with dichloromethane (50ml) and washed with water (50ml). The organic component was dried over magnesium sulphate and concentrated under reduced pressure before being purified by flash chromatography on silica gel eluting with dichloromethane:methanol:0.88 ammonia (a gradient from 100:0:0 to 95:5:0.5, by volume) to provide the title compound (2.0g) as a yellow oil.
¹H NMR (400MHz, CDCl₃): δ =1.40 (m, 12H), 4.42 (m, 4H), 8.22 (s, 1 H), 8.71 (s, 1 H), 9.12 (s, 1 H).
LRMS (APCI): m/z 279 (M-H⁺)

### PREPARATION 110

### 5-{[(tert-Butoxycarbonyl)amino]methyl}nicotinic acid

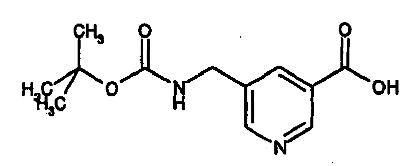

To a stirred solution of the ester from Preparation 109 (2.00g, 7.10mmol) in 1 M aqueous sodium hydroxide solution (15ml, 15mmol) was added methanol (15ml). The reaction mixture was stirred at room temperature for 18 hours, after which time the methanol was removed under reduced pressure. The aqueous solution was washed with diethyl ether (2x25ml), cooled to 0°C and neutralised to pH7 by addition of 2M aqueous hydrochloric acid solution (7.5ml). The mixture was concentrated under reduced pressure to give a yellow oil (1.5g).
¹H NMR (400MHz, (CD₃)₂SO): δ = 1.37 (s, 9H), 4.16 (d, 2H), 7.51 (m, 1H), 8.07 (s, 1 H), 8.50 (s, 1H), 8.88 (s, 1 H).
LRMS (APCI): m/z 251 (M-H⁺)

### PREPARATION 111

### 5-(Aminomethyl)nicotinamide

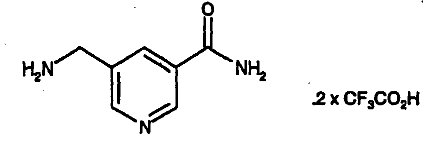

To a stirred solution of the acid from Preparation 110 (770mg, 3.10mmol) in dimethylformamide (15ml) was added carbonyldiimidazole (600mg, 3.70mmol). After 10 minutes 0.880 ammonia (1ml) was added. After a further 1 hour the reaction mixture was concentrated under reduced pressure and the residue was purified by flash chromatography on silica gel eluting with dichloromethane:methanol:0.88 ammonia (a gradient from 95:5:0.5 to 80:20:1, by volume) to provide the boc-protected intermediate. To a stirred solution of this material in dichloromethane (20ml) was added trifluroacetic acid (6ml). After 18 hours a second portion of trifluoroacetic acid (6ml) was added and the reaction mixture was stirred at room temperature for 24 hours. The solution was concentrated under reduced pressure to give an oily residue which was purified by flash chromatography on silica gel eluting with dichloromethane:methanol:0.88 ammonia (100:0:0 then 90:10:1 then 80:20:1, by volume) to provide the title compound (650mg) as a yellow oil.
¹H NMR (400MHz, (CD₃)₂SO): δ = 4.11 (s, 2H), 7.5 (broad s), 7.59 (broad s), 8.14 (broad s), 8.31 (m, 1H), 8.72 (m, 1 H), 8.90 (m, 1H).
LRMS (electrospray): m/z 152 (MH⁺)
HRMS: [MH⁺] 152.0819. C₇H₁₀N₃O requires 152.0818

### PREPARATION 112

### Ethyl 2-{[(tert-butoxycarbonyl)amino]methyl}isonicotinate

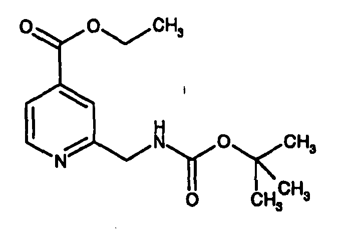

To a stirred solution of ethyl 2-cyanoisonicotinate (2.00g, 11.0mmol, J. Med. Chem., 1976, 19, 483) in ethanol (20ml) was added 2M aqueous hydrochloric acid solution (7.5ml) followed by 5% palladium on carbon (200mg). The reaction mixture was stirred at room temperature under a hydrogen atmosphere (60psi) for 48 hours. The mixture was filtered through arbocel and the filtrate was concentrated under reduced pressure. The residue was dried by azeotropic distillation using toluene under reduced pressure. To a stirred solution of the residue (3.00g) in dichloromethane (22ml) was added triethylamine (4.6ml, 33mmol) followed by di-tert-butyl dicarbonate (2.62g, 12.0mmol). After stirring for 1 hour at room temperature the reaction mixture was diluted with dichloromethane (100ml) and washed with water (50ml). The organic component was washed with brine (50ml), dried over magnesium sulphate and concentrated under reduced pressure to give a brown oily solid. The crude product mixture was purified by flash chromatography on silica gel eluting with dichloromethane:methanol:0.88 ammonia (98:2:0.2 then 97:3:0.3, by volume) to provide the title compound (2.20g) as a yellow oil.
¹H NMR (400MHz, CDCl₃): δ = 1.38 (t, 3H), 1.45 (s, 9H), 4.38 (q, 2H), 4.50 (m, 2H), 5.50 (broad s, 1 H), 7.73 (d, 1 H), 7.81 (s, 1 H), 8.65 (d, 1H).
LRMS (electrospray): m/z 281 (MH⁺)

### PREPARATION 113

### 2-{[(tert-Butoxycarbonyl)amino]methyl}isonicotinic acid

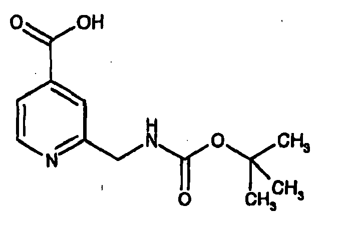

To a stirred solution of the ester from Preparation 112 (1.50g, 5.35mmol) in methanol (10ml) was added 1M aqueous sodium hydroxide solution (10ml). After 1 hour the reaction mixture was cooled to 0°C and neutralised by addition of 2M aqueous hydrochloric acid solution (5ml). The reaction mixture was concentrated under reduced pressure and the residue was purified by flash chromatography on silica gel eluting with dichloromethane:methanol:0.880 ammonia (80:20:1, by volume) to provide the title compound (1.30g) as a yellow foam.
¹H NMR (400MHz, (CD₃OD): δ = 1.43 (s, 9H), 4.36, (s, 2H), 7.68 (m, 1 H), 7.81 (s, 1 H), 8.47 (m, 1 H).
LRMS (electrospray): m/z 251 (M-H⁺)
HRMS: [MH⁺] 253.1179. C₁₂H₁₇N₂O4 requires 253.1183

### PREPARATION 114

### tert-Butyl [4-(aminocarbonyl)-2-pyridinyl]methylcarbamate

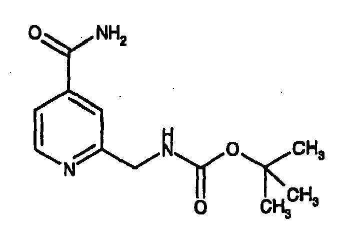

To a stirred solution of the acid from Preparation 113 (1.3g, 5.20mmol) in dimethylformamide (10ml) was added 1-hydroxybenzotriazole (950mg, 6.20mmol) followed by 1-(3-dimethylaminopropyl)-3-athylcarbodiimide hydrochloride salt (1.20g, 6.20mmol). After 1 hour 0.880 ammonia (5ml) was added and the reaction mixture was stirred at room temperature for 1.5 hours. The mixture was concentrated under reduced pressure and dried by azeotropic distillition using toluene under reduced pressure to give a yellow semi-solid. The crude product mixture was purified by flash chromatography on silica gel eluting with dichloromethane:methanol:0.88 ammonia (95:5:0.5, by volume) to provide the title compound (1.1 g) as a clear oil which crystallised on standing. This material was further purified by triturating with diethyl ether (10ml) which gave a sample of the desired product (1.0g) white powder/
¹H NMR (400MHz, D6-DMSO): δ = 1.39 (s, 9H), 4.25 (m, 2H), 7.44 (m, 1H), 7.61 (m, 1 H), 7.66 (broad s, 2H), 8.21 (broad s, 1 H), 8.59 (d, 1H).
LRMS (electrospray): m/z 250 (M-H⁺)
Microanalysis: Found C, 57.26; H, 6.86; N, 16.65. C₁₂H₁₇N₃O₃ requires C, 57.36; H, 6.82; N, 16.72%.

### PREPARATION 115

### 2-(Aminomethyl)isonicotinamide

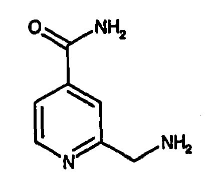

To a stirred solution of the pyridine from Preparation 114 (1.00g, 3.98mmol) in dichloromethane (50ml) was added trifluoroacetic acid (15ml). After stirring at room temperature for 18 hours the reaction mixture was concentrated under reduced pressure and purified by ion-exchange chromatography on Dowex 50-X8-200 eluting with water followed by 0.880 ammonia:methanol:water (5:5:90, by volume) to provide the title compound (265mg) as a white solid.
¹H NMR (400MHz, D6-DMSO) : δ = 2.1 (broad s, 1 H), 3.4 (broad s, 1 H), 3.85 (2H, s), 7.57 (m, 1 H), 7.60 (broad s, 1 H), 7.80 (m, 1 H), 8.16 (broad s, 1 H), 8.59 (m, 1 H).

LRMS (APCI): m/z 152 (MH⁺)

## Claims

1. A compound of the formula (I) 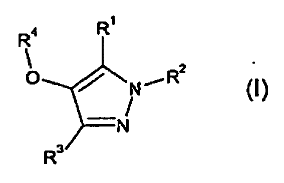 or a pharmaceutically acceptable salt, or solvate thereof, wherein:
either R¹ is H, C₁-C₆ alkyl, C₃-C₇ cycloalkyl or -OR⁷, said C₁-C₆ alkyl and C₃-C₇ cycloalkyl being optionally substituted by halo, -CN, -OR¹⁰, S(O)ₓR¹⁰, -CO₂R¹⁰, - CONR⁵R¹⁰, -OCONR⁵R¹⁰, -NR⁵CO₂R¹⁰, -NR¹⁰R¹¹, -NR⁵COR¹⁰, -SO₂NR⁵R¹⁰, - NR⁵CONR⁵R¹⁰, -NR⁵SO₂R¹⁰ or R¹⁰; and
R² is H, C₁-C₆ alkyl, C₃-C₆ alkenyl or R⁹, said C₁-C₆ alkyl being optionally substituted by halo, -OR⁵, -OR¹², -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹², -NR⁵COR⁵, -NR⁵COR⁸, -NR⁵COR¹², -NR⁵CO₂R⁵, -NR⁵CONR⁵R⁵, -SO₂NR⁵R⁵, -NR⁵SO₂R⁵, R⁸ or R⁹;
or, R¹ and R², when taken together, represent unbranched C₃-C₄ alkylene, optionally substituted by oxo, wherein one methylene group of said C₃-C₄ alkylene is replaced by an oxygen atom or a nitrogen atom, said nitrogen atom being optionally substituted by R¹⁰;
R³ is H or C₁-C₆ alkyl, said C₁-C₆ alkyl being optionally substituted by halo, -CN, - OR⁵, -CO₂R⁵, -CONR⁵R⁵, -OCONR⁵R⁵, -NR⁵CO₂R⁵, -NR⁶R⁶, -NR⁵COR⁵, - SO₂NR⁵R⁵, -NR⁵CONR⁵R⁵, -NR⁵SO₂R⁵, R⁸ or R⁹;
R⁴ is phenyl optionally substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹³, SoₓR⁶, O-(C₁-C₆ alkylene)-CONR⁵R⁵, O-(C₁-C₆ alkylene)-NR⁵R⁵, or O-(C₁-C₆ alkylene)-OR⁶; or naphthyl;
each R⁵ is independently either H, C₁-C₆ alkyl or C₃-C₇ cycloalkyl or, when two R⁵ groups are attached to the same nitrogen atom, those two groups taken together with the nitrogen atom to which they are attached represent azetidinyl, pyrrolidinyl, piperidinyl, homopiperidinyl, piperazinyl, homopiperazinyl or Morpholinyl said azetidinyl, pyrrolidinyl, piperidinyl, homopiperidinyl, piperazinyl, homopiperazinyl and morpholinyl being optionally substituted by C₁-C₆ alkyl or C₃-C₇ cycloalkyl;
each R⁶ is independently either H, C₁-C₆ alkyl or C₃-C₇ cycloalkyl;
R⁷ is C₁-C₆ alkyl or C₃-C₇ cycloalkyl;
R⁸ is a five or six-membered, aromatic heterocyclic group containing (i) from 1 to 4 nitrogen heteroatom(s) or (ii) 1 or 2 nitrogen heteroatom(s) and 1 oxygen or 1 sulphur heteroatom or (iii) 1 or 2 oxygen or sulphur heteroatom(s), said heterocyclic group being optionally substituted by halo, oxo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl;
R⁹ is a four to seven-membered, saturated or partially unsaturated heterocyclic group containing (i) 1 or 2 nitrogen heteroatom(s) or (ii) 1 nitrogen heteroatom and 1 oxygen or 1 sulphur heteroatom or (iii) 1 oxygen or sulphur heteroatom, said heterocyclic group being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN;
R¹⁰ is H, R⁸, R⁹, R¹³, C₁-C₆ alkyl, C₃-C₇ cycloalkyl or -(C₁-C₆ alkyl)-(C₃-C₇ cycloalkyl), said C₁-C₆ alkyl and C₃-C₇ cycloalkyl being optionally substituted by -OR⁵, -OR¹³, R⁸, R⁹, R¹³ or -COR¹³;
R¹¹ is H, C₁-C₆ alkyl or C₃-C₇ cycloalkyl, said C₁-C₆ alkyl and C₃-C₇ cycloalkyl being optionally substituted by -OR⁵, -NR⁵R⁵, -NR⁵COR⁵, -CONR⁵R⁵, R⁸ or R⁹;
R¹² is C₁-C₆ alkyl substituted by R⁸, R⁹, -OR⁵, -CONR⁵R⁵, -NR⁵COR⁵ or -NR⁵R⁵;
R¹³ is phenyl optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl; and
x is 0, 1 or 2;
in combination with one or more additional therapeutic agents.

2. A combination according to claim 1 comprising a compound of formula (I) or a pharmaceutically acceptable salt thereof, wherein said additional therapeutic agent or agents are selected from:
a. reverse transcriptase inhibitors;
b. non-nucleoside reverse transcriptase inhibitors;
c. HIV protease inhibitors;
d. CCR5 antagonists;
e. CXCR4 antagonists;
f. integrate inhibitors;
g. inhibitors of viral fusion; and
h. antifungal or antibacterial agents.

3. A combination according to claim 2 wherein said additional therapeutic agent or agents are selected from zidovudine, didanosine, zalcitabine, stavudine, lamivudine, abacavir, adefovir, nevirapine, delavirdine, efavirenz, indanivir, ritonavir, saquinavir, nelfinavir, TAK-779, AMD-3100, T-20 and fluconazole.

4. A combination according to claim 2 wherein said additional therapeutic agent or agents are selected from CCR5 antagonists.

5. A combination according to any preceding claim wherein R¹, when taken separately, is H, C₁-C₆ alkyl, C₃-C₇ cycloalkyl or -OR⁷, said C₁-C₆ alkyl being optionally substituted by halo, -OR¹⁰, -NR¹⁰R¹¹, -NR⁵COR¹⁰ or R¹⁰.

6. A combination according to any preceding claim wherein R², when taken separately, is H, C₁-C₆ alkyl, C₃-C₆ alkenyl or R⁹, said C₁-C₆ alkyl being optionally substituted by -OR⁵, -OR¹², -CN, -CO₂R⁷, -CONR⁵R⁵, -C(=NR⁵)NR⁵OR⁵, -CONR⁵NR⁵R⁵, -NR⁶R⁶, -NR⁵R¹², -NR⁵COR⁸, -NR⁵COR¹², -NR⁵CO₂R⁵, R⁸ or R⁹.

7. A combination according to claims 1 to 4 or 6 wherein R¹ and R², when taken together, represent unbranched propylene wherein one methylene group is replaced by an oxygen atom or unbranched butylene wherein one methylene group is replaced by a nitrogen atom, said propylene and butylene being optionally substituted by oxo and said nitrogen atom being optionally substituted by R¹⁰.

8. A combination according to any preceding claim wherein R³ is H or C₁-C₆ alkyl.

9. A combination according to any preceding claim wherein R⁴ is phenyl substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹³, SoₓR⁶, O-(C₁-C₆ alkylene)-CONR⁵R⁵, O-(C₁-C₆ alkylene)-NR⁵R⁵, or O-(C₁-C₆ alkylene)-OR⁶.

10. A combination according to any preceding claim wherein R⁴ is phenyl substituted by R⁸, halo, -CN, C₁-C₆ alkyl, C₁-C₆ haloalkyl, C₃-C₇ cycloalkyl or C₁-C₆ alkoxy.

11. A combination according to any preceding claim wherein R⁴ is phenyl substituted by halo, -CN or C₁-C₆ alkyl.

12. A combination according to any preceding claim wherein R⁸ is pyrrolyl, imidazolyl, pyrazolyl, 1,2,3-triazolyl, 1,2,4-triazolyl, tetrazolyl, oxazolyl, isoxazolyl, thiazolyl, isothiazolyl, 1,2,4-oxadiazolyl, 1,3,4-oxadiazolyl, furanyl, thienyl, pyridinyl, pyridazinyl, pyrimidinyl or pyrazinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.

13. A combination according to any preceding claim wherein R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.

14. A combination according to any preceding claim wherein R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OR⁵, -NR⁵R⁵ or C₁-C₆ alkyl.

15. A combination according to any preceding claim wherein R⁹ is azetidinyl, tetrahydropyrrolyl, piperidinyl, azepinyl, oxetanyl, tetrahydrofuranyl, tetrahydropyranyl, oxepinyl, morpholinyl, piperazinyl or diazepinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.

16. A combination according to any preceding claim wherein R⁹ is azetidinyl, piperidinyl, tetrahydrofuranyl, piperazinyl or morpholinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.

17. A combination according to any preceding claim wherein R⁹ is azetidinyl, piperidinyl, tetrahydrofuranyl, piperazinyl or morpholinyl, each being optionally substituted by C₁-C₆ alkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by -OR⁵ or -NR-⁵COR⁵.

18. A combination according to any preceding claim wherein R¹⁰ is H, R⁸, R⁹, R¹³, C₁-C₆ alkyl or -(C₁-C₆ alkyl)-(C₃-C₇ cycloalkyl), said C₁-C₆ alkyl being optionally substituted by -OR⁵, -OR¹³, R⁸, R⁸, R¹³ or -COR¹³.

19. A combination according to any preceding claim wherein R¹⁰ is H, R⁸, R⁹, R¹³, C₁-C₆ alkyl or -(C₁-C₆ alkyl)-(C₃-C₇ cycloalkyl), said C₁-C₆ alkyl being optionally substituted by -OR⁵ or R¹³.

20. A combination according to any preceding claim wherein R¹¹ is H or C₁-C₆ alkyl, said C₁-C₆ alkyl being optionally substituted by -OR⁵, -NR⁵R⁵, -NR⁵COR⁵, -CONR⁵R⁵, R⁸ or R⁹.

21. A combination according to any preceding claim wherein R¹¹ is H or C₁-C₆ alkyl, said C₁-C₆ alkyl being optionally substituted by -OR⁵ or -NR⁵COR⁵.

22. A combination according to any preceding claim wherein R¹² is C₁-C₄ alkyl substituted by R⁸, R⁹, -OR⁵, -CONR⁵R⁵, -NR⁵COR⁵ or -NR⁵R⁵.

23. A combination according to any preceding claim wherein R¹² is C₁-C₄ alkyl substituted by R⁹, -OR⁵, -NR⁵COR⁵ or -NR⁵R⁵.

24. A combination according to any preceding claim wherein R¹³ is phenyl substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.

25. A combination according to any preceding claim wherein R¹³ is phenyl substituted by halo, -CN, -CONR⁵R⁵, -SO₂NR⁵R⁵ or -OR⁵.

26. A combination according to any one of claims 1 to 4 wherein the compound of formula (I) is
3-{[3,5-diethyl-1-(2-hydroxyethyl)-1*H*-pyrazol-4-yl]oxy}-5-fluorobenzonitrile;
3-{[3,5-diethyl-1-(2-hydroxyethyl)-1*H*-pyrazol-4-yl]oxy}-5-methylbenzonitrile;
5-{[3,5-diethyl-1-(2-hydroxyethyl)-1*H*-pyrazol-4-yl]oxy}isophthalonitrile;
3-chloro-5-{[3,5-diethyl-1-(2-hydroxyethyl)-1*H*-pyrazol-4-yl]oxy}benzonitrile;
5-[(3,5-diethyl-1*H*-pyrazol-4-yl)oxy]isophthalonitrile;
3-[(3,5-diethyl-1*H*-pyrazol-4-yl)oxy]-5-methylbenzonitrile;
3-chloro-5-[(3,5-diethyl-1*H*-pyrazol-4-yl)oxy]benzonitrile;
3-{[1-(2-aminoethyl)-3,5-diethyl-1*H*-pyrazol-4-yl]oxy}-5-methylbenzonitrile;
3-{[1-(2-aminoethyl)-3,5-diethyl-1*H*-pyrazol-4-yl]oxy}-5-chlorobenzonitrile;
5-{[1-(2-aminoethyl)-3,5-diethyl-1*H*-pyrazol-4-yl]oxy}isophthalonitrile;
or a pharmaceutically acceptable salt, or solvate thereof.

27. A combination according to any one of claims 1 to 4 wherein the compound of formula (I) is
5-{[3,5-diethyl-1-(2-hydroxyethyl)-1*H*-pyrazol-4-yl]oxy}isophthalonitrile
or a pharmaceutically acceptable salt, or solvate thereof.

28. A combination as defined in any one of claims 1 to 27 for use as a medicament.

29. A combination as defined in any one of claims 1 to 27 for use in the treatment of infection by HIV or related retroviruses.

30. Use of a combination as defined in any one of claims 1 to 27 for the manufacture of a medicament for simultaneous or sequential treatment of infection by HIV or a genetically-related retroviral infection.

## Patentansprüche

1. Verbindung der Formel (I) 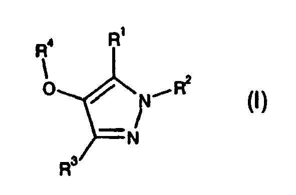 oder ein pharmazeutisch annehmbares Salz oder Solvat davon, worin:
entweder R¹ für H, C₁-C₆-Alkyl, C₃-C₇-Cycloalkyl oder -OR⁷ steht, wobei das C₁-C₆-Alkyl und C₃-C₇-Cycloalkyl gegebenenfalls mit Halogen, -CN, -OR¹⁰, S(O)ₓR¹⁰, -CO₂R¹⁰, -CONR⁵R¹⁰, -OCONR⁵R¹⁰, -NR⁵CO₂R¹⁰, -NR¹⁰R¹¹, -NR⁵COR¹⁰, -SO₂NR⁵R¹⁰, -NR⁵CONR⁵R¹⁰, -NR⁵SO₂R¹⁰ oder R¹⁰ substituiert ist; und
R² für H, C₁-C₆-Alkyl, C₃-C₆-Alkenyl oder R⁹ steht, wobei das C₁-C₆-Alkyl gegebenenfalls mit Halogen, -OR⁵, -OR¹², -CN, -CO₂R⁷, -OCONR⁵R⁵, -CONR⁵R¹⁵, -C(=NR⁵)NR⁵OR⁵, -CONR⁵NR⁵R⁵, -NR⁶R⁶, -NR⁵R¹², -NR⁵COR⁵, -NR⁵COR⁸, -NR⁸COR¹², -NR⁵CO₂R⁵, -NR⁵CONR⁵R⁵, -SO₂NR⁵R⁵, -NR⁵SO₂R⁵, R⁸ oder R⁹ substituiert ist;
oder R¹ und R², wenn sie zusammengenommen werden, für unverzweigtes C₃-C₄-Alkylen stehen, das gegebenenfalls mit Oxo substituiert ist, wobei eine Methylengruppe des C₃-C₄-Alkylens durch ein Sauerstoffatom oder ein Stickstoffatom ersetzt ist, wobei das Stickstoffatom gegebenenfalls mit R¹⁰ substituiert ist;
R³ für H oder C₁-C₆-Alkyl steht, wobei das C₁-C₆-Alkyl gegebenenfalls mit Halogen, -CN, -OR⁵, -CO₂R⁵, -CONR⁵R⁵, -OCONR⁵R⁵, -NR⁵CO₂R⁵, -NR⁶R⁶, -NR⁵COR⁵ -SO₂NR⁵R⁵, -NR⁵CONR⁵R⁵, -NR⁵SO₂R⁵, R⁸ oder R⁹ substituiert ist;
R⁴ für Phenyl, das gegebenenfalls mit R⁸, Halogen, -CN, C₁-C₆-Alkyl, C₁-C₆-Halogenalkyl, C₃-C₇-Cycloalkyl, C₁-C₆-Alkoxy, -CONR⁵R⁵, OR¹³, SOₓR⁶, O- (C₁-C₆-Alkylen) -CONR⁵R⁵, O- (C₁-C₆-Alkylen) -NR⁵R⁵ oder O- (C₁-C₆-Alkylen) -OR⁶ oder substituiert ist, oder Naphthyl steht;
jedes R⁵ unabhängig entweder für H, C₁-C₆-Alkyl oder C₃-C₇-Cycloalkyl steht oder, wenn zwei R⁵-Gruppen an dasselbe Stickstoffatom gebunden sind, diese zwei Gruppen zusammengenommen mit dem Stickstoffatom, an das sie gebunden sind, für Azetidinyl, Pyrrolidinyl, Piperidinyl, Homopiperidinyl, Piperazinyl, Homopiperazinyl oder Morpholinyl stehen, wobei das Azetidinyl, Pyrrolidinyl, Piperidinyl, Homopiperidinyl, Piperazinyl, Homopiperazinyl und Morpholinyl gegebenenfalls mit C₁-C₆-Alkyl oder C₃-C₇-Cycloalkyl substituiert sind;
R⁶ jeweils unabhängig entweder für H, C₁-C₆-Alkyl oder C₃-C₇-Cycloalkyl steht;
R⁷ für C₁-C₆-Alkyl oder C₃-C₇-Cycloalkyl steht;
R⁸ eine fünf- oder sechsgliedrige aromatische heterocyclische Gruppe ist, die (i) 1 bis 4 Stickstoff-Heteroatom(e) oder (ii) 1 oder 2 Stickstoff-Heteroatom(e) und 1 Sauerstoff- oder 1 Schwefel-Heteroatom oder (iii) 1 oder 2 Sauerstoff- oder Schwefel-Heteroatom(e) enthält, wobei die heterocyclische Gruppe gegebenenfalls mit Halogen, Oxo, -CN, -COR⁵, -CONR⁵R⁵, -SO₂NR⁵R⁵, -NR⁵SO₂R⁵, - OR⁵, -NR⁵R⁵, -(C₁-C₆-Alkylen)-NR⁵R⁵, C₁-C₆-Alkyl, Fluor- (C₁-C₆)-alkyl oder C₃-C₇-Cycloalkyl substituiert ist;
R⁹ eine vier- bis siebengliedrige, gesättigte oder teilweise ungesättigte, heterocyclische Gruppe ist, die (i) 1 oder 2 Stickstoff-Heteroatom(e) oder (ii) 1 Stickstoff-Heteroatom und 1 Sauerstoff- oder 1 Schwefel-Heteroatom oder (iii) ein Sauerstoff- oder Schwefel-Heteroatom enthält, wobei die heterocyclische Gruppe gegebenenfalls mit Oxo, C₁-C₆-Alkyl, C₃-C₇-Cycloalkyl, - SO₂R⁵, -CONR⁵R⁵, -COOR⁵, -CO-(C₁-C₆-Alkylen)-OR⁵ oder -COR⁵ substituiert ist und gegebenenfalls an einem Kohlenstoffatom, das nicht zu einem Heteroatom benachbart ist, mit Halogen, -OR⁵, -NR⁵R⁵, -NR⁵COR⁵, -NR⁵COOR⁵, - NR⁵CONR⁵R⁵, -NR⁵SO₂R⁵ oder -CN substituiert ist;
R¹⁰ für H, R⁸, R⁹, R¹³, C₁-C₆-Alkyl, C₃-C₇-Cycloalkyl oder - (C₁-C₆-Alkyl) - (C₃-C₇-cyloalkyl) steht, wobei das C₁-C₆-Alkyl und das C₃-C₇-Cycloalkyl gegebenenfalls mit -OR⁵, -OR¹³, R⁸, R⁹, R¹³ oder -COR¹³ substituiert sind;
R¹¹ für H, C₁-C₆-Alkyl oder C₃-C₇-Cycloalkyl steht, wobei das C₁-C₆-Alkyl und das C₃-C₇-Cycloalkyl gegebenenfalls mit -OR⁵, -NR⁵R⁵, -NR⁵COR⁵, -CONR⁵R⁵ oder R⁹ substituiert sind;
R¹² für C₁-C₆-Alkyl steht, das mit R⁸, R⁹, -OR⁵, -CONR⁵R⁵, -NR⁵COR⁵ oder -NR⁵R⁵ substituiert ist;
R¹³ Phenyl ist, das gegebenenfalls mit Halogen, -CN, -COR⁵, -CONR⁵R⁵, -SO₂NR⁵R⁵, -NR⁵SO₂R⁵, -OR⁵, -NR⁵R⁵, - (C₁-C₆-Alkylen) -NR⁵R⁵, C₁-C₆-Alkyl, Halogen- (C₁-C₆) -alkyl oder C₃-C₇-Cycloalkyl substituiert ist; und
x für 0, 1 oder 2 steht,
in Kombination mit einem oder mehreren zusätzlichen therapeutischen Mittel(n).

2. Kombination nach Anspruch 1, umfassend eine Verbindung der Formel (I) oder ein pharmazeutisch annehmbares Salz davon, wobei das zusätzliche therapeutische Mittel oder die zusätzlichen therapeutischen Mittel ausgewählt ist/sind aus:
a. Inhibitoren der reversen Transkriptase;
b. Nicht-Nukleosid-reverse Transkriptase-Inhibitoren;
c. HIV-Protease-Inhibitoren;
d. CCR5-Antagonisten;
e. CXCR4-Antagonisten;
f. Integrase-Inhibitoren;
g. Inhibitoren der viralen Fusion und
h. antifungalen oder antibakteriellen Mitteln.

3. Kombination nach Anspruch 2, wobei das zusätzliche therapeutische Mittel oder die zusätzlichen therapeutischen Mittel ausgewählt ist/sind aus Zidovudin, Didanosin, Zalcitabin, Stavudin, Lamivudin, Abacavir, Adefovir, Nevirapin, Delaviridin, Efavirenz, Indanivir, Ritonavir, Saquinavir, Nelfinavir, TAK-779, AMD-3100, T-20 und Fluconazol.

4. Kombination nach Anspruch 2, wobei das zusätzliche therapeutische Mittel oder die zusätzlichen therapeutischen Mittel aus CCR5-Antagonisten ausgewählt ist/sind.

5. Kombination nach einem vorangehenden Anspruch, wobei R¹, wenn es getrennt genommen wird, für H, C₁-C₆-Alkyl, C₃-C₇-Cycloalkyl oder -OR⁷ steht, wobei das C₁-C₆-Alkyl gegebenenfalls mit Halogen, -OR¹⁰, -NR¹⁰R¹¹, -NR⁵COR¹⁰ oder R¹⁰ substituiert ist.

6. Kombination nach einem vorangehenden Anspruch, wobei R², wenn es getrennt genommen wird, für H, C₁-C₆-Alkyl, C₃-C₆-Alkenyl oder R⁹ steht, wobei das C₁-C₆-Alkyl gegebenenfalls mit -OR⁵, -OR¹², -CN, -CO₂R⁷, -CONR⁵R⁵, -C (=NR⁵)NR⁵OR⁵, -CONR⁵NR⁵R⁵, -NR⁶R⁶, -NR⁵R¹², -NR⁵COR⁸, -NR⁵COR¹², -NR⁵CO₂R⁵, R⁸ oder R⁹ substituiert ist.

7. Kombination nach den Ansprüchen 1 bis 4 oder Anspruch 6, wobei R¹ und R², wenn sie zusammengenommen werden, für unverzweigtes Propylen, worin eine Methylengruppe durch ein Sauerstoffatom ersetzt ist, oder unverzweigtes Butylen, worin eine Methylengruppe durch ein Stickstoffatom ersetzt ist, stehen, wobei das Propylen und das Butylen gegebenenfalls mit Oxo substituiert sind und das Stickstoffatom gegebenenfalls mit R¹⁰ substituiert ist.

8. Kombination nach einem der vorangehenden Ansprüche, wobei R³ für H oder C₁-C₆-Alkyl steht.

9. Kombination nach einem der vorangehenden Ansprüche, wobei R⁴ für Phenyl steht, das mit R⁸, Halogen, -CN, C₁-C₆-Alkyl, C₁-C₆-Halogenalkyl, C₃-C₇-Cycloalkyl, C₁-C₆-Alkoxy, -CONR⁵R⁵, OR¹³, SOₓR⁶, O- (C₁-C₆-Alkylen) -CONR⁵R⁵, O- (C₁-C₆-Alkylen) -NR⁵R⁵ oder O-(C₁-C₆-Alkylen)-OR⁶ substituiert ist.

10. Kombination nach einem vorangehenden Anspruch, wobei R⁴ für Phenyl steht, das mit R⁸, Halogen, -CN, C₁-C₆-Alkyl, C₁-C₆-Halogenalkyl, C₃-C₇-Cycloalkyl oder C₁-C₆-Alkoxy substituiert ist.

11. Kombination nach einem vorangehenden Anspruch, wobei R⁴ für Phenyl steht, das mit Halogen, -CN oder C₁-C₆-Alkyl substituiert ist.

12. Kombination nach einem der vorangehenden Ansprüche, wobei R⁸ für Pyrrolyl, Imidazolyl, Pyrazolyl, 1,2,3-Triazolyl, 1,2,4-Triazolyl, Tetrazolyl, Oxazolyl, Isoxazolyl, Thiazolyl, Isothiazolyl, 1,2,4-Oxadiazolyl, 1,3,4-Oxadiazolyl, Furanyl, Thienyl, Pyridinyl, Pyridazinyl, Pyrimidinyl oder Pyrazinyl steht, das jeweils gegebenenfalls mit Halogen, -CN, -COR⁵, -CONR⁵R⁵, -SO₂NR⁵R⁵, -NR⁵SO₂R⁵, -OR⁵, -NR⁵R⁵, - (C₁-C₆-Alkylen) -NR⁵R⁵, C₁-C₆-Alkyl, Fluor-(C₁-C₆)-alkyl oder C₃-C₇-Cycloalkyl substituiert ist.

13. Kombination nach einem vorangehenden Anspruch, wobei R⁸ für Imidazolyl, Pyrazolyl, 1,2,4-Triazolyl, 1,2,4-Oxadiazolyl, 1,3,4-Oxadiazolyl, Pyridinyl, Pyrazinyl oder Pyrimidinyl steht, das jeweils gegebenenfalls mit Halogen, -CN, -COR⁵, -CONR⁵R⁵, -SO₂NR⁵R⁵, -NR⁵SO₂R⁵, -OR⁵, - NR⁵R⁵, -(C₁-C₆-Alkylen) -NR⁵R⁵, C₁-C₆-Alkyl, Fluor-(C₁-C₆) - alkyl oder C₃-C₇-Cycloalkyl substituiert ist.

14. Kombination nach einem vorangehenden Anspruch, wobei R⁸ für Imidazolyl, Pyrazolyl, 1,2,4-Triazolyl, 1,2,4-Oxadiazolyl, 1,3,4-Oxadiazolyl, Pyridinyl, Pyrazinyl oder Pyrimidinyl steht, das jeweils gegebenenfalls mit - OR⁵, -NR⁵R⁵ oder C₁-C₆-Alkyl substituiert ist.

15. Kombination nach einem vorangehenden Anspruch, wobei R⁹ für Azetidinyl, Tetrahydropyrrolyl, Piperidinyl, Azepinyl, Oxetanyl, Tetrahydrofuranyl, Tetrahydropyranyl, Oxepinyl, Morpholinyl, Piperazinyl oder Diazepinyl steht, das jeweils gegebenenfalls mit Oxo, C₁-C₆-Alkyl, C₃-C₇-Cycloalkyl, -SO₂R⁵, -CONR⁵R⁵, -COOR⁵, -CO-(C₁-C₆-Alkylen)-OR⁵ oder -COR⁵ substituiert ist und gegebenenfalls an einem Kohlenstoffatom, das nicht zu einem Heteroatom benachbart ist, mit Halogen, -OR⁵, -NR⁵R⁵, -NR⁵COR⁵, -NR⁵COOR⁵, -NR⁵CONR⁵R⁵, -NR⁵SO₂R⁵ oder -CN substituiert ist.

16. Kombination nach einem der vorangehenden Ansprüche, wobei R⁹ für Azetidinyl, Piperidinyl, Tetrahydrofuranyl, Piperazinyl oder Morpholinyl steht, das jeweils gegebenenfalls mit Oxo, C₁-C₆-Alkyl, C₃-C₇-Cycloalkyl, - SO₂R⁵, -CONR⁵R⁵, -COOR⁵, -CO- (C₁-C₆-Alkylen) -OR⁵ oder -COR⁵ substituiert ist und gegebenenfalls an einem Kohlenstoffatom, das nicht zu einem Heteroatom benachbart ist, mit Halogen, -OR⁵, -NR⁵R⁵, -NR⁵COR⁵, -NR⁵COOR⁵, - NR⁵CONR⁵R⁵, -NR⁵SO₂R⁵ oder -CN substituiert ist.

17. Kombination nach einem vorangehenden Anspruch, wobei R⁹ für Azetidinyl, Piperidinyl, Tetrahydrofuranyl, Piperazinyl oder Morpholinyl steht, das jeweils gegebenenfalls mit C₁-C₆-Alkyl, -SO₂R⁵, -CONR⁵R⁵, -COOR⁵, - CO-(C₁-C₆-Alkylen)-OR⁵ oder -COR⁵ substituiert ist und gegebenenfalls an einem Kohlenstoffatom, das nicht zu einem Heteroatom benachbart ist, mit -OR⁵ oder -NR⁵COR⁵ substituiert ist.

18. Kombination nach einem vorangehenden Anspruch, wobei R¹⁰ für H, R⁸, R⁹, R¹³, C₁-C₆-Alkyl oder -(C₁-C₆-Alkyl) - (C₃-C₇-cycloalkyl) steht, wobei das C₁-C₆-Alkyl gegebenenfalls mit -OR⁵, -OR¹³, R⁸, R⁹, R¹³ oder -COR¹³ substituiert ist.

19. Kombination nach einem vorangehenden Anspruch, wobei R¹⁰ für H, R⁸, R⁹, R¹³, C₁-C₆-Alkyl oder - (C₁-C₆-Alkyl) - (C₃-C₇-cycloalkyl) steht, wobei das C₁-C₆-Alkyl gegebenenfalls mit -OR⁵ oder -R¹³ substituiert ist.

20. Kombination nach einem vorangehenden Anspruch, wobei R¹¹ für H oder C₁-C₆-Alkyl steht, wobei das C₁-C₆-Alkyl gegebenenfalls mit -OR⁵, -NR⁵R⁵, -NR⁵COR⁵, -CONR⁵R⁵, R⁸ oder R⁹ substituiert ist.

21. Kombination nach einem vorangehenden Anspruch, wobei R¹¹ für H oder C₁-C₆-Alkyl steht, wobei das C₁-C₆-Alkyl gegebenenfalls mit -OR⁵ oder -NR⁵COR⁵ substituiert ist.

22. Kombination nach einem vorangehenden Anspruch, wobei R¹² für C₁-C₄-Alkyl steht, das mit R⁸, R⁹, -OR⁵, -CONR⁵R⁵, -NR⁵COR⁵ oder -NR⁵R⁵ substituiert ist.

23. Kombination nach einem vorangehenden Anspruch, wobei R¹² für C₁-C₄-Alkyl steht, das mit R⁹, OR⁵, -NR⁵COR⁵ oder -NR⁵R⁵ substituiert ist.

24. Kombination nach einem vorangehenden Anspruch, wobei R¹³ für Phenyl steht, das mit Halogen, -CN, -COR⁵, -CONR⁵R⁵, -SO₂NR⁵R⁵, -NR⁵SO₂R⁵, -OR⁵, -NR⁵R⁵, - (C₁-C₆-Alkylen) -NR⁵R⁵, C₁-C₆-Alkyl, Halogen- (C₁-C₆) -alkyl oder C₃-C₇-Cycloalkyl substituiert ist.

25. Kombination nach einem vorangehenden Anspruch, wobei R¹³ für Phenyl steht, das mit Halogen, -CN, -CONR⁵R⁵, -SO₂NR⁵R⁵ oder -OR⁵ substituiert ist.

26. Kombination nach einem der Ansprüche 1 bis 4, wobei die Verbindung der Formel (I)
3-{[3,5-Diethyl-1-(2-hydroxyethyl)-1*H*-pyrazol-4-yl]oxy}-5-fluorbenzonitril;
3-{[3,5-Diethyl-1-(2-hydroxyethyl)-1*H*-pyrazol-4-yl]oxy}-5-methylbenzonitril;
5-{[3,5-Diethyl-1-(2-hydroxyethyl)-1*H*-pyrazol-4-yl]oxy}-isophthalonitril;
3-Chlor-5-{[3,5-diethyl-1-(2-hydroxyethyl)-1*H*-pyrazol-4-yl]oxy}benzonitril;
5-[(3,5-Diethyl-1*H*-pyrazol-4-yl)oxy]isophthalonitril;
3-[(3,5-Diethyl-1*H*-pyrazol-4-yl)oxy]-5-methylbenzonitril;
3-Chlor-5-[(3,5-diethyl-1*H*-pyrazol-4-yl)oxy]benzonitril;
3-{[1-(2-Aminoethyl)-3,5-diethyl-1*H*-pyrazol-4-yl]oxy}-5-methylbenzonitril;
3-{[1-(2-Aminoethyl)-3,5-diethyl-1*H*-pyrazol-4-yl]oxy}-5-chlorbenzonitril;
5-{[1-(2-Aminoethyl)-3,5-diethyl-1*H*-pyrazol-4-yl]oxy}isophthalonitril;
oder ein pharmazeutisch annehmbares Salz oder Solvat davon ist.

27. Kombination nach einem der Ansprüche 1 bis 4, wobei die Verbindung der Formel (I) 5-{[3,5-Diethyl-1-(2-hydroxyethyl)-1*H*-pyrazol-4-yl]oxy}-isophthalonitril oder ein pharmazeutisch annehmbares Salz oder Solvat davon ist.

28. Kombination, wie sie in einem der Ansprüche 1 bis 27 definiert ist, zur Verwendung als Medikament.

29. Kombination, wie sie in einem der Ansprüche 1 bis 27 definiert ist, zur Verwendung in der Behandlung einer Infektion durch HIV oder verwandte Retroviren.

30. Verwendung einer Kombination, wie sie in einem der Ansprüche 1 bis 27 definiert ist, zur Herstellung eines Medikaments zur gleichzeitigen oder sequenziellen Behandlung einer Infektion durch HIV oder einer genetisch verwandten retroviralen Infektion.

## Revendications

1. Composé de formule (I) 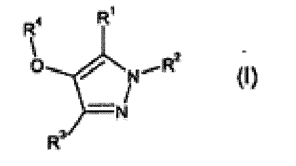 ou un de ses sels ou produits de solvatation pharmaceutiquement acceptables, formule dans laquelle :
soit R¹ représente H, un groupe alkyle en C₁ à C₆, cycloalkyle en C₃ à C₇, ou -OR⁷, lesdits groupes alkyle en C₁ à C₆ et cycloalkyle en C₃ à C₇ étant facultativement substitués avec des substituants halogéno, -CN, -OR¹⁰, S(O)ₓR¹⁰, -CO₂R¹⁰, -CONR⁵R¹⁰, -OCONR⁵R¹⁰, -NR⁵CO₂R¹⁰, -NR¹⁰R¹¹, -NR⁵COR¹⁰, -SO₂NR⁵R¹⁰, -NR⁵CONR⁵R¹⁰, -NR⁵SO₂R¹⁰ ou R¹⁰ ; et
R² représente H, un groupe alkyle en C₁ à C₆, alcényle en C₃ à C₆ ou R⁹, ledit groupe alkyle en C₁ à C₆, étant facultativement substitué avec des substituants halogéno, -OR⁵, -OR¹², -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹², -NR⁵COR⁵, -NR⁵COR⁸, -NR⁵COR¹², -NR⁵CO₂R⁵, -NR⁵CONR⁵R⁵, -SO₂NR⁵R⁵, -NR⁵SO₂R⁵, R⁸ ou R⁹ ;
soit R¹ et R², lorsqu'ils sont pris conjointement, représentent un groupe alkylène en C₃ ou C₄ non ramifié, facultativement substitué avec un substituant oxo, dans lequel un groupe méthylène dudit groupe alkylène en C₃ ou C₄ est remplacé par un atome d'oxygène ou un atome d'azote, ledit atome d'azote étant facultativement substitué avec R¹⁰ ;
R³ représente H ou un groupe alkyle en C₁ à C₆, ledit groupe alkyle en C₁ à C₆ étant facultativement substitué avec des substituants halogéno, -CN, -OR⁵, -CO₂R⁵, -CONR⁵R⁵, -OCONR⁵R⁵, -NR⁵CO₂R⁵, -NR⁶R⁶, -NR⁵COR⁵, -SO²NR⁵R⁵, -NR⁵CONR⁵R⁵, -NR⁵SO²R⁵, R⁸ ou R⁹ ;
R⁴ représente un groupe phényle facultativement substitué avec des substituants R⁸, halogéno, -CN, alkyle en C₁ à C₆, halogénalkyle en C₁ à C₆, cycloalkyle en C₃ à C₇, alkoxy en C₁ à C₆, -CONR⁵R⁵, OR¹³, SoₓR⁶, O-(alkylène en C₁ à C₆)-CONR⁵R⁵, O-(alkylène en C₁ à C₆)-NR⁵R⁵ ou O-(alkylène en C₁ à C₆)-OR⁶ ; ou naphtyle ;
chaque groupe R⁵ représente indépendamment H, un groupe alkyle en C₁ à C₆ ou cycloalkyle en C₃ à C₇, ou bien, lorsque deux groupes R⁵ sont fixés au même atome d'azote, ces deux groupes, pris conjointement avec l'atome d'azote auquel ils sont fixés, représentent un groupe azétidinyle, pyrrolidinyle, pipéridinyle, homopipéridinyle, pipérazinyle, homopipérazinyle ou morpholinyle, lesdits groupes azétidinyle, pyrrolidinyle, pipéridinyle, homopipéridinyle, pipérazinyle, homo- pipérazinyle et morpholinyle étant facultativement substitués avec des substituants alkyle en C₁ à C₆ ou cycloalkyle en C₃ à C₇ ;
chaque groupe R⁶ représente indépendamment H, un groupe alkyle en C₁ à C₆ ou cycloalkyle en C₃ à C₇ ;
R⁷ représente un groupe alkyle en C₁ à C₆ ou cycloalkyle en C₃ à C₇ ;
R⁸ représente un groupe hétérocyclique aromatique penta- ou hexagonal contenant (i) 1 à 4 hétéroatomes d'azote ou (ii) 1 ou 2 hétéroatomes d'azote et 1 hétéroatome d'oxygène ou 1 hétéroatome de soufre ou (iii) 1 ou 2 hétéroatomes d'oxygène ou de soufre, ledit groupe hétérocyclique étant facultativement substitué avec un substituant halogéno, oxo, -CN, -COR⁵, -CONR⁵R⁵, -SO₂NR⁵R⁵, -NR⁵SO₂R⁵, -OR⁵, -NR⁵R⁵, -(alkylène en C₁ à C₆) -NR⁵R⁵, alkyle en C₁ à C₆, fluoralkyle en C₁ à C₆ ou cycloalkyle en C₃ à C₇ ;
R⁹ représente un groupe hétérocyclique tétra- à heptagonal, saturé ou partiellement insaturé, contenant (i) 1 ou 2 hétéroatomes d'azote ou (ii) 1 hétéroatome d'azote et 1 hétéroatome d'oxygène ou 1 hétéroatome de soufre ou (iii) 1 hétéroatome d'oxygène ou de soufre, ledit groupe hétérocyclique étant facultativement substitué avec des substituants oxo, alkyle en C₁ à C₆, cycloalkyle en C₃ à C₇, -SO₂R⁵, -CONR⁵R⁵, -COOR⁵, -CO-(alkylène en C₁ à C₆)-OR⁵ ou -COR⁵ et étant facultativement substitué sur un atome de carbone qui n'est pas adjacent à un hétéroatome avec un substituant halogéno, -OR⁵, -NR⁵R⁵, -NR⁵COR⁵, -NR⁵COOR⁵, -NR⁵CONR⁵R⁵, -NR⁵SO₂R⁵ ou -CN ;
R¹⁰ représente H, un groupe R⁸, R⁹, R¹³, alkyle en C₁ à C₆, cycloalkyle en C₃ à C₇ ou - (alkyle en C₁ à C₆)- (cycloalkyle en C₃ à C₇), lesdits groupes alkyle en C₁ à C₆ et cycloalkyle en C₃ à C₇ étant facultativement substitués avec des substituants -OR⁵, -OR¹³, R⁸, R⁹, R¹³ ou -COR¹³ ;
R¹¹ représente H, un groupe alkyle en C₁ à C₆ ou cycloalkyle en C₃ à C₇, lesdits groupes alkyle en C₁ à C₆ et cycloalkyle en C₃ à C₇ étant facultativement substitués avec des substituants -OR⁵, -NR⁵R⁵, -NR⁵COR⁵, -CONR⁵R⁵, R⁸ ou R⁹ ;
R¹² représente un groupe alkyle en C₁ à C₆ substitué avec un substituant R⁸, R⁹, -OR⁵, -CONR⁵R⁵, -NR⁵COR⁵ ou -NR⁵R⁵ ;
R¹³ représente un groupe phényle facultativement substitué avec un substituant halogéno, -CN, -COR⁵, -CONR⁵R⁵, -SO₂NR⁵R⁵, -NR⁵SO₂R⁵, -OR⁵, -NR⁵R⁵, -(alkylène en C₁ à C₆)- NR⁵R⁵, alkyle en C₁ à C₆, halogénalkyle en C₁ à C₆ ou cycloalkyle en C₃ à C₇ ; et
x est égal à 0, 1 ou 2 ;
en association avec un ou plusieurs agents thérapeutiques supplémentaires.

2. Association suivant la revendication 1, comprenant un composé de formule (I) ou un de ses sels pharmaceutiquement acceptables, dans laquelle ledit ou lesdits agents thérapeutiques supplémentaires sont choisis entre :
a. des inhibiteurs de transcriptase inverse ;
b. des inhibiteurs non nucléosidiques de transcriptase inverse ;
c. des inhibiteurs de protéases du VIH ;
d. des antagonistes de CCR5 ;
e. des antagonistes de CXCR4 ;
f. des inhibiteurs d'intégrase ;
g. des inhibiteurs de fusion virale ; et
h. des agents antifongiques ou antibactériens.

3. Association suivant la revendication 2, dans laquelle ledit ou lesdits agents thérapeutiques supplémentaires sont choisis entre la zidovudine, la didanosine, la zalcitabine, la stavudine, la lamivudine, l'abacavir, l'adéfovir, la névirapine, la délavirdine, l'éfavirenz, l'indanivir, le ritonavir, le saquinavir, le nelfinavir, le TAK-779, le AMD-3100, le T20 et le fluxonazole.

4. Association suivant la revendication 2, dans laquelle ledit ou lesdits agents thérapeutiques supplémentaires sont choisis parmi des antagonistes de CCR5.

5. Association suivant l'une quelconque des revendications précédentes, dans laquelle R¹, lorsqu'il est pris séparément, représente H, un groupe alkyle en C₁ à C₆, cycloalkyle en C₃ à C₇ ou -OR⁷, ledit groupe alkyle en C₁ à C₆ étant facultativement substitué avec un substituant halogéno, -OR¹⁰, -NR¹⁰R¹¹, -NCR⁵COR¹⁰ ou R¹⁰.

6. Association suivant l'une quelconque des revendications précédentes, dans laquelle R², lorsqu'il est pris séparément, représente H, un groupe alkyle en C₁ à C₆, alcényle en C₃ à C₆ ou R⁹, ledit groupe alkyle en C₁ à C₆ étant facultativement substitué avec un substituant -OR⁵, -OR¹², -CN, -CO₂R⁷, -CONR⁵R⁵, -C(=NR⁵)NR⁵OR⁵, -CONR⁵NR⁵R⁵, -NR⁵R¹², -NR⁵COR⁸, -NR⁵COR¹², -NR⁵CO₂R⁵, R⁸ ou R⁹.

7. Association suivant les revendications 1 à 4 ou 6, dans laquelle R¹ et R², lorsqu'ils sont pris conjointement, représentent un groupe propylène non ramifié dans lequel un groupe méthylène est remplacé par un atome d'oxygène ou un groupe butylène non ramifié dans lequel un groupe méthylène est remplacé par un atome d'azote, lesdits groupes propylène et butylène étant facultativement substitués avec un substituant oxo et ledit atome azote étant facultativement R¹⁰.

8. Association suivant l'une quelconque des revendications précédentes, dans laquelle R³ représente H ou un groupe alkyle en C₁ à C₆.

9. Association suivant l'une quelconque des revendications précédentes, dans laquelle R⁴ représente un groupe phényle substitué avec un substituant R⁸, halogéno, -CN, alkyle en C₁ à C₆, halogénalkyle en C₁ à C₆, cycloalkyle en C₃ à C₇, alkoxy en C₁ à C₆, -CONR⁵R⁵, OR¹³, SOₓR⁶, O-(alkylène en C₁ à C₆)-CONR⁵R⁵, O-(alkylène en C₁ à C₆) -NR⁵R⁵ ou O-(alkylène en C₁ à C₆)-OR⁶.

10. Association suivant l'une quelconque des revendications précédentes, dans laquelle R⁴ représente un groupe phényle substitué avec un substituant R⁸, halogéno, -CN, alkyle en C₁ à C₆, halogénalkyle en C₁ à C₆, cycloalkyle en C₃ à C₇ ou alkoxy en C₁ à C₆.

11. Association suivant l'une quelconque des revendications précédentes, dans laquelle R⁴ représente un groupe phényle substitué avec un substituant halogéno, -CN ou alkyle en C₁ à C₆.

12. Association suivant l'une quelconque des revendications précédentes, dans laquelle R⁸ représente un groupe pyrrolyle, imidazolyle, pyrazolyle, 1,2,3-triazolyle, 1,2,4-triazolyle, tétrazolyle, oxazolyle, isoxazolyle, thiazolyle, isothiazolyle, 1,2,4-oxadiazolyle, 1,3,4-oxadiazolyle, furannyle, thiényle, pyridinyle, pyridazinyle, pyrimidinyle ou pyrazinyle, chacun étant facultativement substitué avec un substituant halogéno, -CN, -COR⁵, -CONR⁵R⁵, -SO₂NR⁵R⁵, -NR⁵SO₂R⁵, -OR⁵, -NR⁵R⁵, - (alkylène en C₁ à C₆) -NR⁵R⁵, alkyle en C₁ à C₆, fluoralkyle en C₁ à C₆ ou cycloalkyle en C₃ à C₇.

13. Association suivant l'une quelconque des revendications précédentes, dans laquelle R⁸ représente un groupe imidazolyle, pyrazolyle, 1,2,4-triazolyle, 1,2,4-oxadiazolyle, 1,3,4-oxadiazolyle, pyridinyle, pyrazinyle ou pyridazinyle, chacun étant facultativement substitué avec un substituant halogéno, -CN, -COR⁵, -CONR⁵R⁵, -SO₂NR⁵R⁵, -NR⁵SO₂R⁵, -OR⁵, -NR⁵R⁵, - (alkylène en C₁ à C₆) -NR⁵R⁵, alkyle en C₁ à C₆, fluoralkyle en C₁ à C₆ ou cycloalkyle en C₃ à C₇.

14. Association suivant l'une quelconque des revendications précédentes, dans laquelle R⁸ représente un groupe imidazolyle, pyrazolyle, 1,2,4-triazolyle, 1,2,4-oxadiazolyle, 1,3,4-oxadiazolyle, pyridinyle, pyrazinyle, ou pyrimidinyle, chacun étant facultativement substitué avec un substituant -OR⁵, -NR⁵R⁵ ou alkyle en C₁ à C₆.

15. Association suivant l'une quelconque des revendications précédentes, dans laquelle R⁹ représente un groupe azétidinyle, tétrahydropyrrolyle, pipéridinyle, azépinyle, oxétannyle, tétrahydrofurannyle, tétrahydropyrannyle, oxépinyle, morpholinyle, pipérazinyle ou diazépinyle, chacun étant facultativement substitué avec un substituant oxo, alkyle en C₁ à C₆, cycloalkyle en C₃ à C₇, -SO₂R⁵, -CONR⁵R⁵, -COOR⁵, -CO-(alkylène en C₁ à C₆)-OR⁵ ou -COR⁵ et facultativement substitué sur un atome de carbone qui n'est pas adjacent à un hétéroatome avec un substituant halogéno, -OR⁵, -NR⁵R⁵, -NR⁵COR⁵, -NR⁵COOR⁵, -NR⁵CONR⁵R⁵, -NR⁵SO₂R⁵ ou -CN.

16. Association suivant l'une quelconque des revendications précédentes, dans laquelle R⁹ représente un groupe azétidinyle, pipéridinyle, tétrahydrofurannyle, pipérazinyle ou morpholinyle, chacun étant facultativement substitué avec un substituant oxo, alkyle en C₁ à C₆, cycloalkyle en C₃ à C₇, -SO₂R⁵, -CONR⁵R⁵, -COOR⁵, -CO-(alkylène en C₁ à C₆) -OR⁵ ou -COR⁵ et facultativement substitué sur un atome de carbone qui n'est pas adjacent à un hétéroatome avec un substituant halogéno, -OR⁵, -NR⁵R⁵, -NR⁵COR⁵, -NR⁵COOR⁵, -NR⁵CONR⁵R⁵, -NR⁵SO₂R⁵ ou -CN.

17. Association suivant l'une quelconque des revendications précédentes, dans laquelle R⁹ représente un groupe azétidinyle, pipéridinyle, tétrahydrofurannyle, pipérazinyle ou morpholinyle, chacun étant facultativement substitué avec un substituant alkyle en C₁ à C₆, -SO₂R⁵, -CONR⁵R⁵, -COOR⁵, -CO-(alkylène en C₁ à C₆)-OR⁵ ou -COR⁵ et facultativement substitué sur un atome de carbone qui n'est pas adjacent à un hétéroatome avec un substituant -OR⁵ ou -NR⁵COR⁵.

18. Association suivant l'une quelconque des revendications précédentes, dans laquelle R¹⁰ représente H, un groupe R⁸, R⁹, R¹³, alkyle en C₁ à C₆ ou - (alkyle en C₁ à C₆) - (cycloalkyle en C₃ à C₇), ledit groupe alkyle en C₁ à C₆ étant facultativement substitué avec un substituant -OR⁵, -OR¹³, R⁸, R⁹, R¹³ ou -COR¹³.

19. Association suivant l'une quelconque des revendications précédentes, dans laquelle R¹⁰ représente H, un groupe R⁸, R⁹, R¹³, alkyle en C₁ à C₆ ou - (alkyle en C₁ à C₆) - (cycloalkyle en C₃ à C₇), ledit groupe alkyle en C₁ à C₆ étant facultativement substitué avec un substituant -OR⁵ ou R¹³.

20. Association suivant l'une quelconque des revendications précédentes, dans laquelle R¹¹ représente H ou un groupe alkyle en C₁ à C₆, ledit groupe alkyle en C₁ à C₆ étant facultativement substitué avec un substituant -OR⁵, -NR⁵R⁵, -NR⁵COR⁵, -CONR⁵R⁵, R⁸ ou R⁹.

21. Association suivant l'une quelconque des revendications précédentes, dans laquelle R¹¹ représente H ou un groupe alkyle en C₁ à C₆, ledit groupe alkyle en C₁ à C₆ étant facultativement substitué avec un substituant -OR⁵ ou -NR⁵COR⁵.

22. Association suivant l'une quelconque des revendications précédentes, dans laquelle R¹² représente un groupe alkyle en C₁ à C₄ substitué avec un substituant R⁸, R⁹, -OR⁵, -CONR⁵R⁵, -NR⁵COR⁵ ou -NR⁵R⁵.

23. Association suivant l'une quelconque des revendications précédentes, dans laquelle R¹² représente un groupe alkyle en C₁ à C₄ substitué avec un substituant R⁹, -OR⁵, -NR⁵COR⁵ ou -NR⁵R⁵.

24. Association suivant l'une quelconque des revendications précédentes, dans laquelle R¹³ représente un groupe phényle substitué avec un substituant halogéno, -CN, -COR⁵, -CONR⁵R⁵, -SO₂NR⁵R⁵, -NR⁵SO₂R⁵, -OR⁵, -NR⁵R⁵, -(alkylène en C₁ à C₆) - NR⁵R⁵, alkyle en C₁ à C₆, halogénalkyle en C₁ à C₆ ou cycloalkyle en C₃ à C₇.

25. Association suivant l'une quelconque des revendications précédentes, dans laquelle R¹³ représente un groupe phényle substitué avec un substituant halogéno, -CN, -CONR⁵R⁵, -SO₂NR⁵R⁵ ou -OR⁵.

26. Association suivant l'une quelconque des revendications 1 à 4, dans laquelle le composé de formule (I) est
le 3-{[3,5-diéthyl-1-(2-hydroxyéthyl)-1*H-*pyrazole-4-yl]oxy}-5-fluorobenzonitrile ;
le 3-{[3,5-diéthyl-1-(2-hydroxyéthyl)-1*H-*pyrazole-4-yl]oxy}-5-méthylbenzonitrile ;
le 5-{[3,5-diéthyl-1-(2-hydroxyéthyl)-1*H-*pyrazole-4-yl]oxy}isophtalonitrile ;
le 3-chloro-5-{[3,5-diéthyl-1-(2-hydroxyéthyl)-1*H-*pyrazole-4-yl]oxy}benzonitrile ;
le 5-[(3,5-diéthyl-1*H-*pyrazole-4-yl)oxy]isophtalonitrile ;
le 3-[(3,5-diéthyl-1*H-*pyrazole-4-yl)oxy]-5-méthylbenzonitrile ;
le 3-chloro-5-[(3,5-diéthyl-1*H-*pyrazole-4-yl)oxy]benzonitrile ;
le 3-{[1-(2-aminoéthyl)-3,5-diéthyl-1*H-*pyrazole-4-yl]-oxy}-5-méthylbenzonitrile ;
le 3-{[1-(2-aminoéthyl)-3,5-diéthyl-1*H-*pyrazole-4-yl]-oxy}-5-chlorobenzonitrile ;
le 5-{[1-(2-aminoéthyl)-3,5-diéthyl-1*H-*pyrazole-4-yl]-oxy}isophtalonitrile ;
ou un de ses sels pharmaceutiquement acceptables, ou bien un de ses produits de solvatation.

27. Association suivant l'une quelconque des revendications 1 à 4, dans laquelle le composé de formule (I) est
le 5-{[3,5-diéthyl-1-(2-hydroxyéthyl)-1*H*-pyrazole-4-yl]oxy}isophtalonitrile ;
ou un de ses sels pharmaceutiquement acceptables, ou bien un de ses produits de solvatation.

28. Association suivant l'une quelconque des revendications 1 à 27, pour une utilisation comme médicament.

29. Association suivant l'une quelconque des revendications 1 à 27, pour une utilisation dans le traitement de l'affection par le VIH ou des rétrovirus apparentés.

30. Utilisation d'une association telle que définie dans l'une quelconque des revendications 1 à 27 pour la production d'un médicament pour le traitement simultané ou séquentiel de l'infection par le VIH ou d'une infection par un rétrovirus génétiquement apparenté.
